# EUROPEAN PATENT APPLICATION

(11) **EP 3 466 918 A1**
(43) Date of publication of application: **10.04.2019**
(21) Application number: 17806713.8
(22) Date of filing: 31.05.2017
(51) Int. Cl.: C07C 69/54, C07C 69/732, C09K 19/12, C09K 19/14, C09K 19/18, C09K 19/20, C09K 19/30, C09K 19/32, C09K 19/34, C09K 19/42, C09K 19/54, C09K 19/56, G02F 1/13, G02F 1/1337

(54) **POLYMERIZABLE POLAR COMPOUND, LIQUID CRYSTAL COMPOSITION, AND LIQUID CRYSTAL DISPLAY ELEMENT**

(30) Priority: 03.06.2016 JP 2016111928; 27.10.2016 JP 2016210893
(71) Applicant: JNC Corporation, Chiyoda-ku Tokyo 100-8105 (JP); JNC Petrochemical Corporation, Tokyo 100-0004 (JP)
(72) Inventor: YANO, Masakazu, Ichihara-shi Chiba 290-8551 (JP); KONDOU, Fumitaka, Ichihara-shi Chiba 290-8551 (JP); OGITA, Kazuhiro, Ichihara-shi Chiba 290-8551 (JP)
(74) Representative: Becker, Eberhard
(86) International application number: PCT/JP2017/020186
(87) International publication number: WO 2017/209161

(57) **Abstract**

Provided is a polar compound having high chemically stability, high ability to align liquid crystal molecules, and high solubility in a liquid crystal composition, and which has a large voltage-holding ratio in a liquid crystal display element. Specifically, compound (1) is provided.

**R¹⁻MES-Sp¹⁻P¹** **(1)**

For example, R¹ is a C1-15 alkyl; MES is a mesogenic group having at least one ring; Sp¹ is a single bond or a C1-10 alkylene, in this alkylene, at least one hydrogen is substituted by a group (1a); P¹ is a group (1e) or (If); 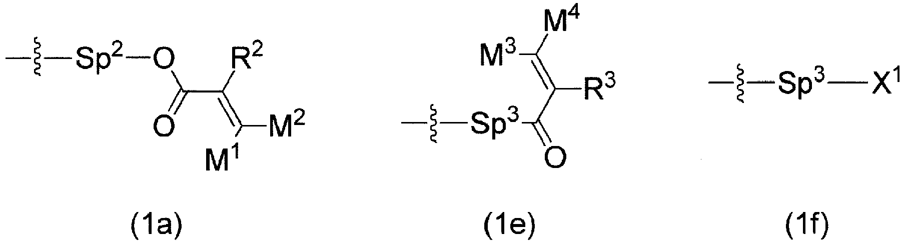 M¹, M², M³ and M⁴ are hydrogen; Sp² and Sp³ are a single bond or a C1-10 alkylene; R² is a C1-15 alkyl; R³ is a group (1g), (1h) or (1i); 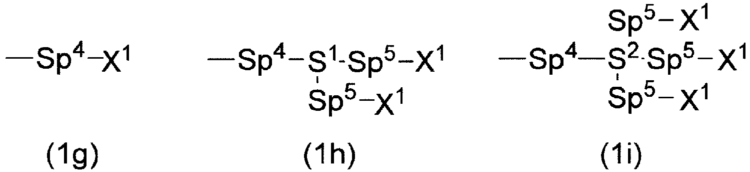 Sp⁴ and Sp⁵ are a single bond or a C1-10 alkylene; S¹ is >CH-, S² is >C<; and X¹ is -OH.

## Description

### Technical Field

The invention relates to a compound having a polymerizable group and a polar group, a liquid crystal composition and a liquid crystal display device. More particularly, the invention relates to a compound having both a plurality of polymerizable groups such as methacryloiloxy and polar groups such as an -OH group, a liquid crystal composition containing the compound and having positive or negative dielectric anisotropy, and a liquid crystal display device including the composition.

### Background Art

In a liquid crystal display device, a classification based on an operating mode for liquid crystal molecules includes a phase change (PC) mode, a twisted nematic (TN) mode, a super twisted nematic (STN) mode, an electrically controlled birefringence (ECB) mode, an optically compensated bend (OCB) mode, an in-plane switching (IPS) mode, a vertical alignment (VA) mode, a fringe field switching (FFS) mode and a field-induced photo-reactive alignment (FPA) mode. A classification based on a driving mode in the device includes a passive matrix (PM) and an active matrix (AM). The PM is classified into static, multiplex and so forth, and the AM is classified into a thin film transistor (TFT), a metal insulator metal (MIM) and so forth. The TFT is further classified into amorphous silicon and polycrystal silicon. The latter is classified into a high temperature type and a low temperature type based on a production process. A classification based on a light source includes a reflective type utilizing natural light, a transmissive type utilizing backlight and a transflective type utilizing both the natural light and the backlight.

The liquid crystal display device includes a liquid crystal composition having a nematic phase. The composition has suitable characteristics. An AM device having good characteristics can be obtained by improving characteristics of the composition. Table 1 below summarizes a relationship in two characteristics. The characteristics of the composition will be further described based on a commercially available AM device. A temperature range of the nematic phase relates to a temperature range in which the device can be used. A preferred maximum temperature of the nematic phase is about 70°C or higher, and a preferred minimum temperature of the nematic phase is about -10°C or lower. Viscosity of the composition relates to a response time in the device. A short response time is preferred for displaying moving images on the device. A shorter response time even by one millisecond is desirable. Accordingly, small viscosity in the composition is preferred. Small viscosity at a low temperature is further preferred.

**Table 1 Characteristics of composition and AM device**

| No. | Characteristics of composition | Characteristics of AM device |
|---|---|---|
| 1 | Wide temperature range of a nematic phase | Wide usable temperature range |
| 2 | Small viscosity ¹⁾ | Short response time |
| 3 | Suitable optical anisotropy | Large contrast ratio |
| 4 | Large positive or negative dielectric anisotropy | Low threshold voltage and small electric power consumption Large contrast ratio |
| 5 | Large specific resistance | Large voltage holding ratio and large contrast ratio |
| 6 | High stability to ultraviolet light and heat | Long service life |
| 7 | Large elastic constant | Large contrast ratio and short response time |

| | | |
|---|---|---|
| 1) A composition can be injected into a liquid crystal display device in a short time. | | |

Optical anisotropy of the composition relates to a contrast ratio in the device. According to a mode of the device, large optical anisotropy or small optical anisotropy, more specifically, suitable optical anisotropy is required. A product (Δn × d) of the optical anisotropy (Δn) of the composition and a cell gap (d) in the device is designed so as to maximize the contrast ratio. A suitable value of the product depends on a type of the operating mode. The value is about 0.45 micrometer in a device having the mode such as the TN mode. The suitable value is in the range of about 0.30 micrometer to about 0.40 micrometer in a device having the VA mode, and is in the range of about 0.20 micrometer to about 0.30 micrometer in a device having the IPS mode or the FFS mode. In the above cases, a composition having large optical anisotropy is preferred for a device having a small cell gap. Large dielectric anisotropy in the composition contributes to a low threshold voltage, small electric power consumption and a large contrast ratio in the device. Accordingly, large positive or negative dielectric anisotropy is preferred. Large specific resistance in the composition contributes to a large voltage holding ratio and the large contrast ratio in the device. Accordingly, a composition having the large specific resistance at room temperature and also at a temperature close to the maximum temperature of the nematic phase in an initial stage is preferred. The composition having the large specific resistance at room temperature and also at a temperature close to the maximum temperature of the nematic phase after the device has been used for a long period of time is preferred. Stability of the composition to ultraviolet light and heat relates to a service life of the device. In the case where the stability is high, the device has a long service life. Such characteristics are preferred for an AM device use in a liquid crystal projector, a liquid crystal television and so forth.

In a liquid crystal display device having a polymer sustained alignment (PSA) mode, a liquid crystal composition containing a polymer is used. First, a composition to which a small amount of a polymerizable compound is added is injected into the device. Then, the composition is irradiated with ultraviolet light while a voltage is applied between substrates of the device. The polymerizable compound is polymerized to form a network structure of the polymer in the composition. In the composition, alignment of liquid crystal molecules can be controlled by the polymer, and therefore the response time of the device is shortened and also image persistence is improved. Such an effect of the polymer can be expected for a device having the mode such as the TN mode, the ECB mode, the OCB mode, the IPS mode, the VA mode, the FFS mode and the FPA mode.

In a general-purpose liquid crystal display device, vertical alignment of liquid crystal molecules is achieved by a polyimide alignment film. Meanwhile, as a liquid crystal display device having no alignment film, a mode in which a polar compound is added to a liquid crystal composition to align liquid crystal molecules is proposed. First, a composition to which a small amount of the polar compound and a small amount of the polymerizable compound are added is injected into the device. Here, the liquid crystal molecules are aligned by action of the polar compound. Then, the composition is irradiated with ultraviolet light while a voltage is applied between substrates of the device. Here, the polymerizable compound is polymerized to stabilize alignment of the liquid crystal molecules. In the composition, the alignment of the liquid crystal molecules can be controlled by the polar compound and the polymer, and therefore the response time of the device is shortened and also the image persistence is improved. Further, in the device having no alignment film, a step of forming the alignment film is not required. The device has no alignment film, and therefore reduction in electric resistance of the device by interaction between the alignment film and the composition is not caused. Such an effect caused by a combination of the polar compound and the polymer can be expected for the device having the mode such as the TN mode, the ECB mode, the OCB mode, the IPS mode, the VA mode, the FFS mode and the FPA mode.

In the liquid crystal display device having no alignment film, various compounds each having an -OH group at a terminal have been prepared so far as a compound capable of vertically aligning liquid crystal molecules. In Patent literature No. 1, biphenyl compound (S-1) having an -OH group at a terminal is described.

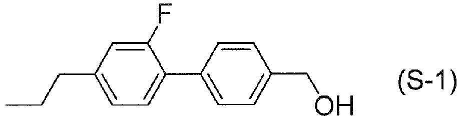

### Citation List

### Patent Literature

Patent literature No. 1: WO 2014/090362 A.
Patent literature No. 2: WO 2014/094959 A.
Patent literature No. 3: WO 2013/004372 A.
Patent literature No. 4: WO 2012/104008 A.
Patent literature No. 5: WO 2012/038026 A.
Patent literature No. 6: JP S50-35076 A.

### Summary of Invention

### Technical Problem

A first object of the invention is to provide a polar compound having at least one of chemically high stability, high capability to align liquid crystal molecules and high solubility in a liquid crystal composition, and having a large voltage holding ratio when used for a liquid crystal display device. A second object is to provide a liquid crystal composition containing the compound, and satisfying at least one of characteristics such as a high maximum temperature of a nematic phase, a low minimum temperature of the nematic phase, small viscosity, suitable optical anisotropy, large positive or negative dielectric anisotropy, large specific resistance, high stability to ultraviolet light, high stability to heat and a large elastic constant. A third object is to provide a liquid crystal display device including the composition, and having at least one of characteristics such as a wide temperature range in which the device can be used, a short response time, high transmittance, a large voltage holding ratio, a low threshold voltage, a large contrast ratio, a long service life and good vertical alignability.

### Solution to Problem

The invention concerns a compound represented by formula (1), a liquid crystal composition containing the compound, and a liquid crystal display device including the composition.

A compound, represented by formula (1):

**R¹-MES-Sp¹-P¹** **(1)**

wherein, in formula (1),
R¹ is alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen;
MES is a mesogen group having at least one ring;
Sp¹ is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen, and in the groups, at least one hydrogen is replaced by a group selected from groups represented by formula (1a), formula (1b), formula (1c) and formula (1d); 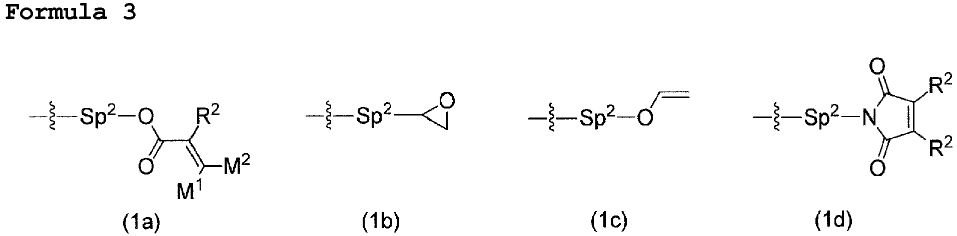
wherein, in formula (1a), formula (1b), formula (1c) and formula (1d),
Sp² is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen;
M¹ and M² are independently hydrogen, halogen, alkyl having 1 to 5 carbons, or alkyl having 1 to 5 carbons in which at least one hydrogen is replaced by halogen;
R² is alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen; and
   in formula (1),
P¹ is a group selected from groups represented by formula (1e) and formula (If); 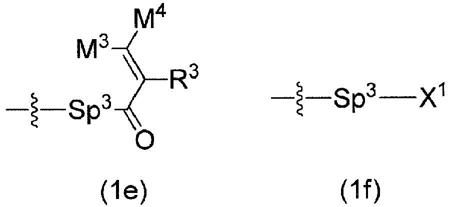 wherein, in formula (1e) and (If),
Sp³ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
M³ and M⁴ are independently hydrogen, halogen, alkyl having 1 to 5 carbons, or alkyl having 1 to 5 carbons in which at least one hydrogen is replaced by halogen;
X¹ is -OH, -NH₂, -OR⁵, -N(R⁵)₂, -COOH, -SH, -B(OH)₂ or -Si(R⁵)₃; and in -N(R⁵)₂,
R³ is a group selected from groups represented by formula (1g), formula (1h) and formula (1i); 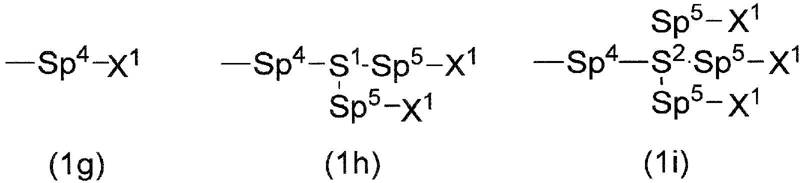 wherein, in formula (1g), formula (1h) and formula (1i),
Sp⁴ and Sp⁵ are independently a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
S¹ is >CH- or >N-;
S² is >C< or >Si<;
X¹ is -OH, -NH₂, -OR⁵, -N(R⁵)₂, -COOH, -SH, -B(OH)₂ or -Si(R⁵)₃; and in -OR⁵, -N(R⁵)₂ and -Si(R⁵)₃,
R⁵ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by halogen.

### Advantageous Effects of Invention

A first advantage of the invention is to provide a polar compound having at least one of chemically high stability, high capability to align liquid crystal molecules and high solubility in a liquid crystal composition, and having a large voltage holding ratio when used for a liquid crystal display device. A second advantage is to provide a liquid crystal composition containing the compound, and satisfying at least one of characteristics such as a high maximum temperature of a nematic phase, a low minimum temperature of the nematic phase, small viscosity, suitable optical anisotropy, large positive or negative dielectric anisotropy, large specific resistance, high stability to ultraviolet light, high stability to heat and large elastic constant. A third advantage is to provide a liquid crystal display device including the composition, and having at least one of characteristics such as a wide temperature range in which the device can be used, a short response time, high transmittance, a large voltage holding ratio, a low threshold voltage, a large contrast ratio, a long service life and good vertical alignability.

### Description of Embodiments

Usage of terms herein is as described below. Terms "liquid crystal composition" and "liquid crystal display device" may be occasionally abbreviated as "composition" and "device," respectively. "Liquid crystal display device" is a generic term for a liquid crystal display panel and a liquid crystal display module. "Liquid crystal compound" is a generic term for a compound having a liquid crystal phase such as a nematic phase and a smectic phase, and a compound having no liquid crystal phase but being mixed with the composition for the purpose of adjusting characteristics such as a temperature range of the nematic phase, viscosity and dielectric anisotropy. The compound has a six-membered ring such as 1,4-cyclohexylene and 1,4-phenylene, and has rod-like molecular structure. "Polymerizable compound" is a compound to be added for the purpose of forming a polymer in the composition. "Polar compound" assists alignment of liquid crystal molecules by interaction of a polar group with a substrate surface.

The liquid crystal composition is prepared by mixing a plurality of liquid crystal compounds. A proportion (content) of the liquid crystal compounds is expressed in terms of weight percent (% by weight) based on the weight of the liquid crystal composition. An additive such as an optically active compound, an antioxidant, an ultraviolet light absorber, a dye, an antifoaming agent, a polymerizable compound, a polymerization initiator, a polymerization inhibitor and a polar compound is added to the liquid crystal composition, when necessary. A proportion (amount of addition) of the additive is expressed in terms of weight percent (% by weight) based on the weight of the liquid crystal composition in a manner similar to the proportion of the liquid crystal compound. Weight parts per million (ppm) may be occasionally used. A proportion of the polymerization initiator and the polymerization inhibitor is exceptionally expressed based on the weight of the polymerizable compound.

A compound represented by formula (1) may be occasionally abbreviated as "compound (1)." "Compound (1)" means one compound, a mixture of two compounds or a mixture of three or more compounds represented by formula (1). A same rule applies also to at least one compound selected from groups of a compound represented by formula (2), or the like. Symbol A¹, B¹, C¹ or the like surrounded by a hexagonal shape corresponds to ring A¹, ring B¹, ring C¹ or the like, respectively. A hexagon represents a six-membered ring such as a cyclohexane ring and a benzene ring, or a condensed ring such as a naphthalene ring. An oblique line crossing one side of the hexagonal shape represents that arbitrary hydrogen on the ring may be replaced by a group such as -Sp¹-P¹. A subscript such as c, d and e represents the number of groups to be replaced. When the subscript is 0 (zero), no such replacement exists. In an expression "ring A and ring C are independently X, Y or Z," the subject is plural, and therefore "independently" is used. When the subject is "ring A," the subject is singular, and therefore "independently" is not used.

A symbol of terminal group R¹¹ is used in a plurality of compounds in chemical formulas of compounds. In the compounds, two groups represented by two pieces of arbitrary R¹¹ may be identical or different. For example, in one case, R¹¹ of compound (2) is ethyl and R¹¹ of compound (3) is ethyl. In another case, R¹¹ of compound (2) is ethyl and R¹¹ of compound (3) is propyl. A same rule applies also to a symbol of R¹², R¹³, Z¹¹ or the like. In compound (8), when i is 2, two of rings D¹ exist. In the compound, two groups represented by two of rings D¹ may be identical or different. A same rule applies also to two of arbitrary rings D¹ when i is larger than 2. A same rule applies also to any other symbols.

An expression "at least one piece of 'A'" means that the number of 'A' is arbitrary. An expression "at least one piece of 'A' may be replaced by 'B'" means that, when the number of 'A' is 1, a position of 'A' is arbitrary, and when the number of 'A' is 2 or more, positions thereof can be selected without limitation. A same rule applies also to an expression "at least one piece of 'A' is replaced by 'B'." An expression "at least one piece of A may be replaced by B, C or D" means inclusion of a case where at least one piece of A is replaced by B, a case where at least one piece of A is replaced by C, and a case where at least one piece of A is replaced by D, and also a case where a plurality of pieces of A are replaced by at least two of B, C or D. For example, alkyl in which at least one piece of -CH₂- (or -CH₂CH₂-) may be replaced by -O- (or -CH=CH-) includes alkyl, alkenyl, alkoxy, alkoxyalkyl, alkoxyalkenyl and alkenyloxyalkyl. In addition, a case where two pieces of consecutive -CH₂- are replaced by -O- to form -O-O- is not preferred. In alkyl or the like, a case where -CH₂- of a methyl part (-CH₂-H) is replaced by -O- to form -O-H is not preferred, either.

Halogen means fluorine, chlorine, bromine or iodine. Preferred halogen is fluorine or chlorine. Further preferred halogen is fluorine. In the liquid crystal compound, alkyl has a straight-chain shape or a branched shape, and contains no cyclic alkyl. In general, straight-chain alkyl is preferred to branched-chain alkyl. A same rule applies also to a terminal group such as alkoxy and alkenyl. With regard to a configuration of 1,4-cyclohexylene, trans is preferred to cis for increasing the maximum temperature of the nematic phase. Then, 2-fluoro-1,4-phenylene means two divalent groups described below. In a chemical formula, fluorine may be leftward (L) or rightward (R). A same rule applies also to a left-right asymmetrical divalent group formed by removing two hydrogens from a ring, such as tetrahydropyran-2,5-diyl.

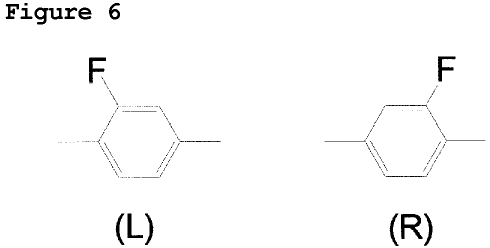

The invention includes items described below.
Item 1. A compound, represented by formula (1):

   **R¹-MES-Sp¹-P¹** **(1)**

   wherein, in formula (1),
   R¹ is alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen;
   MES is a mesogen group having at least one ring;
   Sp¹ is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen, and in the groups, at least one hydrogen is replaced by a group selected from groups represented by formula (1a), formula (1b), formula (1c) and formula (1d); 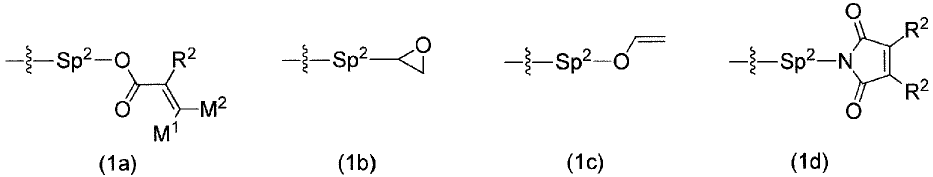
   wherein, in formula (1a), formula (1b), formula (1c) and formula (1d),
   Sp² is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen;
   M¹ and M² are independently hydrogen, halogen, alkyl having 1 to 5 carbons, or alkyl having 1 to 5 carbons in which at least one hydrogen is replaced by halogen;
   R² is hydrogen or alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen; and in formula (1),
   P¹ is a group selected from groups represented by formula (1e) and formula (If); 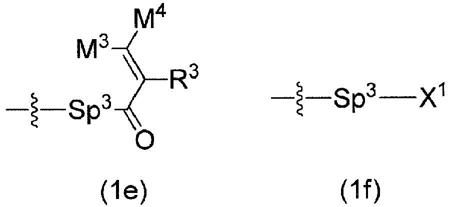
   wherein, in formula (1e) and (If),
   Sp³ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
   M³ and M⁴ are independently hydrogen, halogen, alkyl having 1 to 5 carbons, or alkyl having 1 to 5 carbons in which at least one hydrogen is replaced by halogen;
   X¹ is -OH, -NH₂, -OR⁵, -N(R⁵)₂, -COOH, -SH, -B(OH)₂ or -Si(R⁵)₃; and R³ is a group selected from groups represented by formula (1g), formula (1h) and formula (1i); 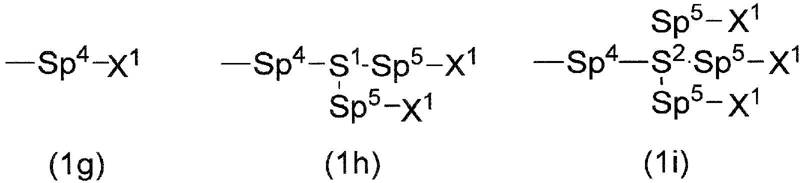
   wherein, in formula (1g), formula (1h) and formula (1i),
   Sp⁴ and Sp⁵ are independently a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
   S¹ is >CH- or >N-;
   S² is >C< or >Si<;
   X¹ is -OH, -NH₂, -OR⁵, -N(R⁵)₂, -COOH, -SH, -B(OH)₂ or -Si(R⁵)₃; and in -OR⁵, -N(R⁵)₂ and -N(R⁵)₂,
   R⁵ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by halogen.
Item 2. The compound according to item 1, represented by formula (1-1) : 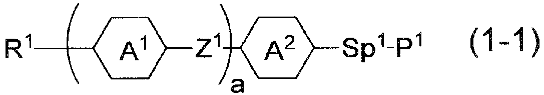 wherein, in formula (1-1),
   R¹ is alkyl having 1 to 12 carbons, and in the alkyl, at least one piece of -CH₂- may be replaced by -O-, and at least one piece of -(CH₂)₂- may be replaced by -CH=CH- or -C≡C-, and in the groups, at least one hydrogen may be replaced by fluorine;
   ring A¹ and ring A² are independently 1,4-cycloxylene, 1,4-cyclohexenylene, 1,4-phenylene, naphthalene-2,6-diyl, decahydronaphthalene-2,6-diyl, 1,2,3,4-tetrahydronaphthalene-2,6-diyl, tetrahydropyran-2,5-diyl, 1,3-dioxane-2,5-diyl, pyrimidine-2,5-diyl, pyridine-2,5-diyl, fluorene-2,7-diyl, phenanthrene-2,7-diyl, anthracene-2,6-diyl, perhydrocyclopenta[a]phenanthrene-3,17-diyl, or 2,3,4,7,8,9,10,11,12,13,14,15,16,17-tetradecahydrocyclopenta[a]phe nanthrene-3,17-diyl, and in the rings, at least one hydrogen may be replaced by fluorine, chlorine, alkyl having 1 to 12 carbons, alkenyl having 2 to 12 carbons, alkoxy having 1 to 11 carbons or alkenyloxy having 2 to 11 carbons, and in the groups, at least one hydrogen may be replaced by fluorine or chlorine;
   a is 0, 1, 2, 3 or 4;
   Z¹ is a single bond or alkylene having 1 to 6 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
   Sp¹ is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine, and in the groups, at least one hydrogen is replaced by a polymerizable group represented by formula (1a) ; 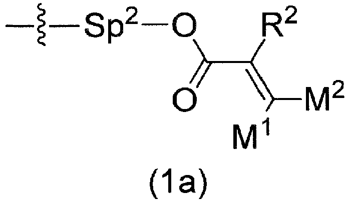
   wherein, in formula (1a),
   Sp² is a single bond or alkylene having 1 to 10 carbons, and in the alkylene, at least one piece of -CH₂- may be replaced by -O-, -NH-,
   -CO-, -COO-, -OCO- or -OCOO-, and at least one piece of -(CH₂)₂- may be replaced by -CH=CH- or -C≡C-, and in the groups, at least one hydrogen may be replaced by halogen;
   M¹ and M² are independently hydrogen, fluorine, chlorine, alkyl having 1 to 5 carbons, or alkyl having 1 to 5 carbons in which at least one hydrogen is replaced by fluorine or chlorine;
   R² is hydrogen or alkylene having 1 to 15 carbons, and in the alkylene, at least one piece of -CH₂- may be replaced by -O- or -S-, and at least one piece of -(CH₂)₂- may be replaced by -CH=CH- or -C≡C-, and in the groups, at least one hydrogen may be replaced by fluorine or chlorine; and
      in formula (1-1),
   P¹ is a group selected from groups represented by formula (1e) and formula (1f); 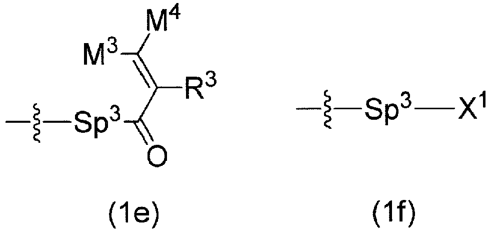
   wherein, in formula (1e) and formula (1f),
   Sp³ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
   M³ and M⁴ are independently hydrogen, fluorine, chlorine, alkyl having 1 to 5 carbons, or alkyl having 1 to 5 carbons in which at least one hydrogen is replaced by fluorine or chlorine;
   X¹ is -OH, -NH₂, -OR⁵, -N(R⁵)₂, -COOH, -SH or -Si(R⁵)₃; and
   R³ is a group selected from groups represented by formula (1g) and formula (1h); 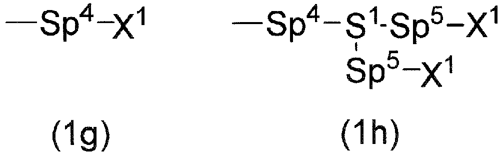
   wherein, in formula (1g) and formula (1h),
   Sp⁴ and Sp⁵ are independently a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
   S¹ is >CH- or >N-;
   X¹ is -OH, -NH₂, -OR⁵, -N(R⁵)₂, -COOH, -SH or -Si(R⁵)₃; and in -OR⁵, -N(R⁵)₂ and -Si(R⁵)₃,
   R⁵ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine or chlorine.
Item 3. The compound according to item 2,
   wherein, in formula (1-1), Z¹ is a single bond, -(CH₂)₂-, - (CH₂)₄-, -CH=CH-, -C≡C-, -COO-, -OCO-, -CF₂O-, -OCF₂-, -CH₂O-, -OCH₂- or -CF=CF-; and
   in formula (1a),
   M¹ and M⁴ are independently hydrogen, fluorine, alkyl having 1 to 5 carbons, or alkyl having 1 to 5 carbons in which at least one hydrogen is replaced by fluorine; and
   in formula (1e),
   M³ and M⁴ are independently hydrogen, fluorine, alkyl having 1 to 5 carbons, or alkyl having 1 to 5 carbons in which at least one hydrogen is replaced by fluorine; and
   R³ is a group represented by formula (1g).
Item 4. The compound according to item 2 or 3,
   wherein, in formula (1-1),
   ring A¹ and ring A² are independently 1,4-cycloxylene, 1,4-cyclohexenylene, 1,4-phenylene, naphthalene-2,6-diyl, decahydronaphthalene-2,6-diyl, tetrahydropyran-2,5-diyl, 1,3-dioxane-2,5-diyl, fluorene-2,7-diyl, phenanthrene-2,7-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine;
   Sp¹ is a single bond or alkylene having 1 to 8 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine, and in the groups, at least one hydrogen is replaced by a group represented by formula (1a); 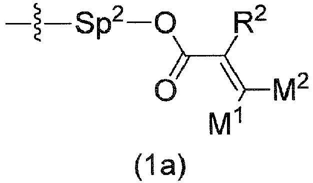 wherein, in formula (1a),
   Sp² is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
   M¹ and M² are independently hydrogen, fluorine, methyl, ethyl or trifluoromethyl;
   R² is hydrogen or alkylene having 1 to 8 carbons, and in the alkylene, at least one piece of -CH₂- may be replaced by -O-, and at least one piece of -(CH₂)₂- may be replaced by -CH=CH- or -C≡C-, and in the groups, at least one hydrogen may be replaced by fluorine; and
      in formula (1-1)
   P¹ is a group selected from groups represented by formula (1e) and formula (If); 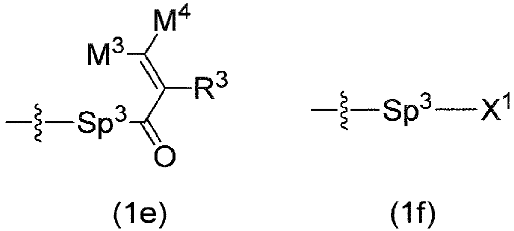
   wherein, in formula (1e) and formula (1f),
   Sp³ is a single bond or alkylene having 1 to 5 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
   M³ and M⁴ are independently hydrogen, fluorine, methyl, ethyl or trifluoromethyl;
   X¹ is -OH, -NH₂ or -N(R⁵)₂;
   R³ is a group represented by formula (1g); and

      **-Sp⁴-X¹** **(1g)**

      wherein, in formula (1g),
      Sp⁴ is a single bond or alkylene having 1 to 5 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
      X¹ is -OH, -NH₂ or -N(R⁵)₂; and
      in -N(R⁵)₂,
      R⁵ is hydrogen or alkyl having 1 to 5 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine.
Item 5. The compound according to item 1, represented by formula (1-2) or formula (1-3): 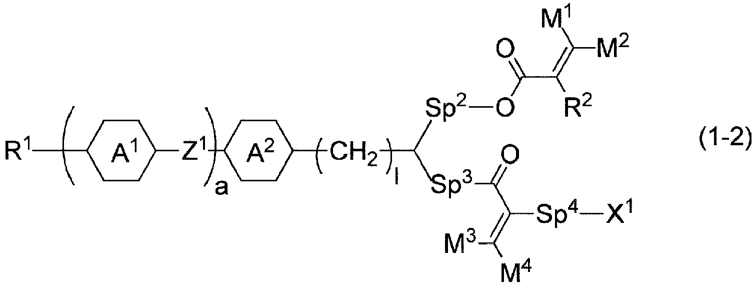 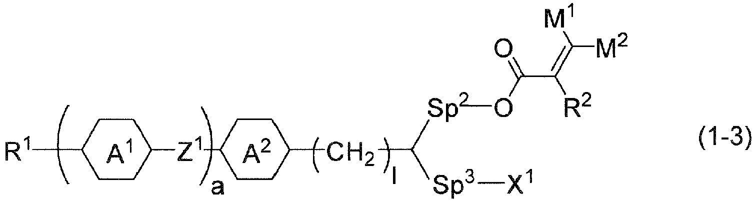 wherein, in formula (1-2) and formula (1-3),
   R¹ is alkyl having 1 to 12 carbons, and in the alkyl, at least one piece of -CH₂- may be replaced by -O-, and at least one piece of -(CH₂)₂- may be replaced by -CH=CH- or -C≡C-, and in the groups, at least one hydrogen may be replaced by fluorine;
   ring A¹ and ring A² are independently 1,4-cycloxylene, 1,4-cyclohexenylene, 1,4-phenylene, naphthalene-2,6-diyl, tetrahydropyran-2,5-diyl, 1,3-dioxane-2,5-diyl, fluorene-2,7-diyl, phenanthrene-2,7-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine, alkyl having 1 to 8 carbons, alkenyl having 2 to 8 carbons, alkoxy having 1 to 7 carbons or alkenyloxy having 2 to 7 carbons, and in the groups, at least one hydrogen may be replaced by fluorine;
   Z¹ is a single bond, -(CH₂)₂-, -(CH₂)₄-, -CH=CH-, -C≡C-, -CF₂O-, -OCF₂-, -CH₂O-, -OCH₂- or -CF=CF-;
   a is 0, 1, 2, 3 or 4;
   1 is 0, 1, 2, 3, 4, 5 or 6, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
   Sp² is a single bond or alkylene having 1 to 5 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
   M¹ and M² are independently hydrogen, fluorine, methyl, ethyl or trifluoromethyl;
   R² is hydrogen or alkyl having 1 to 5 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by fluorine;
   Sp³ is a single bond or alkylene having 1 to 5 carbons, and in the alkylene, at least one piece of -CH₂- may be replaced by -O-, -CO- or -COO-, and in the groups, at least one piece of -(CH₂)₂- may be replaced by -CH=CH- or -C≡C-, and in the groups, at least one hydrogen may be replaced by fluorine;
   M³ and M⁴ are independently hydrogen, fluorine, methyl, ethyl or trifluoromethyl;
   Sp⁴ is a single bond, alkylene having 1 to 5 carbons, and in the alkylene, at least one piece of -CH₂- may be replaced by -O-, -CO- or -COO-, and at least one piece of -(CH₂)₂- may be replaced by -CH=CH- or -C≡C-, and in the groups, at least one hydrogen may be replaced by fluorine;
   X¹ is -OH or -N(R⁵)₂; and
      in N(R⁵)₂,
   R⁵ is hydrogen or alkyl having 1 to 5 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine.
Item 6. The compound according to item 5,
   wherein, in formula (1-2) and formula (1-3),
   R¹ is alkyl having 1 to 10 carbons, alkenyl having 2 to 10 carbons or alkoxy having 1 to 9 carbons, and in the groups, at least one hydrogen may be replaced by fluorine;
   ring A¹ and ring A² are independently 1,4-cycloxylene, 1,4-phenylene, naphthalene-2,6-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine or alkyl having 1 to 5 carbons;
   a is 0, 1, 2, 3 or 4;
   1 is 0, 1, 2, 3, 4, 5 or 6, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
   Z¹ is a single bond, -(CH₂)₂-, -(CH₂)₄-, -CH=CH-, -CF₂O- -OCF₂-, -CH₂O- or -OCH₂-;
   Sp² is a single bond or alkylene having 1 to 5 carbons, and in the alkylene, at least one piece of -CH₂- may be replaced by -O-, and at least one piece of -(CH₂)₂- may be replaced by -CH=CH-;
   M¹ and M² are independently hydrogen, methyl or ethyl;
   R² is hydrogen or alkyl having 1 to 5 carbons, and in the alkyl, at least one piece of -CH₂- may be replaced by -O-, and at least one piece of -(CH₂)₂- may be replaced by -CH=CH-;
   Sp³ is a single bond or alkylene having 1 to 5 carbons, and in the alkylene, at least one piece of -CH₂- may be replaced by -O-, and at least one piece of -(CH₂)₂- may be replaced by -CH=CH-;
   M³ and M⁴ are independently hydrogen, fluorine, methyl or ethyl;
   Sp⁴ is a single bond or alkylene having 1 to 5 carbons, and in the alkylene, at least one piece of -CH₂- may be replaced by -O-, and at least one piece of -(CH₂)₂- may be replaced by -CH=CH-;
   X¹ is -OH or -N(R⁵)₂; and
   in -N(R⁵)₂,
   R⁵ is hydrogen or alkyl having 1 to 3 carbons, and in the alkyl, at least one piece of -CH₂- may be replaced by -O-.
Item 7. The compound according to item 5,
   wherein, in formula (1-2) and formula (1-3),
   R¹ is alkyl having 1 to 10 carbons, alkenyl having 2 to 10 carbons or alkoxy having 1 to 9 carbons;
   ring A¹ and ring A² are independently 1,4-cycloxylene, 1,4-phenylene or naphthalene-2, 6-diyl, and in the rings, at least one hydrogen may be replaced by fluorine or alkyl having 1 to 5 carbons;
   a is 0, 1, 2 or 3; 1 is 0, 1, 2, 3, 4, 5 or 6, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-;
   Z¹ is a single bond, -(CH₂)₂- or -(CH₂)₄-;
   Sp² is a single bond or alkylene having 1 to 3 carbons, and in the alkylene, at least one piece of -CH₂- may be replaced by -O-;
   M¹ and M² are independently hydrogen or methyl;
   R² is hydrogen or alkyl having 1 to 5 carbons, and in the alkyl, at least one piece of -CH₂- may be replaced by -O-;
   Sp³ is a single bond or alkylene having 1 to 3 carbons, and in the alkylene, at least one piece of -CH₂- may be replaced by -O-;
   M³ and M⁴ are independently hydrogen or methyl;
   Sp⁴ is a single bond or alkylene having 1 to 3 carbons, and in the alkylene, at least one piece of -CH₂- may be replaced by -O-; and
   X¹ is -OH.
Item 8. The compound according to item 1, represented by any one of formula (1-4) to formula (1-41): 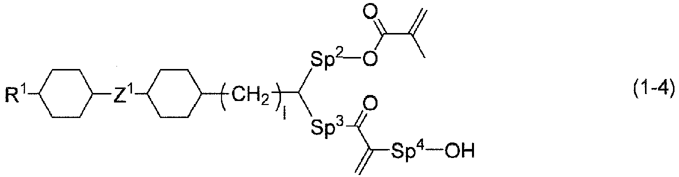 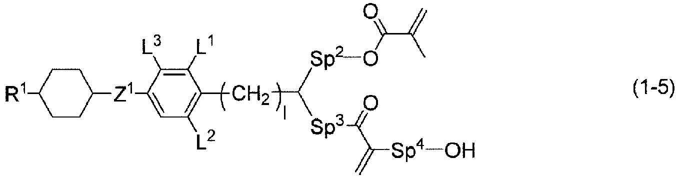 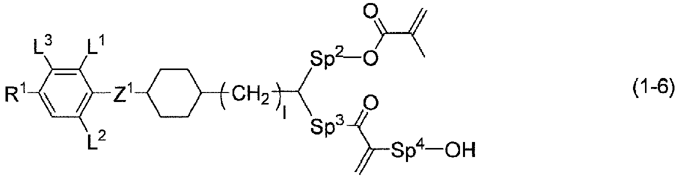 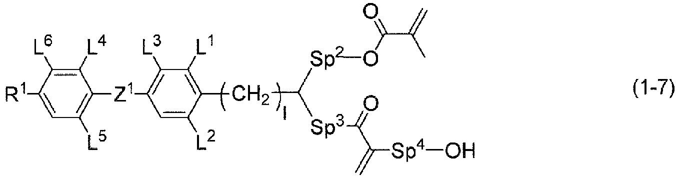 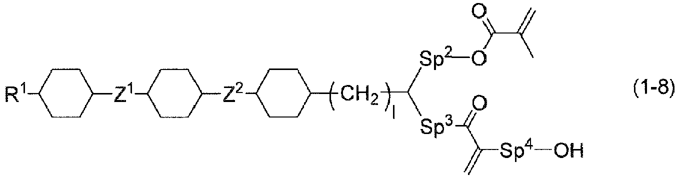 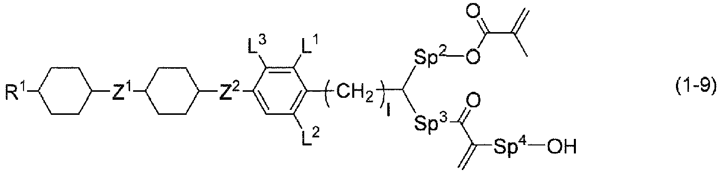 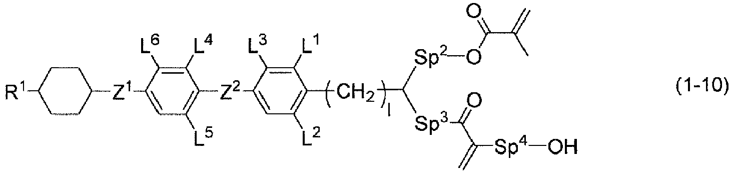 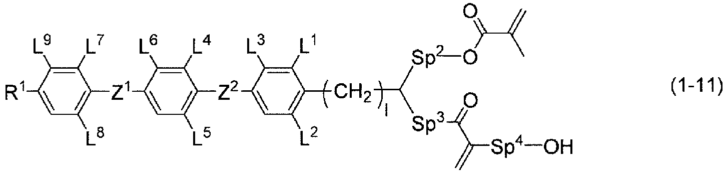 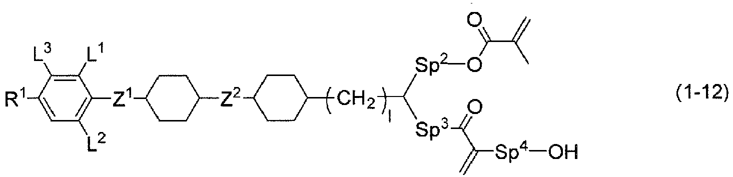 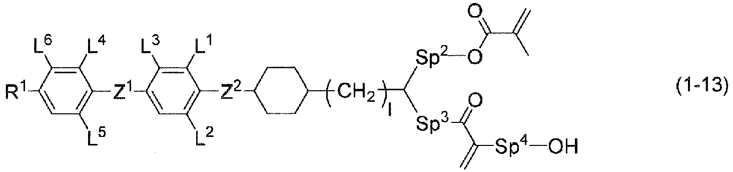 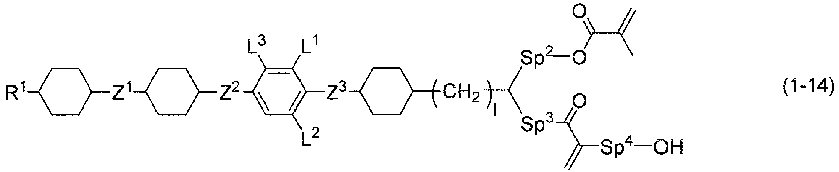 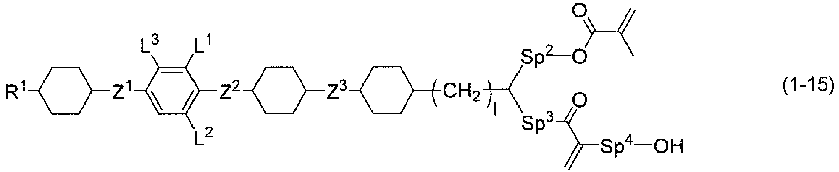 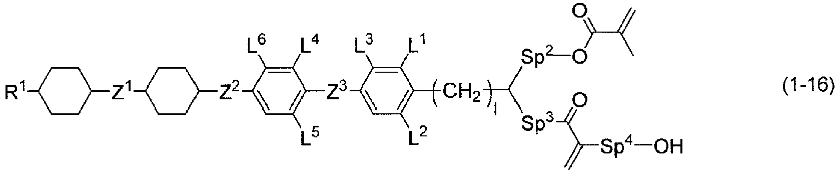 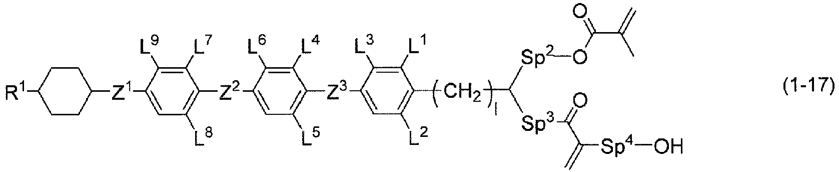 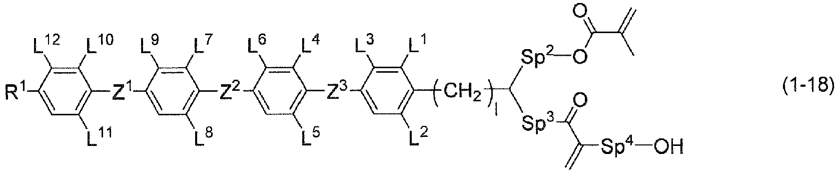 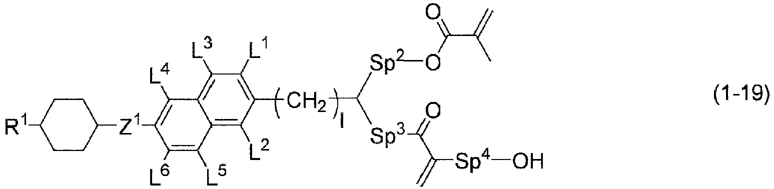 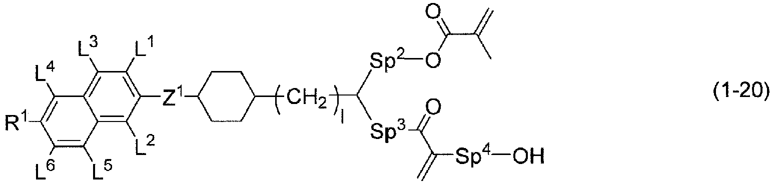 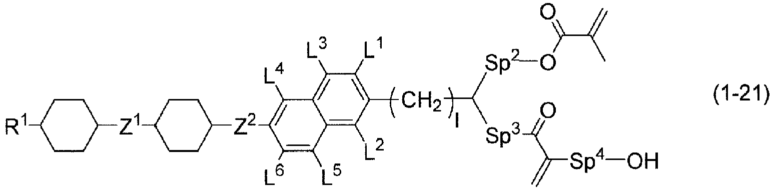 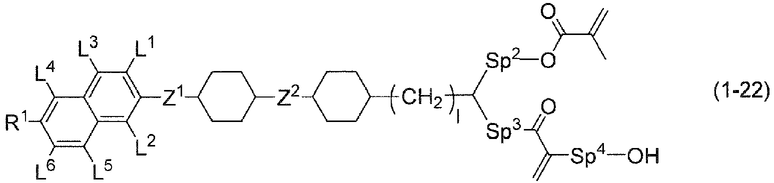 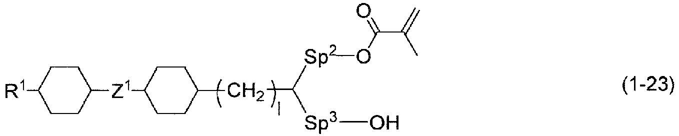 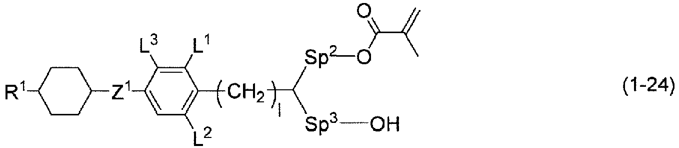 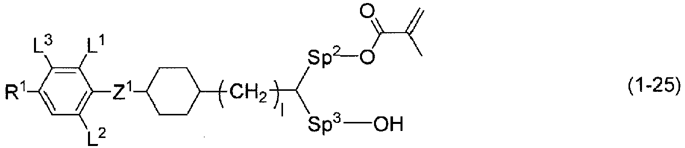 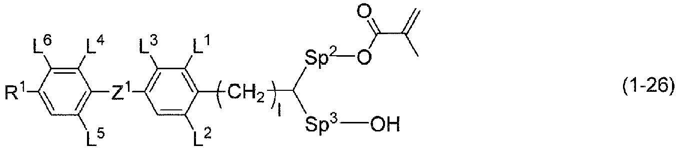 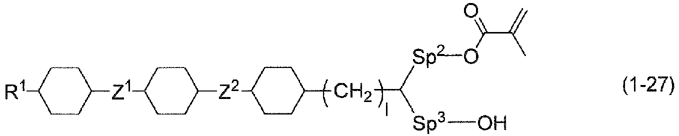 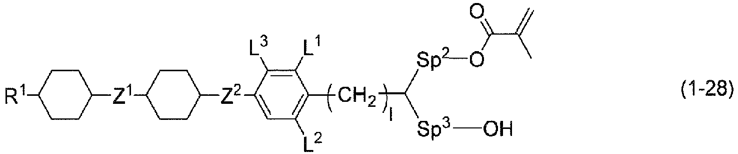 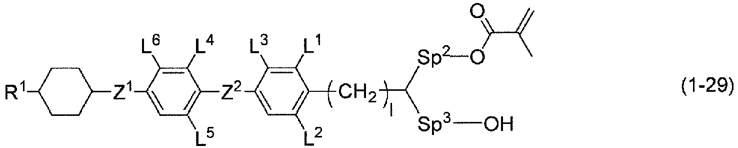 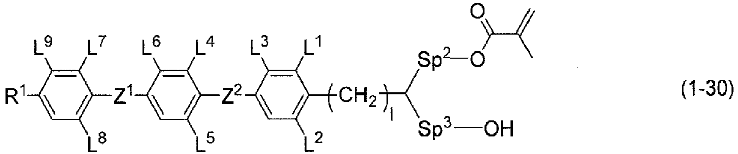 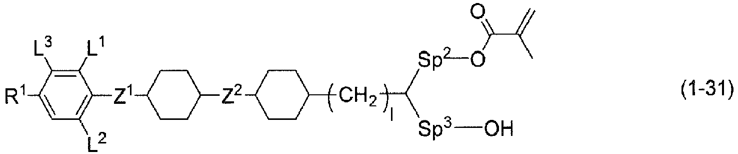 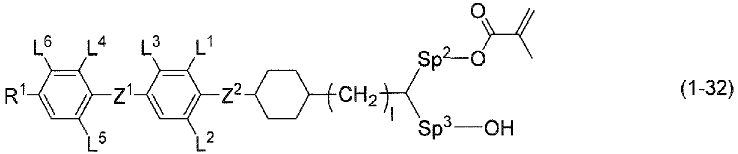 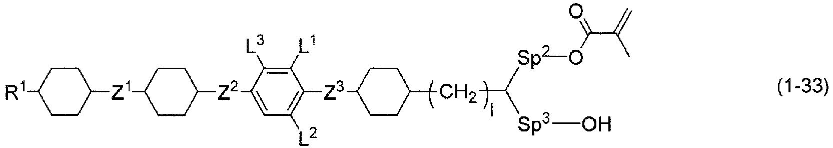 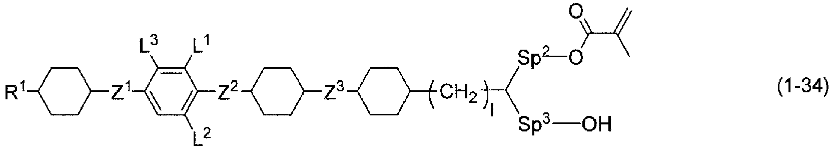 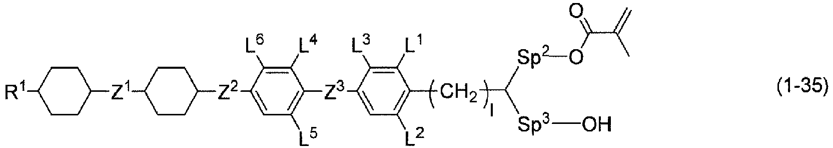 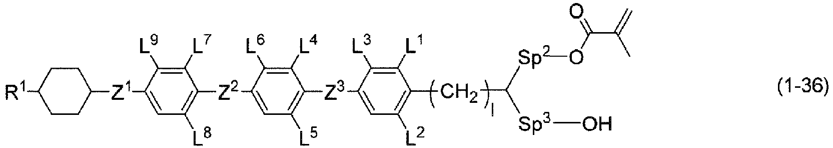 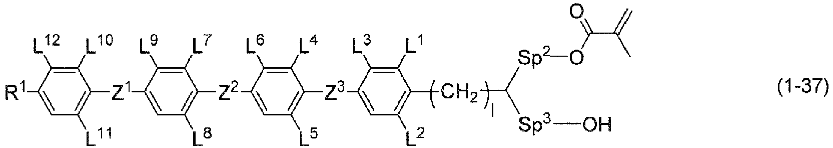 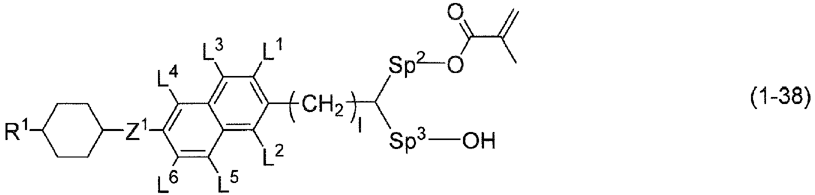 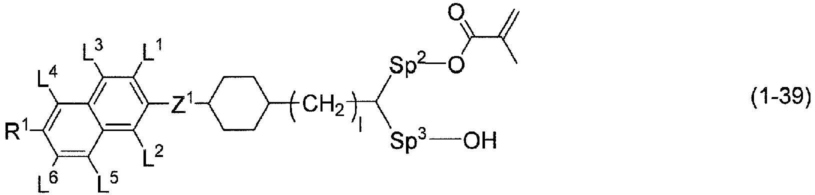 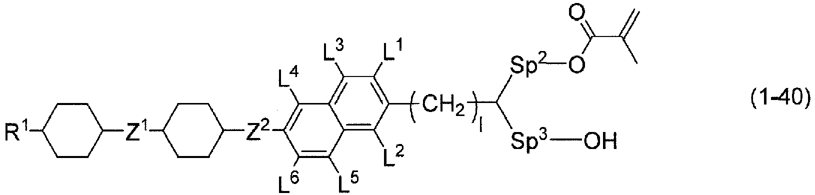 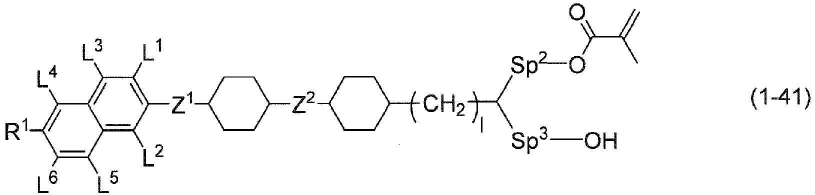 wherein, in formula (1-4) to formula (1-41),
   R¹ is alkyl having 1 to 10 carbons;
   Z¹, Z² and Z³ are independently a single bond, -(CH₂)₂- or -(CH₂)₄-;
   Sp², Sp³ and Sp⁴ are independently alkylene having 1 to 5 carbons, and in the alkylene, at least one piece of -CH₂- may be replaced by -O-;
   L¹, L², L³, L⁴, L⁵, L⁶, L⁷, L⁸, L⁹, L¹⁰, L¹¹ and L¹² are independently hydrogen, fluorine, methyl or ethyl; and 1 is 0, 1, 2, 3, 4, 5 or 6.
Item 9. The compound according to item 1, represented by any one of formula (1-42) to formula (1-60): 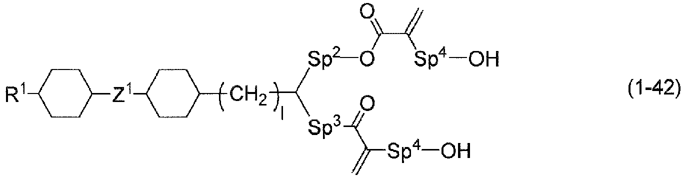 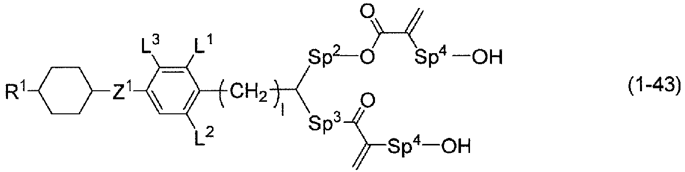 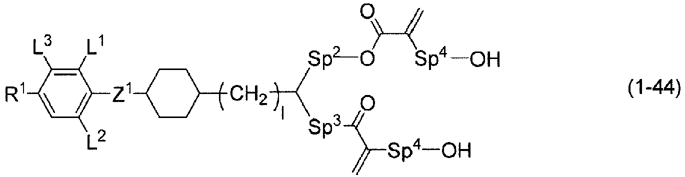 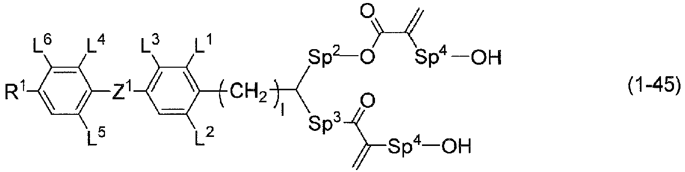 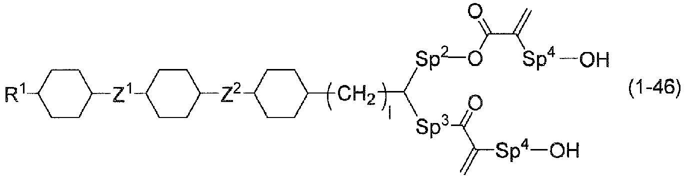 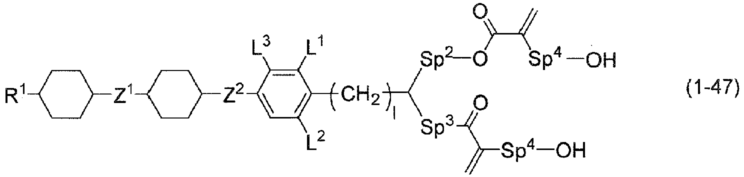 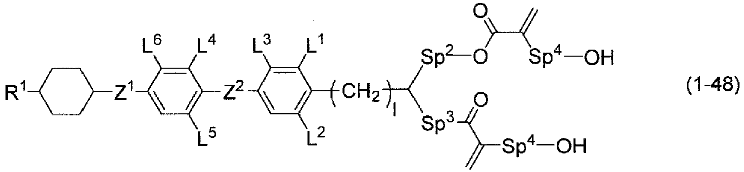 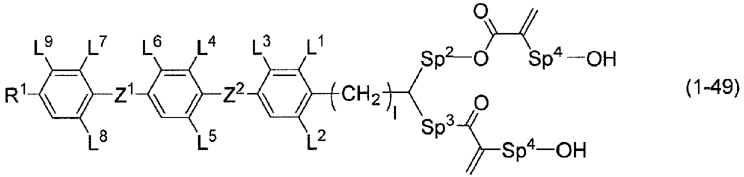 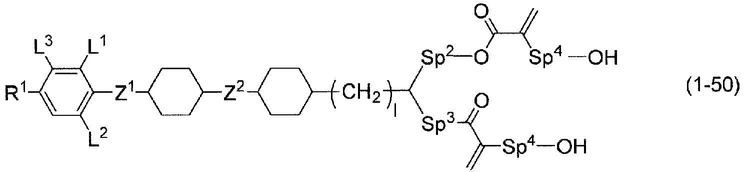 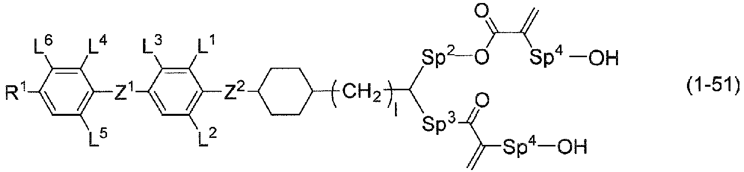 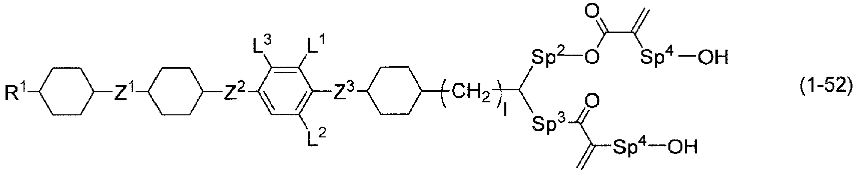 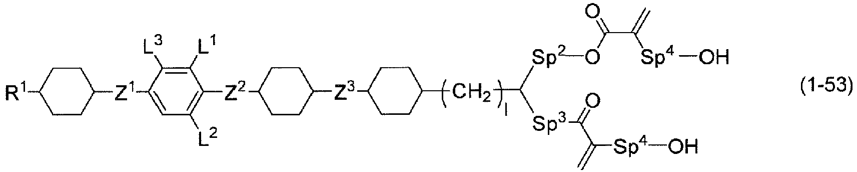 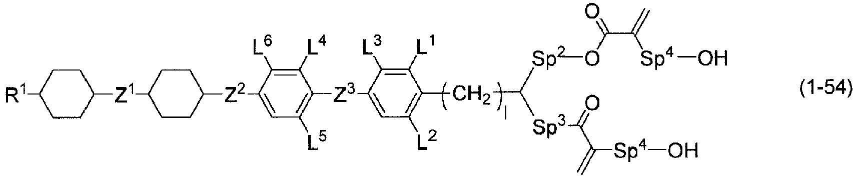 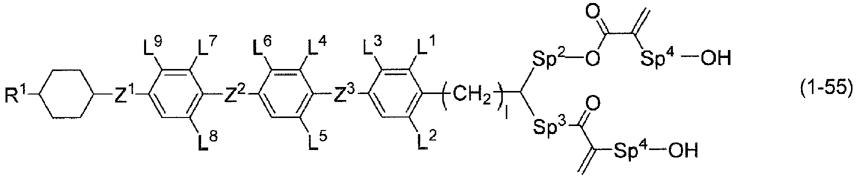 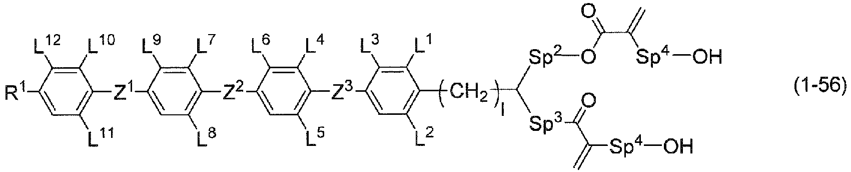 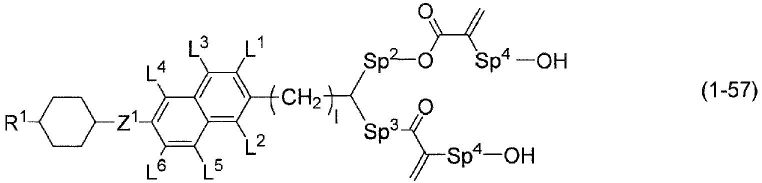 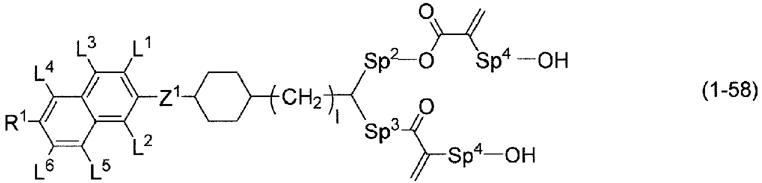 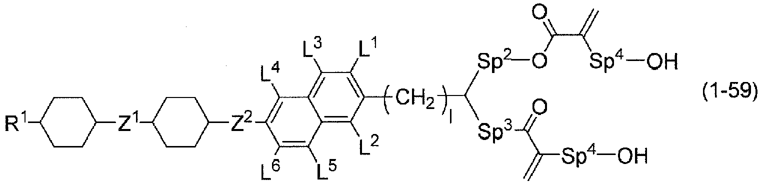 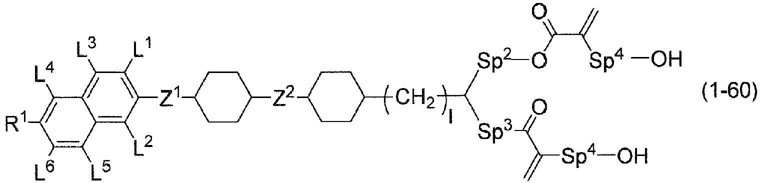 wherein, in formula (1-42) to formula (1-60),
   R¹ is alkyl having 1 to 10 carbons;
   Z¹, Z² and Z³ are independently a single bond, -(CH₂)₂- or -(CH₂)₄-;
   Sp², Sp³ and Sp⁴ are independently alkylene having 1 to 5 carbons, and in the alkylene, at least one piece of -CH₂- may be replaced by -O-;
   L¹, L², L³, L⁴, L⁵, L⁶, L⁷, L⁸, L⁹, L¹⁰, L¹¹ and L¹² are independently hydrogen, fluorine, methyl or ethyl; and
   1 is 0, 1, 2, 3, 4, 5 or 6.
Item 10. The compound according to item 1, represented by any one of formula (1-61) to formula (1-98): 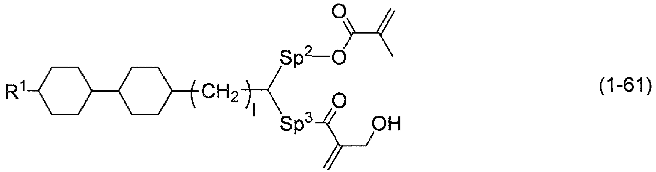 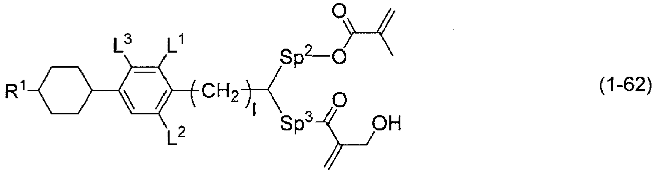 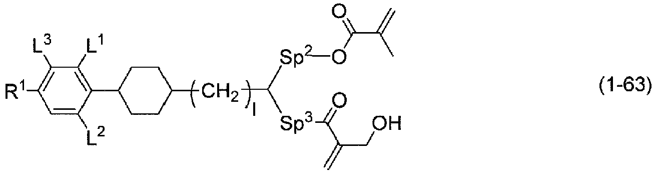 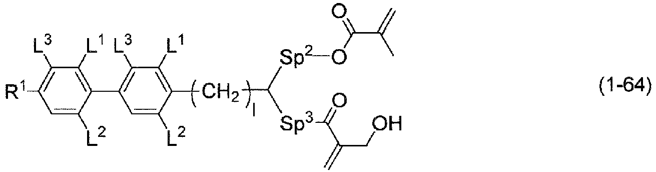 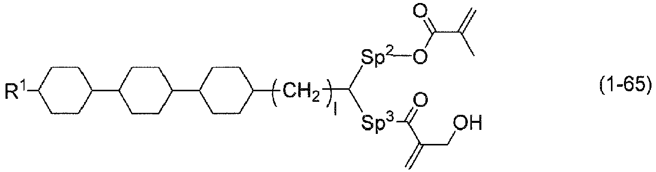 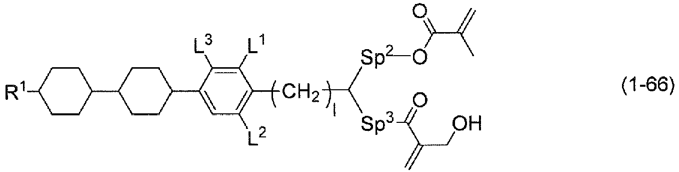 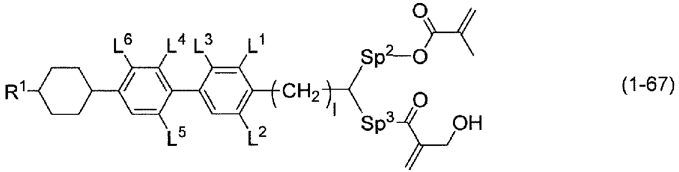 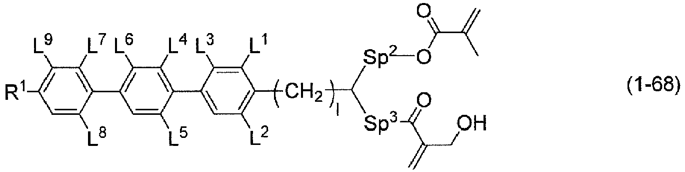 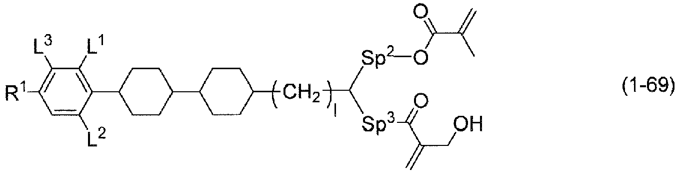 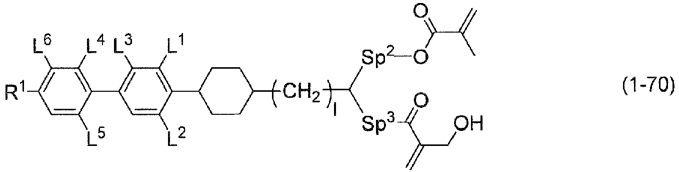 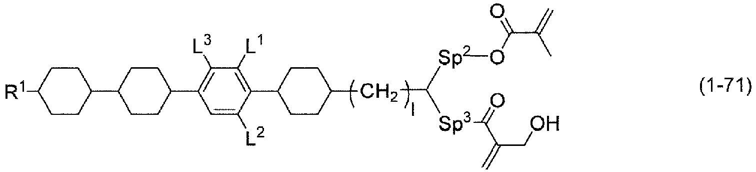 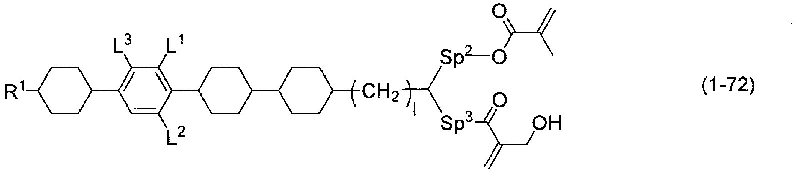 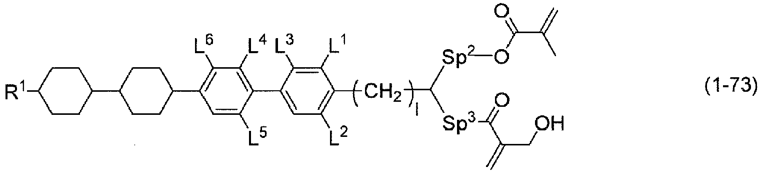 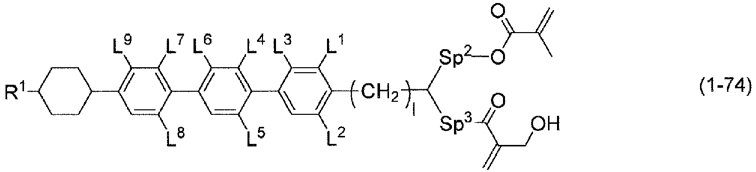 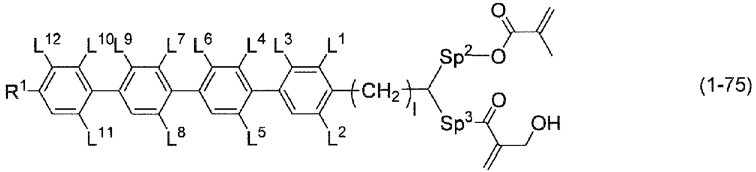 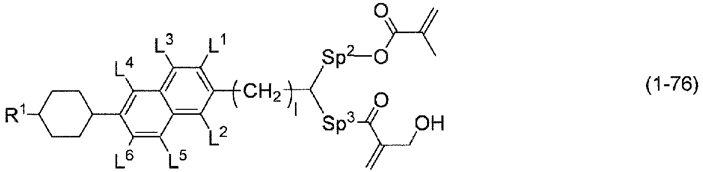 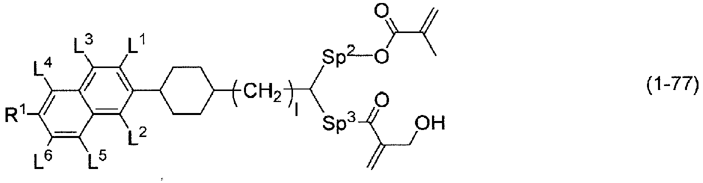 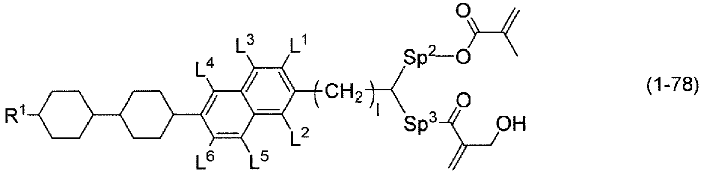 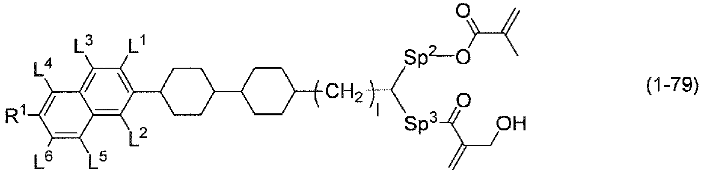 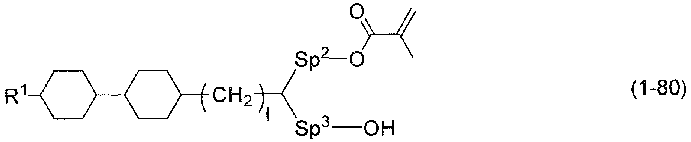 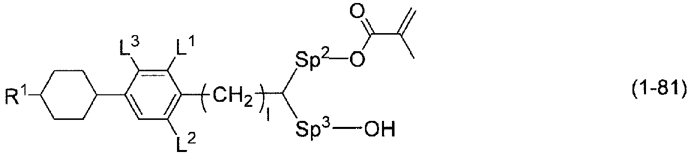 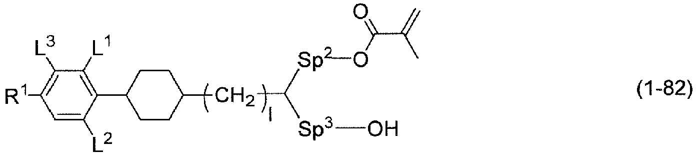 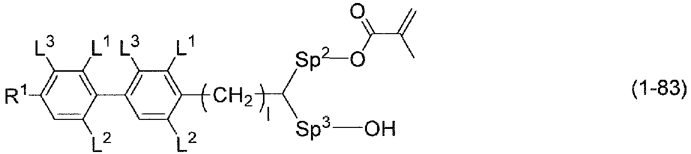 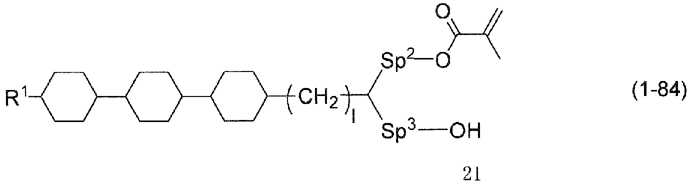 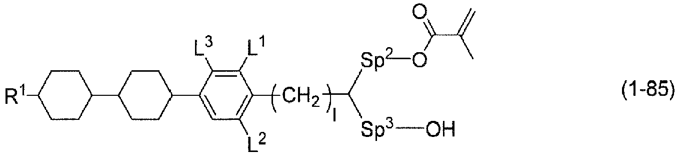 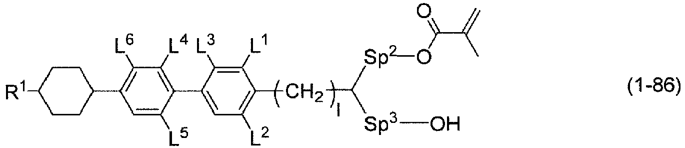 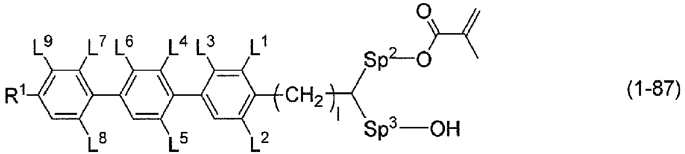 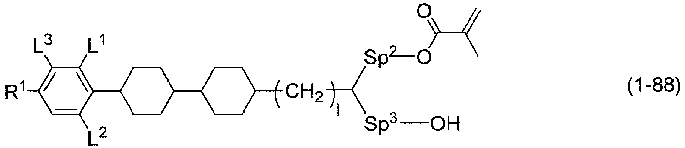 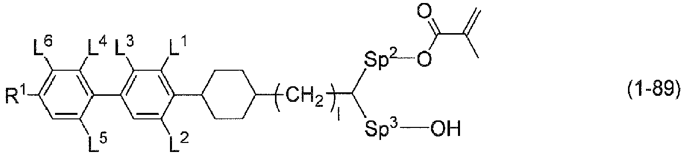 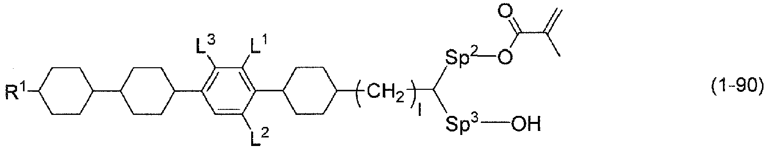 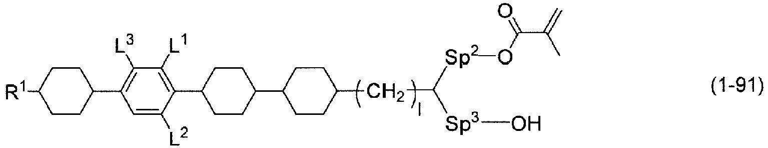 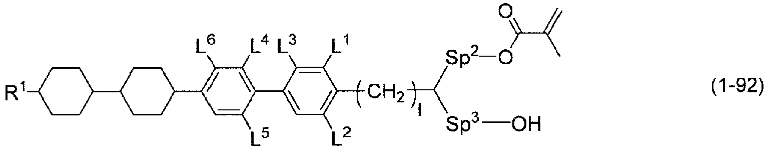 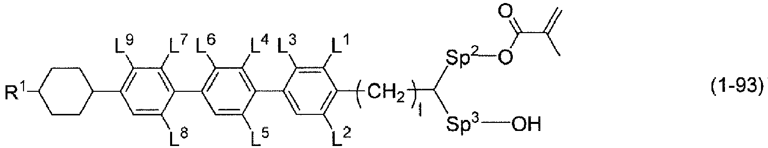 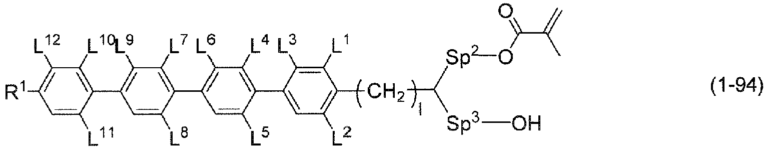 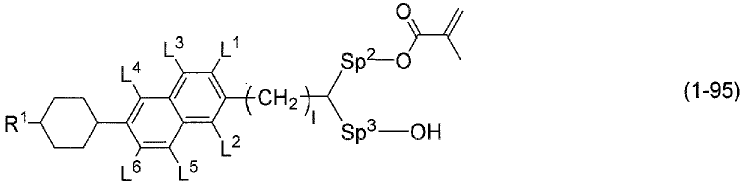 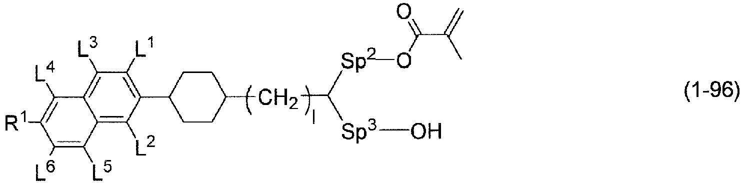 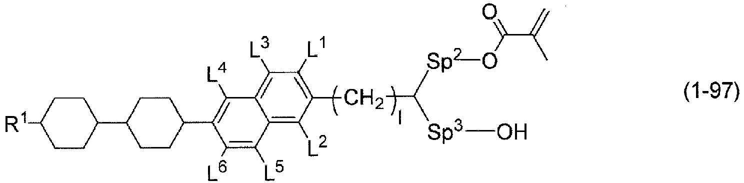 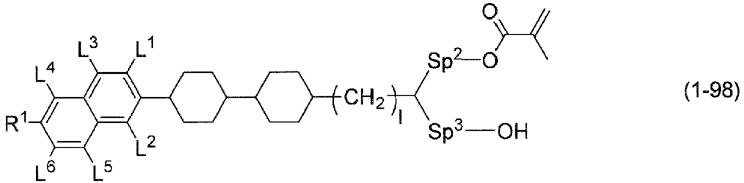 wherein, in formula (1-61) to formula (1-98),
   R¹ is alkyl having 1 to 10 carbons;
   Sp² and Sp³ are independently alkylene having 1 to 3 carbons, and in the alkylene, at least one piece of -CH₂- may be replaced by -O-;
   L¹, L², L³, L⁴, L⁵, L⁶, L⁷, L⁸, L⁹, L¹⁰, L¹¹ and L¹² are independently hydrogen, fluorine or methyl; and
   1 is 1, 2, 3 or 4, and in the alkylene, at least one piece of -CH₂- may be replaced by -O-.
Item 11. The compound according to item 1, represented by any one of formula (1-99) to formula (1-117): 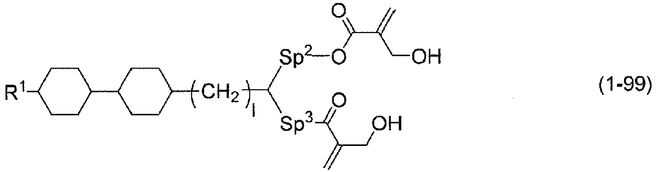 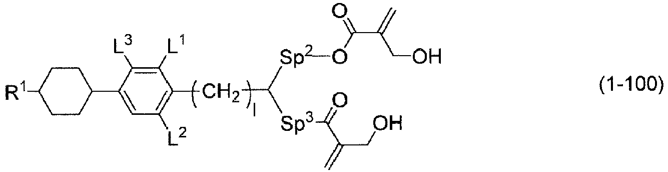 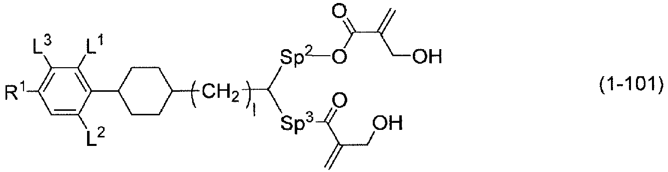 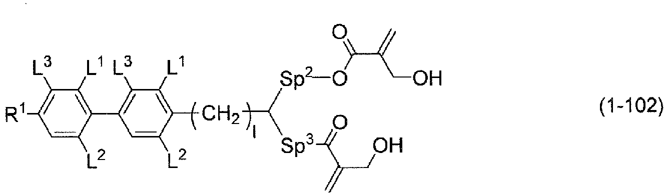 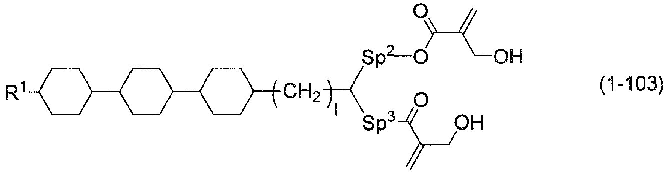 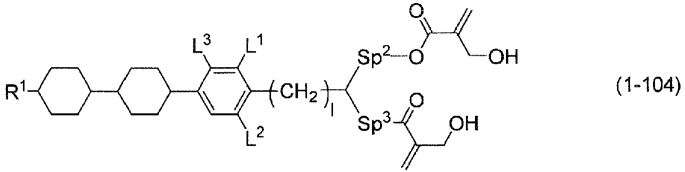 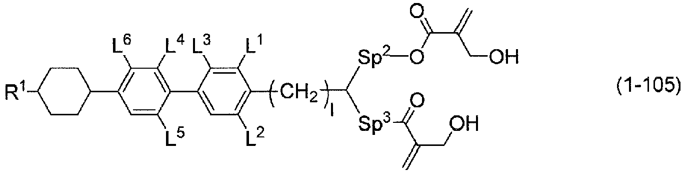 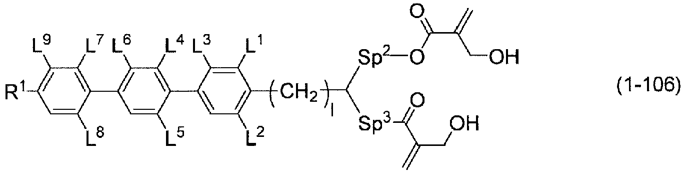 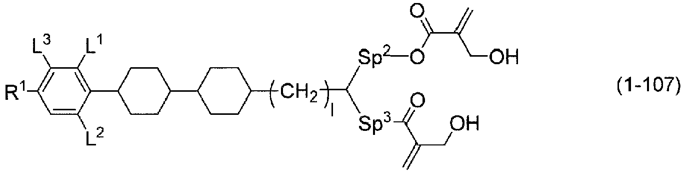 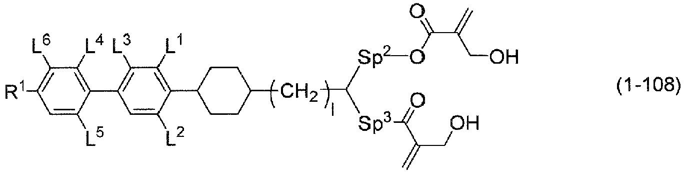 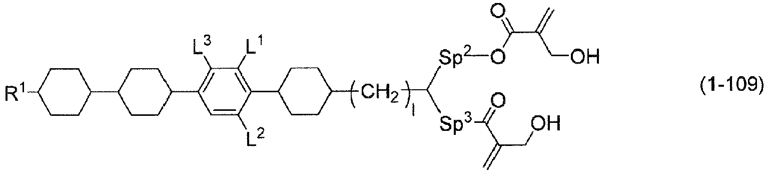 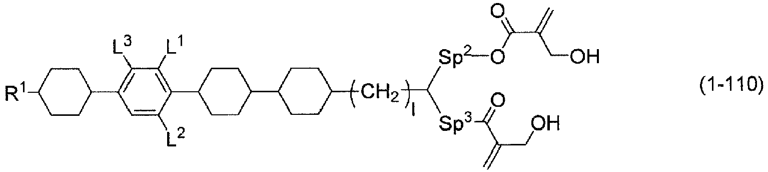 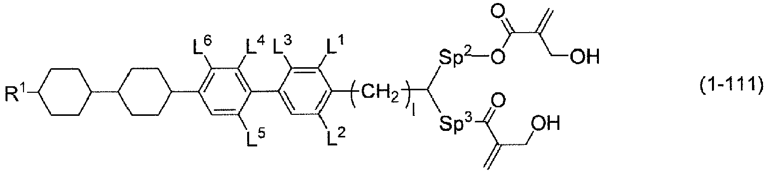 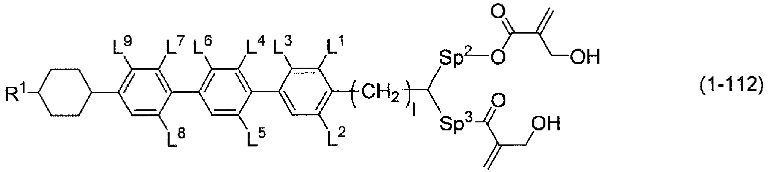 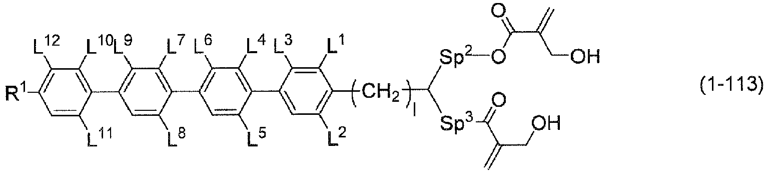 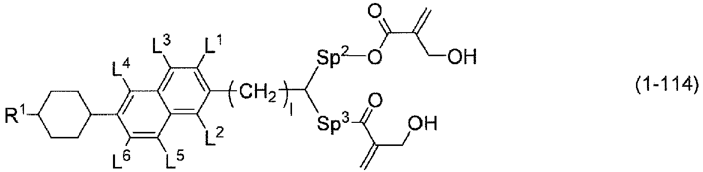 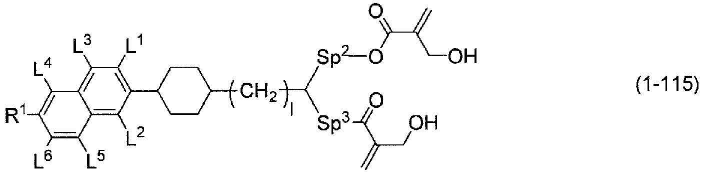 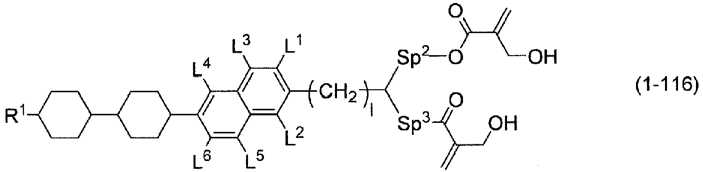 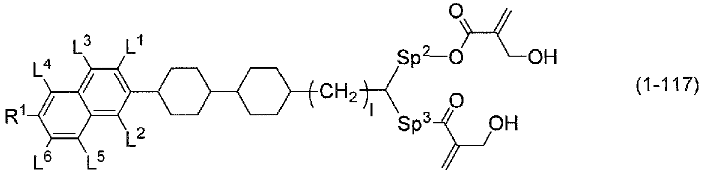 wherein, in formula (1-99) to formula (1-117),
   R¹ is alkyl having 1 to 10 carbons;
   Sp² and Sp³ are independently alkylene having 1 to 3 carbons, and in the alkylene, at least one piece of -CH₂- may be replaced by -O-;
   L¹, L², L³, L⁴, L⁵, L⁶, L⁷, L⁸, L⁹, L¹⁰, L¹¹ and L¹² are independently hydrogen, fluorine or methyl; and
   1 is 1, 2, 3 or 4, and in the alkylene, at least one piece of -CH₂- may be replaced by -O-.
Item 12. A liquid crystal composition, containing at least one of the compounds according to any one of items 1 to 11.
Item 13. The liquid crystal composition according to item 12, further containing at least one compound selected from the group of compounds represented by formula (2) to formula (4): 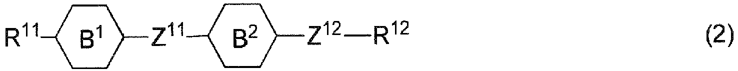 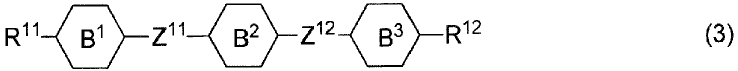 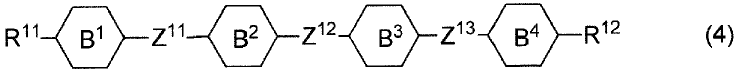 wherein, in formula (2) to formula (4),
   R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
   Z¹¹, Z¹² and Z¹³ are independently a single bond, -CH₂CH₂-, -CH=CH-, -C≡C- or -COO-.
Item 14. The liquid crystal composition according to item 12 or 13, further containing at least one compound selected from the group of compounds represented by formula (5) to formula (7) : 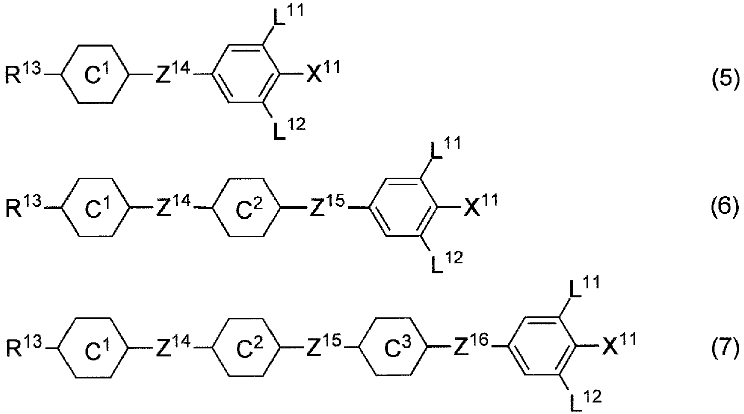 wherein, in formula (5) to formula (7),
   R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   X¹¹ is fluorine, chlorine, -OCF₃, -OCHF₂, -CF₃, -CHF₂, -CH₂F, -OCF₂CHF₂ or -OCF₂CHFCF₃;
   ring C¹, ring C² and ring C³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
   Z¹⁴, Z¹⁵ and Z¹⁶ are independently a single bond, -CH₂CH₂-, -CH=CH-, -C≡C-, -COO-, -CF2O-, -OCF2-, -CH2O- or -(CH₂)₄-; and
   L¹¹ and L¹² are independently hydrogen or fluorine.
Item 15. The liquid crystal composition according to any one of items 12 to 14, further containing at least one compound of compounds represented by formula (8): 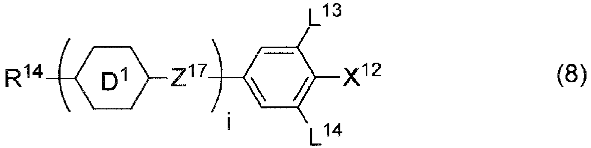 wherein, in formula (8),
   R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   X¹² is -C≡N or -C≡C-C≡N;
   ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
   Z¹⁷ is a single bond, -CH₂CH₂-, -C≡C-, -COO-, -CF₂O-, -OCF₂- or -CH₂O- ;
   L¹³ and L¹⁴ are independently hydrogen or fluorine; and
   i is 1, 2, 3 or 4.
Item 16. The liquid crystal composition according to any one of items 12 to 15, further containing at least one compound selected from the group of compounds represented by formula (9) to formula (15): 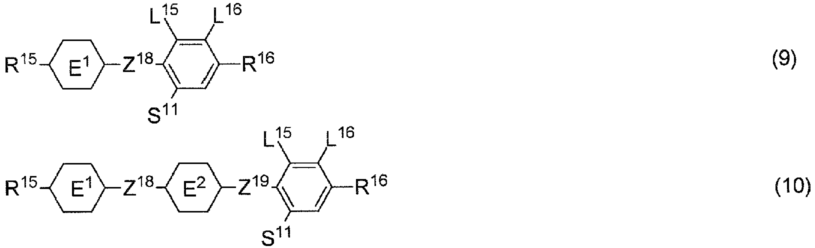 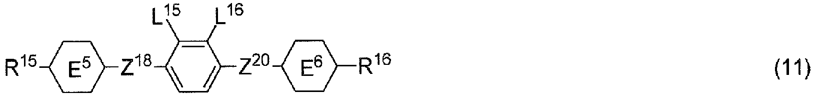 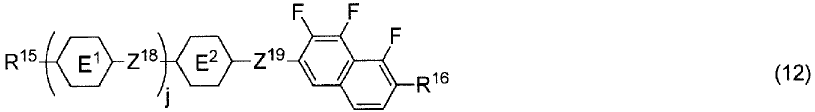 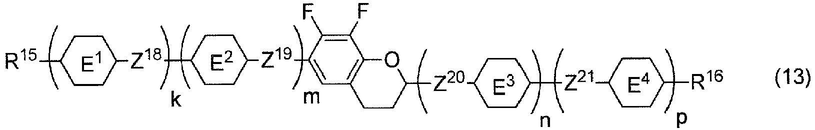 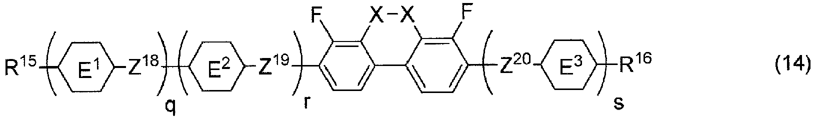 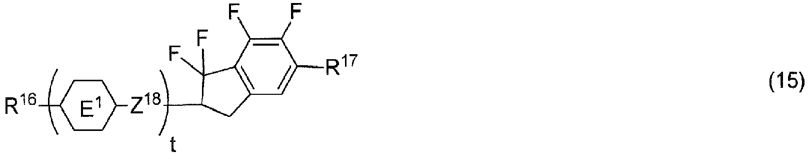 wherein, in formula (9) to formula (15),
   R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine;
   ring E¹, ring E², ring E³ and ring E⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
   ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
   Z¹⁸, Z¹⁹, Z²⁰ and Z²¹ are independently a single bond, -CH₂CH₂-, -COO-, -CH₂O-, -OCF₂- or -OCF₂CH₂CH₂-;
   L¹⁵ and L¹⁶ are independently fluorine or chlorine;
   S¹¹ is hydrogen or methyl;
   X is -CHF- or -CF₂-; and
   j, k, m, n, p, q, r and s are independently 0 or 1, a sum of k, m, n and p is 1 or 2, a sum of q, r and s is 0, 1, 2 or 3, and t is 1, 2 or 3.
Item 17. The liquid crystal composition according to any one of items 12 to 16, further containing at least one compound of polymerizable compounds represented by formula (16): 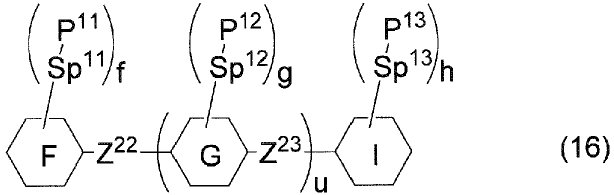 wherein, in formula (16),
   ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   ring G is 1,4-cycloxylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine;
   P¹¹, P¹² and P¹³ are independently a polymerizable group;
   Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
   u is 0, 1 or 2; and
   f, g and h are independently 0, 1, 2, 3 or 4, and a sum of f, g and h is 1 or more.
Item 18. The liquid crystal composition according to item 17, wherein, in formula (16),
   P¹¹, P¹² and P¹³ are independently a group selected from the group of polymerizable groups represented by formula (P-1) to formula (P-5) : 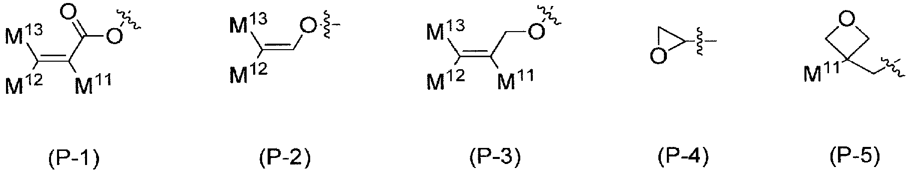 wherein, in formula (P-1) to formula (P-5),
   M¹¹, M¹² and M¹³ are independently hydrogen, fluorine, alkyl having 1 to 5 carbons, or alkyl having 1 to 5 carbons in which at least one hydrogen is replaced by halogen.
Item 19. The liquid crystal composition according to item 17 or 18, wherein the polymerizable compound represented by formula (16) is at least one compound selected from the group of polymerizable compounds represented by formula (16-1) to formula (16-7): 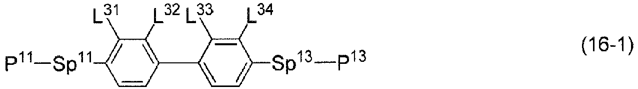 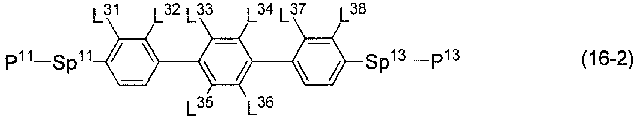 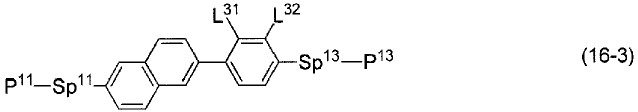 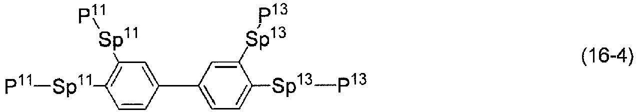 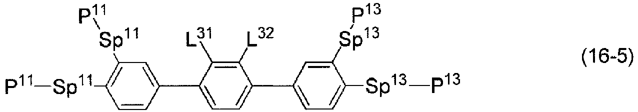 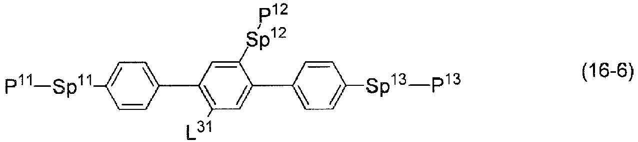 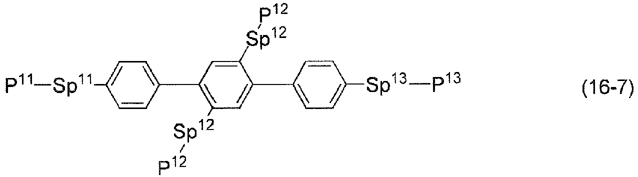 wherein, in formula (16-1) to formula (16-7),
   L³¹, L³², L³³, L³⁴, L³⁵, L³⁶, L³⁷ and L³⁸ are independently hydrogen, fluorine or methyl;
   Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine; and
   P¹¹, P¹² and P¹³ are independently a group selected from the group of polymerizable groups represented by formula (P-1) to formula (P-3) : 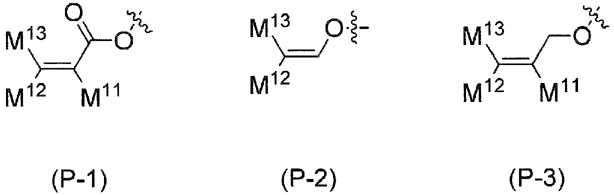
   wherein, in formula (P-1) to formula (P-3),
   M¹¹, M¹² and M¹³ are independently hydrogen, fluorine, alkyl having 1 to 5 carbons, or alkyl having 1 to 5 carbons in which at least one hydrogen is replaced by halogen.
Item 20. The liquid crystal composition according to any one of items 12 to 19, further containing at least one of a polymerizable compound different from the compound represented by formula (1) or formula (16) described below, a polymerization initiator, a polymerization inhibitor, an optically active compound, an antioxidant, an ultraviolet light absorber, a light stabilizer, a heat stabilizer and an antifoaming agent: 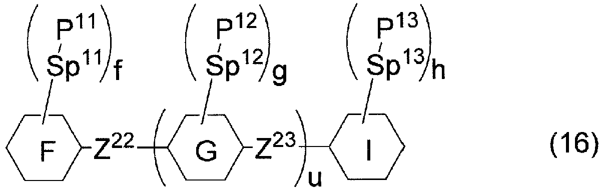 wherein, in formula (16),
   ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   ring G is 1,4-cycloxylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine;
   P¹¹, P¹² and P¹³ are independently a polymerizable group;
   Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
   u is 0, 1 or 2; and
   f, g and h are independently 0, 1, 2, 3 or 4, and a sum of f, g and h is 1 or more.
Item 21. A liquid crystal display device, including at least one of the liquid crystal compositions according to any one of items 12 to 20.

The invention further includes the following items: (a) the liquid crystal composition, further containing at least two of additives such as a polymerizable compound, a polymerization initiator, a polymerization inhibitor, an optically active compound, an antioxidant, an ultraviolet light absorber, a light stabilizer, a heat stabilizer and an antifoaming agent; (b) a polymerizable composition, prepared by adding a polymerizable compound different from compound (1) or compound (16) to the liquid crystal composition; (c) a polymerizable composition, prepared by adding compound (1) and compound (16) to the liquid crystal composition; (d) a liquid crystal composite, prepared by polymerizing the polymerizable composition; (e) a polymer sustained alignment mode device, including the liquid crystal composite; and (f) a polymer sustained alignment mode device, prepared by using a polymerizable composition prepared by adding compound (1), compound (16), and a polymerizable compound different from compound (1) or compound (16) to the liquid crystal composition.

An aspect of compound (1), synthesis of compound (1), the liquid crystal composition and the liquid crystal display device will be described in the following order.

### 1. Aspect of compound (1)

Compound (1) of the invention has a mesogen site formed of at least one ring, and a plurality of polar groups. Compound (1) is useful because the polar group interacts with the substrate surface of glass (or metal oxide) in a noncovalent bonding manner. One of applications is the additive for the liquid crystal composition used in the liquid crystal display device. Compound (1) is added for the purpose of controlling alignment of liquid crystal molecules. Such an additive is preferably chemically stable under conditions in which the additive is sealed in the device, has high solubility in the liquid crystal composition, and has a large voltage holding ratio when the additive is used in the liquid crystal display device. Compound (1) satisfies such characteristics in a significant degree.

Preferred examples of compound (1) will be described. Preferred examples of symbols such as R¹, MES, Sp¹ and P¹ in compound (1) apply also to a subordinate formula of compound (1). In compound (1), characteristics can be arbitrarily adjusted by suitably combining the types of the groups . Compound (1) may contain a larger amount of isotope such as ²H (deuterium) and ¹³C than the amount of natural abundance because no significant difference exists in the characteristics of the compound.

**R¹-MES-Sp¹-P¹** **(1)**

In formula (1), R¹ is hydrogen or alkyl having 1 to 15 carbons, and in the alkyl, at least one piece of -CH₂- may be replaced by -O-, -S- or -NH-, and at least one piece of -(CH₂)₂- may be replaced by -CH=CH-, and in the groups, at least one hydrogen may be replaced by halogen.

Preferred R¹ is hydrogen, alkyl having 1 to 15 carbons, alkenyl having 2 to 15 carbons, alkoxy having 1 to 14 carbons or alkenyloxy having 2 to 14 carbons, and in the groups, at least one hydrogen may be replaced by fluorine or chlorine. Further preferred R¹ is hydrogen, alkyl having 1 to 10 carbons or alkoxy having 1 to 9 carbons, and in the groups, at least one hydrogen may be replaced by fluorine. Particularly preferred R¹ is alkyl having 1 to 10 carbons.

In formula (1), MES is the mesogen group having at least one ring. The mesogen group is known to those skilled in the art. The mesogen group means a part contributing to formation of a liquid crystal phase (mesophase) when the compound has the liquid crystal phase. Preferred examples of compound (1) include compound (1-1).

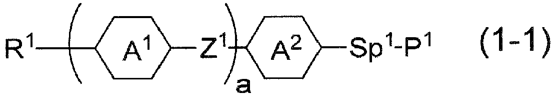

Preferred ring A¹ or ring A² is 1,4-cycloxylene, 1,4-cyclohexenylene, 1,4-phenylene, naphthalene-2,6-diyl, decahydronaphthalene-2,6-diyl, 1,2,3,4-tetrahydronaphthalene-2,6-diyl, tetrahydropyran-2,5-diyl, 1,3-dioxane-2,5-diyl, pyrimidine-2,5-diyl, pyridine-2,5-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine, chlorine, alkyl having 1 to 12 carbons, alkenyl having 2 to 12 carbons, alkoxy having 1 to 11 carbons or alkenyloxy having 2 to 11 carbons, and in the groups, at least one hydrogen may be replaced by fluorine or chlorine. Further preferred ring A¹ or ring A² is 1,4-cycloxylene, 1,4-phenylene, naphthalene-2,6-diyl, perhydrocyclopenta[a]phenanthrene-3,17-diyl, 2,3,4,7,8,9,10,11,12,13,14,15,16,17-tetradecahydrocyclopenta[a]phe nanthrene-3,17-diyl, and in the rings, at least one hydrogen may be replaced by fluorine or alkyl having 1 to 5 carbons. Particularly preferred ring A¹ or ring A² is 1,4-cycloxylene, 1,4-phenylene, naphthalene-2,6-diyl or perhydrocyclopenta[a]phenanthrene-3,17-diyl, and in the rings, at least one hydrogen may be replaced by fluorine, methyl or ethyl.

In formula (1-1), Z¹ is a single bond or alkylene having 1 to 4 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen.

Preferred Z¹ is a single bond, -(CH₂)₂-, -CH=CH-, -C≡C-, -COO-, -OCO-, -CF₂O-, -OCF₂-, -CH₂O-, -OCH₂- or -CF=CF-. Further preferred Z¹ is a single bond, -(CH₂)₂-, -COO- or -OCO-. Particularly preferred Z¹ is a single bond.

In formula (1-1), a is 0, 1, 2, 3 or 4. Preferred a is 0, 1, 2, or 3. Further preferred a is 0, 1 or 2. Particularly preferred a is 1 or 2.

In formula (1-1), Sp¹ is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen, and in the groups, at least one or more hydrogens are replaced by a polymerizable group represented by formula (1a); 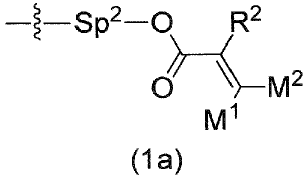 wherein, in formula (1a), Sp² is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen;
M¹ and M² are independently hydrogen, halogen, alkyl having 1 to 5 carbons or alkyl having 1 to 5 carbons in which at least one hydrogen is replaced by halogen;
R² is hydrogen or alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen.

In formula (1-1), preferred Sp¹ is alkylene having 1 to 5 carbons or alkylene having 1 to 5 carbons in which one piece of -CH₂- is replaced by -O-. Further preferred Sp¹ is alkylene having 1 to 3 carbons, or alkylene having 1 to 3 carbons in which one piece of -CH₂- is replaced by -O-, and in the groups, at least one hydrogen is replaced by a polymerizable group represented by formula (1a).

In formula (1a), preferred Sp² is a single bond, alkylene having 1 to 5 carbons, or alkylene having 1 to 5 carbons in which one piece of -CH₂- is replaced by -O-. Further preferred Sp¹ is a single bond, alkylene having 1 to 3 carbons, or alkylene having 1 to 3 carbons in which one piece of -CH₂- is replaced by -O-.

In formula (1a), preferred R² is hydrogen or alkylene having 1 to 5 carbons, or alkylene having 1 to 5 carbons in which one piece of -CH₂- is replaced by -O-. Further preferred R² is hydrogen, alkylene having 1 to 3 carbons, or alkylene having 1 to 3 carbons in which one piece of -CH₂- is replaced by -O-. Particularly preferred R² is hydrogen or methyl. When R² is -CH₂-OH, vertical alignment in low concentration addition is expected by an effect that two hydroxyl groups exist in a molecule.

In formula (1a), M¹ and M² are independently hydrogen, halogen, alkyl having 1 to 5 carbons, or alkyl having 1 to 5 carbons in which at least one hydrogen is replaced by halogen. For increasing reactivity, preferred M¹ or M² is hydrogen or methyl. Further preferred M¹ or M² is hydrogen.

In formula (1), P¹ is a group selected from groups represented by formula (1e) and formula (1f):

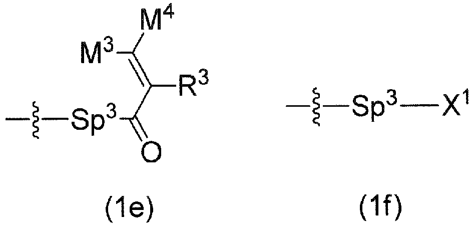

In formula (1e), R³ is a group selected from groups represented by formula (1g), formula (1h) and formula (1i):

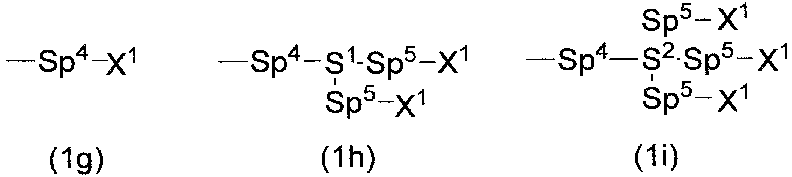

In formula (1e) and formula (1f), preferred Sp³ is alkylene having 1 to 7 carbons, or alkylene having 1 to 5 carbons in which one piece of -CH₂- is replaced by -O-. Further preferred Sp³ is alkylene having 1 to 5 carbons, or alkylene having 1 to 5 carbons in which one piece of -CH₂- is replaced by -O-. Particularly preferred Sp³ is -CH₂O- in formula (1e), and is -CH₂- in formula (1f).

In formula (1e), M³ and M⁴ are independently hydrogen, halogen, alkyl having 1 to 5 carbons, or alkyl having 1 to 5 carbons in which at least one hydrogen is replaced by halogen. For increasing the reactivity, preferred M³ or M⁴ is hydrogen or methyl. Further preferred M³ or M⁴ is hydrogen.

In formula (1e), preferred R³ is a group selected from the group of polar groups represented by formula (1g), formula (1h) and formula (1i). Preferred R³ is a polar group represented by formula (1g) or formula (1h). Further preferred R³ is a polar group represented by formula (1g).

In formula (1g), formula (1h) and formula (1i), preferred Sp⁴ or Sp⁵ is alkylene having 1 to 7 carbons, or alkylene having 1 to 5 carbons in which one piece of -CH₂- is replaced by -O-. Further preferred Sp⁴ or Sp⁵ is alkylene having 1 to 5 carbons, or alkylene having 1 to 5 carbons in which one piece of -CH₂- is replaced by -O-. Particularly preferred Sp⁴ or Sp⁵ is -CH₂-.

In formula (1g) and formula (1i), S¹ is >CH- or >N-, and S² is >C< or >Si<. Preferred S¹ is >CH-, and preferred S² is >C<.

In formula (If), formula (1g) and formula (1i), X¹ is -OH, -NH₂, -OR⁵, -N(R⁵)₂, -COOH, -SH, -B(OH)₂ or -Si(R⁵)₃, in which R⁵ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine or chlorine.

Preferred X¹ is -OH, -NH₂ or -N(R⁵)₂, in which R⁵ is alkyl having 1 to 5 carbons or alkoxy having 1 to 4 carbons. Further preferred X¹ is -OH and -NH₂ or -N(R⁵)₂. Particularly preferred X¹ is -OH.

In formula (2) to formula (15), component compounds of the liquid crystal composition are shown. Compounds (2) to (4) have small dielectric anisotropy. Compounds (5) to (7) have large positive dielectric anisotropy. Compound (8) has a cyano group, and therefore has larger positive dielectric anisotropy. Compounds (9) to (15) have large negative dielectric anisotropy. Specific examples of the compounds will be described later.

In compound (16), P¹¹, P¹² and P¹³ are independently a polymerizable group. Preferred P¹¹, P¹² or P¹³ is a polymerizable group selected from the group of groups represented by formula (P-1) to formula (P-5). Further preferred P¹¹, P¹² or P¹³ is a group represented by formula (P-1), formula (P-2) or formula (P-3). A particularly preferred group represented by formula (P-1) is -OCO-CH=CH₂ or -OCO-C(CH₃)=CH₂. A wavy line in formulas (P-1) to (P-5) shows a site to which bonding is made.

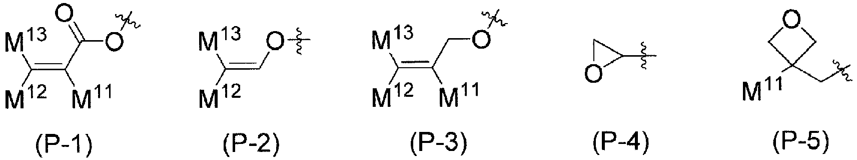

In formula (P-1) to formula (P-5), M¹¹, M¹² and M¹³ are independently hydrogen, fluorine, alkyl having 1 to 5 carbons, or alkyl having 1 to 5 carbons in which at least one hydrogen is replaced by halogen. For increasing the reactivity, preferred M¹¹, M¹² or M¹³ is hydrogen or methyl. Further preferred M¹¹ is methyl, and further preferred M¹² or M¹³ is hydrogen.

Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine. Preferred Sp¹¹, Sp¹² or Sp¹³ is a single bond.

Ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen. Preferred ring F or ring I is phenyl. Ring G is 1, 4-cycloxylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl, 1,3-dioxane-2,5-diyl, pyrimidine-2,5-diyl, or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen. Particularly preferred ring G is 1, 4-phenylene or 2-fluoro-1,4-phenylene.

Z²² and Z²³ are independently a single bond or alkylene having 1 to 10 carbons, and in the alkylene, at least one piece of -CH₂- may be replaced by -O-, -CO-, -COO- or -OCO-, and at least one piece of - (CH₂)₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine. Preferred Z⁷ or Z⁸ is a single bond, -(CH₂)₂-, -CH₂O-, -OCH₂-, -COO- or -OCO-. Further preferred Z²² or Z²³ is a single bond.

Then, u is 0, 1 or 2. Preferred u is 0 or 1. Then, f, g and h are independently 0, 1, 2, 3 or 4, and a sum of f, g and h is 1 or more. Preferred f, g or h is 1 or 2.

### 2. Synthesis of compound (1)

A synthetic method of compound (1) will be described. Compound (1) can be prepared by suitably combining methods in synthetic organic chemistry. The compounds whose synthetic methods are not described above are produced by the methods described in the books such as "Organic Syntheses" (John Wiley & Sons, Inc.), "Organic Reactions" (John Wiley & Sons, Inc.), "Comprehensive Organic Synthesis" (Pergamon Press), and "New Experimental Chemistry Course (Shin Jikken Kagaku Koza in Japanese)" (Maruzen Co., Ltd.).

### 2-1. Formation of a bonding group

An example of a method of forming a bonding group in compound (1) is as described in a scheme described below. In the scheme, MSG¹ (or MSG²) is a monovalent organic group having at least one ring. The monovalent organic groups represented by a plurality of MSG¹ (or MSG²) may be identical or different. Compounds (1A) to (1G) correspond to compound (1) or an intermediate of compound (1).

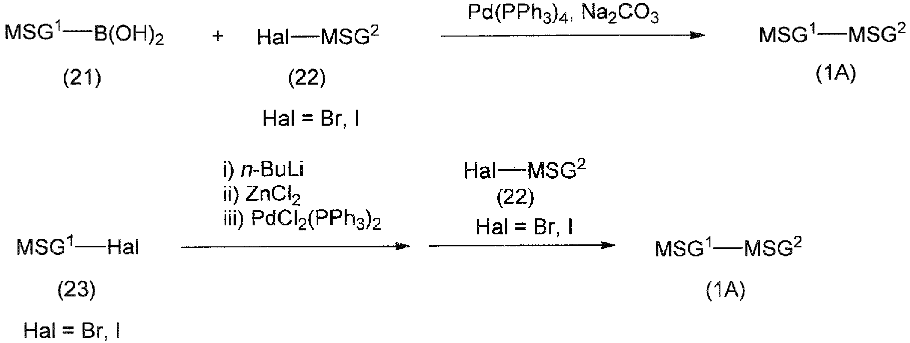

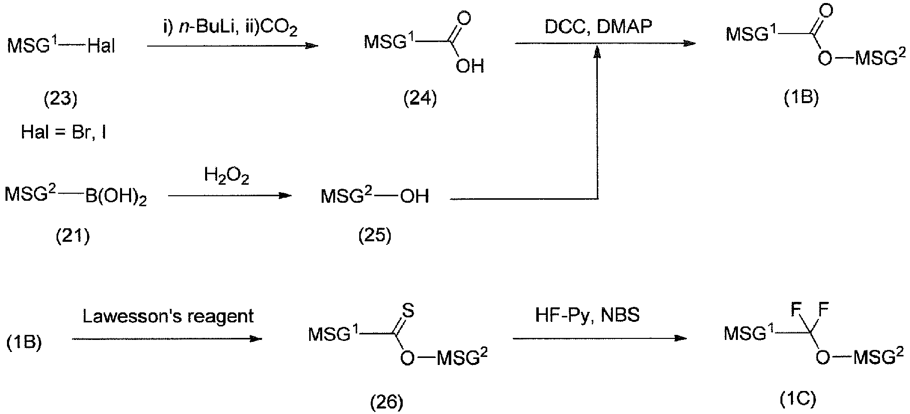

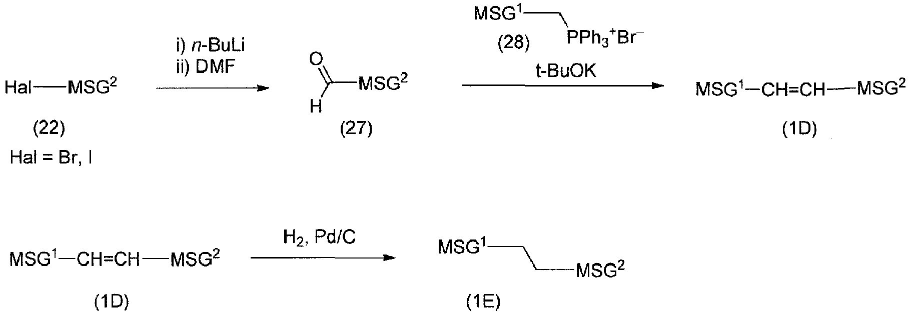

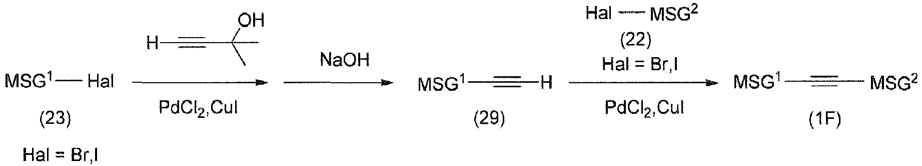

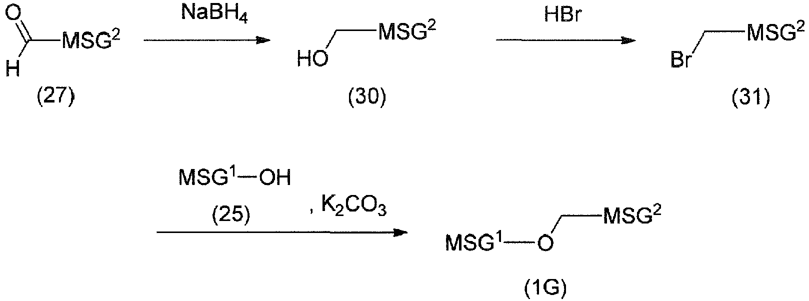

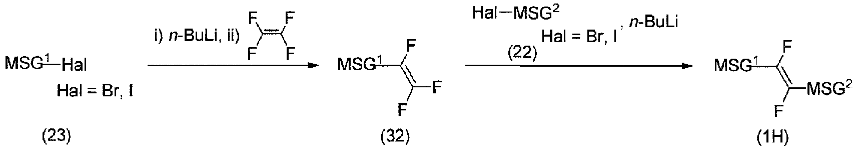

### (I) Formation of a single bond

Compound (1A) is prepared by allowing arylboronic acid (21) to react with compound (22) in the presence of carbonate and a tetrakis(triphenylphosphine)palladium catalyst. Compound (1A) is also prepared by allowing compound (23) to react with n-butyllithium and subsequently with zinc chloride, and further with compound (22) in the presence of a dichlorobis(triphenylphosphine)palladium catalyst.

### (II) Formation of -COO- and -OCO-

Carboxylic acid (24) is obtained by allowing compound (23) to react with n-butyllithium and subsequently with carbon dioxide. Compound (1B) having -COO- is prepared by dehydrating carboxylic acid (24) and phenol (25) derived from compound (21), in the presence of 1,3-dicyclohexylcarbodiimide (DCC) and 4-dimethylaminopyridine (DMAP) . A compound having -OCO- is also prepared according to the above method.

### (III) Formation of -CF₂O- and -OCF₂-

Compound (26) is obtained by thionating compound (1B) with a Lawesson's reagent. Compound (1C) having -CF₂O- is prepared by fluorinating compound (26) with a hydrogen fluoride-pyridine complex and N-bromosuccinimide (NBS). Refer to M. Kuroboshi et al., Chem. Lett., 1992, 827. Compound (1C) is also prepared by fluorinating compound (26) with (diethylamino) sulfur trifluoride (DAST). Refer to W. H. Bunnelle et al., J. Org. Chem. 1990, 55, 768. A compound having -OCF₂- is also prepared according to the above method.

### (IV) Formation of -CH=CH-

Aldehyde (27) is obtained by allowing compound (22) to react with n-butyllithium and subsequently with N,N-dimethylformamide (DMF). Compound (1D) is prepared by allowing phosphorus ylide generated by allowing phosphonium salt (28) to react with potassium tert-butoxide to react with aldehyde (27). A cis isomer may be formed depending on reaction conditions, and therefore the cis isomer is isomerized into a trans isomer according to a publicly known method when necessary.

### (V) Formation of -CH₂CH₂-

Compound (1E) is prepared by hydrogenating compound (1D) in the presence of a palladium on carbon catalyst.

### (VI) Formation of -C≡C-

Compound (29) is obtained by allowing compound (23) to react with 2-methyl-3-butyn-2-ol in the presence of a catalyst of dichloropalladium and copper halide, and then performing deprotection under basic conditions. Compound (1F) is prepared by allowing compound (29) to react with compound (22) in the presence of a catalyst of dichlorobis(triphenylphosphine)palladium and copper halide.

### (VII) Formation of -CH₂O- and -OCH₂-

Compound (30) is obtained by reducing compound (27) with sodium borohydride. Compound (31) is obtained by brominating compound (30) with hydrobromic acid. Compound (1G) is prepared by allowing compound (25) to react with compound (31) in the presence of potassium carbonate. A compound having -OCH₂- is also prepared according to the above method.

### (VIII) Formation of -CF=CF-

Compound (32) is obtained by treating compound (23) with n-butyllithium and subsequently allowing treated compound (23) to react with tetrafluoroethylene. Compound (1H) is prepared by treating compound (22) with n-butyllithium, and then allowing treated compound (22) to react with compound (32).

### 2-2. Formation of ring A²

With regard to a ring such as 1,4-cyclohexylene, 1,4-cyclohexenylene, 1,4-phenylene, 2-fluoro-1,4-phenylene, 2-methyl-1,4-phenylene, 2-ethyl-1,4-phenylene, naphthalene-2, 6-diyl, decahydronaphthalene-2,6-diyl, 1,2,3,4-tetrahydronaphthalene-2,6-diyl, tetrahydropyran-2,5-diyl, 1,3-dioxane-2,5-diyl, pyrimidine-2,5-diyl and pyridine-2,5-diyl, a starting material is commercially available or a synthetic method is known well.

### 2-3. Synthesis Example

Examples of a method of preparing compound (1) are as described below. In the compounds, definitions of R¹, MES, M¹ and M² are identical to the definitions described in item 1.

Compounds (1-51) and (1-52) in which R² is a group represented by formula (1a), Sp⁴ is -CH₂-, and X¹ is -OH can be prepared according to a method described below.

Compound (52) is obtained by allowing compound (51) to react in the presence of formaldehyde and 1,4-diazabicyclo[2.2.2]octane (DABCO) . Compound (53) is obtained by allowing compound (52) to react in the presence of pyridinium p-toluenesulfonate (PPTS) and 3,4-dihydro-2H-pyran.

Compound (1-51) can be obtained by allowing compound (54) to react in the presence of triethylamine (Et₃N) and methacryloyl chloride. Compound (55) is obtained by allowing compound (1-51) to react with compound (53) in the presence of DCC and DMAP, and then compound (55) can be derived to compound (1-52) by performing deprotection by using PPTS.

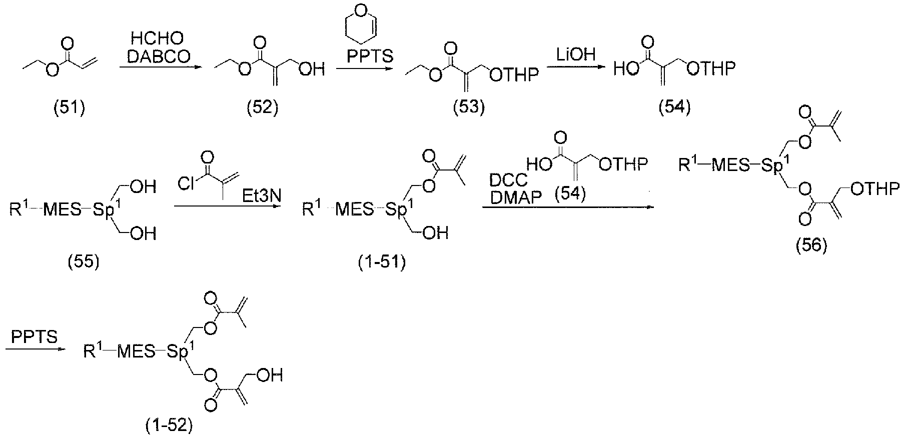

Compound (1-53) in which R² is a group represented by formula (1a), Sp⁴ is -(CH₂)₂-, and X¹ is -OH can be prepared according to a method described below. Compound (56) is obtained by acting phosphorus tribromide on compound (1-52). Then, compound (1-53) can be derived therefrom by acting indium on compound (57), and then allowing the resulting compound to react with formaldehyde.

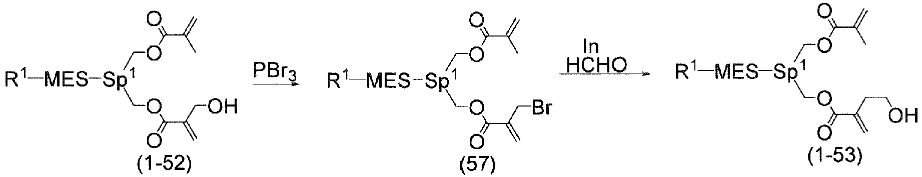

Compound (1-54) in which R² is a group represented by formula (1a), Sp⁴ is -CH₂-, and X¹ is -OH can be prepared according to a method described below.

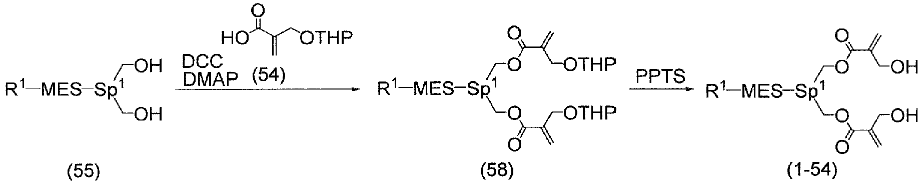

### 3. Liquid crystal composition

### 3-1. Component compound

The liquid crystal composition of the invention contains compound (1) as component A. Compound (1) can control alignment of liquid crystal molecules by interaction with a substrate of the device in the noncovalent bonding manner. The composition contains compound (1) as component A, and preferably further contains a liquid crystal compound selected from component B, C, D and E described below. Component B includes compounds (2) to (4). Component C includes compounds (5) to (7). Component D includes compound (8). Component E includes compounds (9) to (15). The composition may contain any other liquid crystal compound different from compounds (2) to (15). When the composition is prepared, components B, C, D and E are preferably selected by taking magnitude of positive or negative dielectric anisotropy, and so forth into account. The composition in which the components are appropriately selected has a high maximum temperature, a low minimum temperature, small viscosity, suitable optical anisotropy (more specifically, large optical anisotropy or small optical anisotropy), large positive or negative dielectric anisotropy, large specific resistance, high stability to heat and ultraviolet light and a suitable elastic constant (more specifically, a large elastic constant or a small elastic constant).

Compound (1) is added to the composition for the purpose of controlling alignment of liquid crystal molecules. A preferred proportion of compound (1) is 0.05% by weight or more based on the weight of the liquid crystal composition for aligning the liquid crystal molecules, and 10% by weight or less based on the weight of the liquid crystal composition for preventing poor display of the device. A further preferred proportion is in the range of 0.1% by weight to 7% by weight based on the weight of the liquid crystal composition. A particularly preferred proportion is in the range of 0.5% by weight to 5% by weight based on the weight of the liquid crystal composition. When, in addition to compound (1), compound (16) is contained to the composition, the total amount thereof is preferably 0.05% by weight or more and 10% by weight or less based on the weight of the liquid crystal composition. A further preferred proportion is in the range of 0.1% by weight to 7% by weight based on the weight of the liquid crystal composition. A particularly preferred proportion is in the range of 0.5% by weight to 5% by weight based on the weight of the liquid crystal composition.

Component B includes a compound in which two terminal groups are alkyl or the like. Preferred examples of component B include compounds (2-1) to (2-11), compounds (3-1) to (3-19) and compounds (4-1) to (4-7). In the compound of component B, R¹¹ and R¹² are independently alkyl having 1 to 10 carbons or alkenyl having 2 to 10 carbons, and in the alkyl or alkenyl, at least one piece of -CH₂- may be replaced by -O-, and at least one hydrogen may be replaced by fluorine.

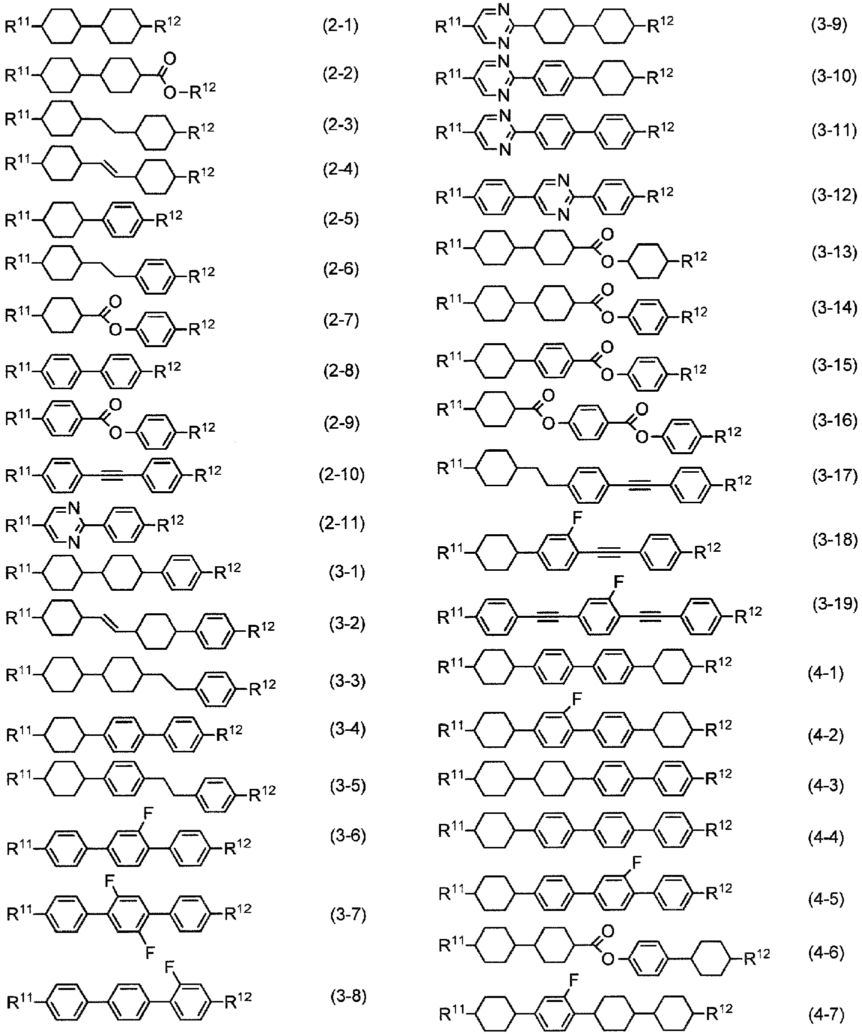

Component B has a small absolute value of the dielectric anisotropy, and therefore is a compound close to neutrality. Compound (2) is effective mainly in decreasing the viscosity or effective in adjusting the optical anisotropy. Compounds (3) and (4) are effective in extending the temperature range of the nematic phase by increasing the maximum temperature, or effective in adjusting the optical anisotropy.

As a content of component B is increased, the dielectric anisotropy of the composition is decreased, but the viscosity is decreased. Thus, as long as a desired value of a threshold voltage of the device is met, the content is preferably as large as possible. When a composition for the IPS mode, the VA mode or the like is prepared, the content of component B is preferably 30% by weight or more, and further preferably 40% by weight or more, based on the weight of the liquid crystal composition.

Component C is a compound having a halogen-containing group or a fluorine-containing group at a right terminal. Specific examples of preferred component C include compounds (5-1) to (5-16), compounds (6-1) to (6-113) and compounds (7-1) to (7-57). In the compound of component C, R¹³ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and X¹¹ is fluorine, chlorine, -OCF₃, -OCHF₂, -CF₃, -CHF₂, -CH₂F, -OCF₂CHF₂ or -OCF₂CHFCF₃.

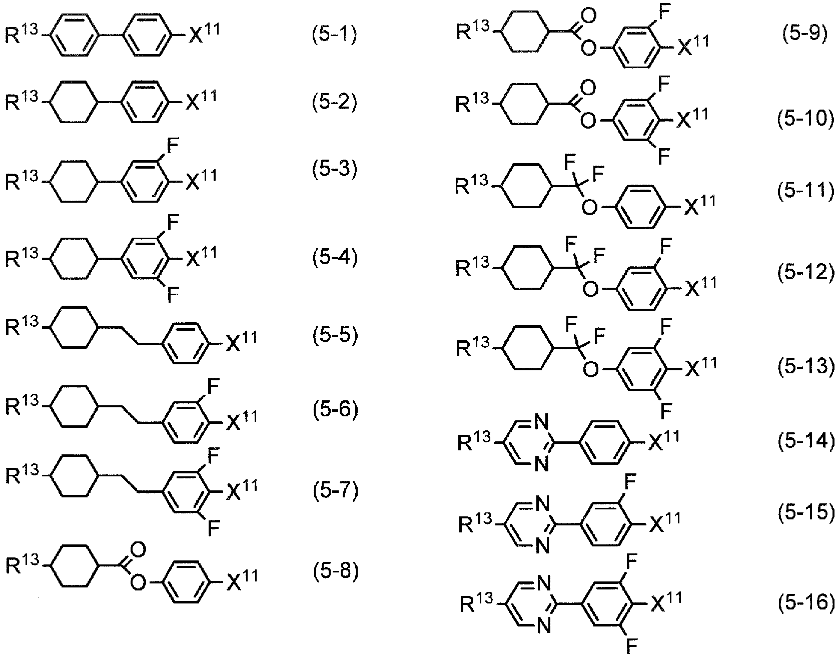

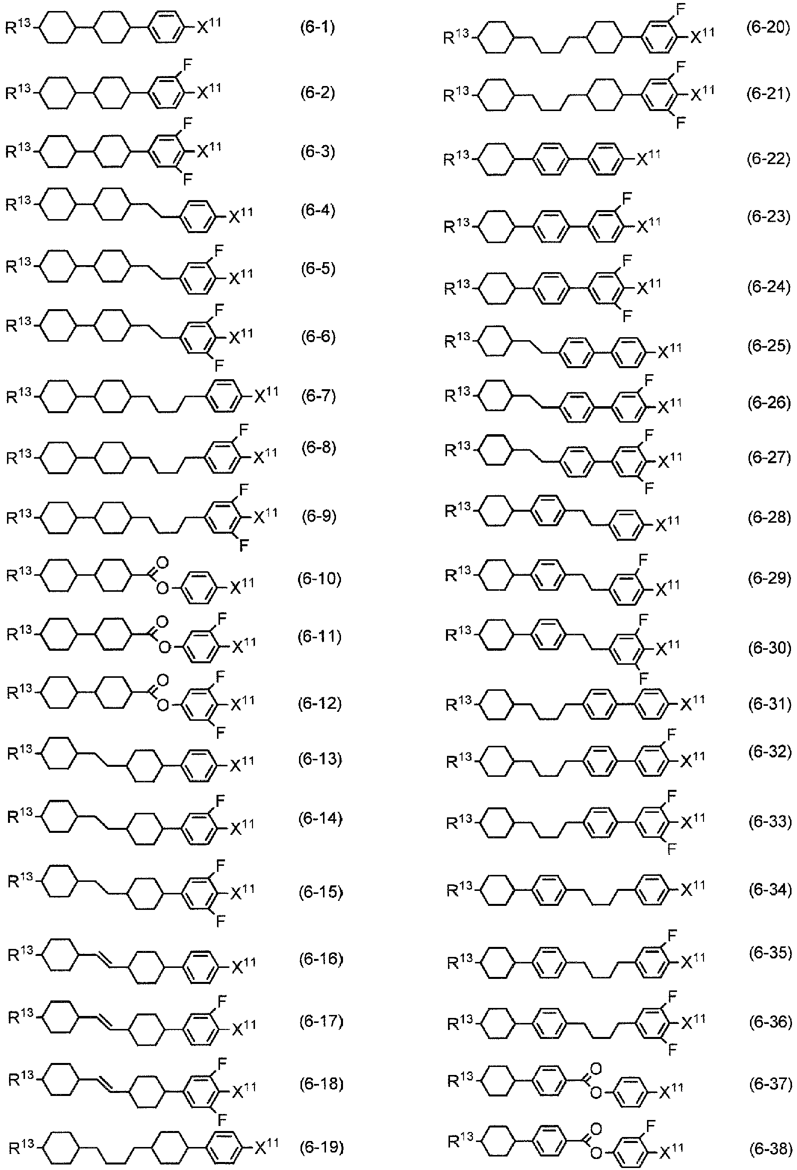

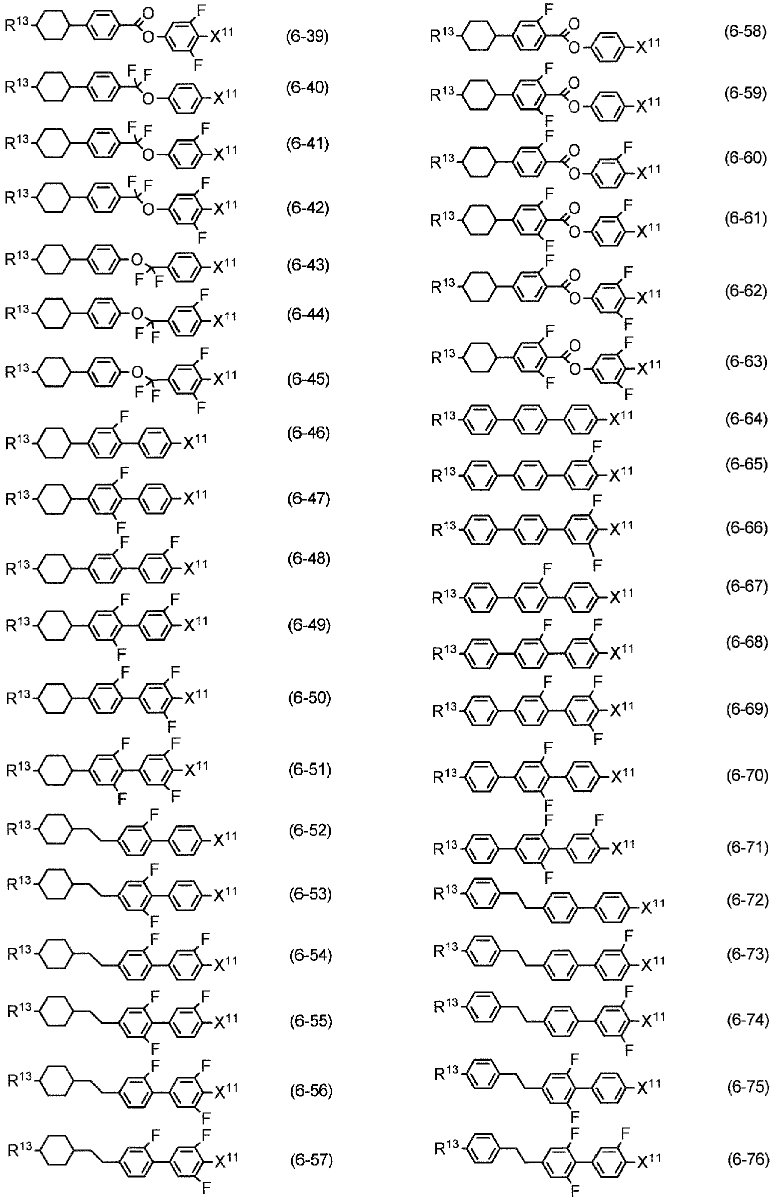

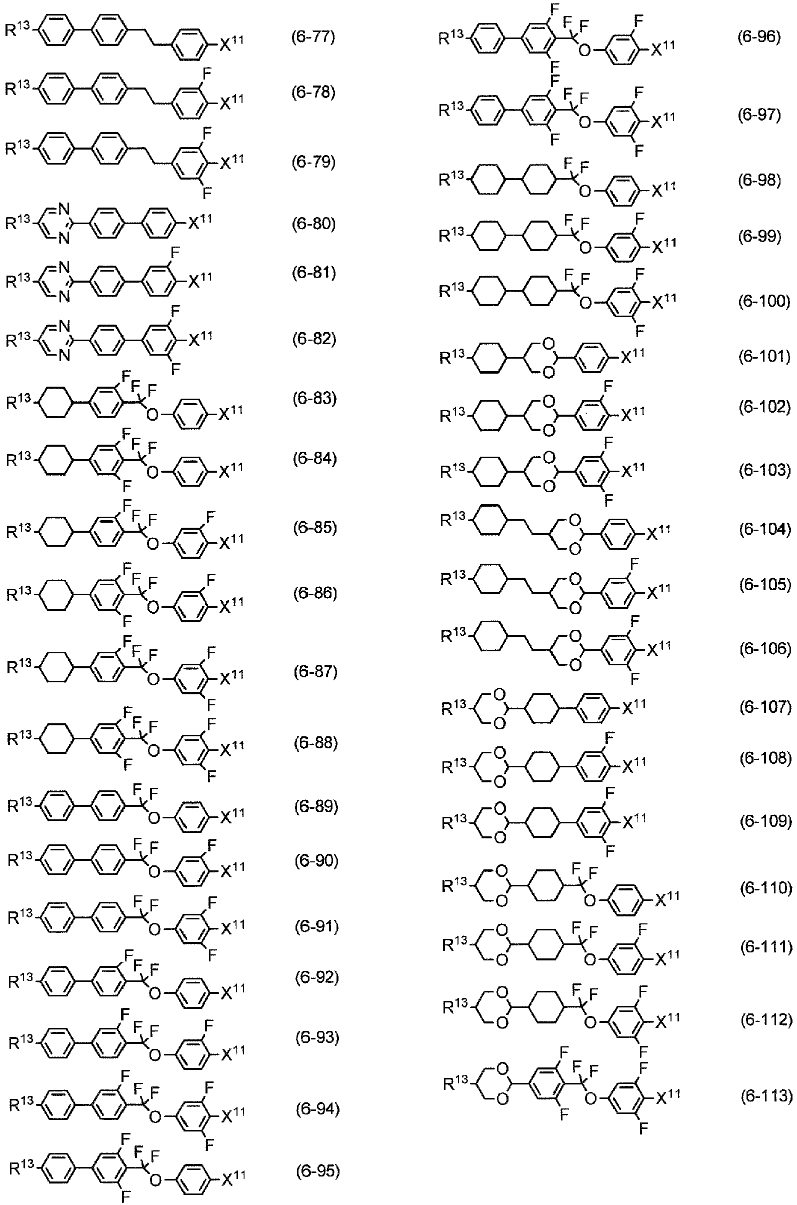

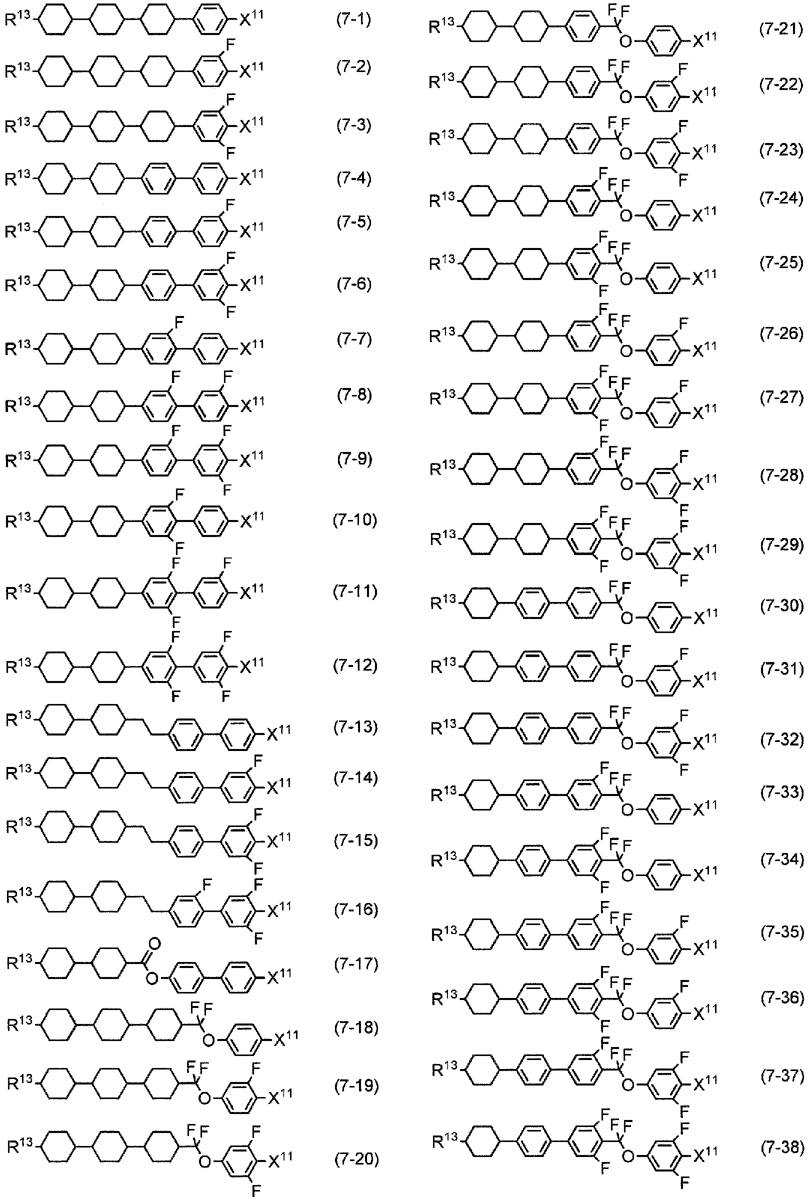

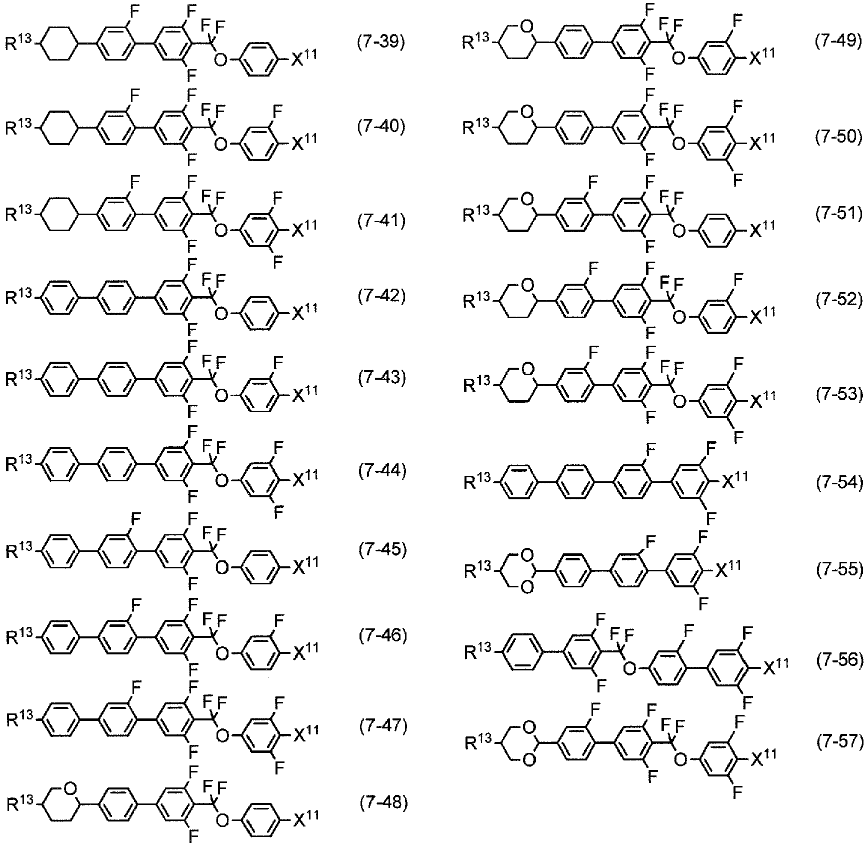

Component C has the positive dielectric anisotropy, and superb stability to heat, light and so forth, and therefore is used when a composition for the IPS mode, the FFS mode, the OCB mode or the like is prepared. A content of component C is suitably in the range of 1% by weight to 99% by weight, preferably in the range of 10% by weight to 97% by weight, and further preferably in the range of 40% by weight to 95% by weight, based on the weight of the liquid crystal composition. When component C is added to the composition having the negative dielectric anisotropy, the content of component C is preferably 30% by weight or less based on the weight of the liquid crystal composition. Addition of component C allows adjustment of the elastic constant of the composition and adjustment of a voltage-transmittance curve of the device.

Component D is compound (8) in which a right-terminal group is -C≡N or -C≡C-C≡N. Preferred examples of component D include compounds (8-1) to (8-64). In the compound of component D, R¹⁴ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and -X¹² is -C≡N or -C≡C-C≡N.

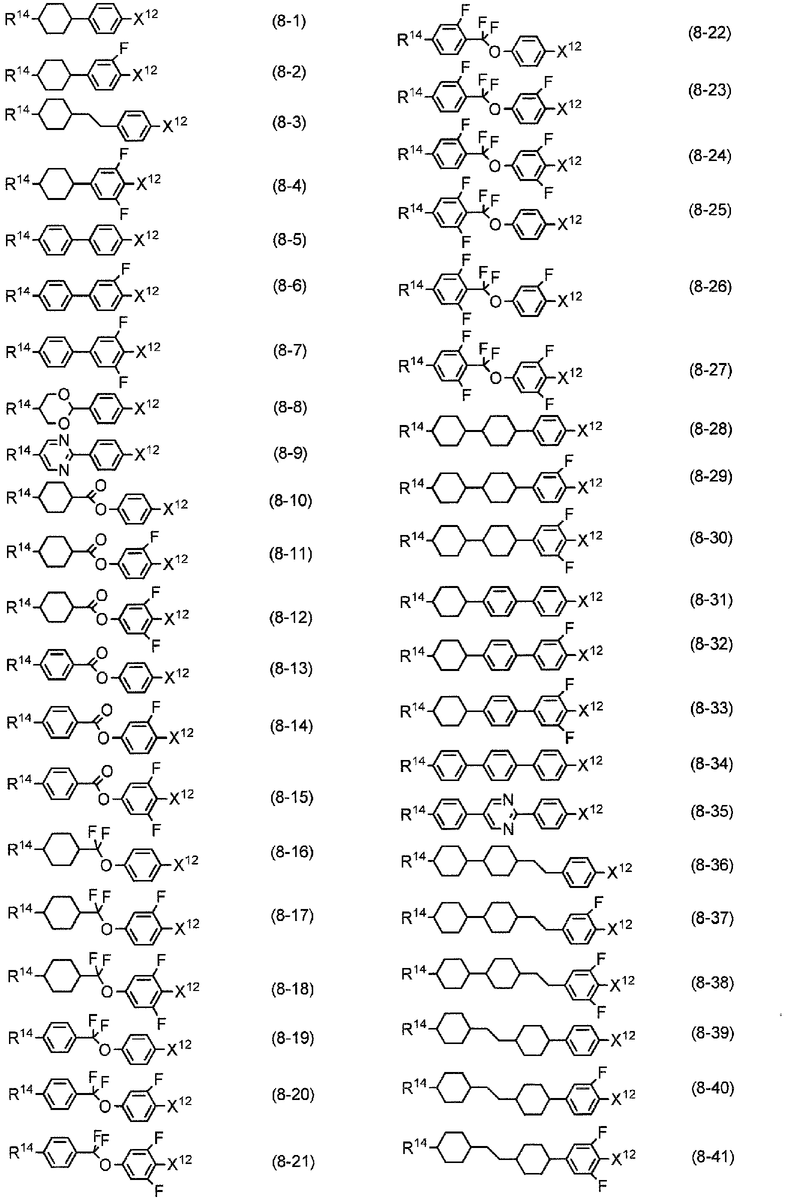

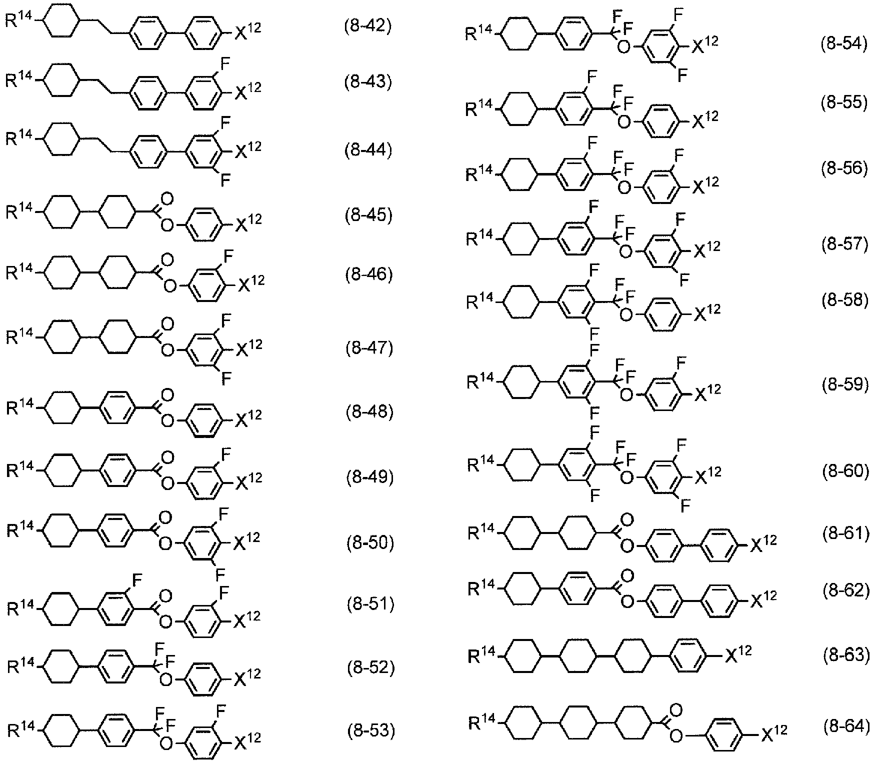

Component D has the positive dielectric anisotropy and a value thereof is large, and therefore is mainly used when the composition for the TN mode or the like is prepared. Addition of component D allows an increase in the dielectric anisotropy of the composition. Component D is effective in extending the temperature range of the liquid crystal phase, adjusting the viscosity or adjusting the optical anisotropy. Component D is also useful for adjustment of the voltage-transmittance curve of the device.

When the composition for the TN mode or the like is prepared, a content of component D is suitably in the range of 1% by weight to 99% by weight, preferably in the range of 10% by weight to 97% by weight, and further preferably in the range of 40% by weight to 95% by weight, based on the weight of the liquid crystal composition. When component D is added to the composition having the negative dielectric anisotropy, the content of component D is preferably 30% by weight or less based on the weight of the liquid crystal composition. Addition of component D allows adjustment of the elastic constant of the composition and adjustment of the voltage-transmittance curve of the device.

Component E includes compounds (9) to (15). The compounds have phenylene in which hydrogen in lateral positions are replaced by two halogens, such as 2,3-difluoro-1,4-phenylene. Preferred examples of component E include compounds (9-1) to (9-8), compounds (10-1) to (10-17), compound (11-1), compounds (12-1) to (12-3), compounds (13-1) to (13-11), compounds (14-1) to (14-3) and compounds (15-1) to (15-3). In the compound of component E, R¹⁵ and R¹⁶ are independently alkyl having 1 to 10 carbons or alkenyl having 2 to 10 carbons, and in the alkyl and the alkenyl, at least piece of -CH₂- may be replaced by -O-, and at least one hydrogen may be replaced by fluorine; and R¹⁷ is hydrogen, fluorine, alkyl having 1 to 10 carbons or alkenyl having 2 to 10 carbons, and in the alkyl and the alkenyl, at least one piece of -CH₂- may be replaced by -O-, and at least one hydrogen may be replaced by fluorine.

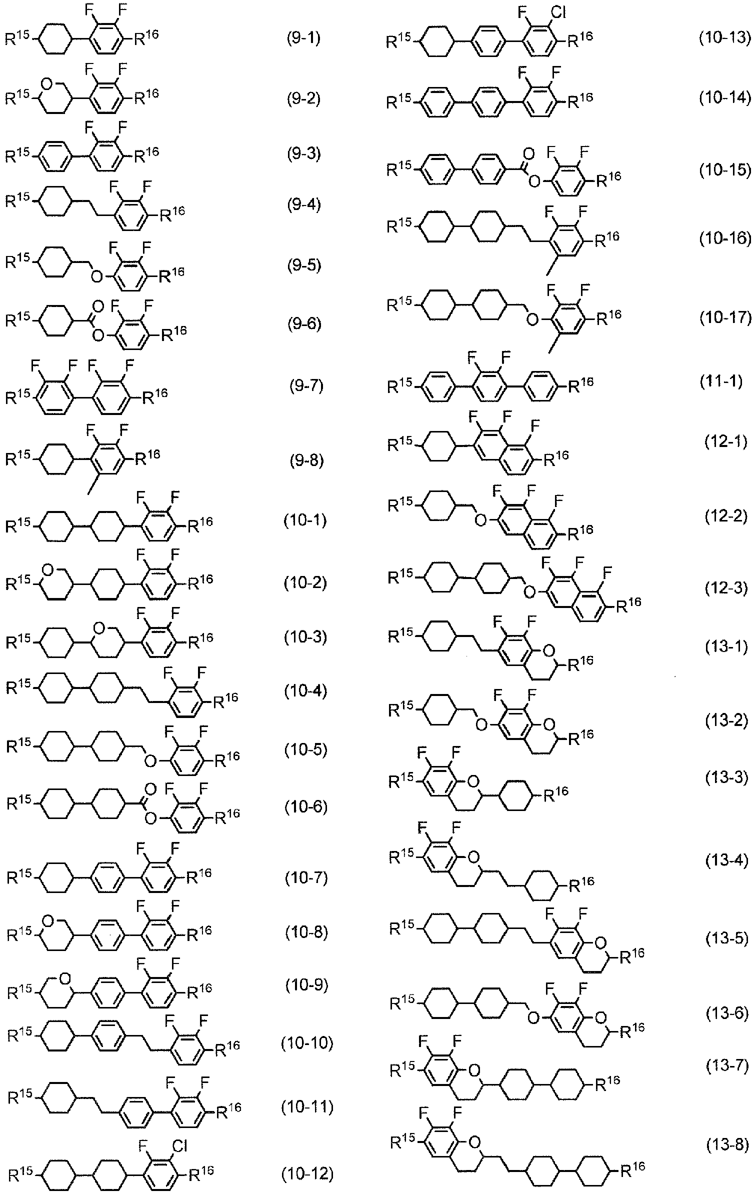

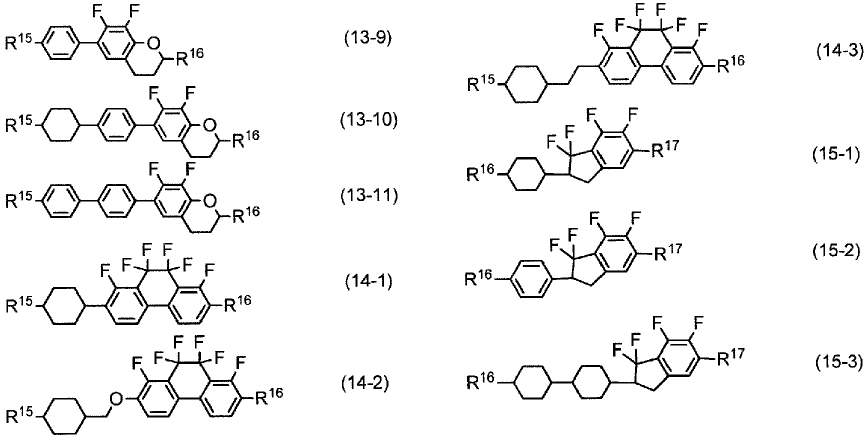

Component E has the negatively large dielectric anisotropy. Component E is used when the composition for the IPS mode, the VA mode, the PSA mode or the like is prepared. As a content of component E is increased, the dielectric anisotropy of the composition is negatively increased, but the viscosity is increased. Thus, as long as a desired value of a threshold voltage of the device is met, the content is preferably as small as possible. When the dielectric anisotropy being about -5 is taken into account, the content is preferably 40% by weight or more based on the weight of the liquid crystal composition for allowing sufficient voltage driving.

Among components E, compound (9) is a bicyclic compound, and therefore is effective mainly in decreasing the viscosity, adjusting the optical anisotropy or increasing the dielectric anisotropy. Compounds (10) and (11) are a tricyclic compound, and therefore are effective in increasing the maximum temperature, increasing the optical anisotropy or increasing the dielectric anisotropy. Compounds (12) to (15) are effective in increasing the dielectric anisotropy.

When the composition for the IPS mode, the VA mode, the PSA mode or the like is prepared, the content of component E is preferably 40% by weight or more, and further preferably in the range of 50% by weight to 95% by weight, based on the weight of the liquid crystal composition. When component E is added to the composition having the positive dielectric anisotropy, the content of component E is preferably 30% by weight or less based on the weight of the liquid crystal composition. Addition of component E allows adjustment of the elastic constant of the composition and adjustment of the voltage-transmittance curve of the device.

The liquid crystal composition satisfying at least one of characteristics such as the high maximum temperature, the low minimum temperature, the small viscosity, the suitable optical anisotropy, the large positive or negative dielectric anisotropy, the large specific resistance, the high stability to ultraviolet light, the high stability to heat and the large elastic constant can be prepared by suitably combining component B, C, D and E described above. A liquid crystal compound different from components B, C, D and E may be added when necessary.

### 3-2. Additive

The liquid crystal composition is prepared according to a publicly known method. For example, the component compounds are mixed and dissolved in each other by heating. According to an application, an additive may be added to the composition. Specific examples of the additives include the polymerizable compound, the polymerization initiator, the polymerization inhibitor, the optically active compound, the antioxidant, the ultraviolet light absorber, the light stabilizer, the heat stabilizer and the antifoaming agent. Such additives are well known to those skilled in the art, and described in literature.

The polymerizable compound is added for the purpose of forming the polymer in the liquid crystal composition. Compound (1) is polymerized by irradiation with ultraviolet light while a voltage is applied between electrodes, and thus the polymer is formed in the liquid crystal composition. On the occasion, compound (1) is fixed while the polar group interacts with the substrate surface of the glass (or metal oxide) in a noncovalent bonding manner. Accordingly, capability to control alignment of liquid crystal molecules is further improved and suitable pretilt is obtained, and therefore a response time is shortened.

Preferred examples of the polymerizable compound include acrylate, methacrylate, a vinyl compound, a vinyloxy compound, propenyl ether, an epoxy compound (oxirane, oxetane) and vinyl ketone. Further preferred examples include a compound having at least one acryloyloxy, and a compound having at least one methacryloyloxy. Still further preferred examples also include a compound having both acryloyloxy and methacryloyloxy.

Compound (1) has the polar group. Meanwhile, compound (16) has no polar group. Preferred examples of compound (16) will be described.

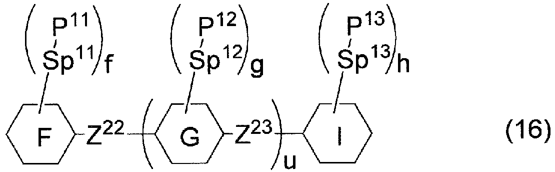

In formula (16), ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring F or ring I is cyclohexyl, cyclohexenyl, phenyl, fluorophenyl, difluorophenyl, 1-naphthyl or 2-naphthyl. Further preferred ring F or ring I is cyclohexyl, cyclohexenyl or phenyl. Particularly preferred ring F or ring I is phenyl.

Ring G is 1,4-cycloxylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring G is 1,4-cycloxylene, 1,4-cyclohexenylene, 1,4-phenylene, 2-fluoro-1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl or naphthalene-2,7-diyl. Further preferred ring G is 1,4-cycloxylene, 1,4-cyclohexenylene, 1,4-phenylene or 2-fluoro-1,4-phenylene. Particularly preferred ring G is 1,4-phenylene or 2-fluoro-1,4-phenylene. Most preferred ring G is 1,4-phenylene.

In formula (16), Z²² and Z²³ are independently a single bond, alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine. Preferred Z⁷ or Z⁸ is a single bond, -CH₂CH₂-, -CH₂O-, -OCH₂-, -COO- or -OCO-. Further preferred Z²² or Z²³ is a single bond.

In compound (16), P¹¹, P¹² and P¹³ are independently a polymerizable group. Preferred P¹¹ to P¹³ are a group selected from the group of polymerizable groups represented by formula (P-1) to formula (P-5). Further preferred P¹¹ to P¹³ are a group represented by formula (P-1) or formula (P-5) . Particularly preferred P¹¹ to P¹³ are a group represented by formula (P-1). The preferred group represented by formula (P-1) is acryloyloxy (-OCO-CH=CH₂) or methacryloiloxy (-OCO-C(CH₃)=CH₂). A wavy line in formulas (P-1) to (P-5) shows a site to be bonded.

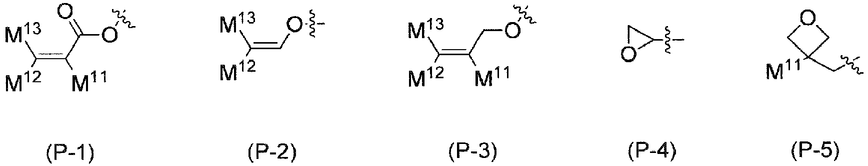

In formula (P-1) to formula (P-5), M¹¹, M¹² and M¹³ are independently hydrogen, fluorine, alkyl having 1 to 5 carbons, or alkyl having 1 to 5 carbons in which at least one hydrogen is replaced by halogen. For increasing the reactivity, preferred M¹¹, M¹² or M¹³ is hydrogen or methyl. Further preferred M¹¹ is hydrogen or methyl, and further preferred M¹² or M¹³ is hydrogen.

In formula (16), Sp¹¹, Sp¹² and Sp¹³ are independently a single bond, alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine. Preferred Sp¹¹, Sp¹² or Sp¹³ is a single bond.

In formula (16), u is 0, 1 or 2. Preferred u is 0 or 1.

Then, f, g and h are independently 0, 1, 2, 3 or 4, and a sum of f, g and h is 1 or more. Preferred f, g or h is 1 or 2. A preferred sum is 2, 3 or 4. A further preferred sum is 2 or 3.

Further preferred examples include compounds (16-1-1) to (16-1-5), compounds (16-2-1) to (16-2-5), compound (16-4-1), compound (16-5-1), compound (16-6-1) and compounds (16-8) to (16-16). In compounds (16-1-1) to (16-1-5), compounds (16-2-1) to (16-2-5), compound (16-4-1), compound (16-5-1), compound (16-6-1) and compounds (16-8) to (16-16), R²⁵ to R³¹ are independently hydrogen or methyl; v and x are independently 0 or 1; t and u are independently an integer of 1 to 10; and L³¹ to L³⁶ are independently hydrogen or fluorine, and L³⁷ and L³⁸ are independently hydrogen, fluorine or methyl.

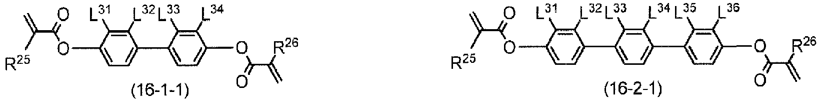

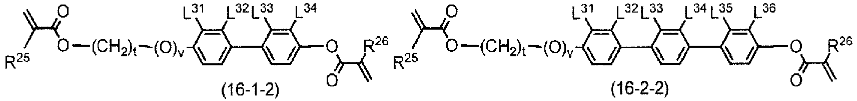

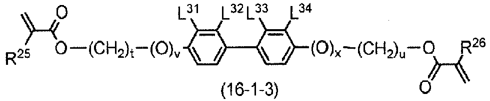

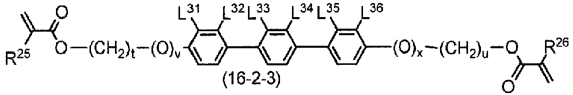

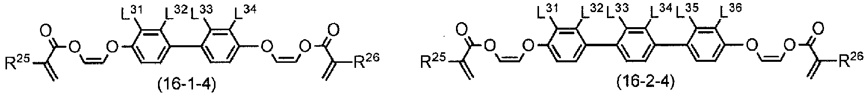

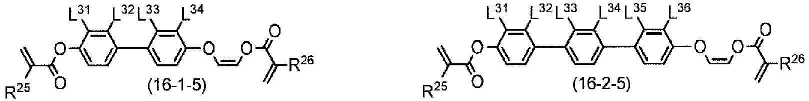

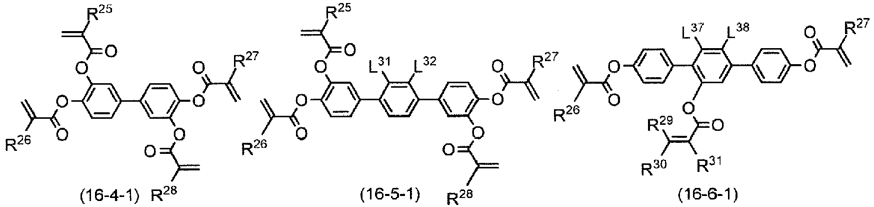

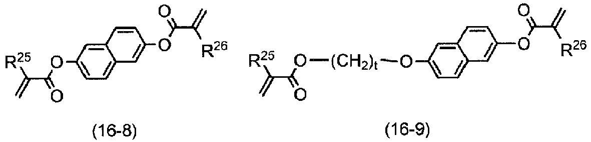

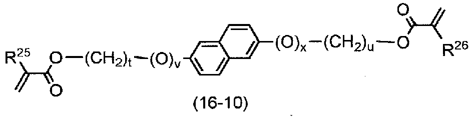

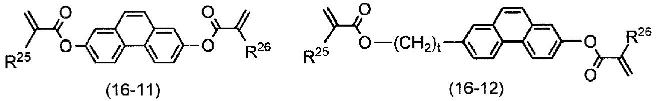

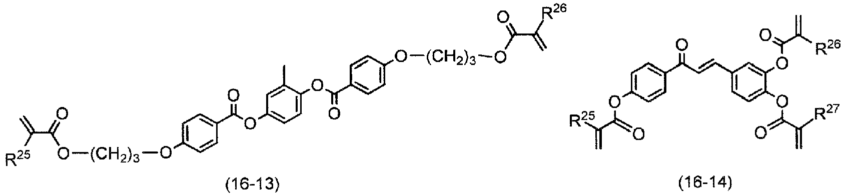

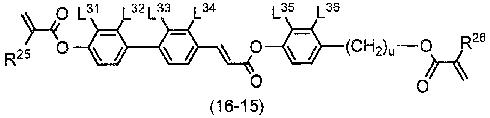

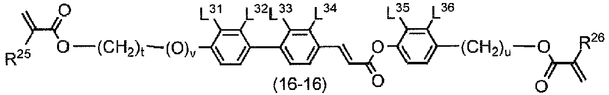

The polymerizable compound can be rapidly polymerized by adding the polymerization initiator. An amount of the remaining polymerizable compound can be decreased by optimizing a reaction temperature. Examples of a photoradical polymerization initiator include TPO, 1173 and 4265 from Darocur series of BASF SE, and 184, 369, 500, 651, 784, 819, 907, 1300, 1700, 1800, 1850 and 2959 from Irgacure series thereof.

Additional examples of the photoradical polymerization initiator include 4-methoxyphenyl-2,4-bis(trichloromethyl)triazine, 2-(4-butoxystyryl)-5-trichloromethyl-1,3,4-oxadiazole, 9-phenylacridine, 9,10-benzphenazine, a benzophenone-Michler's ketone mixture, a hexaarylbiimidazole-mercaptobenzimidazole mixture, 1-(4-isopropylphenyl)-2-hydroxy-2-methylpropane-1-one, benzyl dimethyl ketal, 2-methyl-1-[4-(methylthio)phenyl]-2-morpholinopropane-1-one, a mixture of 2,4-diethylxanthone and methyl p-dimethylaminobenzoate, and a mixture of benzophenone and methyltriethanolamine.

After the photoradical polymerization initiator is added to the liquid crystal composition, polymerization can be performed by irradiation with ultraviolet light while an electric field is applied. However, an unreacted polymerization initiator or a decomposition product of the polymerization initiator may cause poor display such as image persistence in the device. In order to prevent such an event, photopolymerization may be performed with no addition of the polymerization initiator. A preferred wavelength of irradiation light is in the range of 150 nanometers to 500 nanometers . A further preferred wavelength is in the range of 250 nanometers to 450 nanometers, and a most preferred wavelength is in the range of 300 nanometers to 400 nanometers.

Upon storing the polymerizable compound, the polymerization inhibitor may be added thereto for preventing polymerization. The polymerizable compound is ordinarily added to the composition without removing the polymerization inhibitor. Specific examples of the polymerization inhibitor include hydroquinone, a hydroquinone derivative such as methylhydroquinone, 4-t-butylcatechol, 4-methoxyphenol and phenothiazine.

The optically active compound is effective in inducing a helical structure in liquid crystal molecules to give a required twist angle, and thereby preventing a reverse twist. A helical pitch can be adjusted by adding the optically active compound thereto. Two or more optically active compounds may be added for the purpose of adjusting temperature dependence of the helical pitch. Preferred examples of the optically active compound include compounds (Op-1) to (Op-18) described below. In compound (Op-18), ring J is 1, 4-cyclohexylene or 1, 4-phenylene, and R²⁸ is alkyl having 1 to 10 carbons.

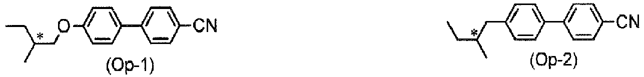

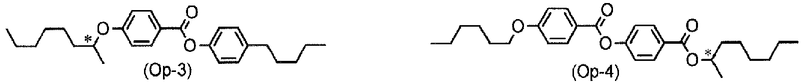

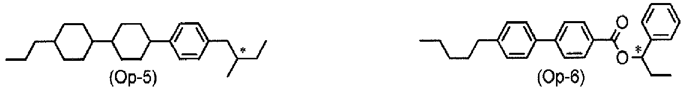

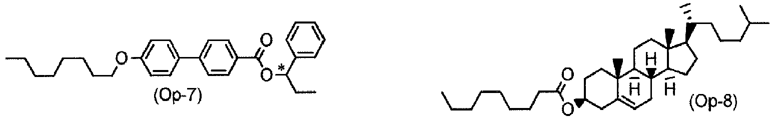

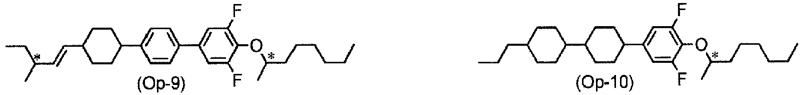

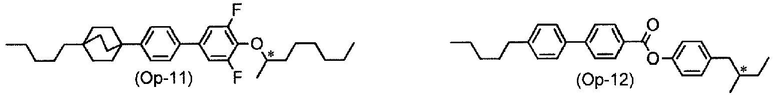

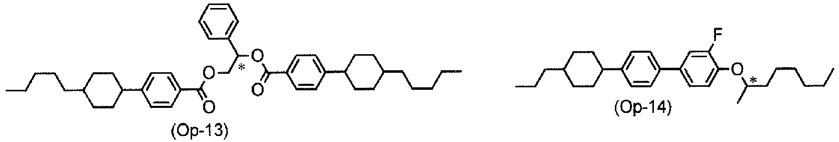

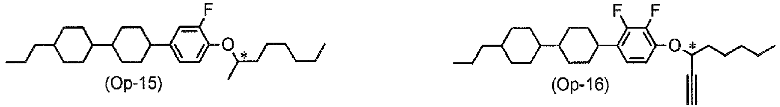

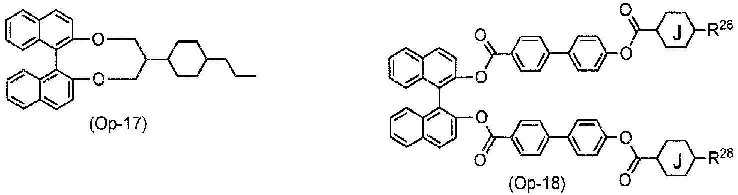

The antioxidant is effective for maintaining the large voltage holding ratio. Preferred examples of the antioxidant include compounds (AO-1) and (AO-2) described below; IRGANOX 415, IRGANOX 565, IRGANOX 1010, IRGANOX 1035, IRGANOX 3114 and IRGANOX 1098 (trade names: BASF SE). The ultraviolet light absorber is effective for preventing a decrease in the maximum temperature. Preferred examples of the ultraviolet light absorber include a benzophenone derivative, a benzoate derivative and a triazole derivative. Specific examples include compounds (AO-3) and (AO-4) described below; TINUVIN 329, TINUVIN P, TINUVIN 326, TINUVIN 234, TINUVIN 213, TINUVIN 400, TINUVIN 328 and TINUVIN 99-2 (trade names: BASF SE); and 1,4-diazabicyclo[2.2.2]octane (DABCO).

The light stabilizer such as an amine having steric hindrance is preferred for maintaining the large voltage holding ratio. Preferred examples of the light stabilizers include compounds (AO-5) and (AO-6) described below; TINUVIN 144, TINUVIN 765 and TINUVIN 770DF (trade names: BASF SE). The heat stabilizer is also effective for maintaining the large voltage holding ratio, and preferred examples include IRGAFOS 168 (trade name: BASF SE). The antifoaming agent is effective for preventing foam formation. Preferred examples of the antifoaming agent include dimethyl silicone oil and methylphenyl silicone oil.

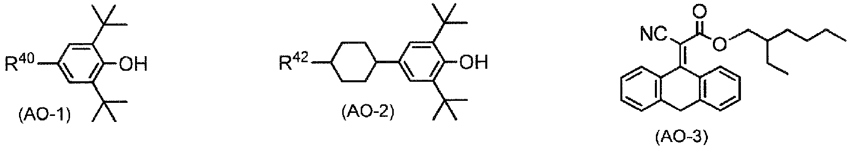

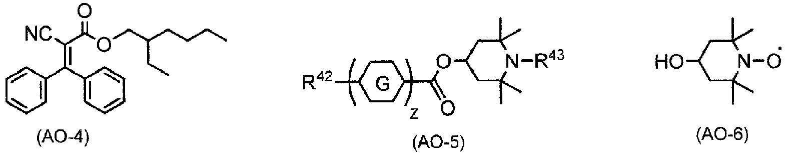

In compound (AO-1), R⁴⁰ is alkyl having 1 to 20 carbons, alkoxy having 1 to 20 carbons, -COOR⁴¹ or -CH₂CH₂COOR⁴¹, and R⁴¹ is alkyl having 1 to 20 carbons. In compounds (AO-2) and (AO-5), R⁴² is alkyl having 1 to 20 carbons. In compound (AO-5), R⁴³ is hydrogen, methyl or O^{·} (oxygen radical), and ring G is 1,4-cycloxylene or 1,4-phenylene, and z is 1, 2 or 3.

### 4. Liquid crystal display device

The liquid crystal composition can be used in a liquid crystal display device having an operating mode such as the PC mode, the TN mode, the STN mode, the OCB mode and the PSA mode, and driven by an active matrix mode. The composition can also be used in a liquid crystal display device having the operating mode such as the PC mode, the TN mode, the STN mode, the OCB mode, the VA mode and the IPS mode, and driven by a passive matrix mode. The devices can be applied to any of a reflective type, a transmissive type and a transflective type.

The composition can be also used for a nematic curvilinear aligned phase (NCAP) device made by microencapsulating nematic liquid crystals, a polymer dispersed liquid crystal display device (PDLCD) made by forming a three-dimensional network polymer in liquid crystals, and a polymer network liquid crystal display device (PNLCD). When an amount of addition of the polymerizable compound is about 10 % by weight or less based on the weight of the liquid crystal composition, the liquid crystal display device having the PSA mode is made. A preferred proportion is in the range of about 0.1% by weight to about 2% by weight. A further preferred proportion is in the range of about 0.2% by weight to about 1.0% by weight. The device having the PSA mode can be driven by a driving mode such as the active matrix mode and the passive matrix mode. Such devices can be applied to any of the reflective type, the transmissive type and the transflective type. A polymer dispersed mode device can be also made by increasing the amount of addition of the polymerizable compound.

In the polymer sustained alignment mode device, the polymer contained in the composition aligns liquid crystal molecules. The polar compound supports alignment of the liquid crystal molecules. More specifically, the polymer compound can be used in place of an alignment film. One example of a method of producing such a device is as described below. A device having two substrates referred to as an array substrate and a color filter substrate is prepared. The substrates have no alignment film. At least one of the substrates has an electrode layer. The liquid crystal compounds are mixed to prepare the liquid crystal composition. The polymerizable compound and the polar compound are added to the composition. The additive may be further added thereto when necessary. The composition is injected into the device. The device is irradiated with light while a voltage is applied to the device. Ultraviolet light is preferred. The polymerizable compound is polymerized by light irradiation. The composition containing the polymer is formed by the polymerization, and the device having the PSA mode is made.

In the above procedure, the polar compounds are arranged because the polar group interacts with the substrate surface. The polar compound aligns the liquid crystal molecules. When a plurality of polar groups exist, the interaction with the substrate surface is further strengthened to allow alignment at a low concentration. When a voltage is applied thereto, the alignment of the liquid crystal molecules is further promoted by an effect of an electric field. The polymerizable compounds are also aligned according to the alignment. The polymerizable compound is polymerized by the ultraviolet light in the above state, and therefore the polymer maintained in the alignment is formed. The alignment of the liquid crystal molecules is additionally stabilized by an effect of the polymer, and therefore the response time of the device is shortened. Simultaneously, image persistence is also improved by the effect of the polymer because the image persistence is operation failure of the liquid crystal molecules. Compound (1) is polymerizable, and therefore is consumed by polymerization. Compound (1) is also consumed by copolymerization with any other polymerizable compound. Accordingly, compound (1) has the polar group, but is consumed, and therefore the liquid crystal display device having the large voltage holding ratio is obtained.

### Examples

The invention will be described in greater detail by way of Examples (including Synthesis Examples and Use Examples). However, the invention is not limited by the Examples. The invention includes a mixture of a composition in Use Example 1 and a composition in Use Example 2. The invention also includes a mixture prepared by mixing at least two of the compositions in the Use Examples.

### 1. Example of compound (1)

Unless otherwise described, a reaction was carried out under a nitrogen atmosphere. Compound (1) was prepared by a procedure shown in Example 1 or the like. The thus prepared compound was identified by methods such as an NMR analysis. Characteristics of compound (1), a liquid crystal compound, a composition and a device were measured according to methods described below.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sex and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber was set to 300°C, and a temperature of a detector (FID) was set to 300°C. A sample was dissolved in acetone and prepared to be a 1 weight % solution, and then 1 microliter of the solution obtained was injected into the sample vaporizing chamber. As a recorder, GC Solution System made by Shimadzu Corporation or the like was used.

HPLC analysis: For measurement, Prominence (LC-20AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set to 254 nanometers. A sample was dissolved in acetonitrile and prepared to be a 0.1 weight % solution, and then 1 microliter of the solution was introduced into a sample chamber. As a recorder, C-R7Aplus made by Shimadzu Corporation was used.

Ultraviolet-visible spectrophotometry: For measurement, PharmaSpec UV-1700 made by Shimadzu Corporation was used. A detection wavelength was adjusted in the range of 190 nanometers to 700 nanometers. A sample was dissolved in acetonitrile and prepared to be a 0.01 mmol/L solution, and measurement was carried out by putting the solution in a quartz cell (optical path length: 1 cm).

Sample for measurement: Upon measuring phase structure and a transition temperature (a clearing point, a melting point, a polymerization starting temperature or the like), the compound itself was used as a sample.

Measuring method: Characteristics were measured according to the methods described below. Most of the methods are described in the Standard of Japan Electronics and Information Technology Industries Association (hereinafter, abbreviated as JEITA) discussed and established in JEITA (JEITA ED-2521B), or modified thereon. No thin film transistor (TFT) was attached to a TN device used for measurement.

### (1) Phase structure

A sample was placed on a hot plate of a melting point apparatus (FP-52 Hot Stage made by Mettler-Toledo International Inc.) equipped with a polarizing microscope. A state of phase and a change thereof were observed with the polarizing microscope while the sample was heated at a rate of 3°C per minute, and a kind of the phase was specified.

### (2) Transition temperature (°C)

For measurement, a scanning calorimeter, Diamond DSC System, made by PerkinElmer, Inc., or a high sensitivity differential scanning calorimeter, X-DSC7000, made by SSI NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. A melting point and a polymerization starting temperature of a compound were also measured using the apparatus. Temperature at which a compound undergoes transition from a solid to a liquid crystal phase such as a smectic phase and a nematic phase may be occasionally abbreviated as "minimum temperature of the liquid crystal phase." Temperature at which the compound undergoes transition from the liquid crystal phase to liquid may be occasionally abbreviated as "clearing point."

A crystal was expressed as C. When kinds of the crystals were distinguishable, each of the crystals was expressed as C₁ or C₂. The smectic phase or the nematic phase was expressed as S or N. When a smectic A phase, a smectic B phase, a smectic C phase or a smectic F phase was distinguishable among the smectic phases, the phases were expressed as S_{A}, S_{B}, S_{C} or S_{F}, respectively. A liquid (isotropic) was expressed as I. A transition temperature was expressed as "C 50.0 N 100.0 I," for example. The expression indicates that a transition temperature from the crystals to the nematic phase is 50.0°C, and a transition temperature from the nematic phase to the liquid is 100.0°C.

### (3) Maximum temperature of nematic phase (T_{NI} or NI; °C)

A sample was placed on a hot plate in a melting point apparatus equipped with a polarizing microscope, and heated at a rate of 1°C per minute. Temperature when part of the sample changed from a nematic phase to an isotropic liquid was measured. A maximum temperature of the nematic phase may be occasionally abbreviated as "maximum temperature." When the sample was a mixture of compound (1) and the base liquid crystal, the maximum temperature was expressed in terms of a symbol T_{NI}. When the sample was a mixture of compound (1) and a compound such as component B, compound C and compound D, the maximum temperature was expressed using a symbol NI.

### (4) Minimum temperature of a nematic phase (T_{C}; °C)

Samples each having a nematic phase were put in glass vials and kept in freezers at temperatures of 0°C, -10°C, -20°C, -30°C and -40°C for 10 days, and then liquid crystal phases were observed. For example, when the sample was maintained in the nematic phase at -20°C and changed to crystals or a smectic phase at -30°C, T_{C} was expressed as T_{C} ≤ -20°C. A minimum temperature of the nematic phase may be occasionally abbreviated as "minimum temperature."

### (5) Viscosity (bulk viscosity; η; measured at 20°C; mPa·s)

For measurement, a cone-plate (E type) rotational viscometer made by Tokyo Keiki Inc. was used.

### (6) Optical anisotropy (refractive index anisotropy; Δn; measured at 25°C)

Measurement was carried out by an Abbe refractometer with a polarizing plate mounted on an ocular, using light at a wavelength of 589 nanometers. A surface of a main prism was rubbed in one direction, and then a sample was added dropwise onto the main prism. A refractive index (n∥) was measured when a direction of polarized light was parallel to a direction of rubbing. A refractive index (n⊥) was measured when the direction of polarized light was perpendicular to the direction of rubbing. A value of optical anisotropy was calculated from an equation: Δn = n∥ - n⊥.

### (7) Specific resistance (ρ measured at 25°C; Qcm)

Into a vessel equipped with electrodes, 1.0 milliliter of a sample was injected. A direct current voltage (10 V) was applied to the vessel, and a direct current after 10 seconds was measured. Specific resistance was calculated from the following equation: (specific resistance) = { (voltage) × (electric capacity of a vessel) } / { (direct current) × (dielectric constant of vacuum)}.

The measuring method of the characteristics may be different between a sample having positive dielectric anisotropy and a sample having negative dielectric anisotropy. When the dielectric anisotropy was positive, the measuring method was described in measurement (8a) to measurement (12a). When the dielectric anisotropy was negative, the measuring method was described in measurement (8b) to measurement (12b) .

### (8a) Viscosity (rotational viscosity; γ1; measured at 25°C; mPa·s)

Positive dielectric anisotropy: Measurement was carried out according to a method described in M. Imai et al., Molecular Crystals and Liquid Crystals, Vol. 259, p. 37 (1995). A sample was put in a TN device in which a twist angle was 0 degrees and a distance (cell gap) between two glass substrates was 5 micrometers. A voltage was applied stepwise to the device in the range of 16 V to 19.5 V at an increment of 0.5 V. After a period of 0.2 second with no voltage application, a voltage was repeatedly applied under conditions of only one rectangular wave (rectangular pulse; 0.2 second) and no voltage application (2 seconds) . A peak current and a peak time of transient current generated by the applied voltage were measured. A value of rotational viscosity was obtained from the measured values and calculation equation (8) described on page 40 of the paper presented by M. Imai et al. A value of dielectric anisotropy required for the calculation was determined using the device by which the rotational viscosity was measured and by a method described below.

### (8b) Viscosity (rotational viscosity; γ1; measured at 25°C; mPa·s)

Negative dielectric anisotropy: Measurement was carried out according to a method described in M. Imai et al., Molecular Crystals and Liquid Crystals, Vol. 259, p. 37 (1995). A sample was put in a VA device in which a distance (cell gap) between two glass substrates was 20 micrometers. A voltage was applied stepwise to the device in the range of 39 V to 50 V at an increment of 1 V. After a period of 0.2 second with no voltage application, a voltage was repeatedly applied under conditions of only one rectangular wave (rectangular pulse; 0.2 second) and no voltage application (2 seconds). A peak current and a peak time of transient current generated by the applied voltage were measured. A value of rotational viscosity was obtained from the measured values and calculation equation (8) described on page 40 of the paper presented by M. Imai et al. As dielectric anisotropy required for the calculation, a value measured in a section of dielectric anisotropy described below was used.

### (9a) Dielectric anisotropy (Δε; measured at 25°C)

Positive dielectric anisotropy: A sample was put in a TN device in which a distance (cell gap) between two glass substrates was 9 micrometers and a twist angle was 80 degrees. Sine waves (10 V, 1 kHz) were applied to the device, and after 2 seconds, a dielectric constant (ε∥) of liquid crystal molecules in a major axis direction was measured. Sine waves (0.5 V, 1 kHz) were applied to the device, and after 2 seconds, a dielectric constant (ε⊥) of liquid crystal molecules in a minor axis direction was measured. A value of dielectric anisotropy was calculated from an equation: Δε = ε∥ - ε⊥.

### (9b) Dielectric anisotropy (Δε; measured at 25°C)

Negative dielectric anisotropy: A value of dielectric anisotropy was calculated from an equation: Δε = ε∥ - ε⊥. A dielectric constant (ε∥ and ε⊥) was measured as described below.
(1) Measurement of dielectric constant (ε∥): An ethanol (20 mL) solution of octadecyltriethoxysilane (0.16 mL) was applied to a well-cleaned glass substrate. After rotating the glass substrate with a spinner, the glass substrate was heated at 150°C for 1 hour. A sample was put in a VA device in which a distance (cell gap) between two glass substrates was 4 micrometers, and the device was sealed with an ultraviolet-curable adhesive. Sine waves (0.5 V, 1 kHz) were applied to the device, and after 2 seconds, a dielectric constant (ε∥) of liquid crystal molecules in a major axis direction was measured.
(2) Measurement of dielectric constant (ε⊥): A polyimide solution was applied to a well-cleaned glass substrate. After calcining the glass substrate, rubbing treatment was applied to the alignment film obtained. A sample was put in a TN device in which a distance (cell gap) between two glass substrates was 9 micrometers and a twist angle was 80 degrees. Sine waves (0.5 V, 1 kHz) were applied to the device, and after 2 seconds, a dielectric constant (ε⊥) of liquid crystal molecules in a minor axis direction was measured.

### (10a) Elastic constant (K; measured at 25°C; pN)

Positive dielectric anisotropy: For measurement, HP4284A LCR Meter made by Yokogawa-Hewlett-Packard Co. was used. A sample was put in a horizontal alignment device in which a distance (cell gap) between two glass substrates was 20 micrometers. An electric charge of 0 V to 20 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook" (Ekisho Debaisu Handobukku in Japanese; The Nikkan Kogyo Shimbun, Ltd.) and values of K₁₁ and K₃₃ were obtained from equation (2.99). Next, K₂₂ was calculated using the previously determined values of K₁₁ and K₃₃ in equation (3.18) on page 171. Elastic constant K was expressed in terms of a mean value of the thus determined K₁₁, K₂₂ and K₃₃.

### (10b) Elastic constant (K₁₁ and K₃₃; measured at 25°C; pN)

Negative dielectric anisotropy: For measurement, Elastic Constant Measurement System Model EC-1 made by TOYO Corporation was used. A sample was put in a vertical alignment device in which a distance (cell gap) between two glass substrates was 20 micrometers. An electric charge of 20 V to 0 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook (Ekisho Debaisu Handobukku in Japanese; Nikkan Kogyo Shimbun, Ltd.)," and values of elastic constant were obtained from equation (2.100).

### (11a) Threshold voltage (Vth; measured at 25°C; V)

Positive dielectric anisotropy: For measurement, an LCD-5100 luminance meter made by Otsuka Electronics Co., Ltd. was used. A light source was a halogen lamp. A sample was put in a normally white mode TN device in which a distance (cell gap) between two glass substrates was 0.45/Δn (µm) and a twist angle was 80 degrees. A voltage (32 Hz, rectangular waves) to be applied to the device was stepwise increased from 0 V to 10 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of a voltage at 90% transmittance.

### (11b) Threshold voltage (Vth; measured at 25°C; V)

Negative dielectric anisotropy: For measurement, an LCD-5100 luminance meter made by Otsuka Electronics Co., Ltd. was used. A light source was a halogen lamp. A sample was put in a normally black mode VA device in which a distance (cell gap) between two glass substrates was 4 micrometers and a rubbing direction was anti-parallel, and the device was sealed with an ultraviolet-curable adhesive. A voltage (60 Hz, rectangular waves) to be applied to the device was stepwise increased from 0 V to 20 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of a voltage at 10% transmittance.

### (12a) Response time (τ; measured at 25°C; ms)

Positive dielectric anisotropy: For measurement, an LCD-5100 luminance meter made by Otsuka Electronics Co., Ltd. was used. A light source was a halogen lamp. A low-pass filter was set to 5 kHz. A sample was put in a normally white mode TN device in which a distance (cell gap) between two glass substrates was 5.0 micrometers and a twist angle was 80 degrees. A voltage (rectangular waves; 60 Hz, 5 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A rise time (τr; millisecond) was expressed in terms of time required for a change from 90% transmittance to 10% transmittance. A fall time (τf; millisecond) was expressed in terms of time required for a change from 10% transmittance to 90% transmittance. A response time was expressed by a sum of the rise time and the fall time thus determined.

### (12b) Response time (τ; measured at 25°C; ms)

Negative dielectric anisotropy: For measurement, an LCD-5100 luminance meter made by Otsuka Electronics Co., Ltd. was used. A light source was a halogen lamp. A low-pass filter was set to 5 kHz. A sample was put in a normally black mode PVA device in which a distance (cell gap) between two glass substrates was 3.2 micrometers, and a rubbing direction was anti-parallel. The device was sealed with an ultraviolet-curable adhesive. The device was applied with a voltage of a little exceeding a threshold voltage for 1 minute, and then was irradiated with ultraviolet light of 23.5 mW/cm² for 8 minutes, while applying a voltage of 5.6 V. A voltage (rectangular waves; 60 Hz, 10 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A response time was expressed in terms of time required for a change from 90% transmittance to 10% transmittance (fall time; millisecond).

### (13) Voltage holding ratio

A polymerizable compound was polymerized by irradiation with ultraviolet light by using Black Light F40T10/BL (a peak wavelength of 369 nm) made by EYE GRAPHICS CO., LTD. The device was charged by applying a pulse voltage (60 microseconds at 1 V) at 60°C. A decaying voltage was measured for 16.7 milliseconds with a high-speed voltmeter, and area A between a voltage curve and a horizontal axis in a unit cycle was determined. Area B is an area without decay. A voltage holding ratio is expressed in terms of a percentage of area A to area B.

### Raw material

SolmixA-11 (registered trademark) is a mixture of ethanol (85.5%), methanol (13.4%) and isopropanol (1.1%), and was purchased from Japan Alcohol Trading Co., Ltd.

### Synthesis Example 1

### Synthesis of compound (1-6-1)

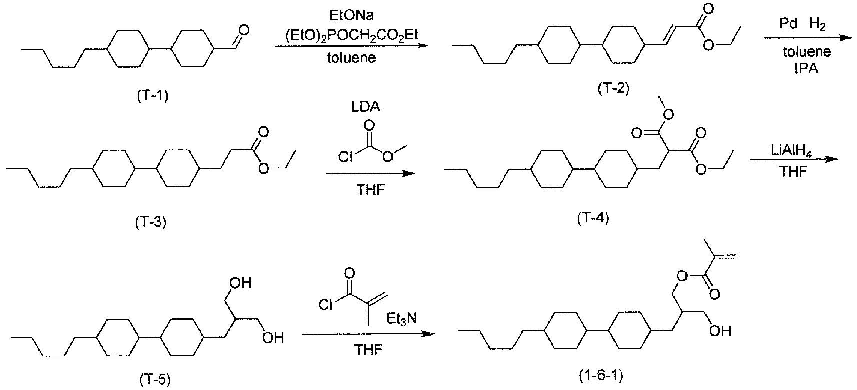

### First step

Compound (T-1) (40.0 g), triethyl phosphonoacetate (40.7 g) and toluene (800 mL) were put in a reaction vessel, and the resulting mixture was cooled to 0°C. Sodium ethoxide (20% ethanol solution) (61.8 g) was slowly added dropwise thereto, and the resulting mixture was stirred for 12 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio) to obtain compound (T-2) (42.0 g; 83%).

### Second step

Compound (T-2) (42.0 g), toluene (400 mL) and isopropanol (400 mL) were put in a reaction vessel, and Pd/C (0.7 g) was added thereto, and the resulting mixture was stirred under a hydrogen atmosphere at room temperature for 24 hours. The reaction mixture was poured into water, and an aqueous layer was subjected to extraction with toluene. Organic layers combined were washed with brine, and dried over anhydrous magnesium sulfate. The resulting solution was concentrated under reduced pressure, and the residue was purified by silica gel chromatography (toluene : heptane = 4 : 1 in a volume ratio) to obtain compound (T-3) (40.1 g; 95%).

### Third step

Compound (T-3) (40.1 g) and THF (400 mL) were put in a reaction vessel, and the resulting mixture was cooled to -60°C. Lithium diisopropylamide (LDA) (1.13 M; a THF solution; 142 mL) was slowly added dropwise thereto, and the resulting mixture was stirred for 1 hour. Methyl chloroformate (11.0 mL) was slowly added dropwise thereto, and the resulting mixture was stirred for 5 hours while returning to room temperature. After an insoluble matter was filtered off, the reaction mixture was poured into water and the aqueous layer was extracted with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio) to obtain compound (T-4) (30.5 g; 65%).

### Fourth step

Lithium aluminum hydride (1.7 g) and THF (300 mL) were put in a reaction vessel, and the resulting mixture was cooled with ice. A THF (600 mL) solution of compound (T-4) (30.5 g) was slowly added thereto, and the resulting mixture was stirred for 3 hours while returning to room temperature. The reaction mixture was poured into water, and an aqueous layer was subjected to extraction with ethyl acetate. Organic layers combined were washed with brine, and dried over anhydrous magnesium sulfate. The resulting solution was concentrated under reduced pressure, and the residue was purified by silica gel chromatography (toluene : ethyl acetate =1 : 1 in a volume ratio). The resulting solution was further purified by recrystallization from heptane to obtain compound (T-5) (20.1 g; 80%).

### Fifth step

Compound (T-5) (20.1 g), triethylamine (10.3 mL) and THF (200 mL) were put in a reaction vessel, and the resulting mixture was cooled to 0°C. Methacryloyl chloride (6.0 mL) was slowly added dropwise thereto, and the resulting mixture was stirred for 4 hours while returning to room temperature. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 9 : 1 in a volume ratio) to obtain compound (1-6-1) (7.7 g; 32%).

An NMR analysis value of compound (1-6-1) obtained is as described below. ¹H-NMR: Chemical shifts δ (ppm; CDCl₃) : 6.11 (s, 1H), 5.58 (s, 1H), 4.29 - 4.26 (m, 1H), 4.14 - 4.11 (m, 1H), 3.60 - 3.57 (m, 1H), 3.50 - 3.47 (m, 1H), 1.98 - 1.95 (m, 5H), 1.78 - 1.67 (m, 8H), 1.32 - 1.11 (m, 12H), 0.99 - 0.81 (m, 13H).

Physical properties of compound (1-6-1) were as described below. Transition temperature: C 65.0 I.

### Synthesis Example 2

### Synthesis of compound (1-2-1)

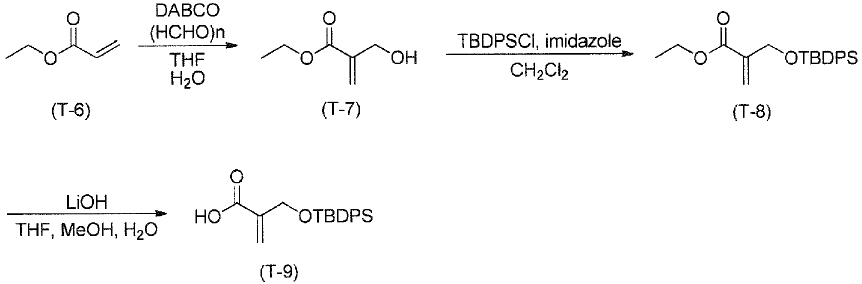

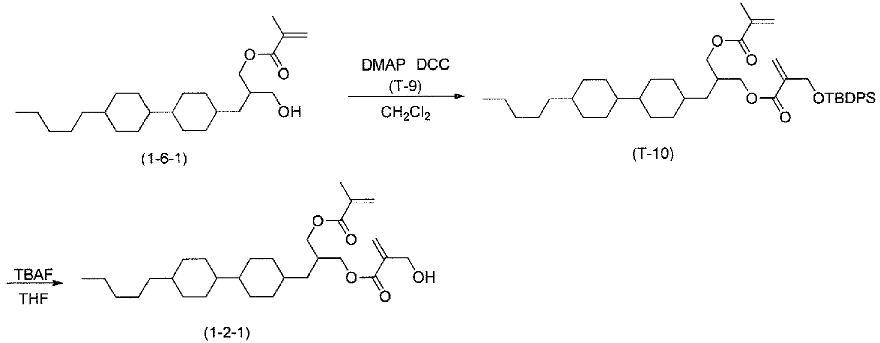

### First step

Paraformaldehyde (30.0 g), DABCO (56.0 g) and water (600 mL) were put in a reaction vessel, and the resulting mixture was stirred for 15 minutes at room temperature. A THF (1200 mL) solution of compound (T-6) (50.0 g) was added dropwise thereto, and the resulting mixture was stirred at room temperature for 72 hours. The reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 4 : 1 in a volume ratio) to obtain compound (T-7) (43.2 g; 65%).

### Second step

Compound (T-7) (42.2 g) was used as a raw material, imidazole (26.3 g) and dichloromethane (800 mL) were put in a reaction vessel, and the resulting mixture was cooled to 0°C. A dichloromethane (100 mL) solution of t-butyldiphenylchlorosilane (106.4 g) was slowly added dropwise thereto, and the resulting mixture was stirred for 12 hours while returning to room temperature. The reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (heptane : ethyl acetate = 10 : 1 in a volume ratio) to obtain compound (T-8) (107.0 g; 90%).

### Third step

Compound (T-8) (107.0 g), THF (800 mL), methanol (200 mL) and water (100 mL) were put in a reaction vessel, and the resulting mixture was cooled to 0°C. Lithium hydroxide monohydrate (24.3 g) was added thereto, and the resulting mixture was stirred for 12 hours while returning to room temperature. The reaction mixture was poured into water, and after 6 N hydrochloric acid (100 mL) was slowly added thereto to acidify the resulting mixture, an aqueous layer was subjected to extraction with ethyl acetate. Organic layers combined were washed with water, and dried over anhydrous magnesium sulfate. The resulting solution was concentrated under reduced pressure, and the resulting solution was purified by recrystallization from heptane to obtain compound (T-9) (47.4 g; 48%).

### Fourth step

Compound (1-6-1) (7.7 g), compound (T-9) (8.0 g), DMAP (1.0 g) and dichloromethane (200 mL) were put in a reaction vessel, and the resulting mixture was cooled to 0°C. A dichloromethane (60 mL) solution of DCC (4.8 g) was slowly added dropwise thereto, and the resulting mixture was stirred for 12 hours while returning to room temperature. After an insoluble matter was filtered off, the reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (heptane : ethyl acetate = 19 : 1 in a volume ratio) to obtain compound (T-10) (9.8 g; 70%).

### Fifth step

Compound (T-10) (9.8 g) and THF (100 mL) were put in a reaction vessel, and the resulting mixture was cooled to 0°C. TBAF (1.00 M; a THF solution; 16.5 mL) was slowly added thereto, and the resulting mixture was stirred for 1 hour while returning to room temperature. The reaction mixture was poured into water, and an aqueous layer was subjected to extraction with ethyl acetate. Organic layers combined were washed with brine, and dried over anhydrous magnesium sulfate. The resulting solution was concentrated under reduced pressure, and the residue was purified by silica gel chromatography (toluene : ethyl acetate =9 : 1 in a volume ratio) . The resulting solution was further purified by recrystallization from heptane to obtain compound (1-2-1) (3.1 g; 47%).

An NMR analysis value of compound (1-2-1) obtained is as described below.
¹H-NMR: Chemical shifts δ (ppm; CDCl₃) : 6.25 (s, 1H), 6.10 (s, 1H), 5.85 (s, 1H), 5.57 (s, 1H), 4.33 (d, J = 4.5 Hz, 2H), 4.27 - 4.16 (m, 2H), 4.13 - 4.08 (m, 2H), 2.31 (s, 1H), 2.26 - 2.22 (m, 1H), 1.94 (s, 3H), 1.81 - 1.61 (m, 8H), 1.32 - 1.08 (m, 12H), 1.00 - 0.79 (m, 13H).

Physical properties of compound (1-2-1) were as described below. Transition temperature: C 49.6 I.

### Synthesis Example 3

### Synthesis of compound (1-2-2)

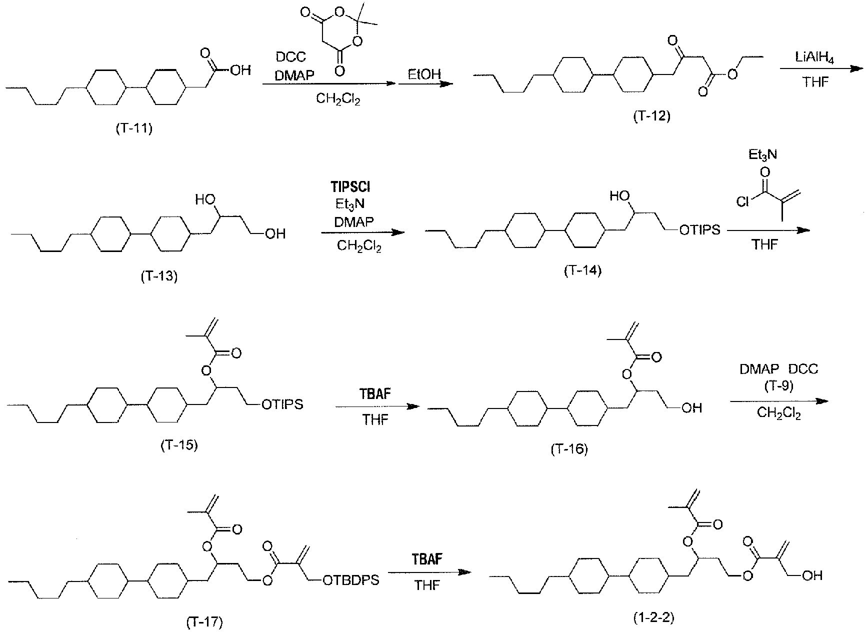

### First step

Compound (T-11) (15.0 g), DMAP (9.33 g), Meldrum's acid (9.54 g), and dichloromethane (250 mL) were put in a reaction vessel, and the resulting mixture was cooled to 0°C. DCC (15.7 g) was slowly added thereto, and the resulting mixture was stirred for 12 hours while returning to room temperature. After an insoluble matter was filtered off, the reaction mixture was poured into water, and an aqueous layer was subjected to extraction with dichloromethane. Organic layers combined were washed with water, and dried over anhydrous magnesium sulfate. The resulting solution was concentrated under reduced pressure. The residue and ethanol (250 mL) were put in a reaction vessel, and the resulting mixture was stirred at 70°C. After an insoluble matter was filtered off, the reaction mixture was poured into brine, and an aqueous layer was subjected to extraction with ethyl acetate. Organic layers combined were dried over anhydrous magnesium sulfate. The resulting solution was concentrated under reduced pressure, and the residue was purified by silica gel chromatography (heptane : toluene = 1 : 1 in a volume ratio) to obtain compound (T-12) (10.2 g; 55%).

### Second step

Lithium aluminum hydride (0.6 g) and THF (100 mL) were put in a reaction vessel, and the resulting mixture was cooled with ice. A THF (100 mL) solution of compound (T-12) (10.2 g) was slowly added thereto, and the resulting mixture was stirred for 3 hours while returning to room temperature. After an insoluble matter was filtered off, the reaction mixture was poured into water, and an aqueous layer was subjected to extraction with ethyl acetate. Organic layers combined were washed with brine, and dried over anhydrous magnesium sulfate. The resulting solution was concentrated under reduced pressure, and the residue was purified by silica gel chromatography (toluene : ethyl acetate = 1 : 1 in a volume ratio) to obtain compound (T-13) (7.35 g; 81%).

### Third step

Compound (T-13) (7.35 g), triethylamine (3.75 mL), N,N-dimethyl-4-aminopyridine (DMAP) (0.27 g) and dichloromethane (200 mL) were put in a reaction vessel, and the resulting mixture was cooled to 0°C. Triisopropylsilyl chloride (TIPSCL) (5.05 mL) was slowly added dropwise thereto, and the resulting mixture was stirred for 24 hours while returning to room temperature. After an insoluble matter was filtered off, the reaction mixture was poured into water, and an aqueous layer was subjected to extraction with ethyl acetate. Organic layers combined were washed with brine, and dried over anhydrous magnesium sulfate. The resulting solution was concentrated under reduced pressure, and the residue was purified by silica gel chromatography (toluene : ethyl acetate = 19 : 1 in a volume ratio) to obtain compound (T-14) (6.50 g; 60%).

### Fourth step

Compound (T-14) (6.50 g), triethylamine (3.77 mL) and THF (200 mL) were put in a reaction vessel, and the resulting mixture was cooled to 0°C. Methacryloyl chloride (2.00 mL) was slowly added dropwise thereto, and the resulting mixture was stirred for 4 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 1 in a volume ratio) to obtain compound (T-15) (4.70 g; 63%).

### Fifth step

Compound (T-15) (4.70 g) and THF (100 mL) were put in a reaction vessel, and the resulting mixture was cooled at 0°C. TBAF (1.00 M; a THF solution; 10.3 mL) was slowly added thereto, and the resulting mixture was stirred for 1 hour while returning to room temperature. The reaction mixture was poured into water, and an aqueous layer was subjected to extraction with ethyl acetate. Organic layers combined were washed with brine, and dried over anhydrous magnesium sulfate. The resulting solution was concentrated under reduced pressure, and the residue was purified by silica gel chromatography (toluene : ethyl acetate = 9 : 1 in a volume ratio) to obtain compound (T-16) (1.50 g; 45%).

### Sixth step

Compound (T-17) (1.51 g; 55%) was obtained by using compound (T-16) (1.50 g) as a raw material in a manner similar to the technique in the fourth step in Synthesis Example 2.

### Seventh step

Compound (1-2-2) (0.45 g; 45%) was obtained by using compound (T-17) (1.51 g) as a raw material in a manner similar to the technique in the fifth step in Synthesis Example 2.

An NMR analysis value of compound (1-2-2) obtained is as described below.
¹H-NMR: Chemical shifts δ (ppm; CDCl₃) : 6.25 (s, 1H), 6.09 (s, 1H), 5.82 (d, J = 1.1 Hz, 1H), 5.55 (s, 1H), 5.22 - 5.17 (m, 1H), 4.32 - 4.26 (m, 3H), 4.17 - 4.12 (m, 3H), 2.50 (s, 1H), 2.03 - 1.89 (m, 5H), 1.83 - 1.58 (m, 9H), 1.41 - 1.08 (m, 11H), 0.96 - 0.78 (m, 13H).

Physical properties of compound (1-2-2) were as described below. Transition temperature: C 61.2 I.

### Synthesis Example 4

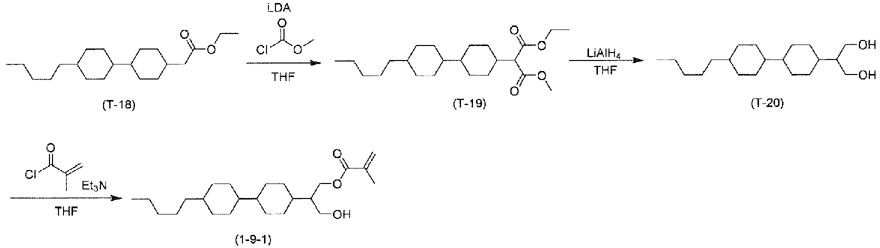

### First step

Compound (T-18) (20.0 g) and THF (200 mL) were put in a reaction vessel, and the resulting mixture was cooled to -70°C, and lithium diisopropylamide (LDA) (1.10 M; a THF solution; 68.0 mL) was slowly added dropwise thereto, and the resulting mixture was stirred for 1 hour. Methyl chloroformate (7.00 g) was slowly added thereto, and the resulting mixture was stirred for 4 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 9 : 1 in a volume ratio) to obtain compound (T-19) (19.4 g; 82%).

### Second step

Lithium aluminum hydride (1.93 g) and THF (200 mL) were put in a reaction vessel, and the resulting mixture was cooled to 0°C. A THF (100 mL) solution of compound (T-19) (19.4 g) was slowly added thereto, and the resulting mixture was stirred for 3 hours while returning to room temperature. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 1 : 1 in a volume ratio) to obtain compound (T-20) (6.0 g; 38%).

### Third step

Compound (T-20) (6.0 g), triethylamine (3.2 mL), and THF (100 mL) were put in a reaction vessel, and the resulting mixture was cooled to 0°C. Methacryloyl chloride (1.8 mL) was slowly added thereto, and the resulting mixture was stirred for 5 hours while returning to room temperature. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 9 : 1 in a volume ratio) to obtain compound (1-9-1) (2.5 g; 34%).

An NMR analysis value of compound (1-9-1) obtained is as described below.
¹H-NMR: Chemical shifts δ (ppm; CDCl₃): 6.10 (s, 1H), 5.57 (d, J = 1.1 Hz, 1H), 4.38 (dd, J = 11.4 Hz, J = 4.3 Hz, 1H), 4.23 (dd, J = 11.3 Hz, J = 6.7 Hz, 1H), 3.71 - 3.68 (m, 1H), 3.63 - 3.60 (m, 1H), 1.97 (s, 1H), 1.94 (s, 3H), 1.82 - 1.62 (m, 9H), 1.41 - 1.18 (m, 7H), 1.14 - 0.79 (m, 16H).

Physical properties of compound (1-9-1) were as described below. Transition temperature: C 68.4 S_{A} 89.3 I.

### Synthesis Example 5

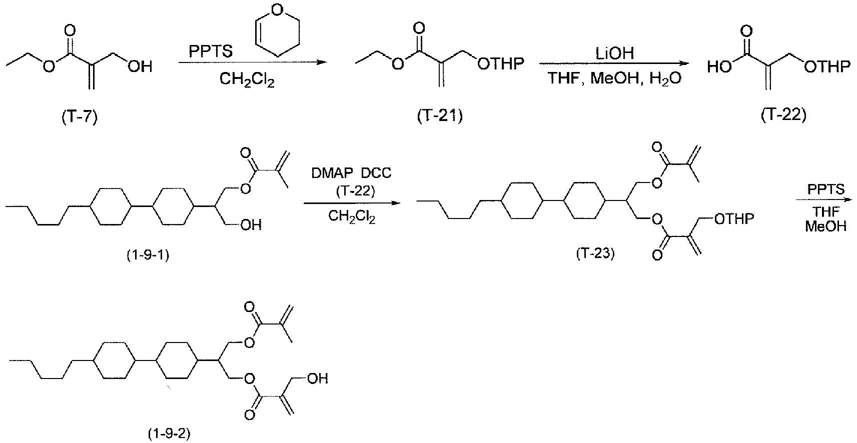

### First step

Compound (T-7), 3,4-dihydro-2H-pyran (23.3 g) and pyridinium p-toluenesulfonate (PPTS) (5.80 g) was put in a reaction vessel, and the resulting mixture was stirred at 50°C for 10 hours. After an insoluble matter was filtered off, the reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (heptane : ethyl acetate = 2 : 1 in a volume ratio) to obtain compound (T-21) (39.5 g; 80%).

### Second step

Compound (T-21) (39.5 g), THF (400 mL) and water (400 mL) were put in a reaction vessel, and the resulting mixture was cooled to 0°C. Lithium hydroxide monohydrate (15.4 g) was added thereto, and the resulting mixture was stirred for 12 hours while returning to room temperature. The reaction mixture was poured into water, and after 6N hydrochloric acid (60 mL) was slowly added thereto to acidify the resulting mixture, an aqueous layer was subjected to extraction with ethyl acetate. Organic layers combined were washed with water, and dried over anhydrous magnesium sulfate. The resulting solution was concentrated under reduced pressure to obtain compound (T-22) (32.6 g; 95%).

### Third step

Compound (1-9-1) (2.0 g), compound (T-22) (1.18 g), DMAP (0.32 g), and dichloromethane (100 mL) were put in a reaction vessel, and the resulting mixture was cooled at 0°C. A dichloromethane (60 mL) solution of DCC (1.30 g) was slowly added dropwise thereto, and the resulting mixture was stirred for 12 hours while returning to room temperature. After an insoluble matter was filtered off, the reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (toluene : ethyl acetate = 19 : 1 in a volume ratio) to obtain compound (T-23) (2.37 g; 82%).

### Fourth step

Compound (T-23) (2.37 g), pyridinium p-toluenesulfonate (PPTS) (0.54 g), THF (50 mL) and methanol (50 mL) were put in a reaction vessel, and the resulting mixture was stirred at 50°C for 5 hours. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 9 : 1 in a volume ratio) to obtain compound (1-9-2) (1.50 g; 75%).

An NMR analysis value of compound (1-9-2) obtained is as described below. ¹H-NMR: Chemical shifts δ (ppm; CDCl₃) : 6.24 (s, 1H), 6.09 (s, 1H), 5.84 (s, 1H), 5.57 (s, 1H), 4.33 - 4.27 (m, 4H), 4.20 - 4.16 (m, 2H), 2.34 - 2.31 (m, 1H), 1.97 - 1.90 (m, 4H), 1.82 - 1.67 (m, 8H), 1.43 - 1.39 (m, 1H), 1.31 - 1.18 (m, 6H), 1.15 - 0.75 (m, 16H).

Physical properties of compound (1-9-2) were as described below. Transition temperature: C 66.5 I.

### Synthesis Example 6

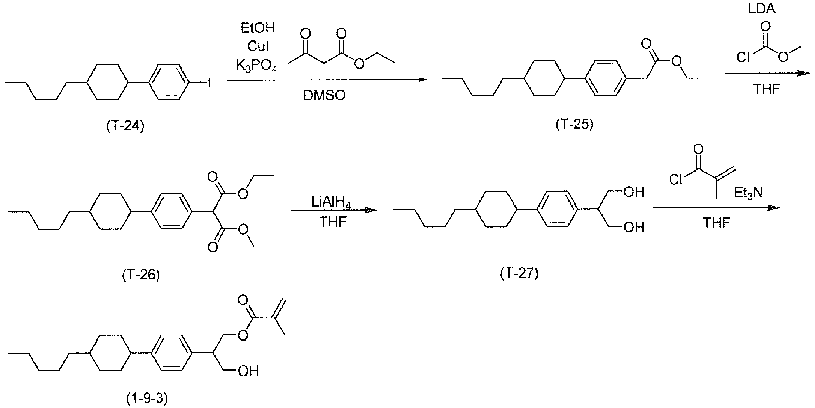

### First step

Compound (T-24) (30.0 g), ethanol (14.4 mL), potassium phosphate (53.6 g), copper iodide (1.60 g), ethyl acetoacetate (32.8 g) and dimethyl sulfoxide (DMSO) (500 mL) were put in a reaction vessel, and the resulting mixture was stirred at 80°C for 6 hours. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio) to obtain compound (T-25) (19.5 g; 73%).

### Second step

Compound (T-26) (16.2 g; 70%) was obtained by using compound (T-25) (19.5 g) as a raw material in a manner similar to the technique in the first step in Synthesis Example 4.

### Third step

Compound (T-27) (6.0 g; 45%) was obtained by using compound (T-26) (16.2g) as a raw material in a manner similar to the technique in the second step in Synthesis Example 4.

### Third step

Compound (1-9-3) (2.3 g; 31%) was obtained by using compound (T-27) (6.0 g) as a raw material in a manner similar to the technique in the third step in Synthesis Example 4.

An NMR analysis value of compound (1-9-3) obtained is as described below. ¹H-NMR: Chemical shifts δ (ppm; CDCl₃) : 7.18 - 7.17 (m, 4H), 6.09 (s, 1H), 5.57 (s, 1H), 4.47 - 4.38 (m, 2H), 3.91 - 3.85 (m, 2H), 3.19 - 3.14 (m, 1H), 2.44 (tt, J = 12.2 Hz, J = 3.0 Hz, 1H), 1.93 - 1.86 (m, 8H), 1.48 - 1.38 (m, 2H), 1.34 - 1.19 (m, 9H), 1.07 - 0.99 (m, 2H), 0.89 (t, J = 6.8 Hz, 3H).

Physical properties of compound (1-9-3) were as described below. Transition temperature: C 36.1 I.

### Synthesis Example 7

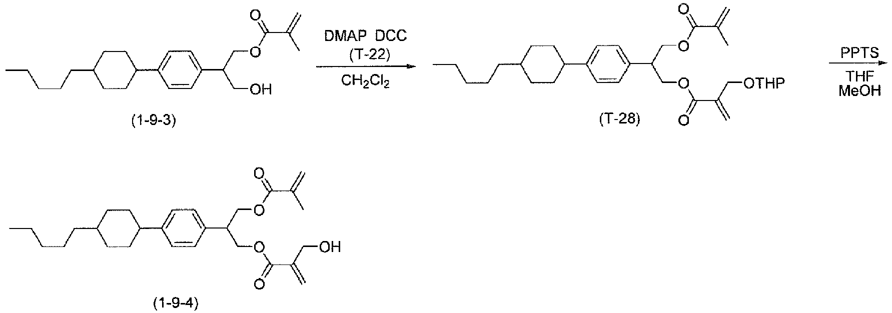

### First step

Compound (T-28) (2.2 g; 76%) was obtained by using compound (1-9-3) (2.0 g) as a raw material in a manner similar to the technique in the third step in Synthesis Example 5.

### Second step

Compound (1-9-4) (1.3 g; 70%) was obtained by using compound (T-28) (2.2 g) as a raw material in a manner similar to the technique in the fourth step in Synthesis Example 5.

An NMR analysis value of compound (1-9-4) obtained is as follows.
¹H-NMR : Chemical shifts δ (ppm; CDCl₃) : 7.17 - 7.16 (m, 4H), 6.21 (s, 1H), 6.07 (s, 1H), 5.81 (d, J = 1.0 Hz, 1H), 5.55 (s, 1H), 4.46 - 4.39 (m, 4H), 4.27 (d, J = 6.2 Hz, 2H), 3.42 - 3.37 (m, 1H), 2.44 (tt, J = 12.2 Hz, J = 3.1 Hz, 1H), 2.22 - 2.21 (m, 1H), 1.95 (s, 3H), 1.87 - 1.85 (m, 4H), 1.46 - 1.38 (m, 2H), 1.34 - 1.19 (m, 9H), 1.07 - 0.99 (m, 2H), 0.89 (t, J = 7.0 Hz, 3H).

Physical properties of compound (1-9-4) were as described below. Transition temperature: C 52.3 I.

### Synthesis Example 8

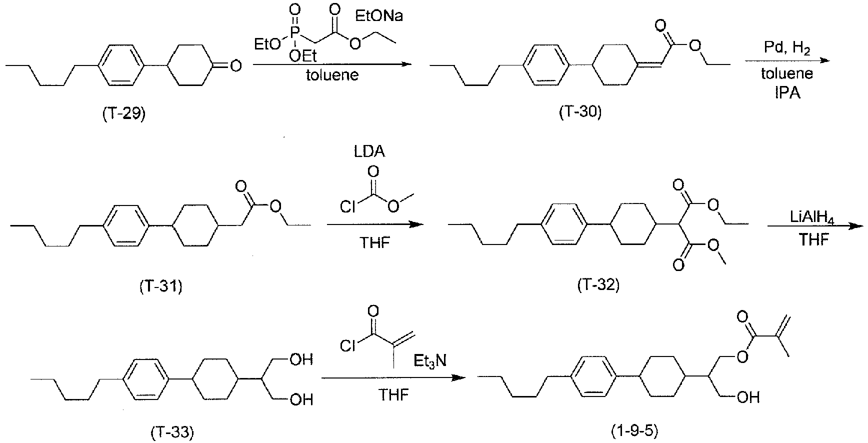

### First step

Compound (T-29) (30.0 g), triethyl phosphonoacetate (33.0 g) and toluene (500 mL) were put in a reaction vessel, and the resulting mixture was cooled to 0°C. Sodium ethoxide (20% ethanol solution) (50.1 g) was slowly added dropwise thereto, and the resulting mixture was stirred for 6 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio) to obtain compound (T-30) (32.8 g; 85%).

### Second step

Compound (T-30) (32.8 g), toluene (300 mL), IPA (300 mL) and Pd/C (0.55 g) were put in a reaction vessel, and the resulting mixture was stirred under a hydrogen atmosphere for 12 hours. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio). The resulting solution was further purified by recrystallization from heptane to obtain compound (T-31) (16.8 g; 51%).

### Third step

Compound (T-32) (14.1 g; 71%) was obtained by using compound (T-31) (16.8 g) as a raw material in a manner similar to the technique in the first step in Synthesis Example 4.

### Fourth step

Compound (T-33) (6.0 g; 52%) was obtained by using compound (T-32) (14.1 g) as a raw material in a manner similar to the technique in the second step in Synthesis Example 4.

### Fifth step

Compound (1-9-5) (2.3 g; 32%) was obtained by using compound (T-33) (6.0 g) as a raw material in a manner similar to the technique in the third step in Synthesis Example 4.

An NMR analysis value of compound (1-9-5) obtained is as described below. ¹H-NMR: Chemical shifts δ (ppm; CDCl₃) : 7.14 - 7.10 (m, 4H), 6.12 (s, 1H), 5.59 (s, 1H), 4.43 - 4.40 (m, 1H), 4.28 - 4.25 (m, 1H), 3.75 - 3.64 (m, 2H), 2.55 (t, J = 7.6 Hz, 2H), 2.47 - 2.42 (m, 1H), 2.14 (s, 1H), 1.96 - 1.91 (m, 7H), 1.74 - 1.69 (m, 1H), 1.62 - 1.22 (m, 11H), 0.88 (t, J = 6.8 Hz, 3H).

Physical properties of compound (1-9-5) were as described below. Transition temperature: C < -50.0 I.

### Synthesis Example 9

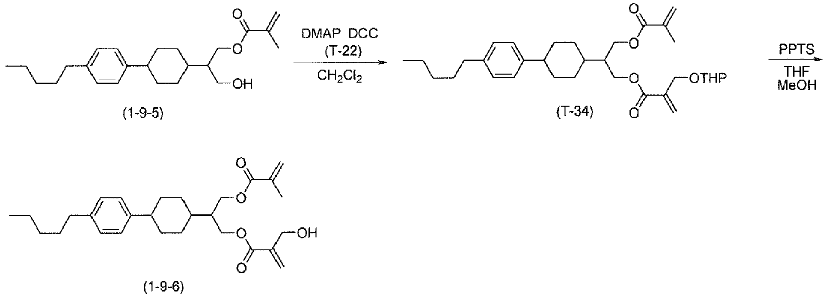

### First step

Compound (T-34) (1.9 g; 68%) was obtained by compound (1-9-5) (2.0 g) as a raw material according to the third step in Synthesis Example 5.

### Second step

Compound (1-9-6) (1.2 g; 75%) was obtained by using compound (T-34) (1.9 g) as a raw material according to the fourth step in Synthesis Example 5.

An NMR analysis value of compound (1-9-6) obtained is as described below.
¹H-NMR: Chemical shifts δ (ppm; CDCl₃) : 7.13 - 7.10 (m, 4H), 6.27 (s, 1H), 6.11 (s, 1H), 5.86 (s, 1H), 5.58 (s, 1H), 4.40 - 4.32 (m, 4H), 4.25 - 4.20 (m, 2H), 2.56 (t, J = 7.6 Hz, 2H), 2.45 (tt, J = 12.1 Hz, J = 2.9 Hz, 1H), 2.35 - 2.32 (m, 1H), 2.04 - 1.91 (m, 7H), 1.62 - 1.26 (m, 12H), 0.88 (t, J = 6.8 Hz, 3H).

Physical properties of compound (1-9-6) were as described below. Transition temperature: C 35.8 I.

### Synthesis Example 10

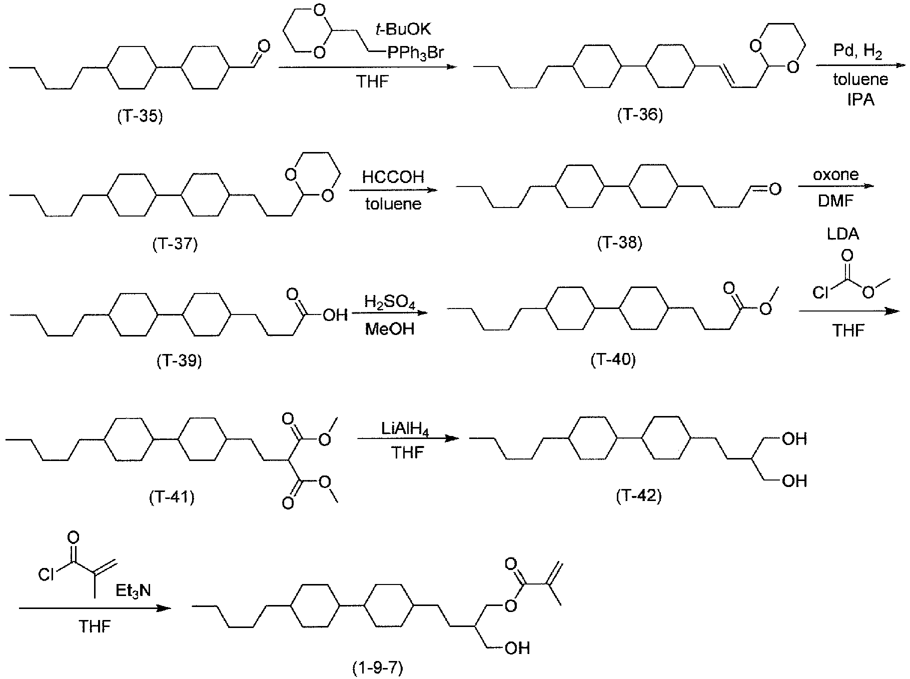

### First step

In a reaction vessel, 2-(1,3-dioxane-2-yl)ethyltriphenylphosphonium bromide (103.7 g) and THF (500 mL) were put, and the resulting mixture was cooled to -30°C, and potassium t-butoxide (25.4 g) was added thereto, and the resulting mixture was stirred for 1 hour. A THF (300 mL) solution of compound (T-35) (50.0 g) was slowly added dropwise thereto, and the resulting mixture was stirred for 6 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 1 in a volume ratio) to obtain compound (T-36) (63.0 g; 92%).

### Second step

Compound (T-36) (63.0 g), toluene (500 mL), IPA (500 mL) and Pd/C (0.55 g) were put in a reaction vessel, and the resulting mixture was stirred under a hydrogen atmosphere for 16 hours. After an insoluble matter was filtered out, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 1 in a volume ratio) to obtain compound (T-37) (60.1 g; 95%).

### Third step

Compound (T-37) (60.1 g), formic acid (75.8 g) and toluene (1,000 mL) were put in a reaction vessel, and the resulting mixture was stirred at 100°C for 6 hours. After an insoluble matter was filtered off, the resulting solution was neutralized with a sodium hydrogencarbonate aqueous solution, and an aqueous layer was subjected to extraction with toluene. Organic layers combined were washed with water, and dried over anhydrous magnesium sulfate. The resulting solution was concentrated under reduced pressure, and the residue was purified by silica gel chromatography with toluene to obtain compound (T-38) (45.0 g; 89%).

### Fourth step

Compound (T-38) (45.0 g), potassium peroxymonosulfate (OXONE) (108.3 g) and DMF (1,000 mL) were put in a reaction vessel, and the resulting mixture was stirred at room temperature for 8 hours. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure to obtain compound (T-39) (28.5 g; 60%).

### Fifth step

Compound (T-39) (28.5 g), sulfuric acid (0.5 mL) and methanol (500 mL) were put in a reaction vessel, and the resulting mixture was stirred at 60°C for 5 hours. After an insoluble matter was filtered off, the resulting solution was concentrated, and the residue was purified by silica gel chromatography with toluene to obtain compound (T-40) (22.3 g; 75%).

### Sixth step

Compound (T-41) (18.3 g; 70%) was obtained by using compound (T-40) (22.3 g) as a raw material in a manner similar to the technique in the first step in Synthesis Example 4.

### Seventh step

Compound (T-42) (5.9 g; 38%) was obtained by using compound (T-41) (18.3 g) as a raw material in a manner similar to the technique in the second step in Synthesis Example 4.

### Eighth step

Compound (1-9-7) (2.4 g; 34%) was obtained by using compound (T-42) (5.9 g) as a raw material in a manner similar to the technique in the third step in Synthesis Example 4.

An NMR analysis value of compound (1-9-7) obtained is as described below.
¹H-NMR: Chemical shifts δ (ppm; CDCl₃) : 6.11 (s, 1H), 5.81 (s, 1H), 4.31 - 4.28 (m, 1H), 4.17 - 4.14 (m, 1H), 3.63 - 3.58 (m, 1H), 3.54 - 3.49 (m, 1H), 1.98 - 1.95 (m, 4H), 1.84 - 1.69 (m, 9H), 1.41 - 1.18 (m, 10H), 1.15 - 1.06 (m, 4H), 1.02 - 0.80 (m, 13H).

Physical properties of compound (1-9-7) were as described below. Transition temperature: C 33.6 S_{A} 101 I.

### Synthesis Example 11

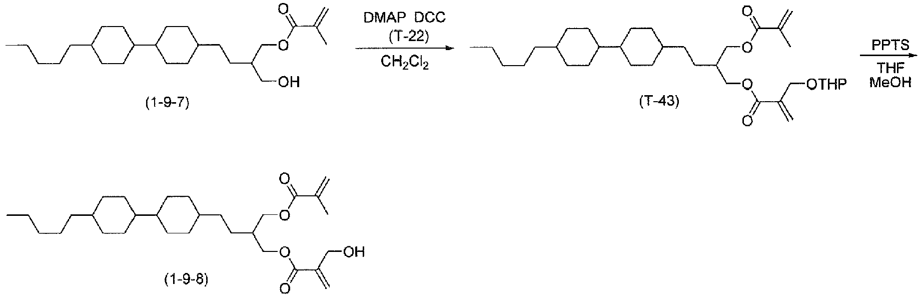

### First step

Compound (T-43) (2.1 g; 74%) was obtained by using compound (1-9-7) (2.0 g) as a raw material in a manner similar to the technique in the third step in Synthesis Example 5.

### Second step

Compound (1-9-8) (1.3 g; 72%) was obtained by using compound (T-43) (2.1 g) as a raw material in a manner similar to the technique in the fourth step in Synthesis Example 5.

An NMR analysis value of compound (1-9-8) obtained is as described below. ¹H-NMR: Chemical shifts δ (ppm; CDCl₃) : 6.25 (s, 1H), 6.10 (s, 1H), 5.85 (d, J = 1.1 Hz, 1H), 5.57 (s, 1H), 4.33 (d, J = 6.5 Hz, 2H), 4.24 - 4.11 (m, 4H), 2.28 (t, J = 6.6 Hz, 1H), 2.09 - 2.03 (m, 1H), 1.94 (s, 3H), 1.75 - 1.67 (m, 8H), 1.44 - 1.39 (m, 2H), 1.32 - 1.18 (m, 8H), 1.15 - 1.06 (m, 4H), 1.02 - 0.79 (m, 13H).

Physical properties of compound (1-9-8) were as described below.
Transition temperature: C 71.4 I.

### Synthesis Example 12

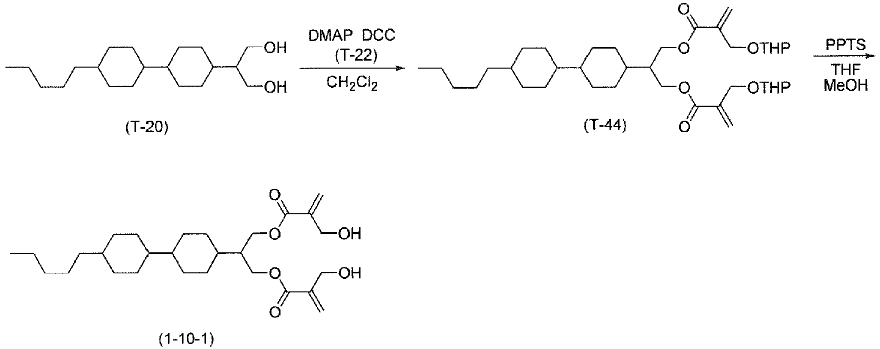

### First step

Compound (T-20) (2.0 g), compound (T-22) (2.63 g), DMAP (0.78 g) and dichloromethane (100 mL) were put in a reaction vessel, and the resulting mixture was cooled to 0°C. A dichloromethane (60 mL) solution of DCC (2.92 g) was slowly added dropwise thereto, and the resulting mixture was stirred for 12 hours while returning to room temperature. After an insoluble matter was filtered off, the reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (toluene : ethyl acetate = 9 : 1 in a volume ratio) to obtain compound (T-44) (2.83 g; 68%).

### Second step

Compound (T-44) (2.83 g), pyridinium p-toluenesulfonate (PPTS) (1.09 g), THF (50 mL) and methanol (50 mL) were put in a reaction vessel, and the resulting mixture was stirred at 50°C for 8 hours. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 1 : 1 in a volume ratio) to obtain compound (1-10-1) (1.47 g; 70%).

An NMR analysis value of compound (1-10-1) obtained is as described below.
¹H-NMR: Chemical shifts δ (ppm; CDCl₃) : 6.24 (s, 2H), 5.82 (s, 2H), 4.35 - 4.31 (m, 6H), 4.22 - 4.19 (m, 2H), 2.36 (s, 2H), 1.97 - 1.91 (s, 1H), 1.82 - 1.63 (m, 8H), 1.43 - 1.18 (m, 7H), 1.15 - 0.79 (m, 16H) .

Physical properties of compound (1-10-1) were as described below. Transition temperature: C 102 I.

### Synthesis Example 13

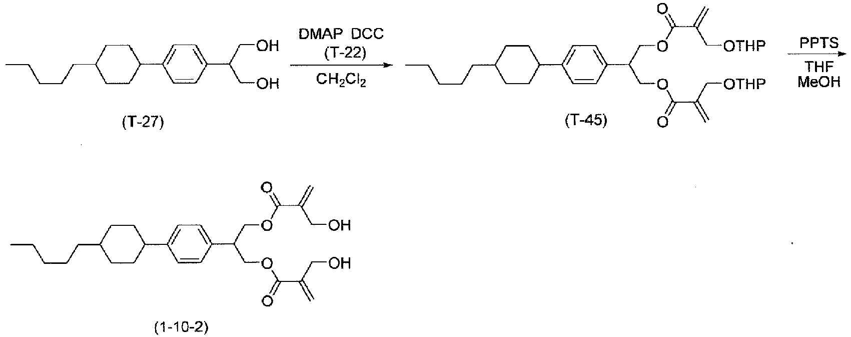

### First step

Compound (T-45) (2.7 g; 64%) was obtained by using compound (T-27) (2.0 g) as a raw material in a manner similar to the technique in the first step in Synthesis Example 12.

### Second step

Compound (1-10-2) (1.3 g; 65%) was obtained by using compound (T-45) (2.7 g) as a raw material in a manner similar to the technique in the second step in Synthesis Example 12.

An NMR analysis value of compound (1-10-2) obtained is as described below.
¹H-NMR: Chemical shifts δ (ppm; CDCl₃) : 7.20 - 7.16 (m, 4H), 6.26 (s, 2H), 5.83 (d, J = 0.8 Hz, 2H), 4.46 (d, J = 6.6 Hz, 4H), 4.28 (d, J = 6.3 Hz, 4H), 3.44 - 3.39 (m, 1H), 2.44 (tt, J = 12.2 Hz, J = 3.1 Hz, 1H), 2.16 - 2.13 (m, 2H), 1.87 - 1.85 (m, 4H), 1.46 - 1.19 (m, 11H), 1.07 - 0.99 (m, 2H), 0.89 (t, J = 6.8 Hz, 3H).

Physical properties of compound (1-10-2) were as described below. Transition temperature: C 65.8 I.

### Synthesis Example 14

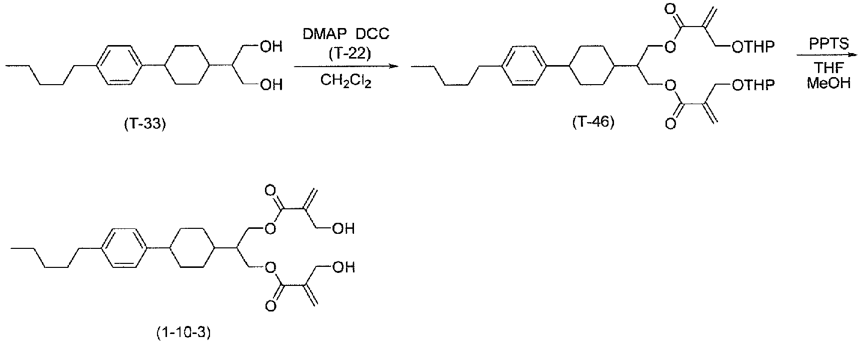

### First step

Compound (T-46) (2.5 g; 59%) was obtained by using compound (T-33) (2.0 g) as a raw material according to the first step in Synthesis Example 12.

### Second step

Compound (1-10-3) (1.1 g; 60%) was obtained by using compound compound (T-46) (2.7 g) as a raw material in a manner similar to the technique in the second step in Synthesis Example 12.

An NMR analysis value of compound (1-10-3) obtained is as described below.
¹H-NMR: Chemical shifts δ (ppm; CDCl₃) : 7.14 - 7.10 (m, 4H), 6.27 (s, 2H), 5.87 (d, J = 1.1 Hz, 2H), 4.39 - 4.33 (m, 6H), 4.27 - 4.20 (m, 2H), 2.57 - 2.54 (m, 2H), 2.45 (tt, J = 12.2 Hz, J = 3.1 Hz, 1H), 2.38 - 2.35 (m, 2H), 2.05 - 1.91 (m, 5H), 1.63 - 1.1.26 (m, 11H), 0.88 (t, J = 6.8 Hz, 3H).

Physical properties of compound (1-10-3) were as described below. Transition temperature: C 65.6 I.

### Synthesis Example 15

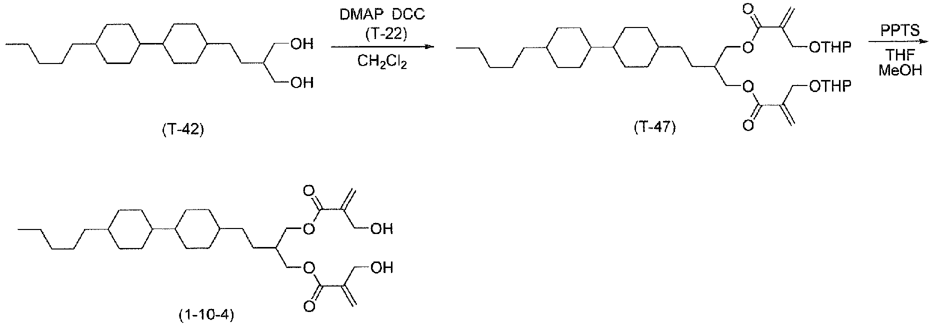

### First step

Compound (T-47) (2.7 g; 67%) was obtained by using compound (T-head-42) (2.0 g) as a raw material in a manner similar to the technique in the first step in Synthesis Example 12.

### Second step

Compound (1-10-4) (1.3 g; 64%) was obtained by using compound (T-47) (2.7 g) as a raw material in a manner similar to the technique in the second step in Synthesis Example 12.

An NMR analysis value of compound (1-10-4) obtained is as described below.
¹H-NMR: Chemical shifts δ (ppm; CDCl₃): 6.25 (s, 2H), 5.85 (d, J = 1.1 Hz, 2H), 4.33 (d, J = 6.3 Hz, 4H), 4.25 - 4.22 (m, 2H), 4.18 - 4.14 (m, 2H), 2.30 - 2.28 (m, 2H), 2.11 - 2.06 (m, 1H), 1.75 - 1.67 (m, 8H), 1.44 - 1.39 (m, 2H), 1.32 - 0.79 (m, 25H).

Physical properties of compound (1-10-4) were as described below. Transition temperature: C 85.7 S_{A} 125 I.

### Synthesis example 16

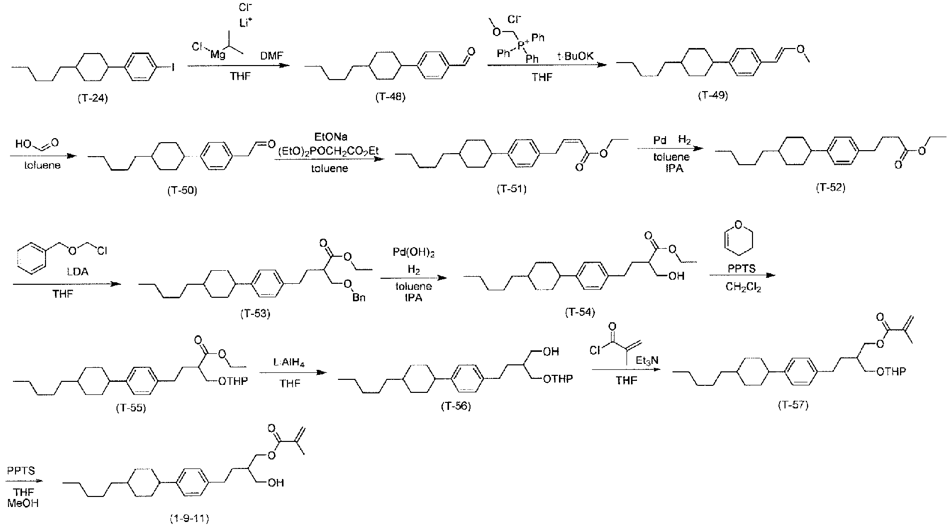

### First step

Compound (T-24) (50.0 g) and THF (1,000 mL) were put in a reaction vessel, and the resulting mixture was cooled to -70°C. Isopropylmagnesium chloride-lithium chloride (1.3 M; a THF solution; 130.0 mL) was slowly added dropwise thereto, and the resulting mixture was stirred for 1 hour. DMF (13.0 mL) was added dropwise thereto, and the resulting mixture was stirred for 4 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio) to obtain compound (T-48) (34.5 g; 95%).

### Second step

Methoxymethyl triphenylphosphonium chloride (54.9 g) and THF (1,000 mL) were put in a reaction vessel, and the resulting mixture was cooled to -30°C. Potassium t-butoxide (18.0 g) was added thereto, and the resulting mixture was stirred for 1 hour. A THF (500 mL) solution of compound (T-48) (34.5 g) was added dropwise thereto, and the resulting mixture was stirred for 5 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 4 in a volume ratio) to obtain compound (T-49) (31.7 g; 83%).

### Third step

Compound (T-49) (31.7 g), formic acid (50.9 g) and toluene (1,000 mL) were put in a reaction vessel, and the resulting mixture was stirred at 100°C for 6 hours. After an insoluble matter was filtered off, the reaction mixture was poured into water, and the resulting solution was neutralized with sodium hydrogencarbonate water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 4 : 1 in a volume ratio) to obtain compound (T-50) (26.5 g; 88%).

### Fourth step

Compound (T-50) (26.5 g), triethyl phosphonoacetate (26.2 g), toluene (500 mL) were put in a reaction vessel, and the resulting mixture was cooled to 0°C. Sodium ethoxide (a 20% ethanol solution) (39.7 g) was slowly added dropwise thereto, and the resulting mixture was stirred for 6 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 9 : 1 in a volume ratio) to obtain compound (T-51) (30.6 g; 92%).

### Fifth step

Compound (T-51) (30.6 g), Pd/C (0.55 g), toluene (250 mL) and IPA (250 mL) were put in a reaction vessel, and the resulting mixture was stirred under a hydrogen atmosphere for 12 hours. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 9 : 1 in a volume ratio) to obtain compound (T-52) (29.2 g; 95%).

### Sixth step

Compound (T-52) (29.2 g) and THF (250 mL) were put in a reaction vessel, and the resulting mixture was cooled to -70°C. Lithium diisopropylamide (LDA) (1.1 M; a THF solution; 92.5 mL) was slowly added dropwise thereto. The resulting mixture was stirred for 1 hour, and benzyl chloromethyl ether (16.0 g) was added dropwise thereto, and the resulting mixture was stirred for 12 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 9 : 1 in a volume ratio) to obtain compound (T-53) (31.5 g; 80%).

### Seventh step

Compound (T-53) (31.5 g), palladium hydroxide (0.28 g), toluene (250 mL) and IPA (250 mL) were put in a reaction vessel, and the resulting mixture was stirred under a hydrogen atmosphere for 12 hours. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : ethyl acetate = 4 : 1 in a volume ratio) to obtain compound (T-54) (23.3 g; 92%).

### Eighth step

Compound (T-54) (23.3 g), 3,4-dihydro-2H-pyran (5.8 g), pyridinium p-toluenesulfonate (PPTS) (1.5 g) and dichloromethane (500 mL) were put in a reaction vessel, and were stirred at room temperature for 8 hours. The reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (toluene : ethyl acetate = 9 : 1 in a volume ratio) to obtain compound (T-55) (25.4 g; 89%).

### Ninth step

Lithium aluminum hydride (LAH) (1.2 g) and THF (300 mL) were put in a reaction vessel, and the resulting mixture was cooled to 0°C. A THF (100 mL) solution of compound (T-55) (25.4 g) was slowly added dropwise thereto, and the resulting mixture was stirred for 3 hours while returning to room temperature. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 4 : 1 in a volume ratio) to obtain compound (T-56) (19.2 g; 83%).

### Tenth step

Compound (T-56) (19.2 g), triethylamine (7.6mL), and THF (200 mL) were put in a reaction vessel, and the resulting mixture was cooled to 0°C. Methacryloyl chloride (5.3 mL) was slowly added thereto, and the resulting mixture was stirred for 5 hours while returning to room temperature. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 19 : 1 in a volume ratio) to obtain compound (T-57) (17.9 g; 80%).

### Eleventh step

Compound (T-57) (17.9 g), pyridinium p-toluenesulfonate (PPTS) (4.6 g), THF (200 mL) and methanol (200 mL) were put in a reaction vessel, and the resulting mixture was stirred at 50°C for 6 hours. After an insoluble matter was filtered off, the reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 9 : 1 in a volume ratio) to obtain compound (1-9-11) (12.4 g; 84%).

An NMR analysis value of compound (1-9-11) obtained is as described below.
¹H-NMR: Chemical shifts δ (ppm; CDCl₃) : 7.13 - 7.09 (m, 4H), 6.10 (s, 1H), 5.57 (s, 1H), 4.33 - 4.30 (m, 1H), 4.23 - 4.20 (m, 1H), 3.65 - 3.62 (m, 1H), 3.58 - 3.54 (m, 1H), 2.66 (t, J = 8.0 Hz, 2H), 2.45 - 2.39 (m, 1H), 1.94 - 1.81 (m, 8H), 1.74 - 1.60 (m, 2H), 1.46 - 1.19 (m, 12H), 1.07 - 0.99 (m, 2H), 0.89 (t, J = 6.9 Hz, 3H) .

Physical properties of compound (1-9-11) were as described below. Transition temperature: C -24.7 I.

### Synthesis Example 17

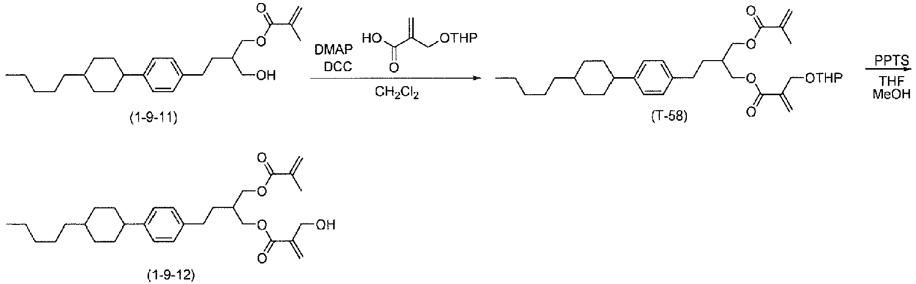

### First step

Compound (T-58) (2.1 g; 74%) was obtained by using compound (1-9-11) (2.0 g) as a raw material in a manner similar to the technique in the third step in Synthesis Example 5.

### Second step

Compound (1-9-12) (1.4 g; 78%) was obtained by using compound (T-58) (2.1 g) as a raw material in a manner similar to the technique in the fourth step in Synthesis Example 5.

An NMR analysis value of compound (1-9-12) obtained is as described below. ¹H-NMR: Chemical shifts δ (ppm; CDCl₃) : 7.14 - 7.08 (m, 4H), 6.24 (s, 1H), 6.10 (s, 1H), 5.85 (s, 1H), 5.57 (s, 1H), 4.32 (d, J = 6.1 Hz, 2H), 4.27 - 4.18 (m, 4H), 2.67 (t, J = 7.4 Hz, 2H), 2.45 - 2.40 (m, 2H), 2.18 - 2.12 (m, 1H), 1.93 (s, 3H), 1.88 - 1.84 (m, 4H), 1.77 - 1.72 (m, 2H), 1.46 - 1.38 (m, 2H), 1.34 - 1.19 (m, 9H), 1.07 - 0.99 (m, 2H), 0.89 (t, J = 6.8 Hz, 3H).

Physical properties of compound (1-9-12) were as described below. Transition temperature: C -30.2 S_{A} -26.3 I.

### Synthesis Example 18

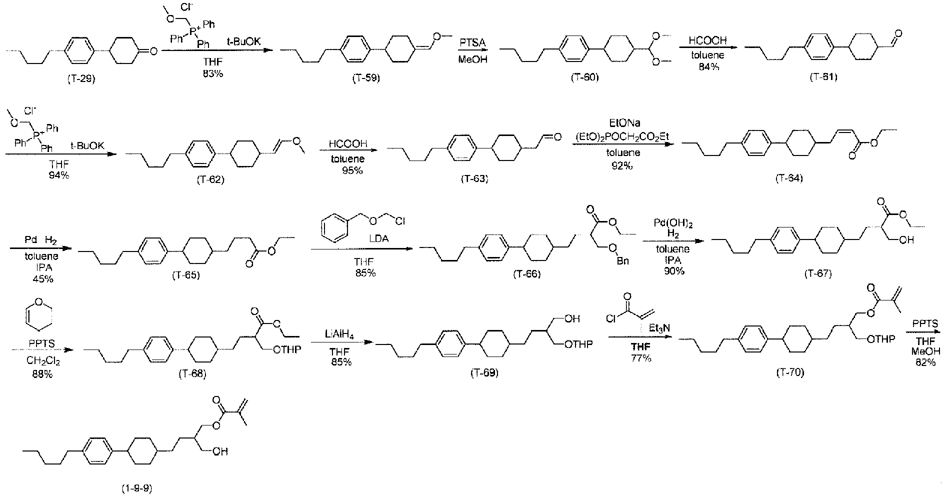

### First step

Methoxymethyl triphenylphosphonium chloride (84.2 g) and THF (1,000 mL) were put in a reaction vessel, and the resulting mixture was cooled to -30°C. Potassium t-butoxide (27.6 g) was added thereto, and the resulting mixture was stirred for 1 hour. A THF (500 mL) solution of compound (T-29) (50.0 g) was added dropwise thereto, and the resulting mixture was stirred for 5 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 4 in a volume ratio) to obtain compound (T-59) (46.3 g; 83%).

### Second step

Compound (T-59) (46.3 g), p-toluenesulfonic acid (PTSA) (3.2 g), and toluene (1,000 mL) were put in a reaction vessel, and the resulting mixture was stirred at 100°C for 12 hours. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 1 in a volume ratio) to obtain compound (T-60) (49.2 g; 95%).

### Third step

Compound (T-60) (49.2 g), formic acid (74.4 g) and toluene (1,000 mL) were put in a reaction vessel, and the resulting mixture was stirred at room temperature for 8 hours . After an insoluble matter was filtered off, the reaction mixture was poured into water, and the resulting solution was neutralized with sodium hydrogencarbonate water. An aqueous layer was subjected to extraction with toluene, and Organic layers combined were washed with water, and dried over anhydrous magnesium sulfate. The resulting solution was concentrated under reduced pressure, and the residue was purified by silica gel chromatography (toluene : heptane = 1 : 1 in a volume ratio) to obtain compound (T-61) (35.0 g; 84%).

### Fourth step

Methoxymethyl triphenylphosphonium chloride (55.7 g) and THF (1,000 mL) were put in a reaction vessel, and the resulting mixture was cooled to -30°C. Potassium t-butoxide (18.2 g) was added thereto, and the resulting mixture was stirred for 1 hour. A THF (500 mL) solution of compound (T-61) (35.0 g) was added dropwise thereto, and the resulting mixture was stirred for 5 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 1 : 4 in a volume ratio) to obtain compound (T-62) (36.5 g; 94%).

### Fifth step

Compound (T-62) (36.5 g), formic acid (58.6 g) and toluene (1,000 mL) were put in a reaction vessel, and the resulting mixture was stirred at 100°C for 4 hours. After an insoluble matter was filtered off, the reaction mixture was poured into water, and the resulting solution was neutralized with sodium hydrogencarbonate water. An aqueous layer was subjected to extraction with toluene, and organic layers combined were washed with water, and dried over anhydrous magnesium sulfate. The resulting solution was concentrated under reduced pressure, and the residue was purified by silica gel chromatography (toluene : heptane = 1 : 1 in a volume ratio) to obtain compound (T-63) (33.0 g; 95%).

### Sixth step

Compound (T-64) (38.2 g; 92%) was obtained by using compound (T-63) (33.0 g) as a raw material in a manner similar to the technique in the fourth step in Synthesis Example 16.

### Seventh step

Compound (T-65) (18.7 g; 48%) was obtained by using compound (T-64) (38.2 g) as a raw material in a manner similar to the technique in the fifth step in Synthesis Example 16.

### Eighth step

Compound (T-66) (21.5 g; 85%) was obtained by using compound (T-65) (18.7 g) as a raw material in a manner similar to the technique in the sixth step in Synthesis Example 16.

### Ninth step

Compound (T-67) (15.6 g; 90%) was obtained by using compound (T-66) (21.5 g) as a raw material in a manner similar to the technique in the seventh step in Synthesis Example 16.

### Tenth step

Compound (T-68) (16.8 g; 88%) was obtained by using compound (T-67) (15.6 g) as a raw material in a manner similar to the technique in the eighth step in Synthesis Example 16.

### Tenth step

Compound (T-69) (13.0 g; 85%) was obtained by using compound (T-68) (16.8 g) as a raw material in a manner similar to the technique in the ninth step in Synthesis Example 16.

### Eleventh step

Compound (T-70) (11.6 g; 77%) was obtained by using compound (T-69) (13.0 g) as a raw material in a manner similar to the technique in the tenth step in Synthesis Example 16.

### Twelfth step

Compound (1-9-9) (7.8 g; 82%) was obtained by using compound (T-70) (11.6 g) as a raw material in a manner similar to the technique in the eleventh step in Synthesis Example 16.

An NMR analysis value of compound (1-9-9) obtained is as described below. ¹H-NMR: Chemical shifts δ (ppm; CDCl₃) : 7.11 - 7.08 (m, 4H), 6.11 (s, 1H), 5.57 (s, 1H), 4.31 - 4.28 (m, 1H), 4.19 - 4.16 (m, 1H), 3.63 - 3.60 (m, 1H), 3.55 - 3.52 (m, 1H), 1.95 (s, 3H), 1.89 - 1.81 (m, 5H), 1.62 - 1.56 (m, 2H), 1.47 - 1.28 (m, 12H), 1.09 - 1.01 (m, 2H), 0.88 (t, J = 6.7 Hz, 3H).

Physical properties of compound (1-9-9) were as described below. Transition temperature: C < -50 I.

### Synthesis example 19

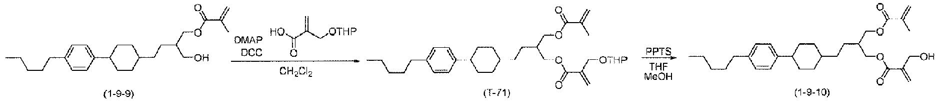

### First step

Compound (T-71) (3.2 g; 75%) was obtained by using compound (1-9-9) (3.0 g) as a raw material in a manner similar to the technique in the third step in Synthesis Example 5.

### Second step

Compound (1-9-10) (1.9 g; 70%) was obtained by using compound (T-71) (3.2 g) as a raw material in a manner similar to the technique in the fourth step in Synthesis Example 5.

An NMR analysis value of compound (1-9-10) obtained is as described below.
¹H-NMR: Chemical shifts δ (ppm; CDCl₃) : 7.12 - 7.08 (m, 4H), 6.26 (s, 1H), 6.11 (s, 1H), 5.86 (s, 1H), 5.58 (s, 1H), 4.34 (d, J = 6.5 Hz, 2H), 4.26 - 4.14 (m, 4H), 2.55 (t, J = 7.8 Hz, 2H), 2.45 - 2.40 (m, 1H), 2.34 (s, 1H), 2.13 - 2.08 (m, 1H), 1.95 (s, 3H), 1.90 - 1.84 (m, 4H), 1.63 - 1.56 (m, 2H), 1.48 - 1.39 (m, 4H), 1.35 - 1.27 (m, 7H), 1.09 - 1.01 (m, 2H), 0.88 (t, J = 6.8 Hz, 3H) .

Physical properties of compound (1-9-10) were as described below. Transition temperature: C 35.9 I.

### Synthesis Example 20

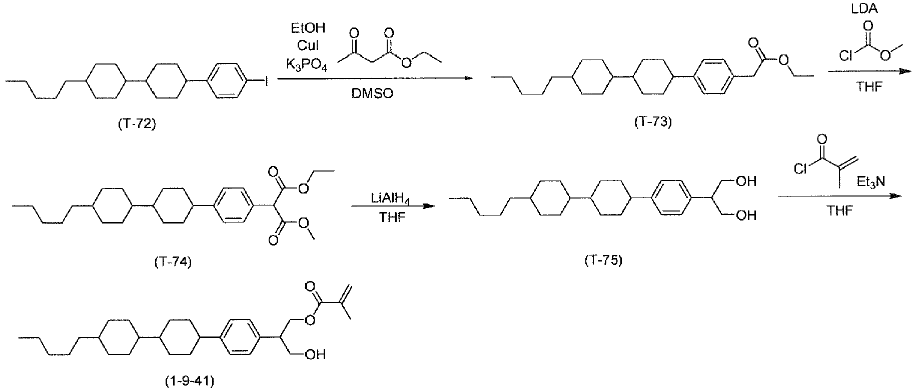

### First step

Compound (T-73) (31.8 g; 70%) was obtained by using compound (T-72) (50.0 g) as a raw material in a manner similar to the technique in the first step in Synthesis Example 6.

### Second step

Compound (T-74) (26.2 g; 72%) was obtained by using compound (T-73) (31.8 g) as a raw material in a manner similar to the technique in the second step in Synthesis Example 6.

### Third step

Compound (T-75) (10.1 g; 46%) was obtained by using compound (T-74) (26.2 g) as a raw material in a manner similar to the technique in the third step in Synthesis Example 6.

### Fourth step

Compound (1-9-41) (3.8 g; 32%) was obtained by using compound (T-75) (10.1 g) as a raw material in a manner similar to the technique in the fourth step in Synthesis Example 6.

An NMR analysis value of compound (1-9-41) obtained is as described below. ¹H-NMR: Chemical shifts δ (ppm; CDCl₃) : 7.18 - 7.16 (m, 4H), 6.09 (s, 1H), 5.57 (s, 1H), 4.47 - 4.38 (m, 2H), 3.91 - 3.83 (m, 2H), 3.20 - 3.14 (m, 1H), 2.45 - 2.40 (m, 1H), 1.97 - 1.72 (m, 12H), 1.42 - 0.82 (m, 22H).

Physical properties of compound (1-9-41) were as described below. Transition temperature: C 46.3 I.

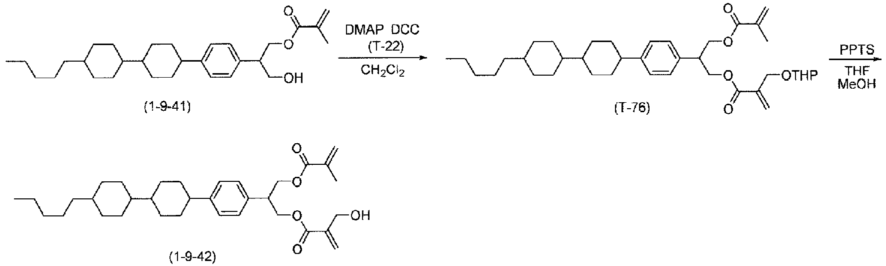

### Synthesis Example 21

### First step

Compound (T-76) (3.1 g; 75%) was obtained by using compound (1-9-41) (3.0 g) as a raw material in a manner similar to the technique in the third step in Synthesis Example 5.

### Second step

Compound (1-9-42) (1.9 g; 71%) was obtained by using compound (T-76) (3.1 g) as a raw material in a manner similar to the technique in the fourth step in Synthesis Example 5.

An NMR analysis value of compound (1-9-42) obtained is as described below. ¹H-NMR: Chemical shifts δ (ppm; CDCl₃) : 7.17 (s, 4H), 6.21 (s, 1H), 6.07 (s, 1H), 5.80 (s, 1H), 5.56 (s, 1H), 4.46 - 4.39 (m, 4H), 4.27 (d, J = 6.6 Hz, 2H), 3.42 - 3.37 (m, 1H), 2.45 - 2.39 (m, 1H), 2.12 (t, J = 6.6 Hz, 1H), 1.91 - 1.72 (m, 11H), 1.46 - 0.95 (m, 16H), 0.89 - 0.82 (m, 5H).

Physical properties of compound (1-9-42) were as described below. Transition temperature: C 80.0 I.

### Synthesis Example 22

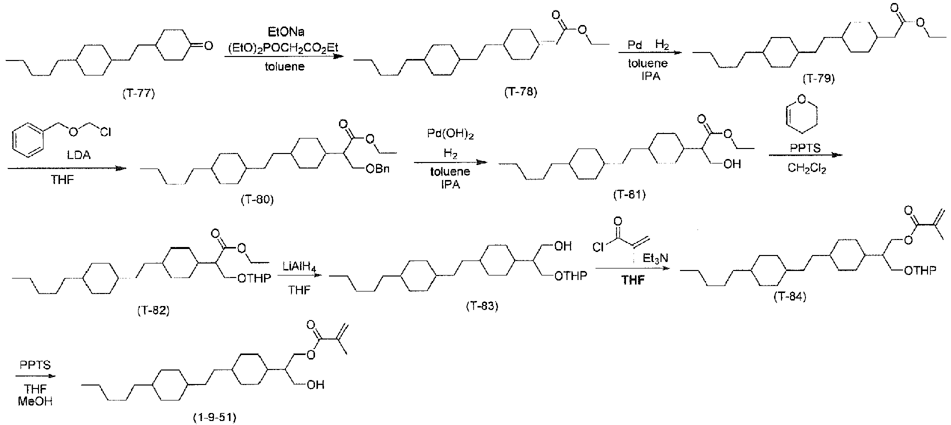

### First step

Compound (T-77) (50.0 g), triethyl phosphonoacetate (48.3 g) and toluene (500 mL) were put in a reaction vessel, and the resulting mixture was cooled to 0°C. Sodium ethoxide (20% ethanol solution) (73.3 g) was slowly added dropwise thereto, and the resulting mixture was stirred for 6 hours while returning to room temperature. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 9 : 1 in a volume ratio) to obtain compound (T-78) (60.7 g; 97%).

### Second step

Compound (T-78) (60.7 g), Pd/C (0.51 g), toluene (500 mL) and IPA (50 mL) were put in a reaction vessel, and the resulting mixture was stirred under a hydrogen atmosphere for 12 hours. After an insoluble matter was filtered off, the reaction mixture was poured into water, and an aqueous layer was subjected to extraction with toluene. Organic layers combined were washed with water, and dried over anhydrous magnesium sulfate. The resulting solution was concentrated under reduced pressure, and the residue was purified by silica gel chromatography (toluene : heptane = 9 : 1 in a volume ratio), and the resulting solution was further purified by recrystallization from Solmix to obtain compound (T-79) (33.6 g; 55%).

### Third step

Compound (T-80) (38.8 g; 86%) was obtained by using compound (T-79) (33.6 g) as a raw material in a manner similar to the technique in the sixth step in Synthesis Example 16.

### Fourth step

Compound (T-81) (29.8 g; 95%) was obtained by using compound (T-80) (38.8 g) as a raw material in a manner similar to the technique in the seventh step in Synthesis Example 16.

### Fifth step

Compound (T-82) (34.6 g; 95%) was obtained by using compound (T-81) (29.8 g) as a raw material in a manner similar to the technique in the eighth step in Synthesis Example 16.

### Sixth step

Compound (T-83) (30.5 g; 97%) was obtained by using compound (T-82) (34.6 g) as a raw material in a manner similar to the technique in the ninth step in Synthesis Example 16.

### Seventh step

Compound (T-84) (26.6 g; 75%) was obtained by using compound (T-83) (30.5 g) as a raw material in a manner similar to the technique in the tenth step in Synthesis Example 16.

### Eighth step

Compound (1-9-51) (18.3 g; 83%) was obtained by using compound (T-84) (26.6 g) as a raw material according to the eleventh step in Synthesis Example 16.

An NMR analysis value of compound (1-9-51) obtained is as described below.
¹H-NMR: Chemical shifts δ (ppm; CDCl₃) : 6.10 (s, 1H), 5.57 (s, 1H), 4.40 - 4.37 (m, 1H), 4.24 - 4.20 (m, 1H), 3.69 - 3.61 (m, 2H), 1.9 - 1.94 (m, 4H), 1.78 - 1.58 (m, 9H), 1.43 - 1.37 (m, 1H), 1.32 - 1.02 (m, 17H), 0.90 - 0.79 (m, 9H).

Physical properties of compound (1-9-51) were as described below. Transition temperature: C 54.5 S_{A} 81.0 I.

### Synthesis Example 23

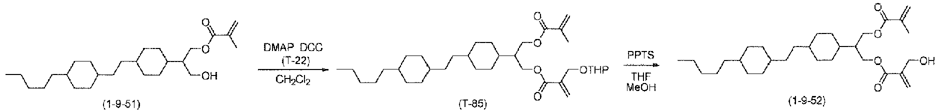

### First step

Compound (T-85) (3.3 g; 78%) was obtained by using compound (1-9-51) (3.0 g) as a raw material in a manner similar to the technique in the third step in Synthesis Example 5.

### Second step

Compound (1-9-52) (2.1 g; 75%) was obtained by using compound (T-85) (3.3 g) as a raw material in a manner similar to the technique in the fourth step in Synthesis Example 5.

An NMR analysis value of compound (1-9-52) obtained is as described below. ¹H-NMR: Chemical shifts δ (ppm; CDCl₃) : 6.24 (s, 1H), 6.09 (s, 1H), 5.84 (s, 1H), 5.56 (s, 1H), 4.33 - 4.28 (m, 4H), 4.20 - 4.16 (m, 2H), 2.28 (t, J= 6.6 Hz, 1H), 1.97 - 1.91 (m, 4H), 1.79 - 1.69 (m, 8H), 1.47 - 1.41 (m, 1H), 1.32 - 1.07 (m, 17H), 0.90 - 0.82 (m, 9H) .

Physical properties of compound (1-9-52) were as described below. Transition temperature: C 64.0 I.

### Synthesis Example 24

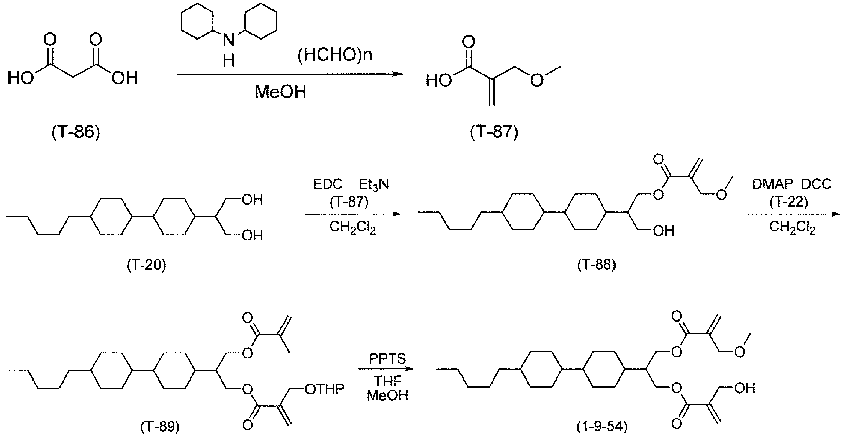

### First step

Compound (T-86) (50.0 g), paraformaldehyde (31.7 g), dicyclohexylamine (95.7 mL) and methanol (500 mL) were put in a reaction vessel, and the resulting mixture was stirred at 65°C for 5 hours. After the reaction mixture was concentrated under reduced pressure, the resulting mixture was poured into water, and an aqueous layer was washed with t-butyl methyl ether. Then, 3N hydrochloric acid (200 mL) was added to the aqueous layer, and precipitated salt was removed by filtration. An aqueous layer obtained was subjected to extraction with ethyl acetate, and dried over anhydrous magnesium sulfate. The resulting solution was concentrated under reduced pressure, and purified by distillation to obtain compound (T-87) (40.4 g; 72%).

### Second step

Compound (T-20) (8.00 g), compound (T-87) (3.29 g) and dichloromethane (320 mL) were put in a reaction vessel, and the resulting mixture was cooled to 0°C. Thereto, 1-(3-dimethylaminopropyl)-3-ethylcarbodiimide hydrochloride (EDC) (5.43 g) and triethylamine (6.1 mL) were added, and the resulting mixture was stirred for 12 hours while returning to room temperature. The reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (toluene : ethyl acetate = 3 : 1 in a volume ratio) to obtain compound (T-88) (4.72 g; 40%).

### Third step

Compound (T-88) (4.72 g), compound (T-22) (2.58 g), DMAP (0.71 g) and dichloromethane (75 mL) were put in a reaction vessel, and the resulting mixture was cooled to 0°C. A dichloromethane (20 mL) solution of DCC (3.58 g) was slowly added dropwise thereto, and the resulting mixture was stirred for 12 hours while returning to room temperature. After an insoluble matter was filtered off, the reaction mixture was poured into water, and an aqueous layer was subjected to extraction with dichloromethane. Organic layers combined were washed with water, and dried over anhydrous magnesium sulfate. The resulting solution was concentrated under reduced pressure, and the residue was purified by silica gel chromatography (toluene : ethyl acetate = 10 : 1 in a volume ratio) to obtain compound (T-89) (6.50 g; 98%).

### Fourth step

Compound (T-89) (6.50 g), pyridinium p-toluenesulfonate (PPTS) (1.41 g), THF (50 mL) and methanol (50 mL) were put in a reaction vessel, and the resulting mixture was stirred at 50 °C for 4 hours. The reaction mixture was poured into water, and an aqueous layer was subjected to extraction with ethyl acetate. Organic layers combined were washed with water, and dried over anhydrous magnesium sulfate. The resulting solution was concentrated under reduced pressure, and the residue was purified by silica gel chromatography (toluene : ethyl acetate = 3 : 1 in a volume ratio), and the resulting solution was further purified by recrystallization from heptane to obtain compound (1-9-54) (4.69 g; 85%).

An NMR analysis value of compound (1-9-54) obtained is as described below. ¹H-NMR: Chemical shifts δ (ppm; CDCl₃) : 6.30 (s, 1H), 6.25 (s, 1H), 5.86 (d, J = 1.4 Hz, 1H), 5.85 (s, 1H), 4.38 - 4.29 (m, 4H), 4.24 - 4.16 (m, 2H), 4.12 (s, 2H), 3.40 (s, 3H), 2.34 - 2.29 (m, 1H), 1.97 - 1.90 (m, 1H), 1.85 - 1.64 (m, 8H), 1.47 - 1.38 (m, 1H), 1.33 - 1.18 (m, 6H), 1.18 - 0.78 (m, 16H).

Physical properties of compound (1-9-54) were as follows. Transition temperature: C 37.3 I.

### Comparative Example 1

As a comparative compound, compound (S-1) was prepared, and characteristics were measured. The compound was selected because the compound is described in WO 2014/090362 A, and is similar to the compound of the invention.

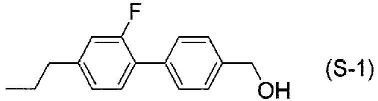

An NMR analysis value of comparative compound (S-1) was as described below.
¹H-NMR: Chemical shifts δ (ppm; CDCl₃) : 7.57 - 7.52 (m, 2H), 7.45 - 7.42 (m, 2H), 7.36 - 7.30 (m, 1H), 7.04 - 6.95 (m, 2H), 4.75 (d, 6.0 Hz, 2H), 2.62 (t, J = 7.8 Hz, 2H), 1.75 - 1.64 (m, 3H), 0.98 (t, J = 7.4 Hz, 3H).

Vertical alignability and voltage holding ratios of compound (No. 1-2-1) and compound (No. 1-6-1) and comparative compound (S-1) were compared. In addition, for evaluation, composition (i) and polymerizable compound (RM-1) were used.

A proportion of components of composition (i) is expressed in terms of weight percent (% by weight).

| | |
|---|---|
| 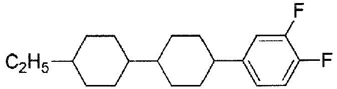 | 16.67% |
| 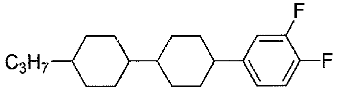 | 16.67% |
| 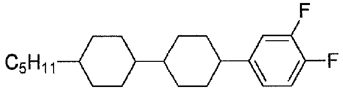 | 16.67% |
| 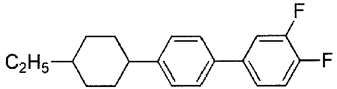 | 6.25% |
| 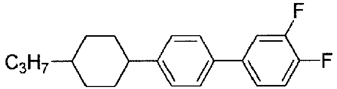 | 6.25% |
| 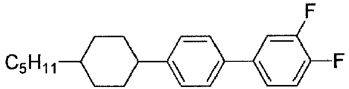 | 12.50% |
| 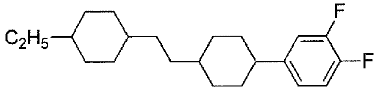 | 12.50% |
| 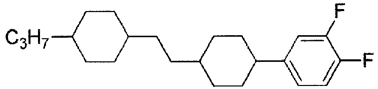 | 6.25% |
| 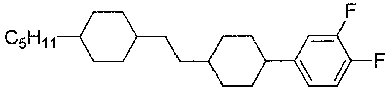 | 6.25% |

Polymerizable compound (RM-1) is shown below.

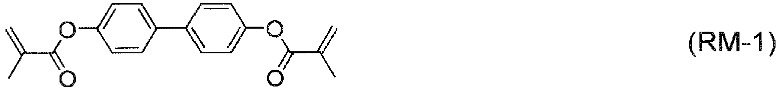

### Vertical alignability

Polymerizable compound (RM-1) was added to composition (i) in a proportion of 0.4% by weight. Compound (1-2-1), compound (1-6-1) or comparative compound (S-1) was added thereto in a proportion of 3.0% by weight. The resulting mixture was injected into a device having no alignment film in which a distance (cell gap) between two glass substrates was 3.5 micrometers. The polymerizable compound was polymerized by irradiation with ultraviolet light (20J) by using Black Light F40T10/BL (peak wavelength: 369 nm) made by EYE GRAPHICS CO., LTD. The device was charged by applying a pulse voltage (60 microseconds at 1 V) at 60°C. A decaying voltage was measured for 16.7 milliseconds with a high-speed voltmeter, and area A between a voltage curve and a horizontal axis in a unit cycle was determined. Area B is an area without decay. The voltage holding ratio is expressed in terms of a percentage of area A to area B. The device was set to the polarizing microscope, and the device was irradiated with light from below, and presence or absence of light leakage was observed. When liquid crystal molecules were sufficiently aligned and no light passed through the device, vertical alignability was determined as "good." When light passing through the device was observed, vertical alignability was expressed as "poor."

### Table 2

**Table 2 Physical properties of compound (1-2-1), compound (1-6-1) and comparative compound (S-1)**

| | 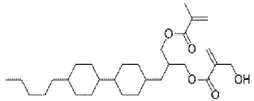 | 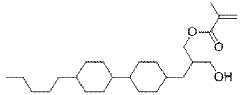 | 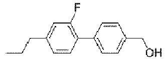 |
|---|---|---|---|
| | Compound (1-2-1) | Compound (1-6-1) | Compound (S-1) |
| Vertical alignability | Good | Good | Good |
| Voltage holding ratio (VHR) | 83.1% | 86.6% | 29.6% |

Physical properties of compound (1-2-1) in Synthesis Example 1, compound (1-6-1) and comparative compound (S-1) are summarized in Table 2. Both the compounds exhibited good vertical alignability in the device having no alignment film. Meanwhile, when compound (1-2-1) and compound (1-6-1) were used, the voltage holding ratio is higher in comparison with the case of using comparative compound (S-1). The reason is that, while the polar compound having such an -OH group as in comparative compound (S-1) significantly reduces the voltage holding ratio of the device, reduction of the voltage holding ratio is suppressed by incorporation of the polar compound into the polymer formed by the polymerizable compound by providing the polar compound with polymerizability as in compound (1-2-1) and compound (1-6-1). Accordingly, compound (1-2-1) and compound (1-6-1) are reasonably a superb compound that exhibits good vertical alignability without reducing the voltage holding ratio of the device.

### Comparative Example 2

### Vertical alignability

Polymerizable compound (RM-1) was added to composition (i) in a proportion of 0.4% by weight. Compound (1-1-2), compound (1-6-1) or comparative compound (S-1) was added thereto in a proportion of 0.3% by weight to 5.0% by weight. The resulting mixture was injected into a device having no alignment film, in which a distance (cell gap) between two glass substrates was 3.5 micrometers. The polymerizable compound was polymerized by irradiation (20J) with ultraviolet light by using Black Light F40T10/BL (peak wavelength: 369 nm) made by EYE GRAPHICS CO., LTD. The device was set to a polarizing microscope, and the device was irradiated with light from below, and presence or absence of light leakage was observed. When liquid crystal molecules were sufficiently aligned and no light passed through the device, vertical alignability was determined as "good." When light passing through the device was observed, vertical alignability was expressed as "poor."

### Table 3

**Table 3 Physical properties of compound (1-10-1) and comparative compound (S-1)**

| Addition concentration (% by weight) | 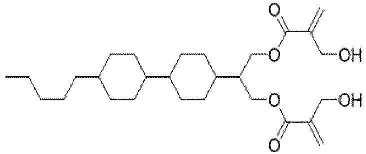 | 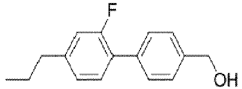 |
|---|---|---|
| | Compound (1-10-1) | Compound (S-1) |
| 0.30% | Good | Poor |
| 0.50% | Good | Poor |
| 1% | Good | Poor |
| 3% | Good | Good |
| 5% | Good | Good |

Physical properties of the compound (1-10-1) in Synthesis Example 12 and comparative compound (S-1) are summarized in Table 3. While good vertical alignability was not confirmed unless comparative compound (S-1) was added to the liquid crystal composition in a proportion of 3% by weight or more, good vertical alignability was confirmed when compound (1-10-1) was added to the liquid crystal composition in a proportion of 0.3% by weight or more. Accordingly, compound (1-10-1) is reasonably a superb compound that vertically aligns at a concentration lower than the concentration of comparative compound (S-1).

According to the synthesis method described in Example 1, compounds (1-1-1) to (1-1-20), compounds (1-2-1) to (1-2-200), compounds (1-3-1) to (1-3-140), compounds (1-4-1) to (1-4-134), (1-5-1) to (1-5-20), compounds (1-6-1) to (1-6-180), compounds (1-7-1) to (1-7-140), compounds (1-8-1) to (1-8-134), compounds (1-9-1) to (1-9-80), compounds (1-10-1) to (1-10-180), compound (1-11-1) to (1-11-140) and compounds (1-12-1) to (1-12-220) described below can be prepared.

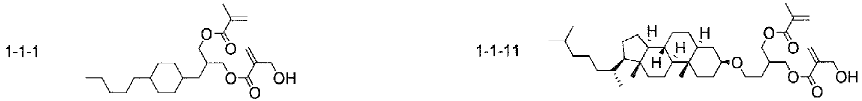

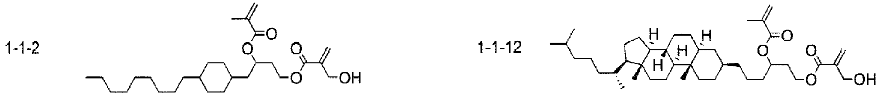

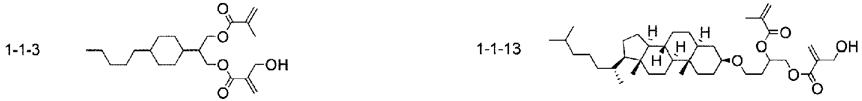

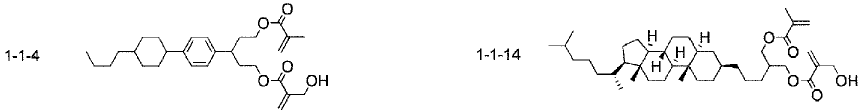

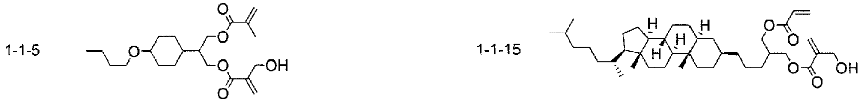

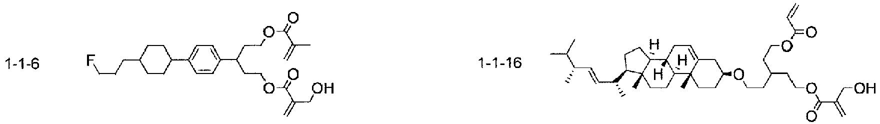

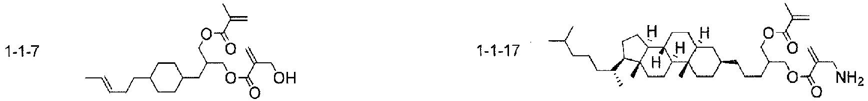

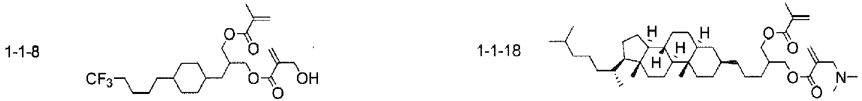

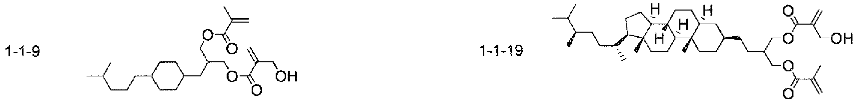

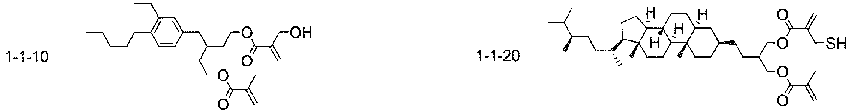

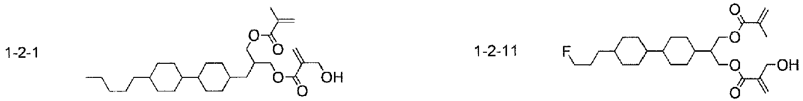

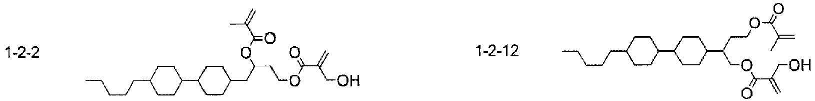

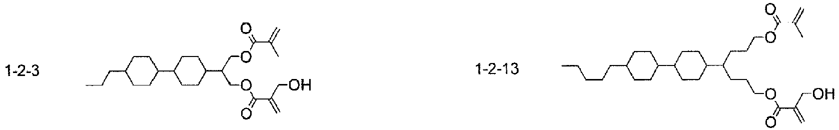

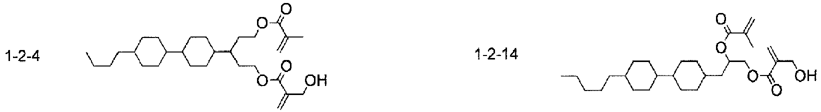

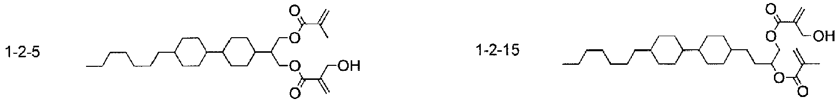

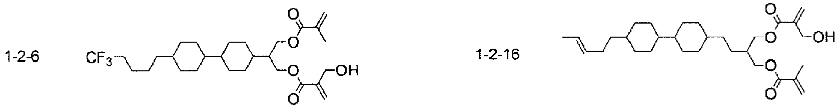

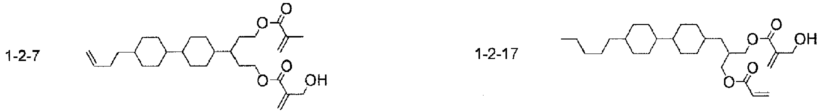

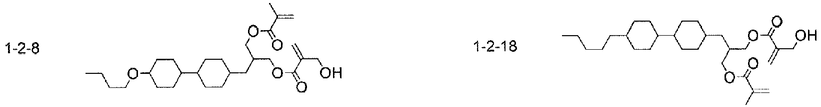

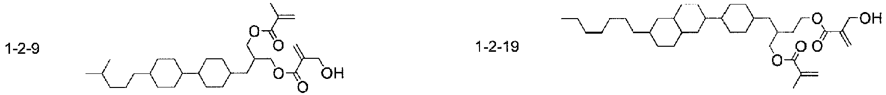

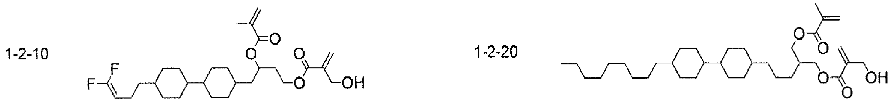

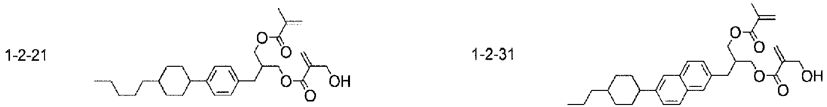

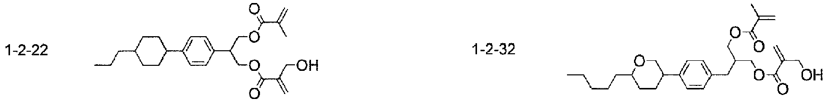

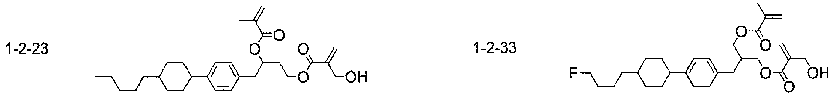

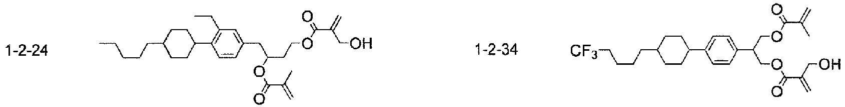

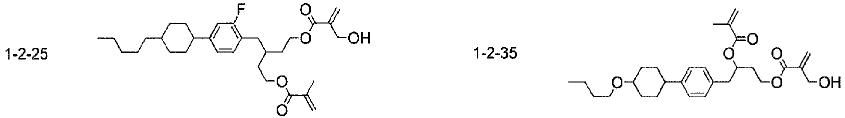

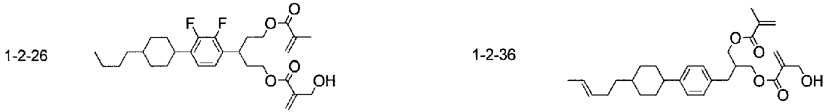

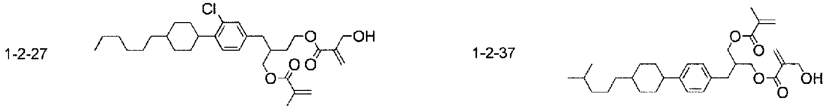

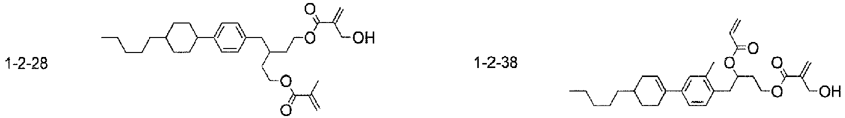

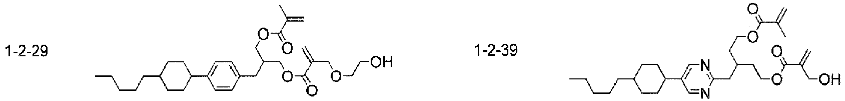

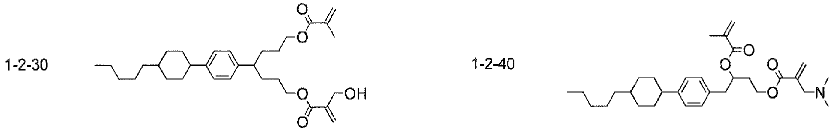

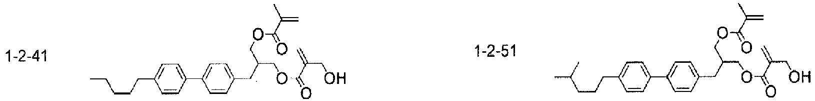

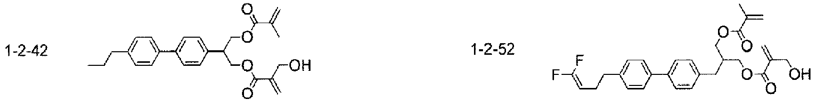

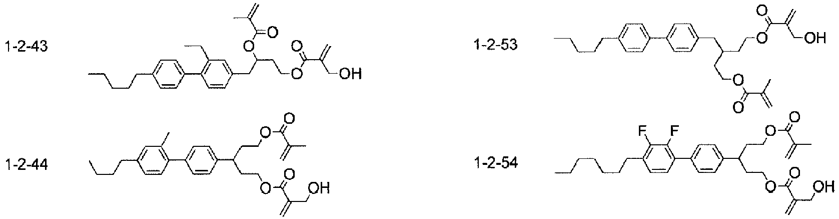

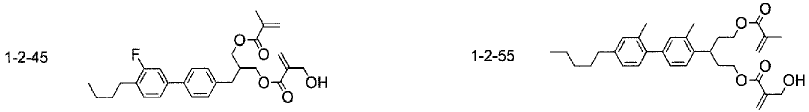

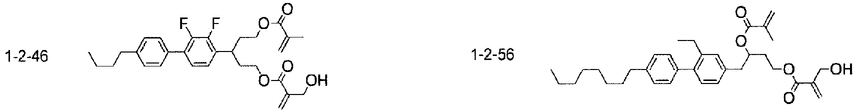

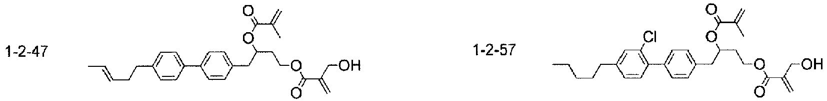

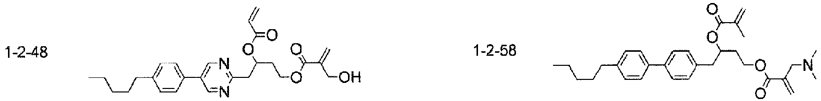

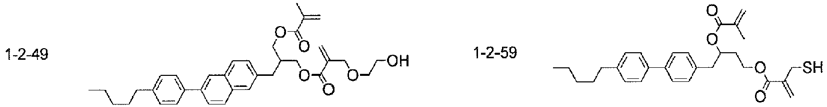

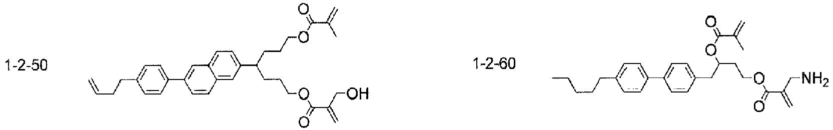

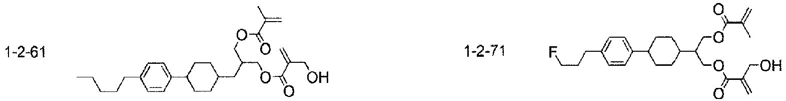

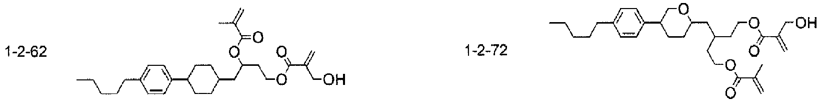

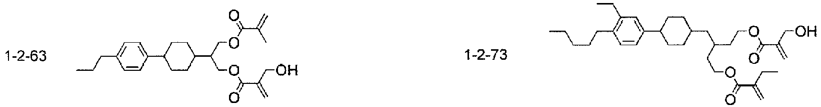

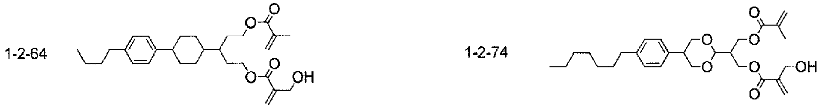

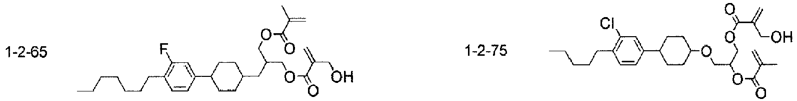

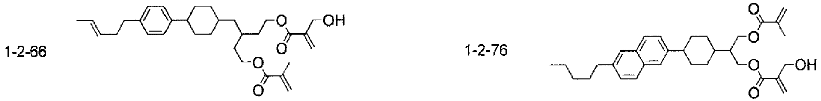

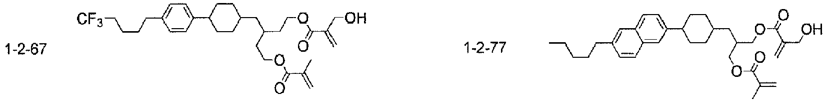

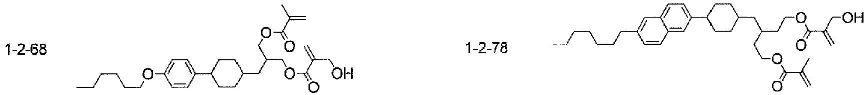

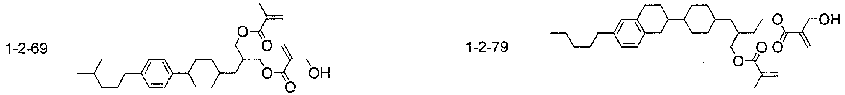

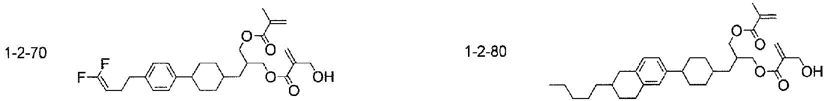

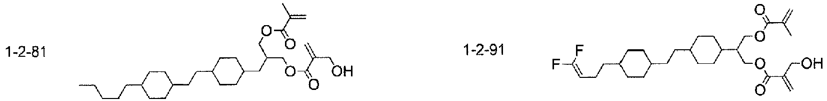

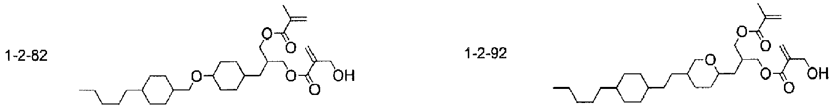

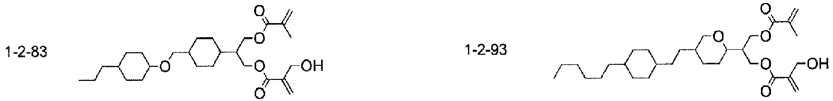

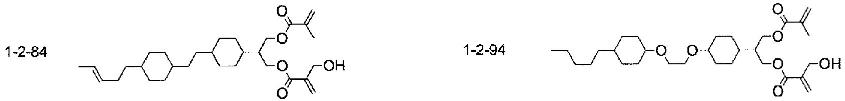

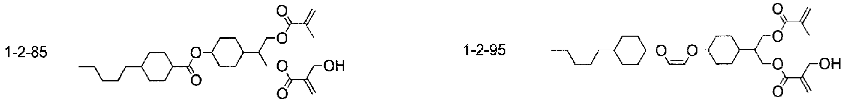

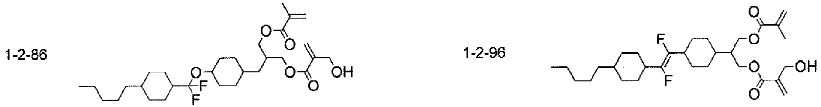

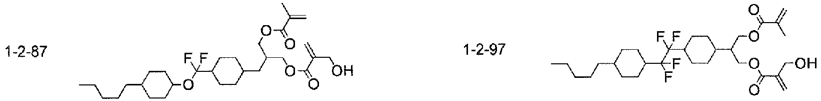

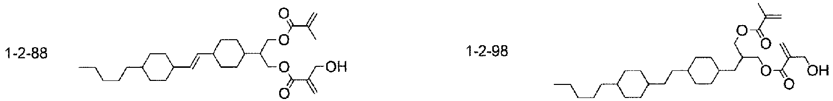

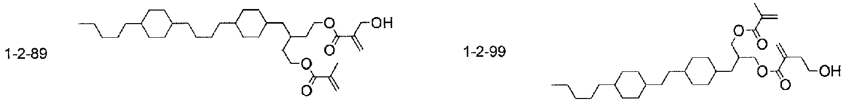

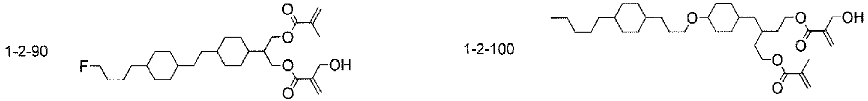

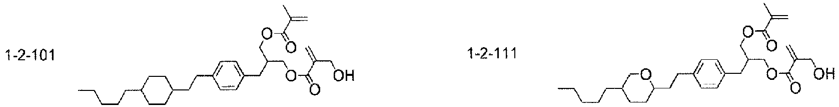

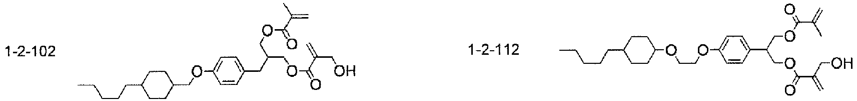

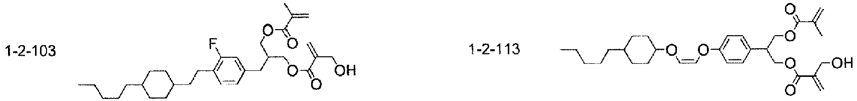

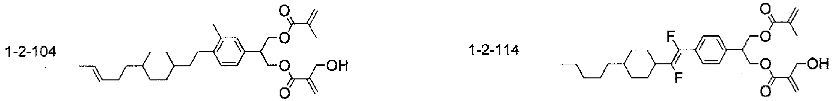

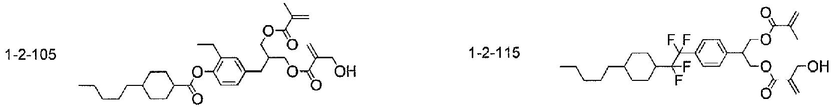

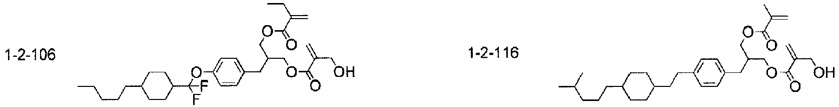

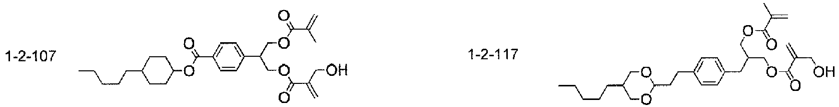

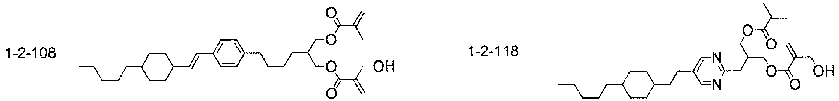

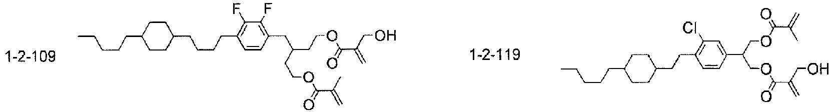

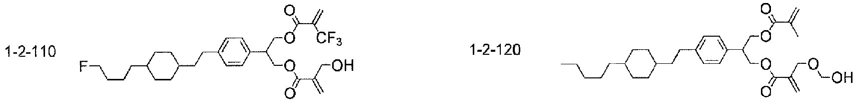

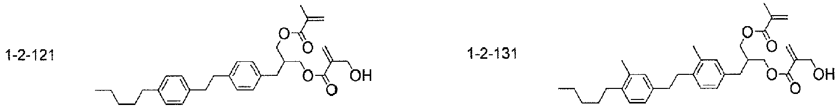

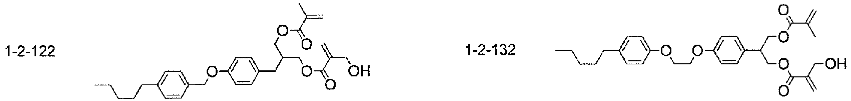

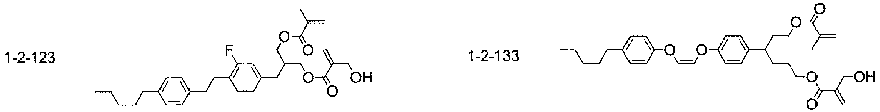

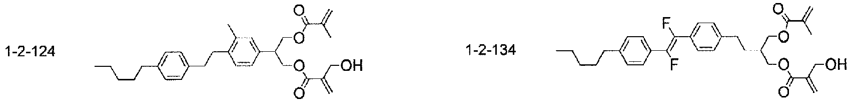

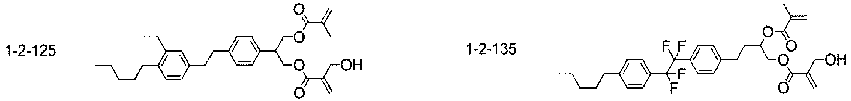

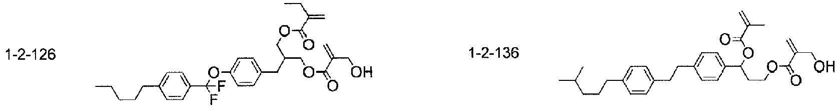

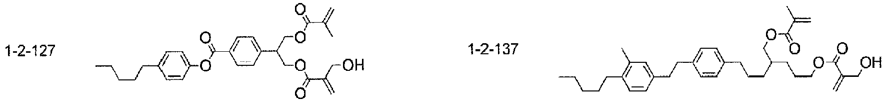

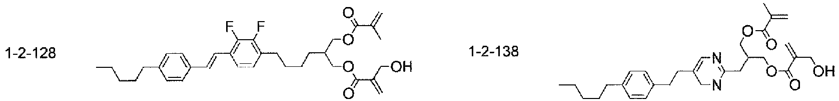

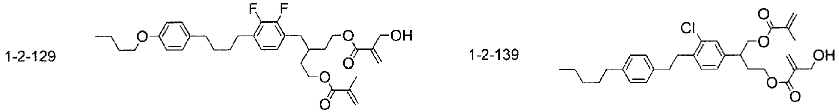

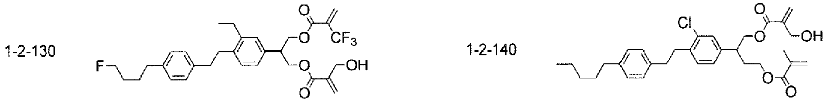

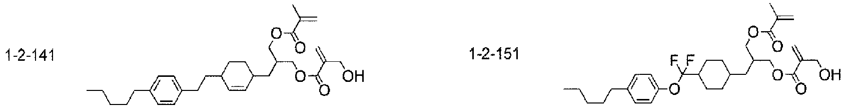

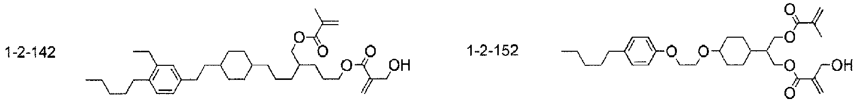

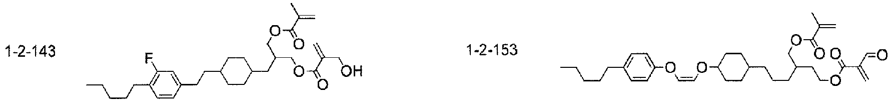

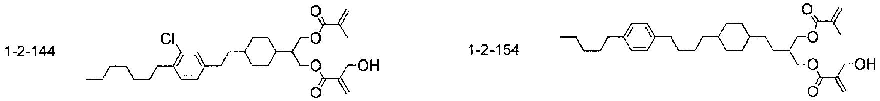

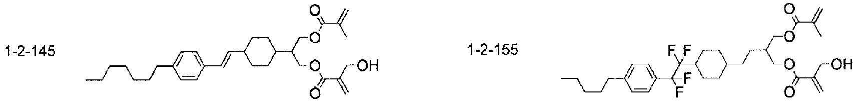

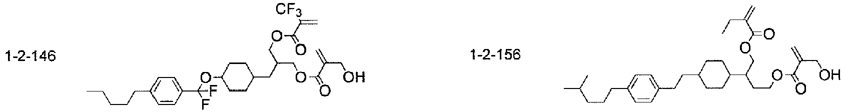

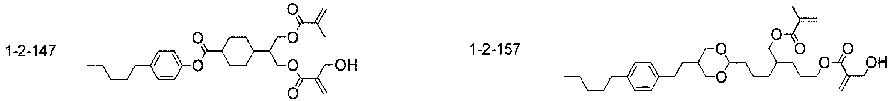

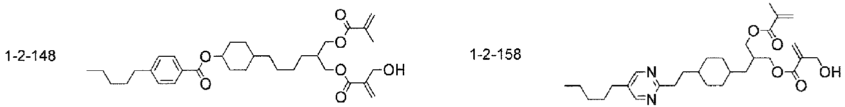

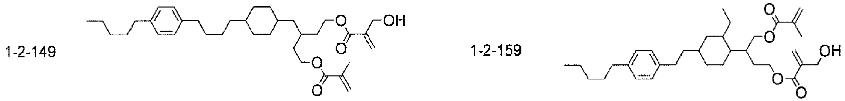

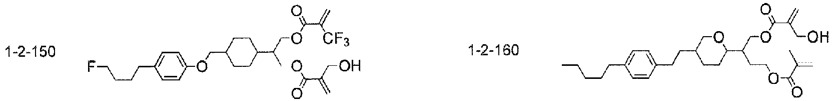

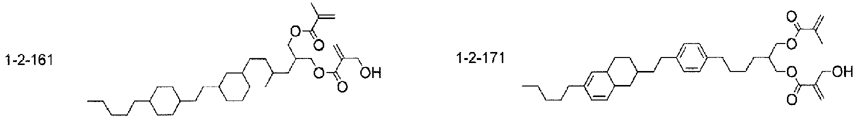

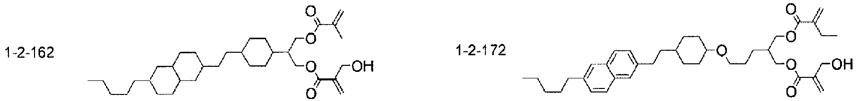

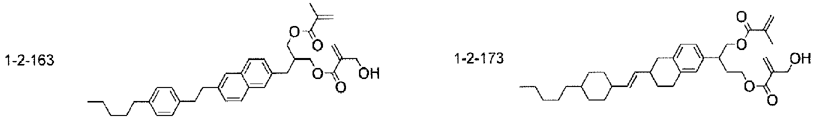

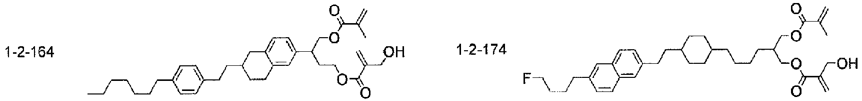

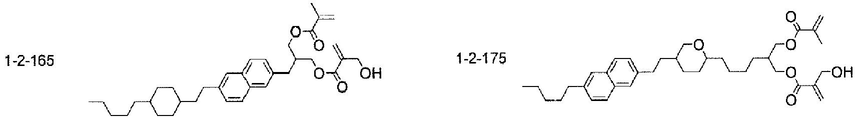

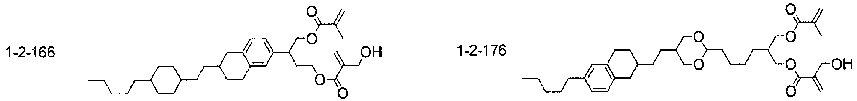

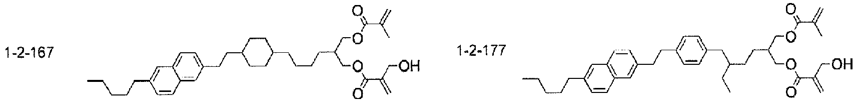

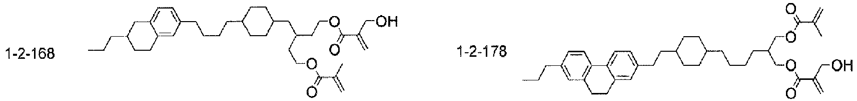

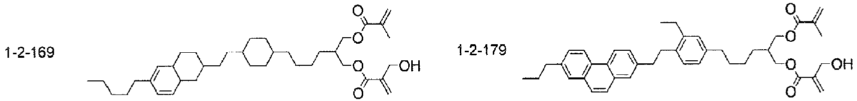

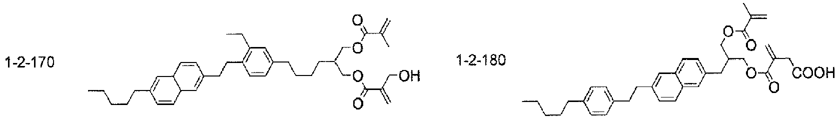

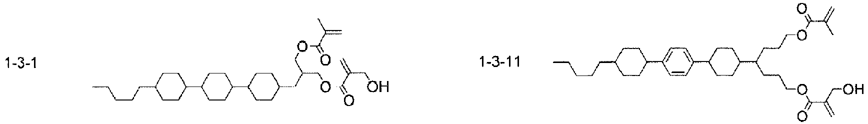

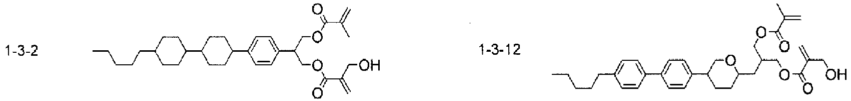

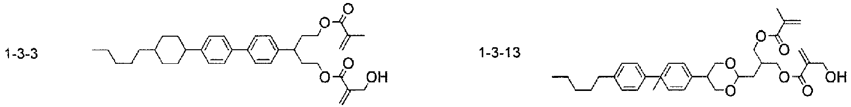

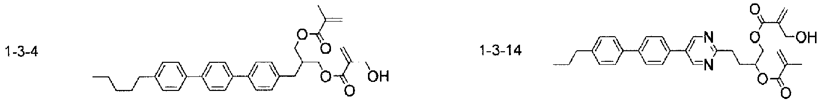

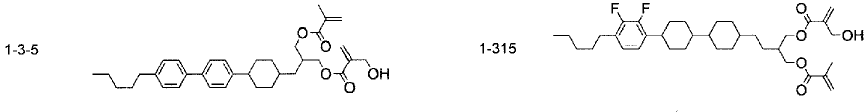

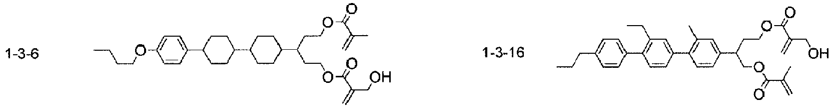

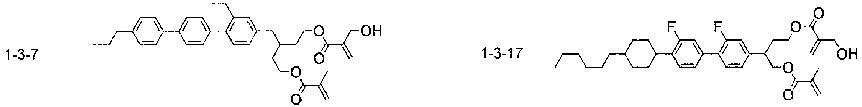

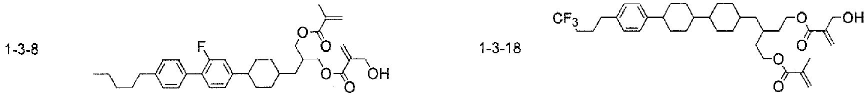

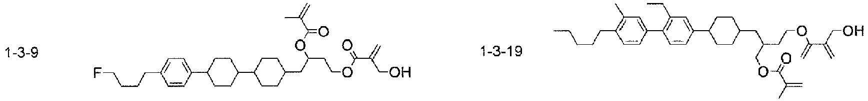

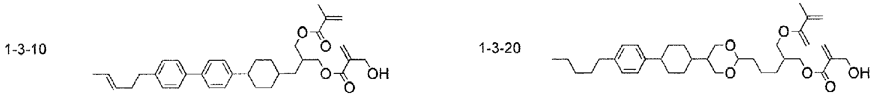

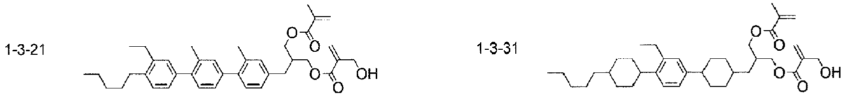

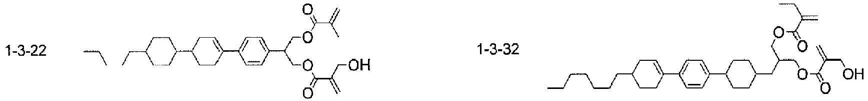

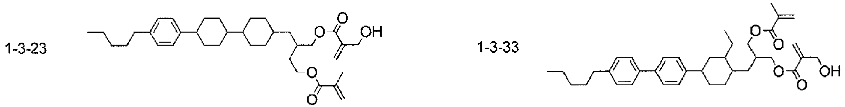

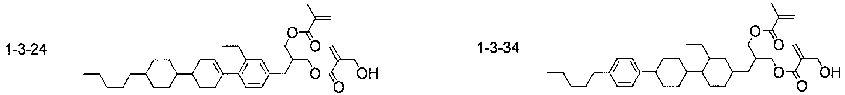

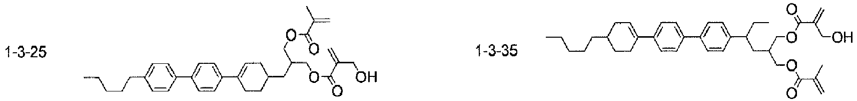

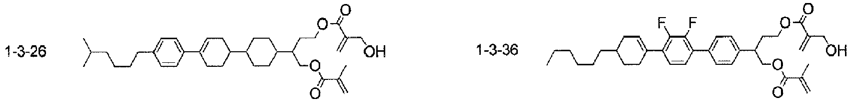

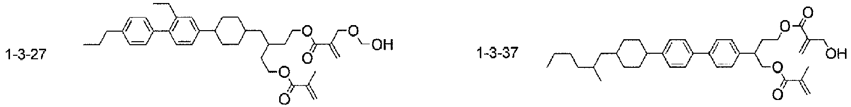

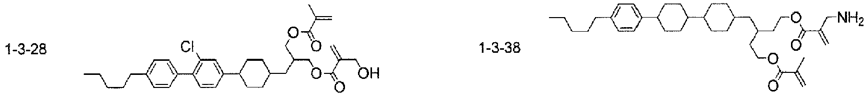

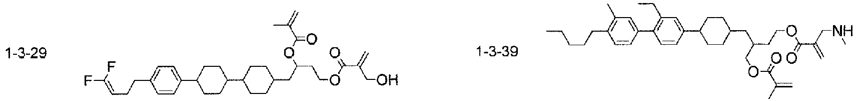

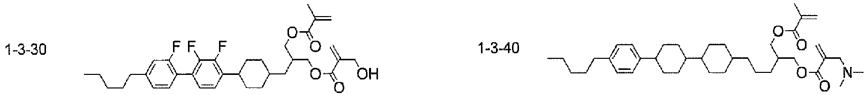

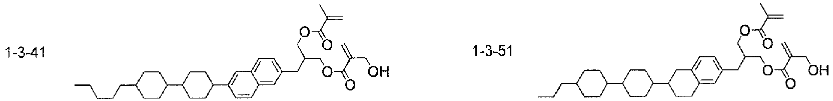

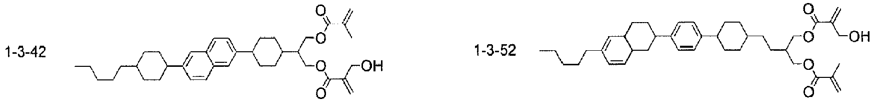

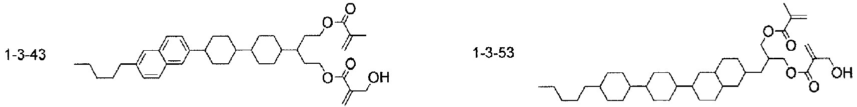

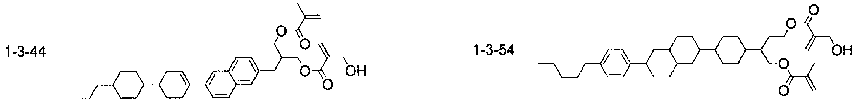

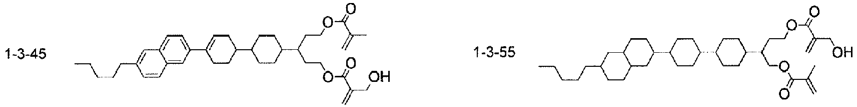

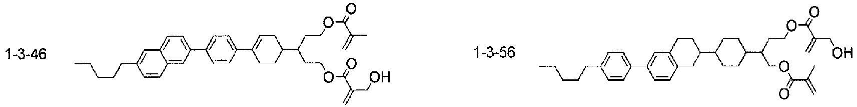

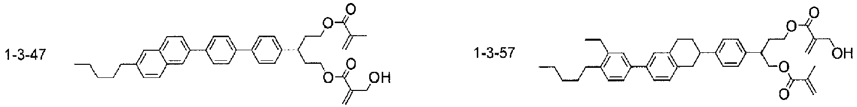

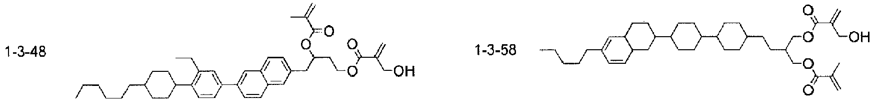

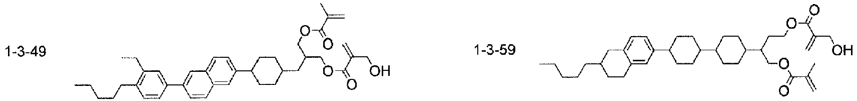

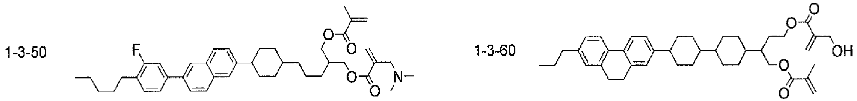

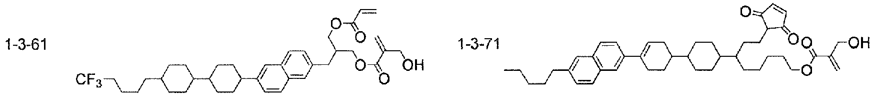

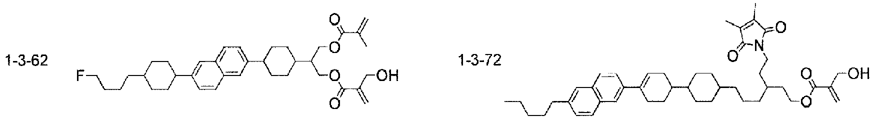

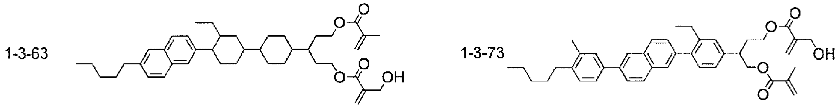

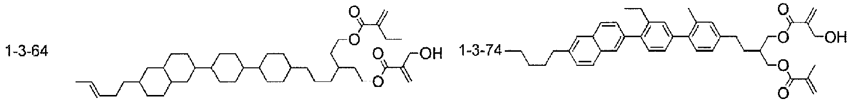

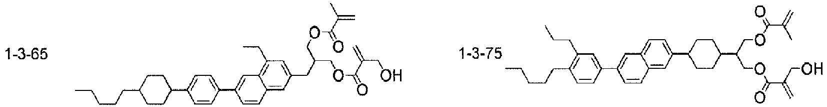

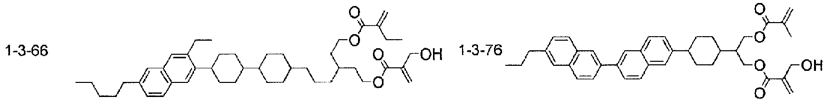

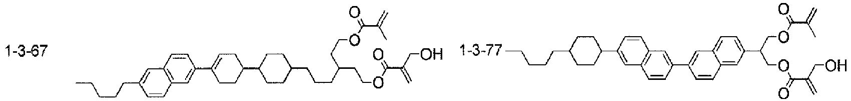

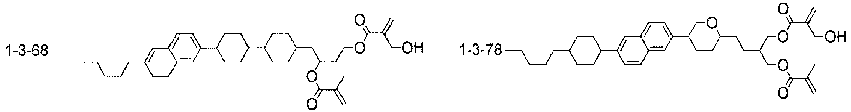

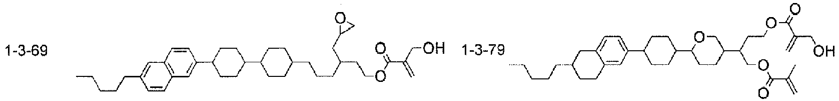

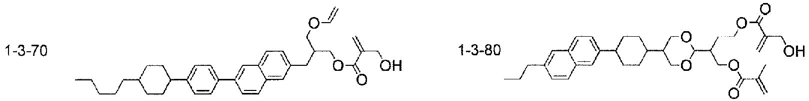

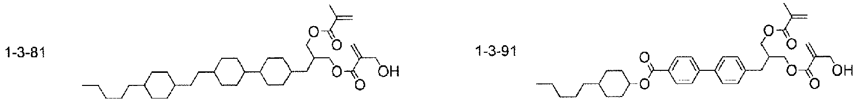

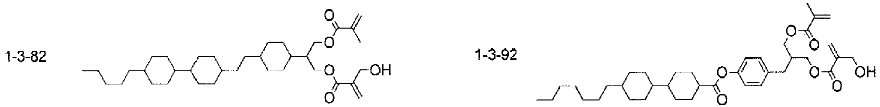

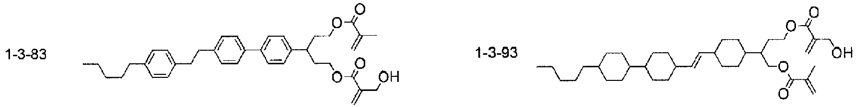

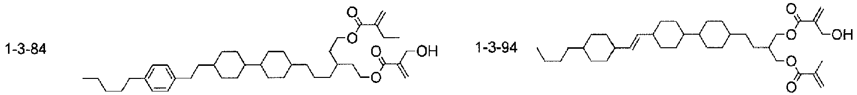

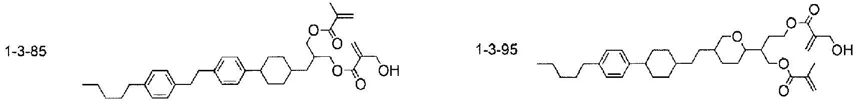

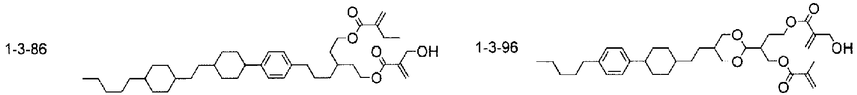

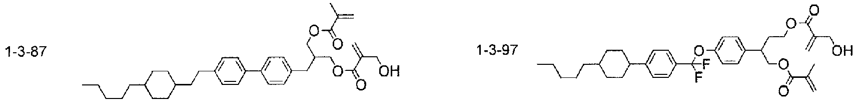

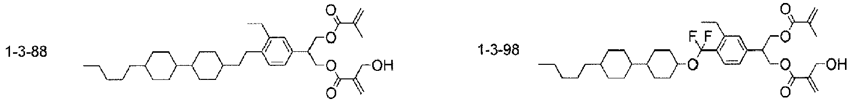

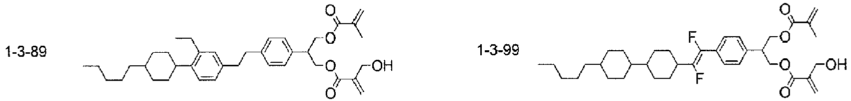

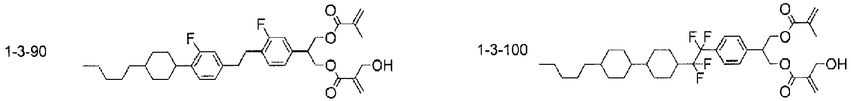

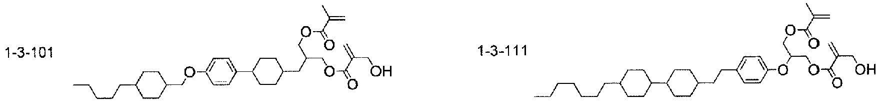

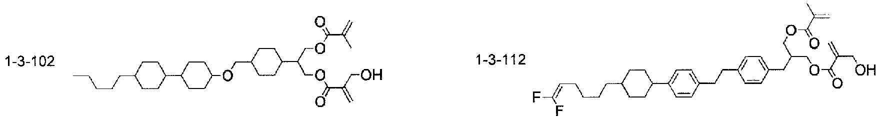

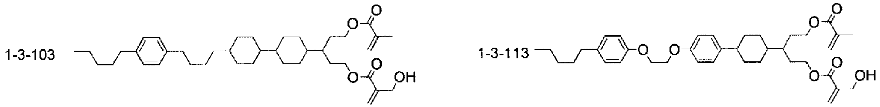

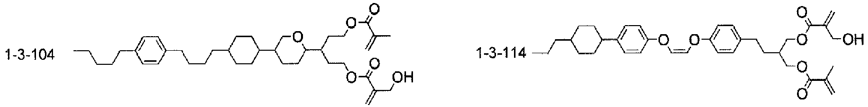

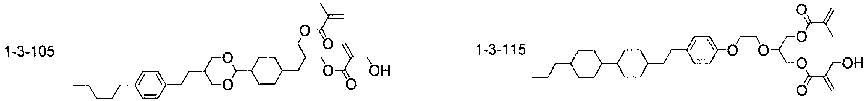

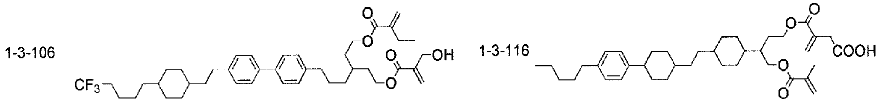

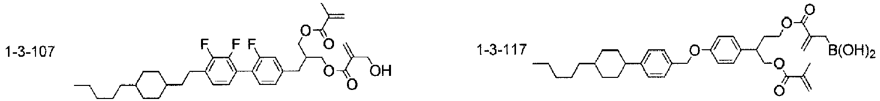

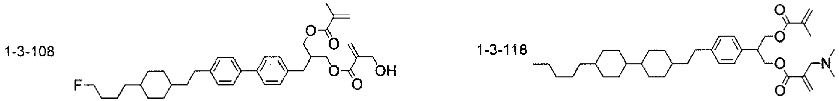

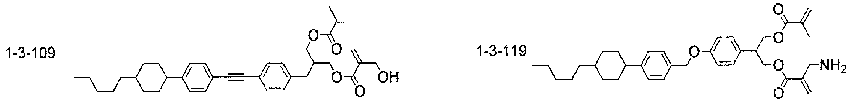

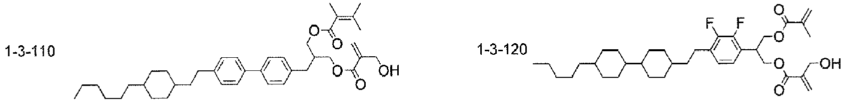

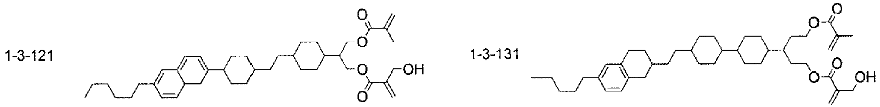

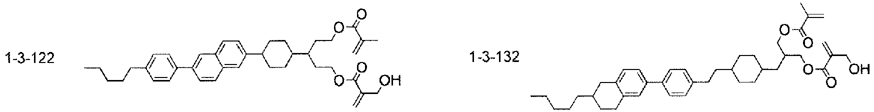

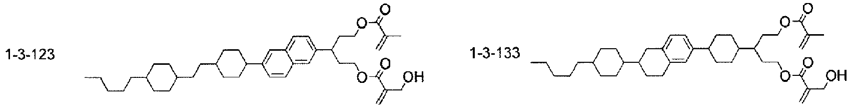

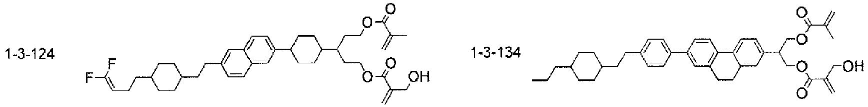

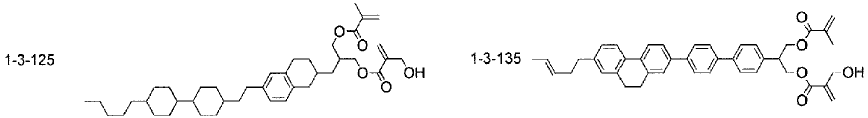

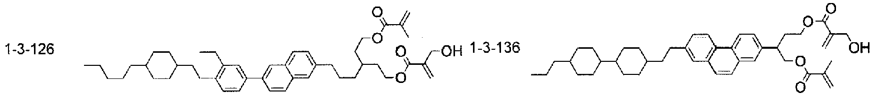

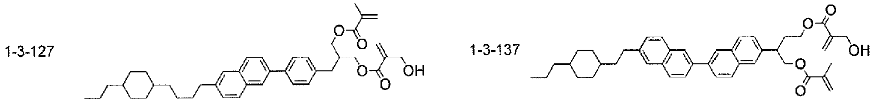

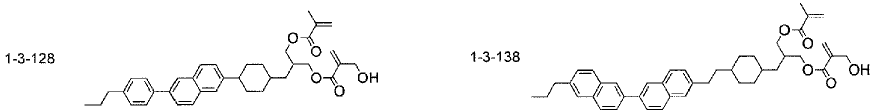

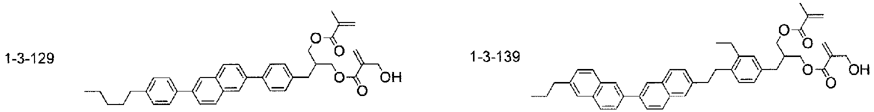

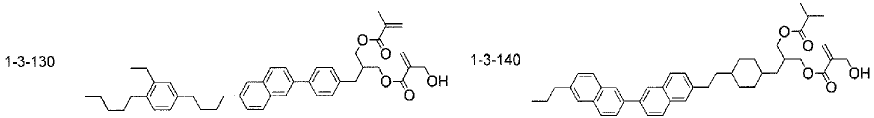

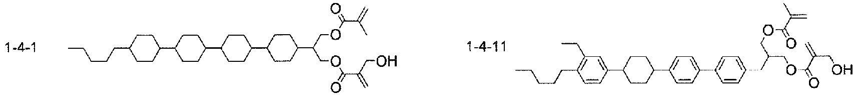

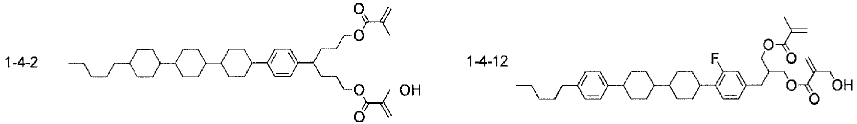

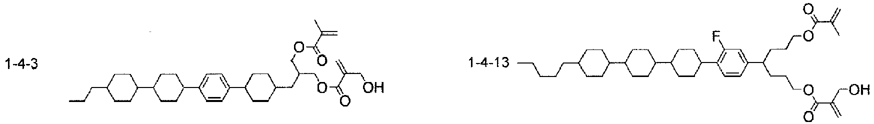

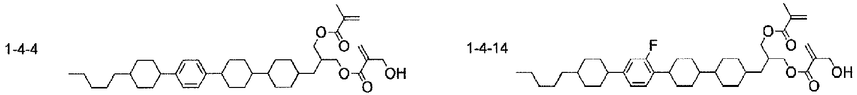

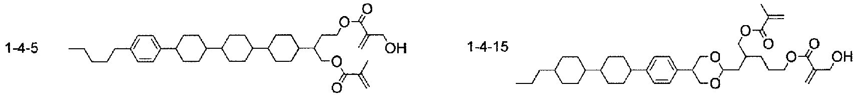

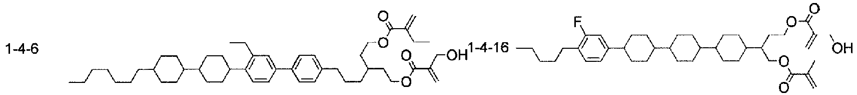

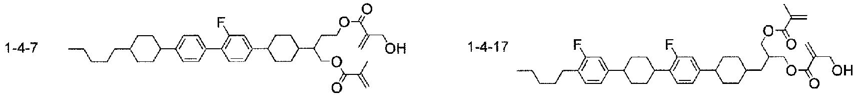

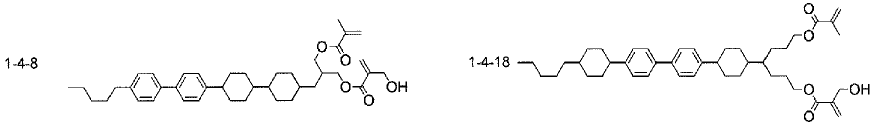

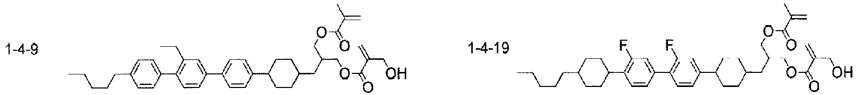

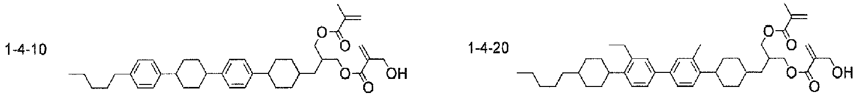

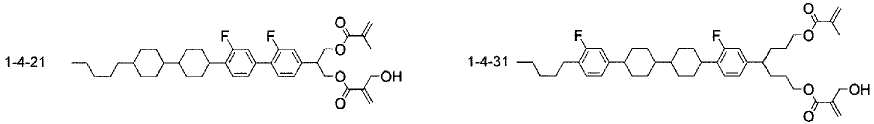

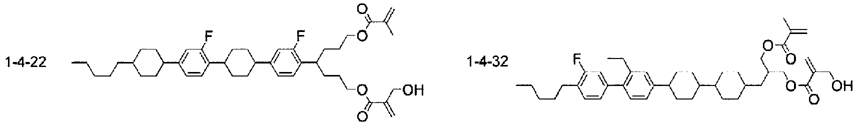

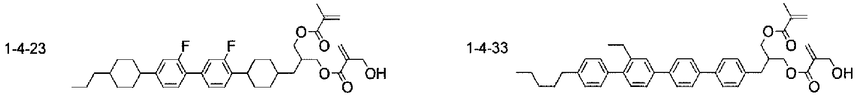

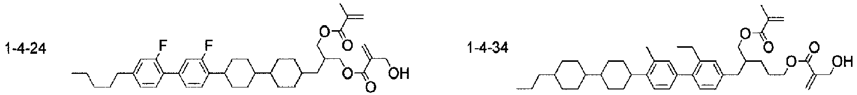

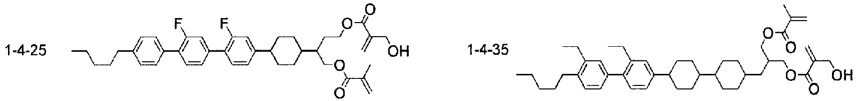

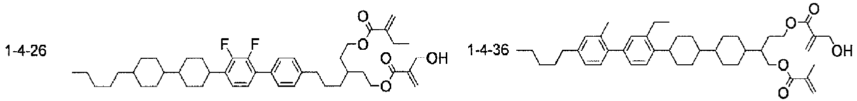

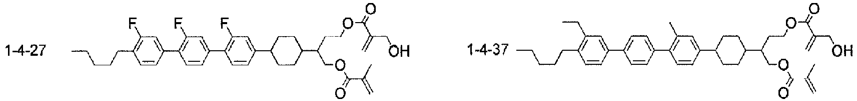

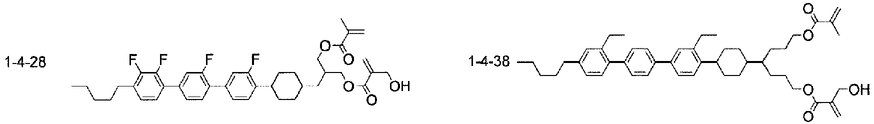

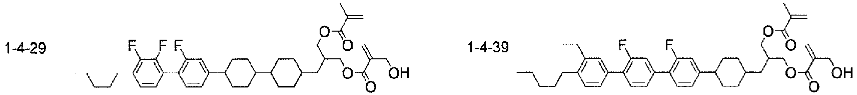

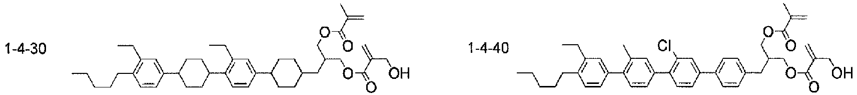

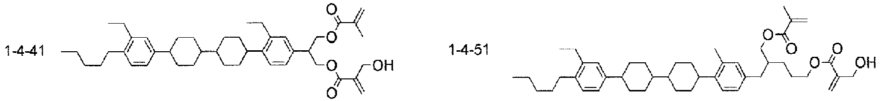

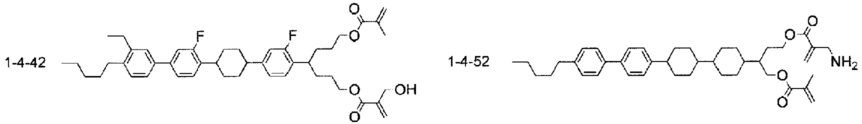

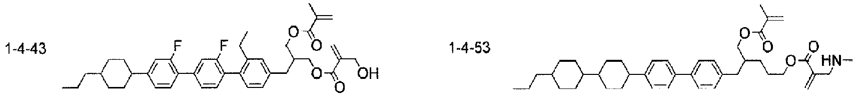

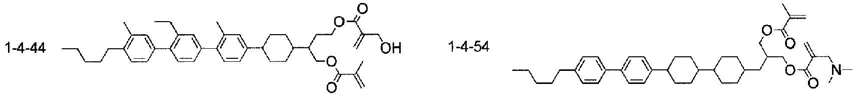

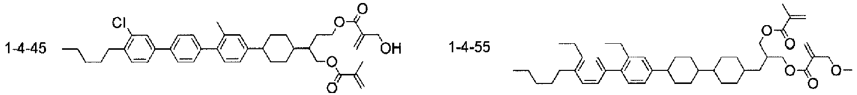

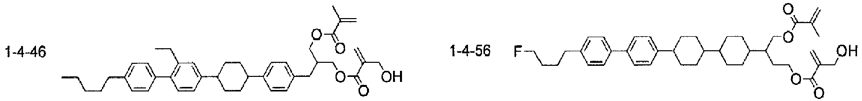

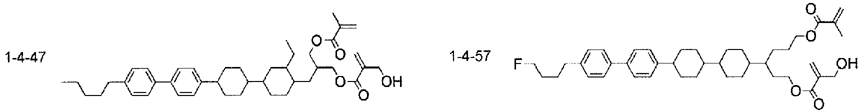

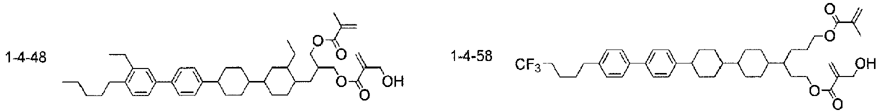

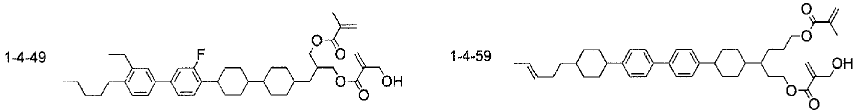

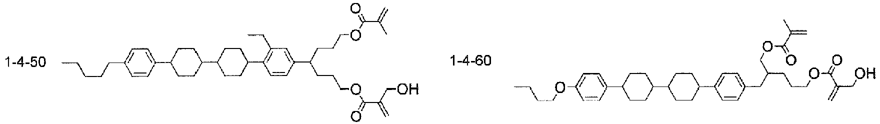

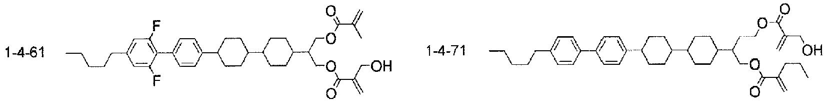

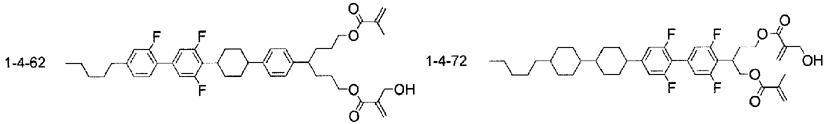

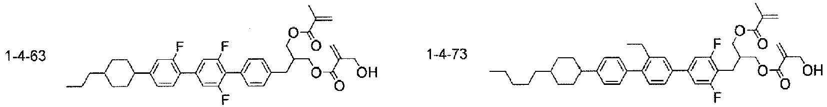

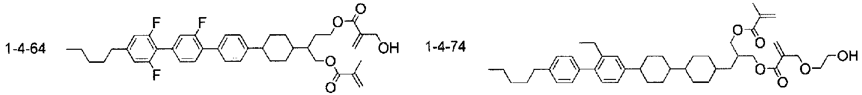

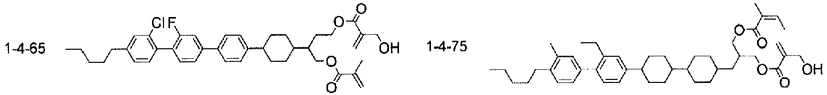

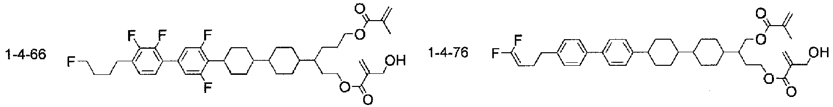

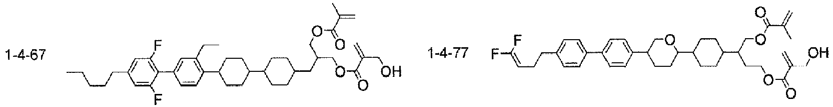

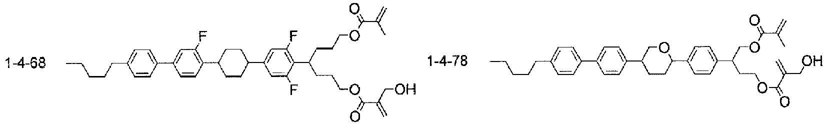

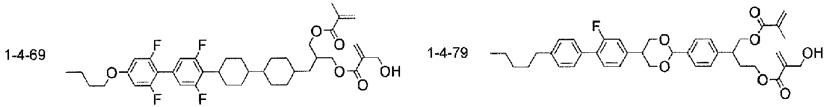

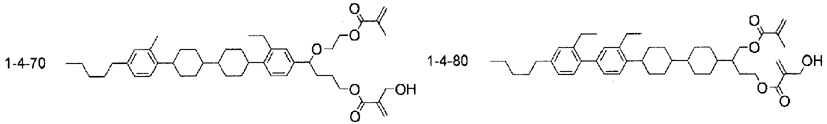

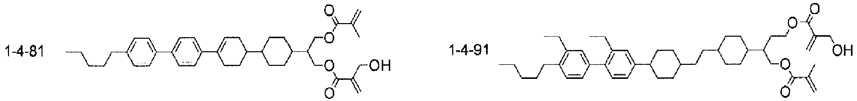

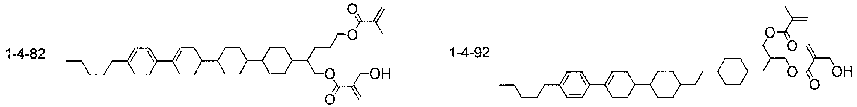

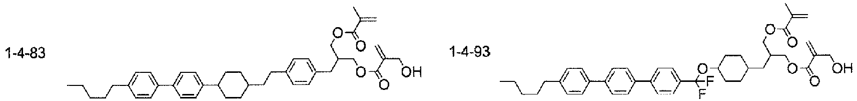

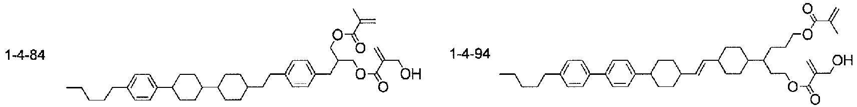

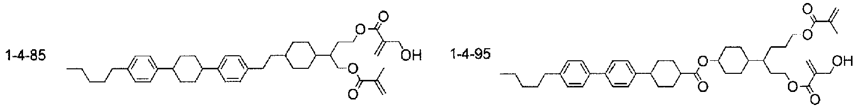

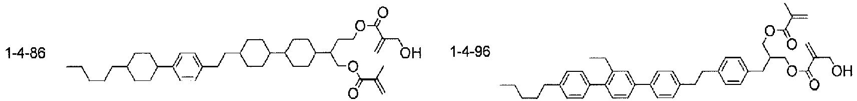

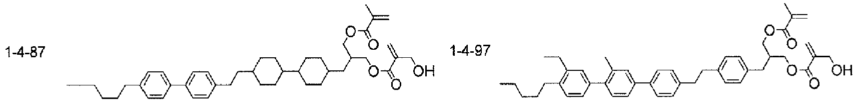

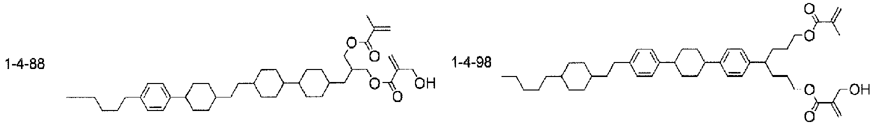

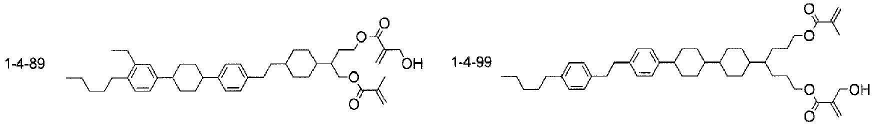

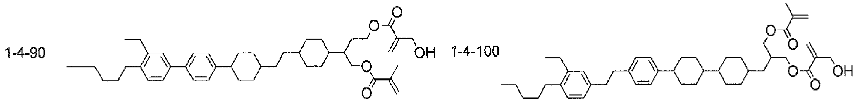

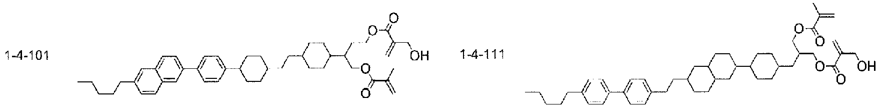

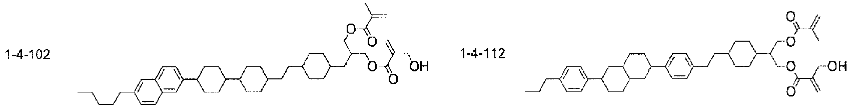

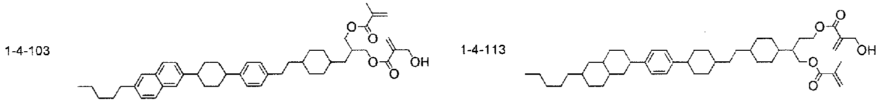

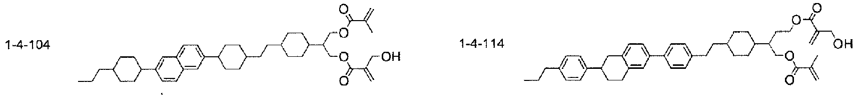

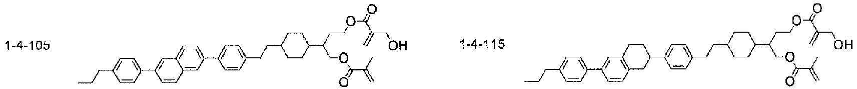

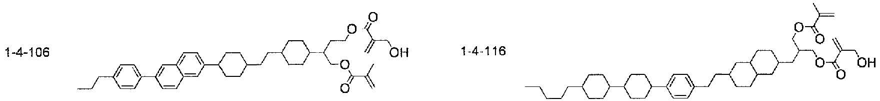

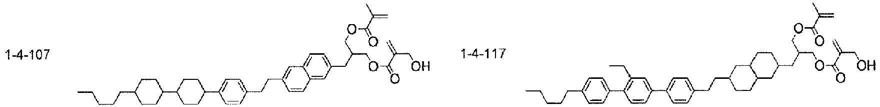

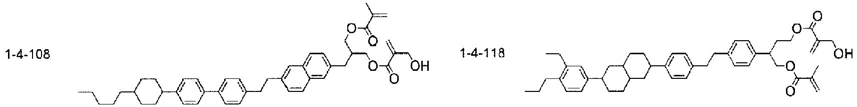

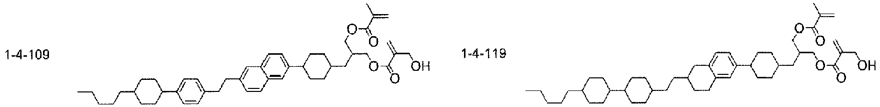

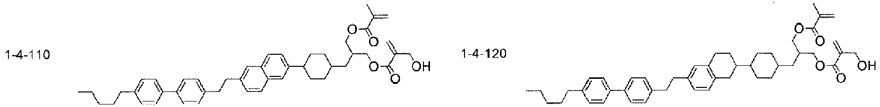

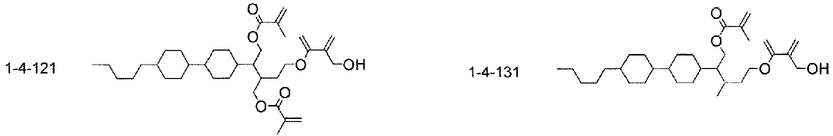

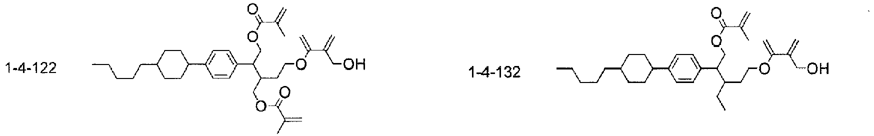

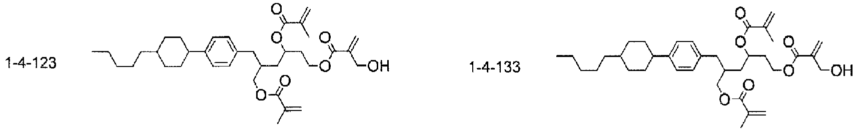

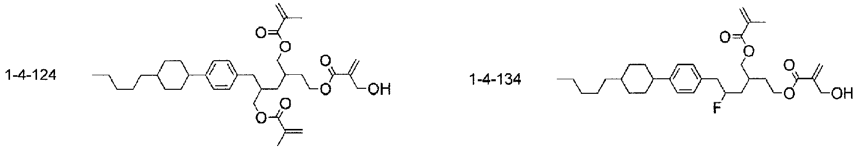

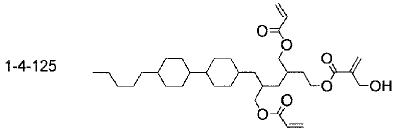

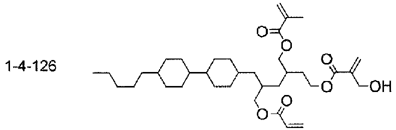

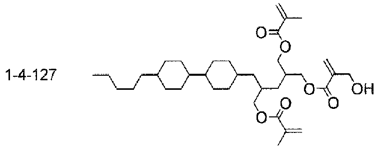

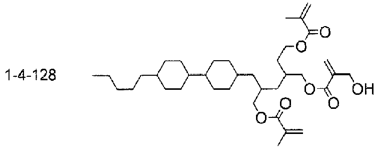

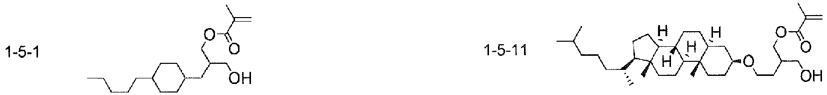

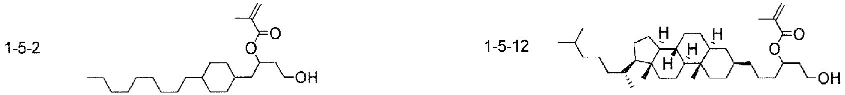

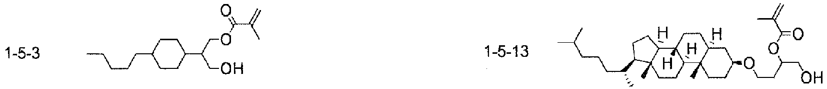

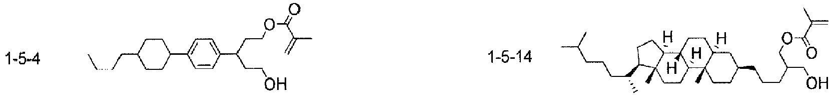

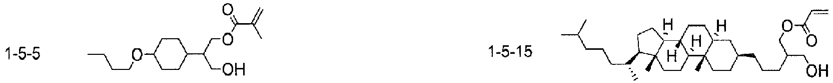

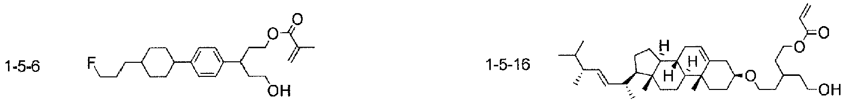

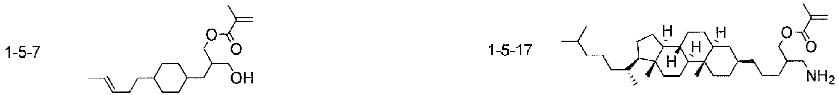

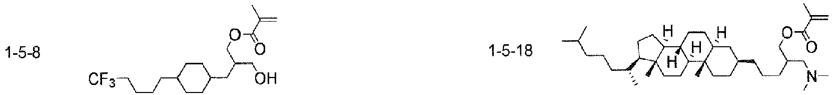

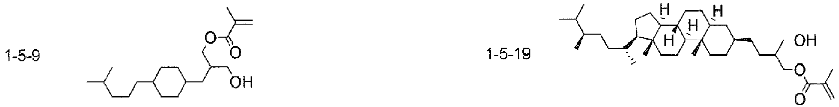

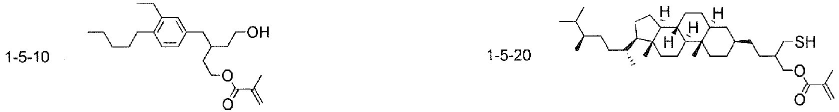

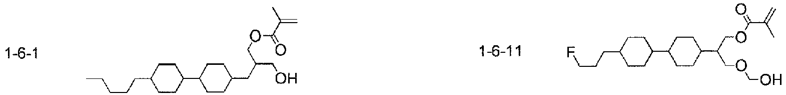

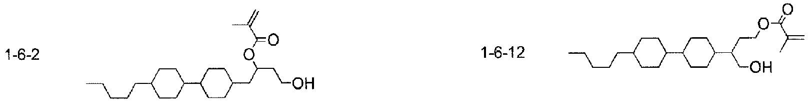

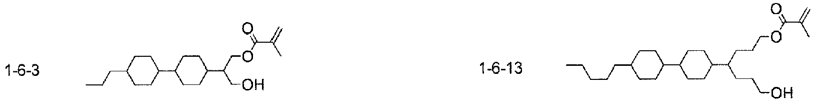

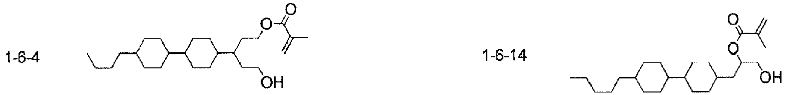

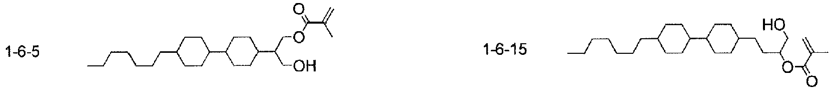

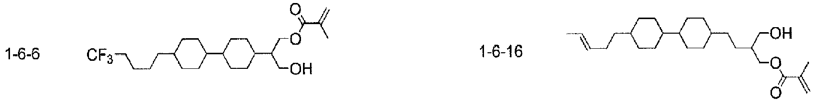

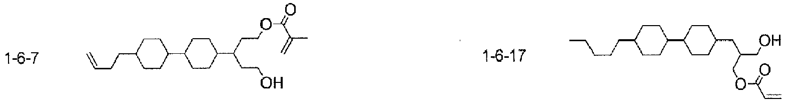

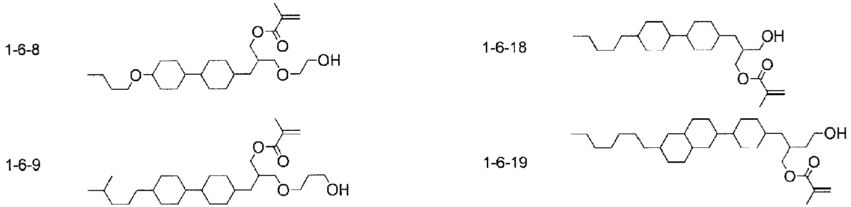

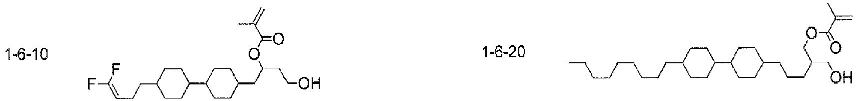

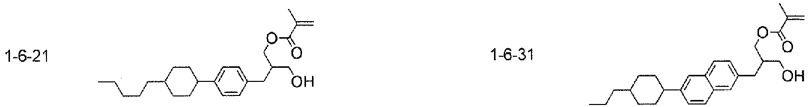

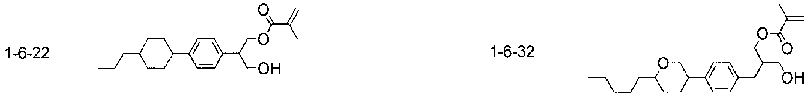

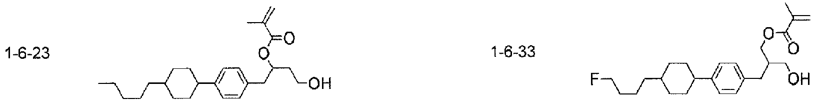

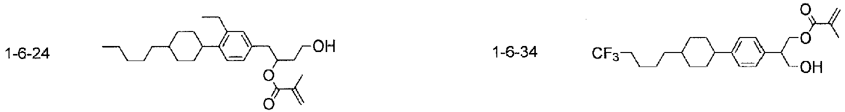

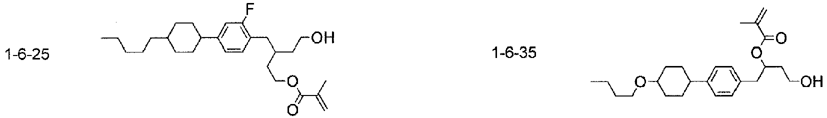

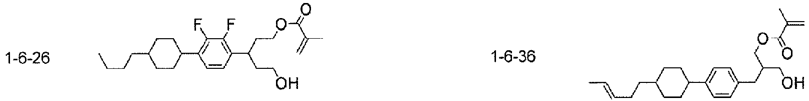

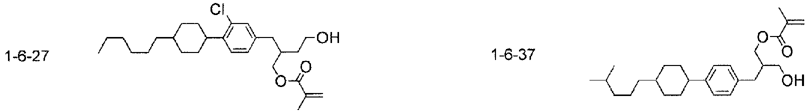

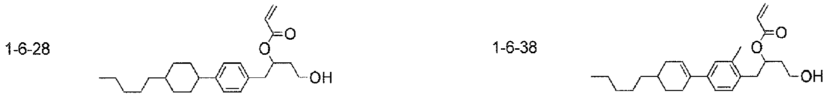

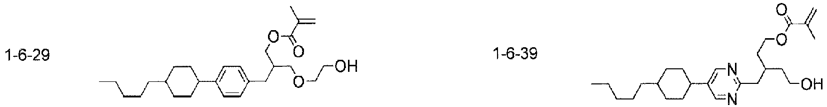

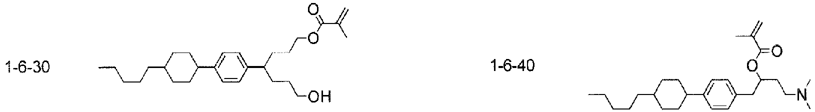

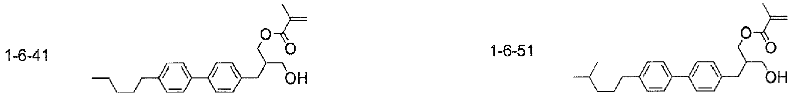

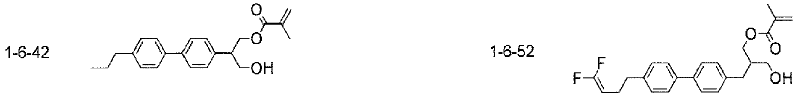

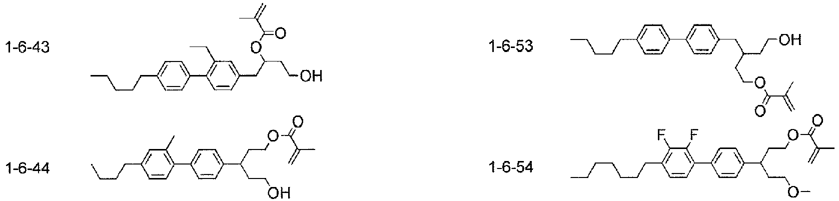

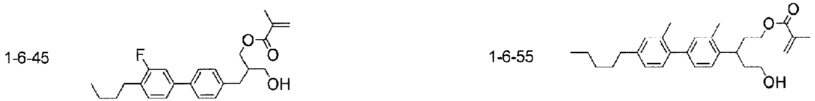

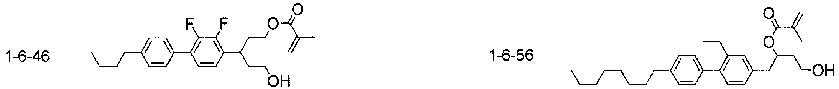

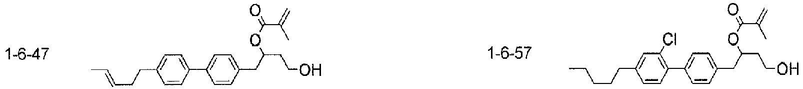

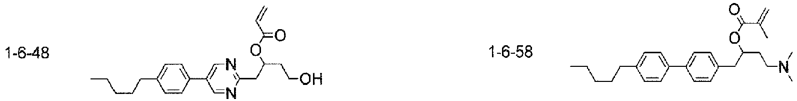

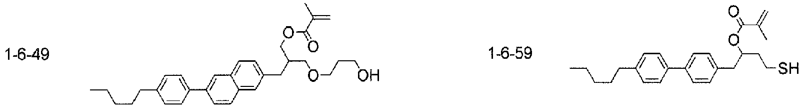

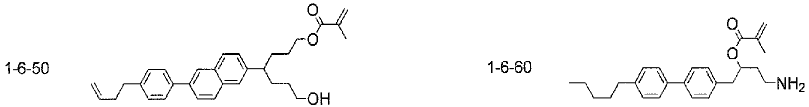

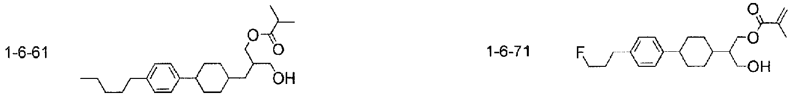

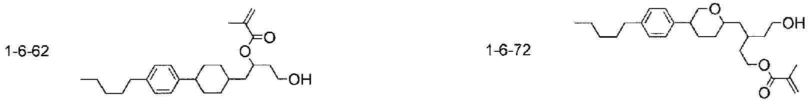

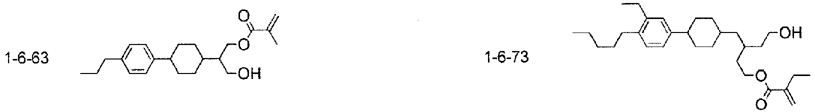

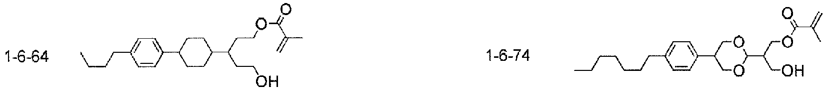

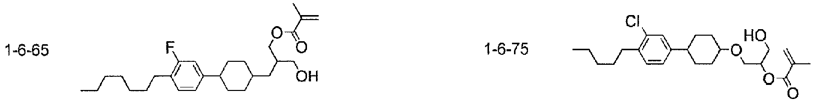

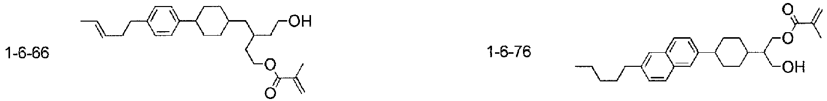

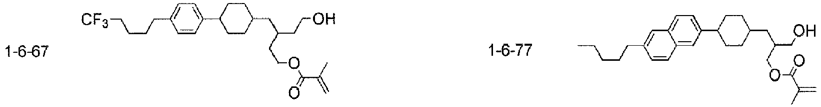

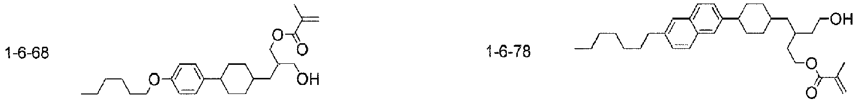

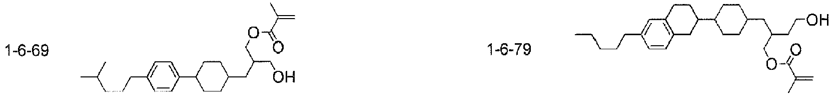

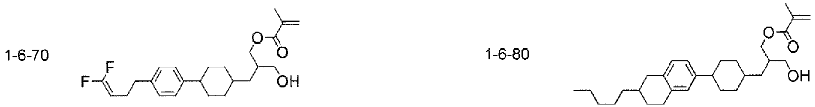

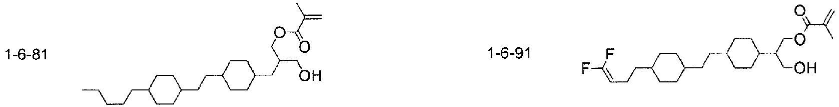

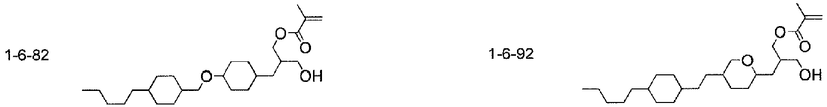

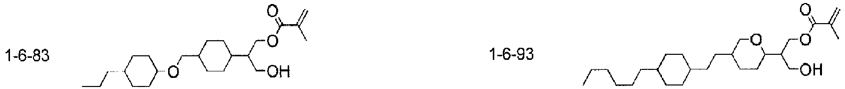

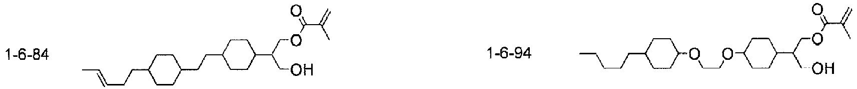

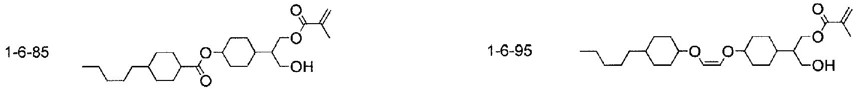

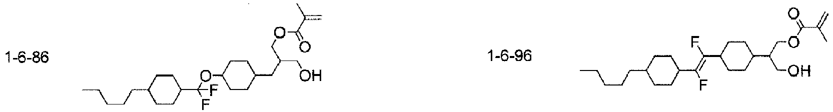

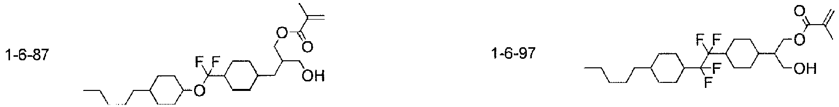

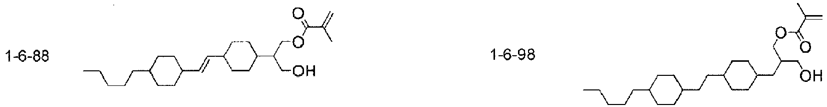

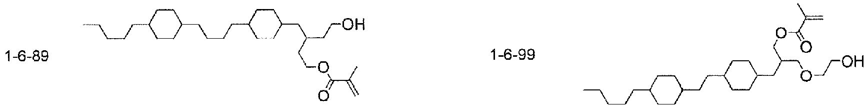

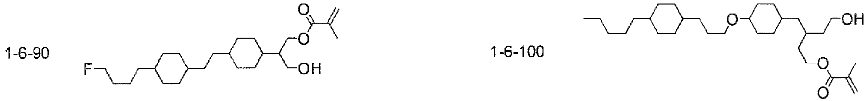

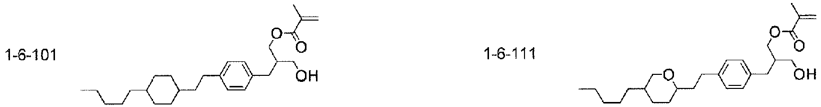

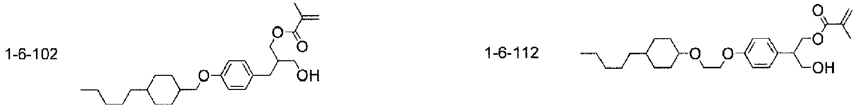

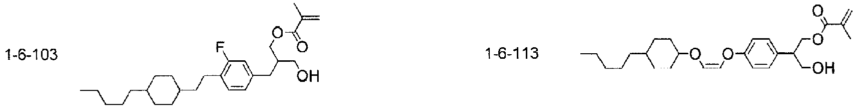

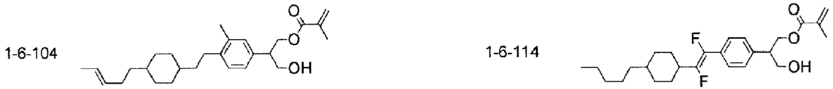

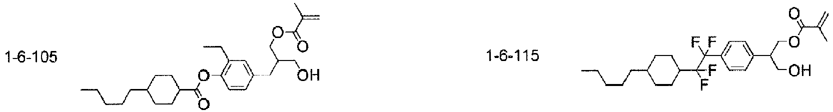

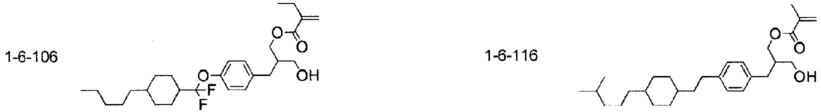

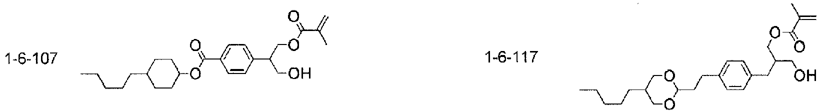

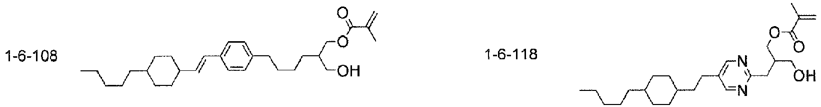

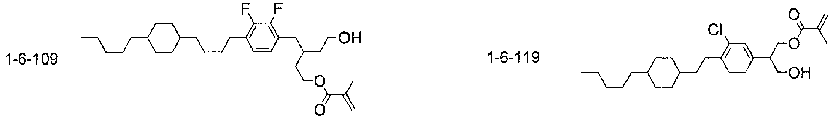

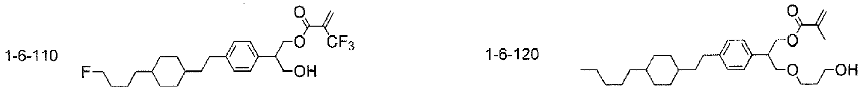

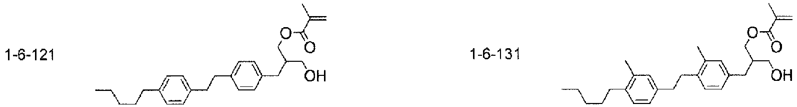

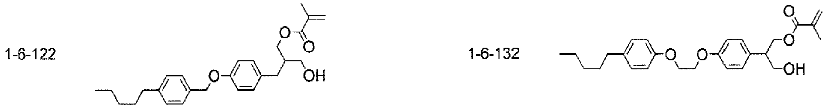

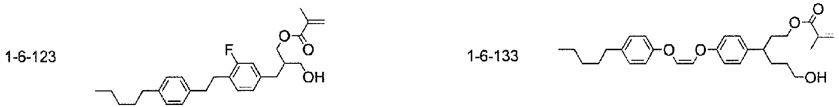

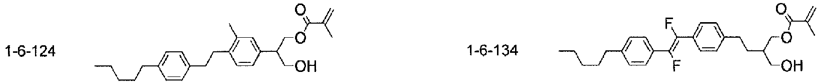

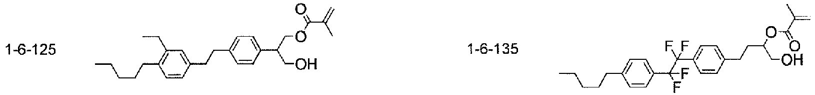

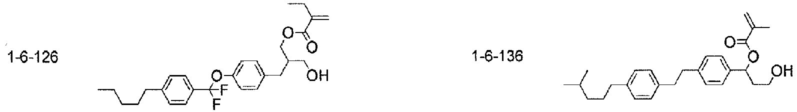

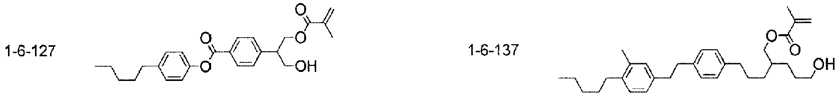

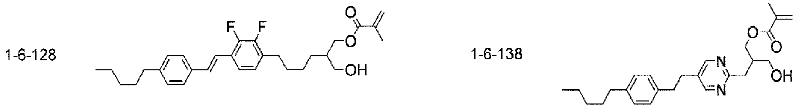

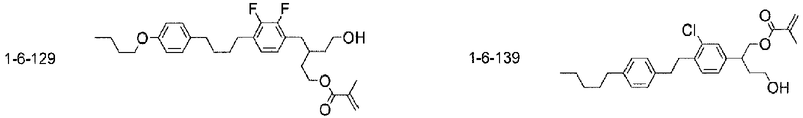

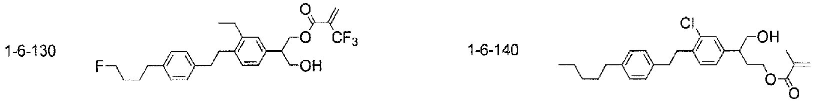

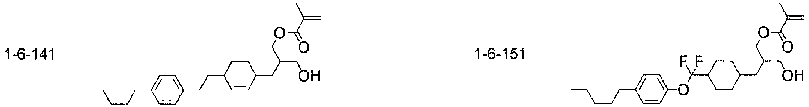

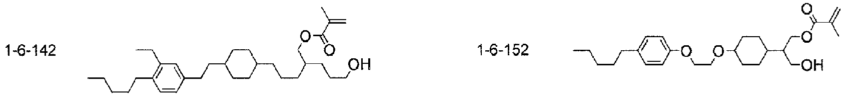

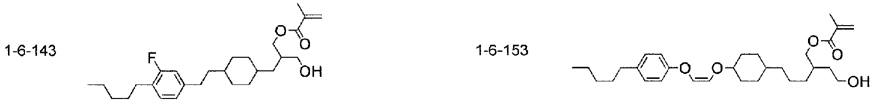

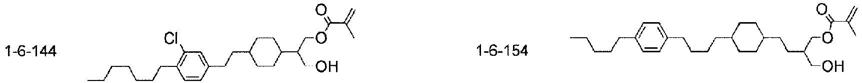

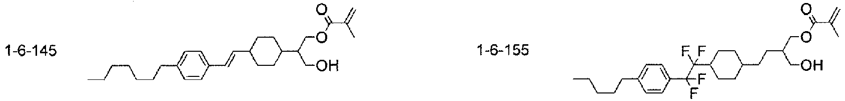

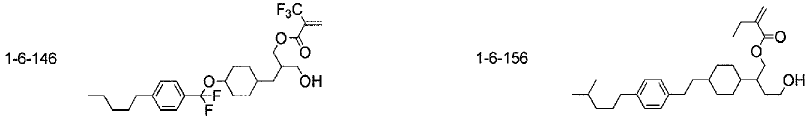

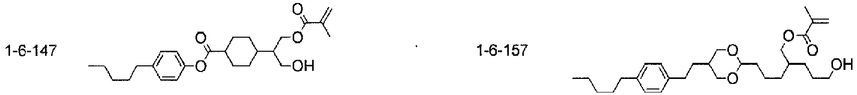

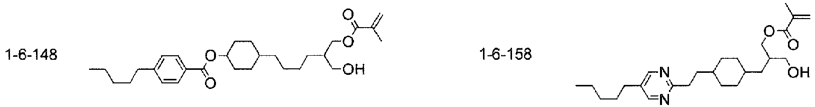

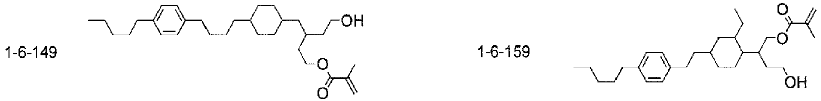

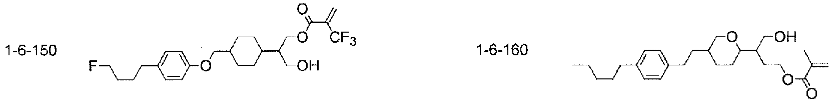

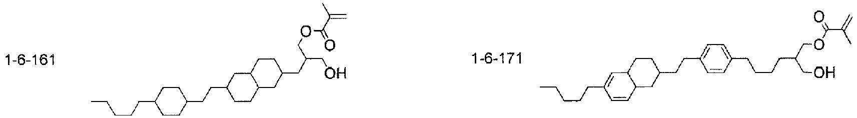

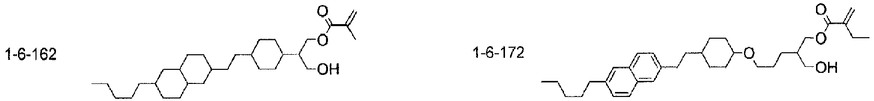

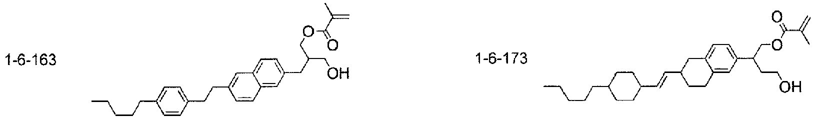

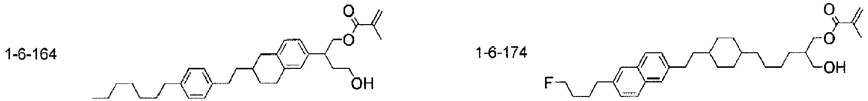

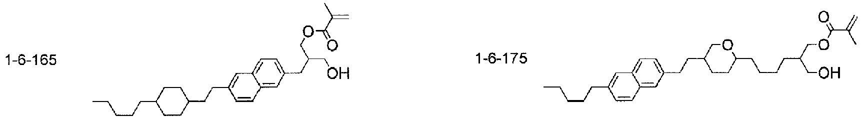

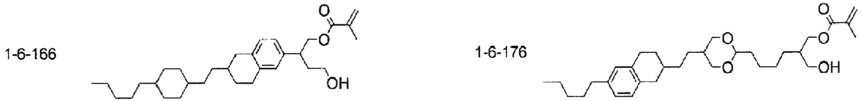

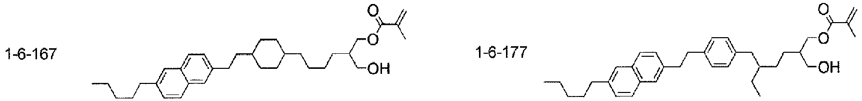

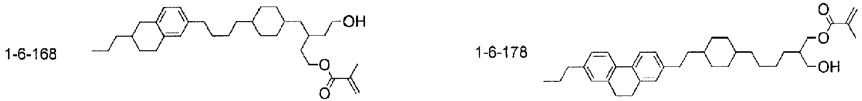

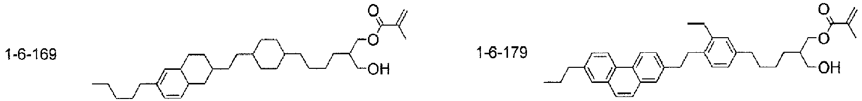

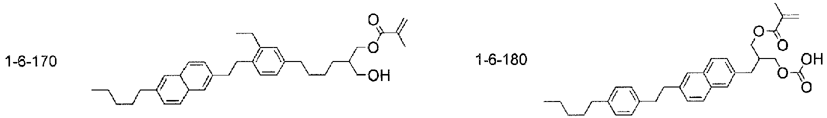

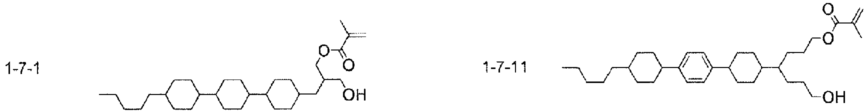

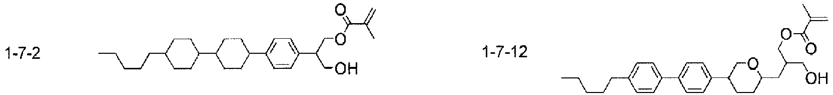

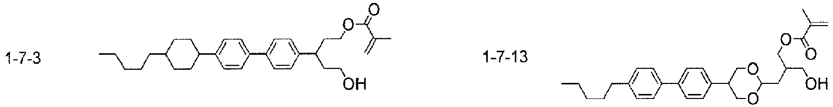

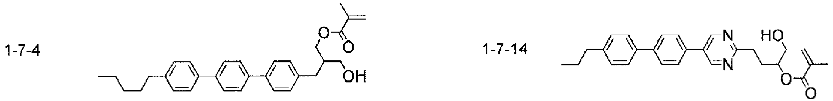

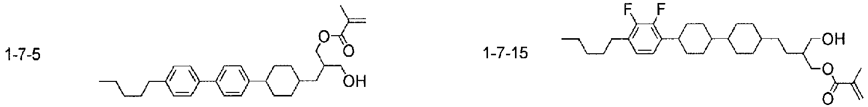

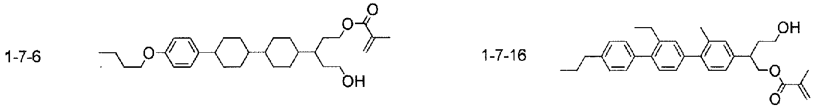

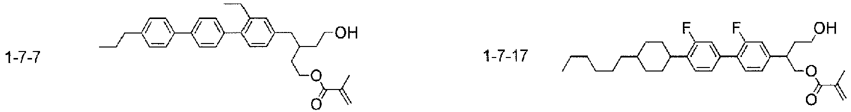

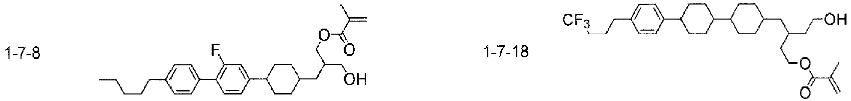

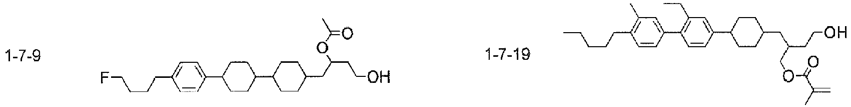

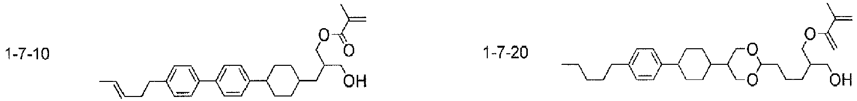

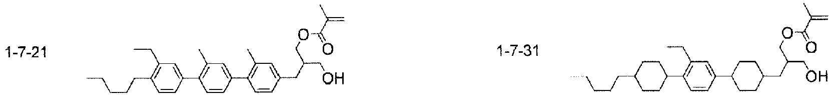

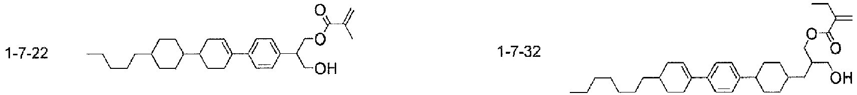

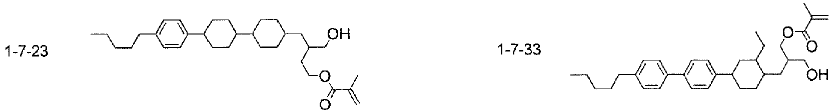

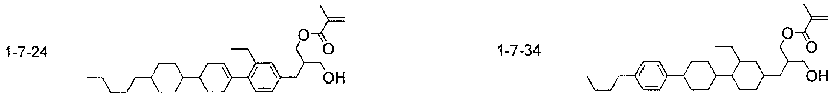

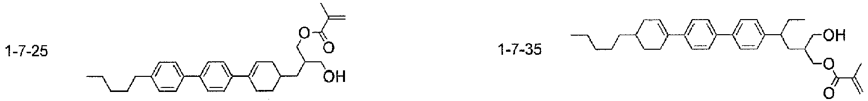

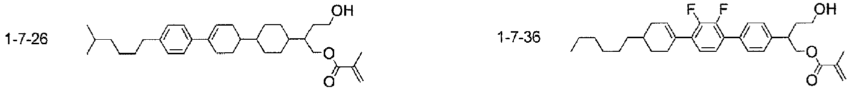

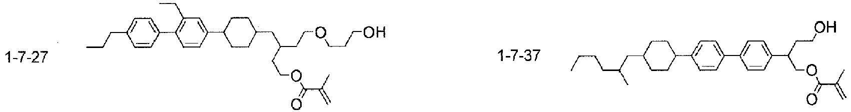

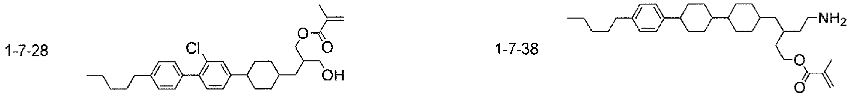

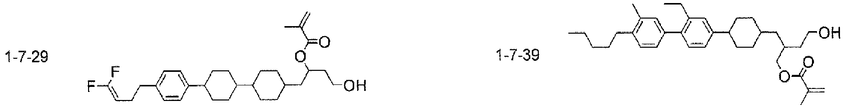

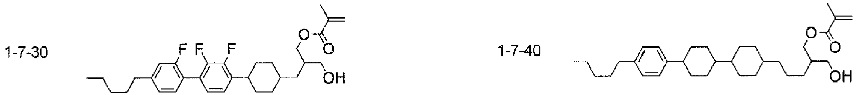

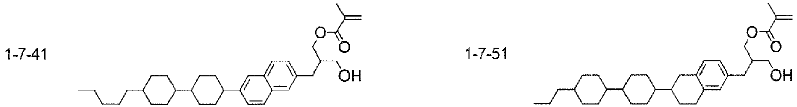

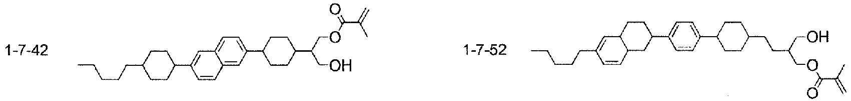

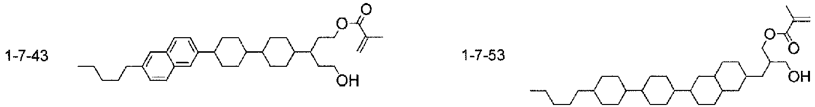

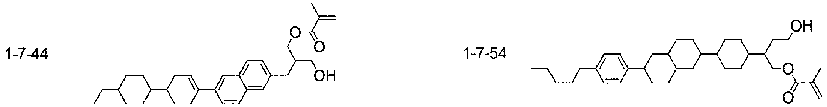

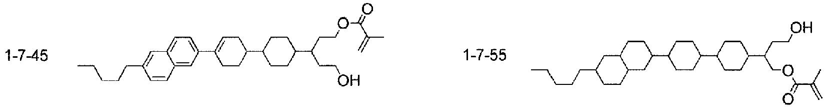

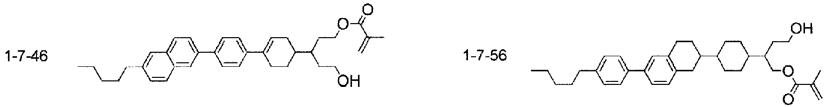

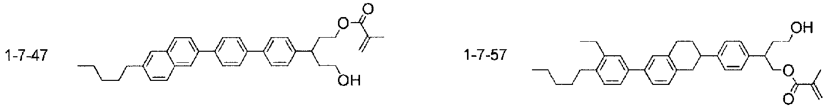

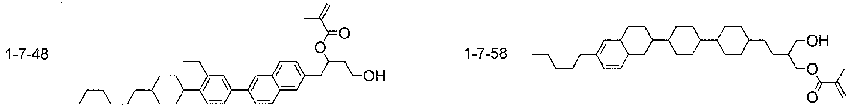

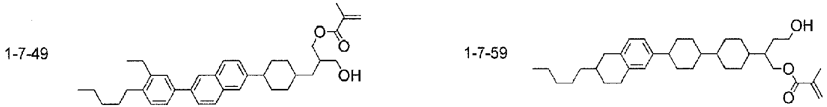

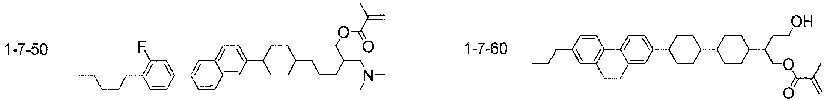

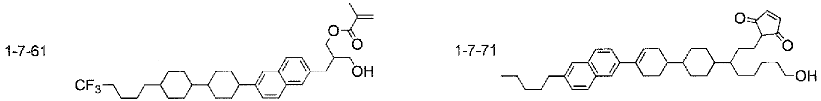

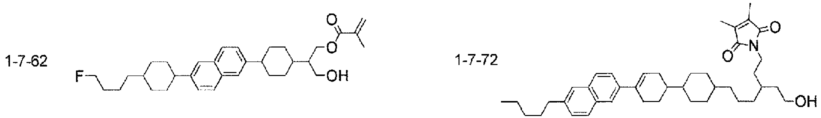

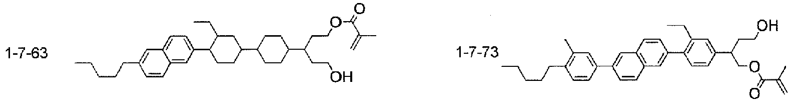

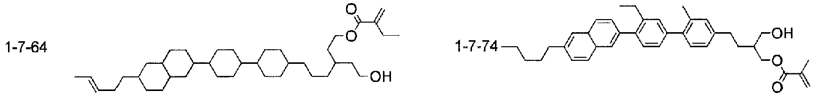

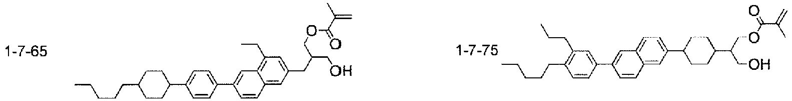

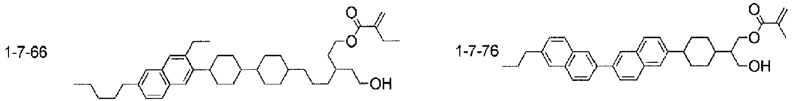

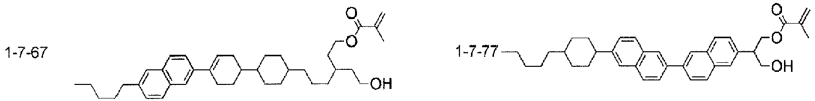

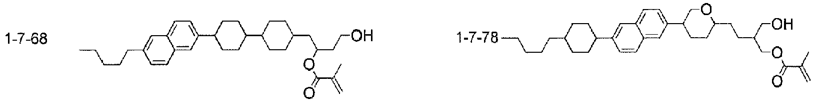

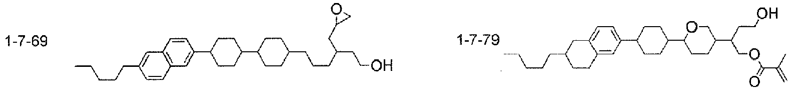

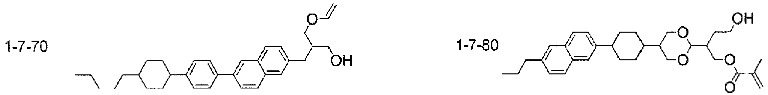

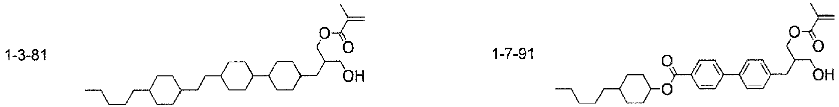

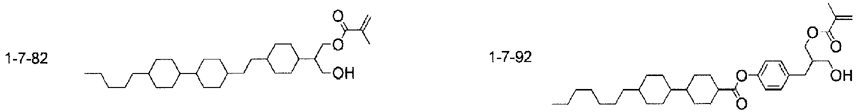

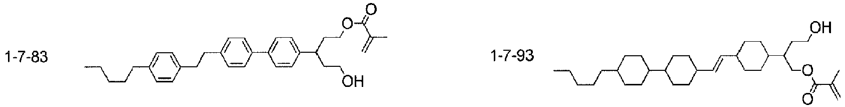

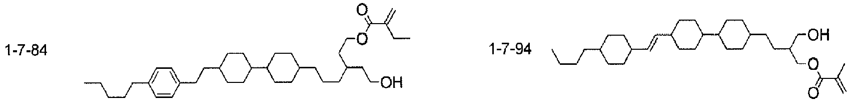

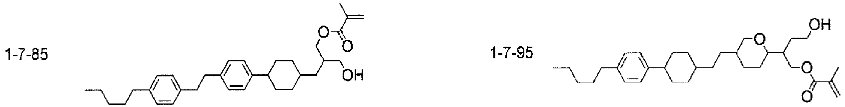

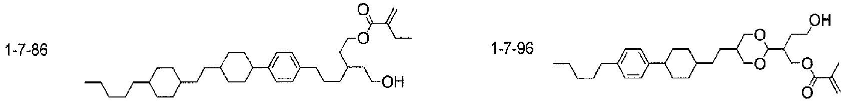

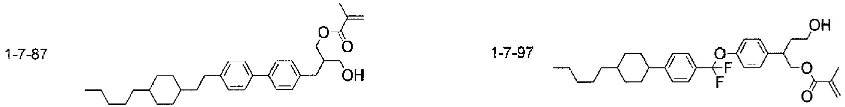

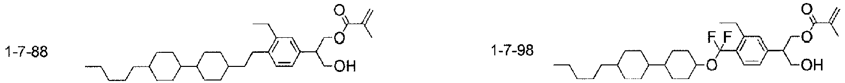

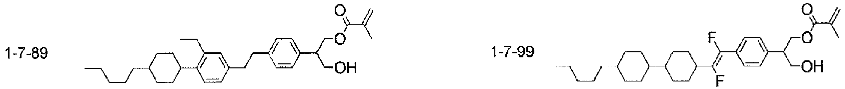

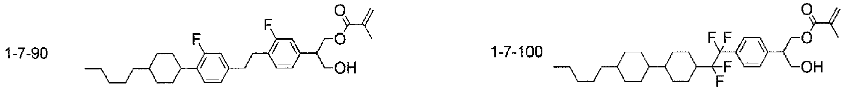

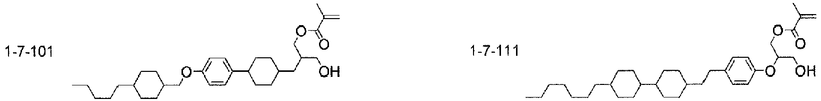

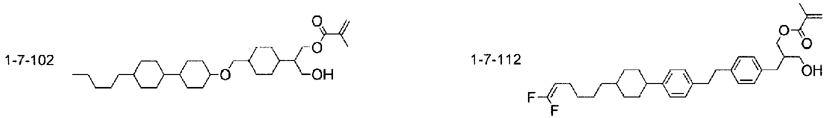

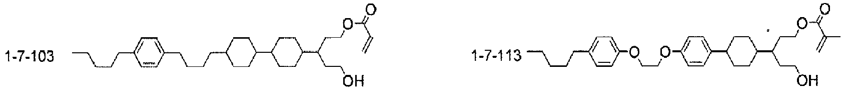

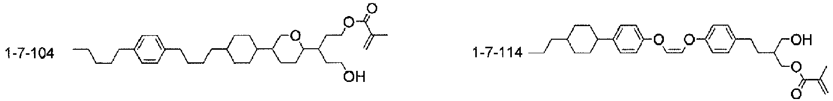

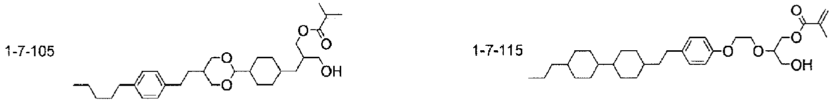

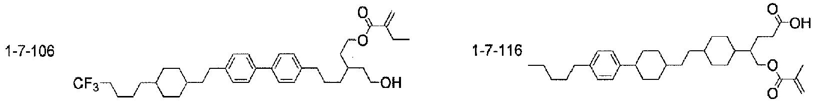

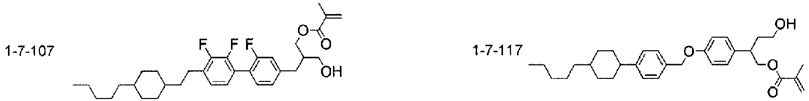

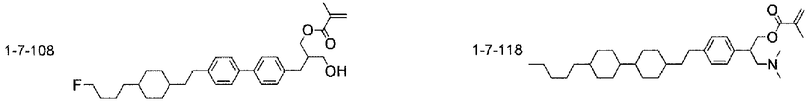

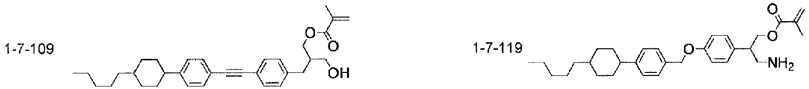

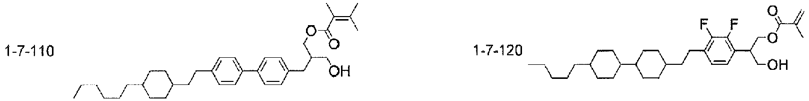

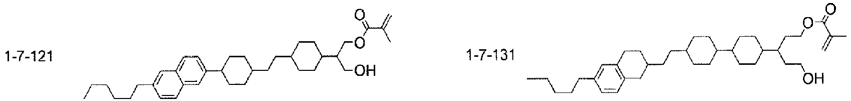

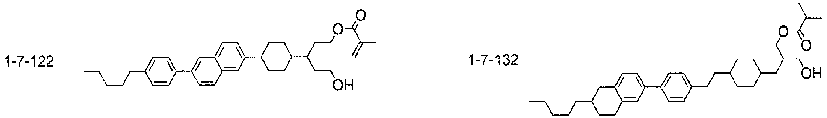

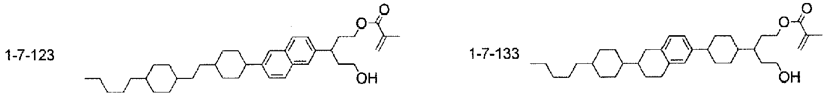

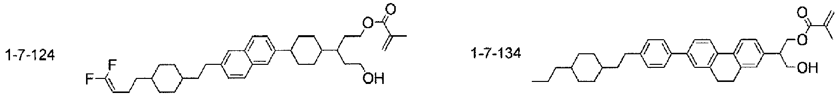

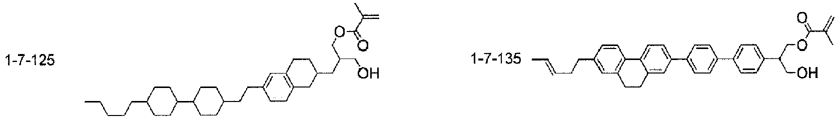

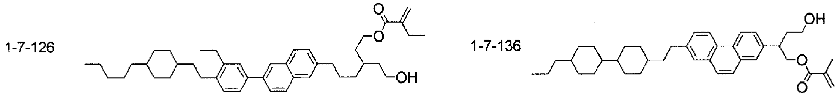

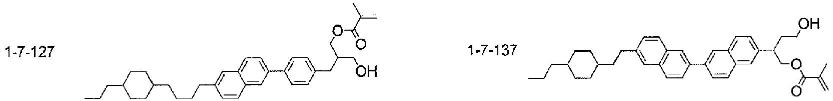

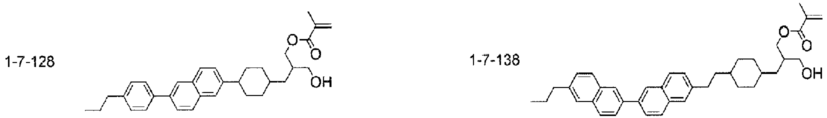

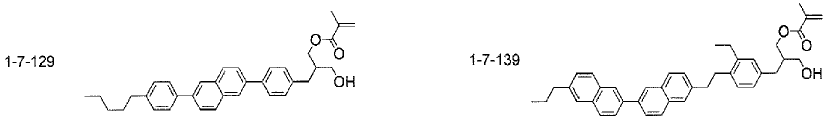

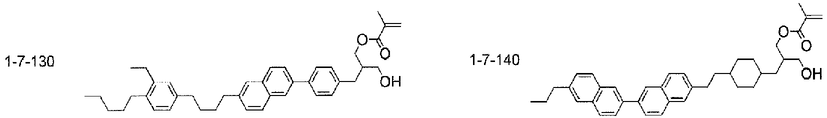

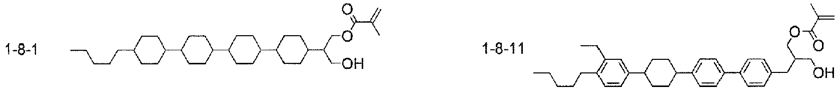

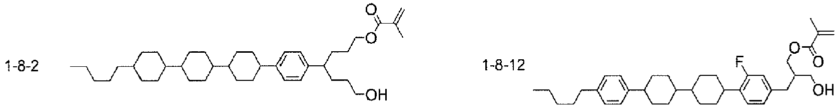

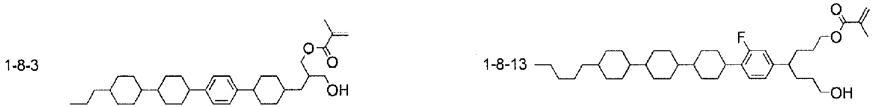

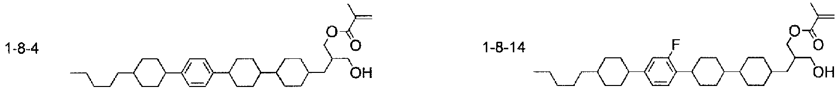

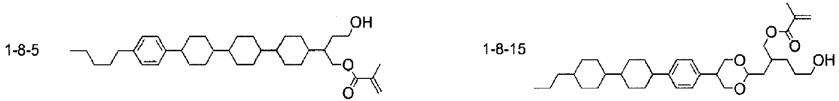

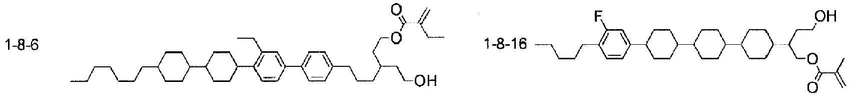

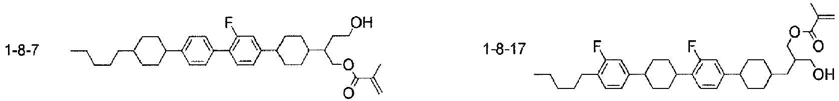

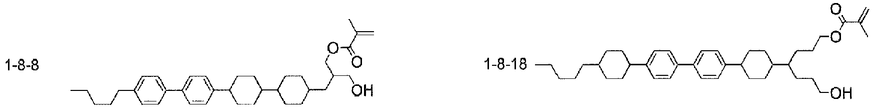

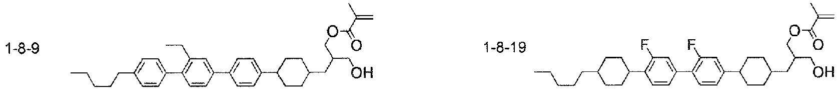

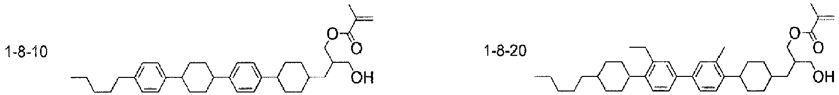

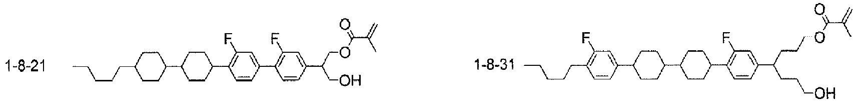

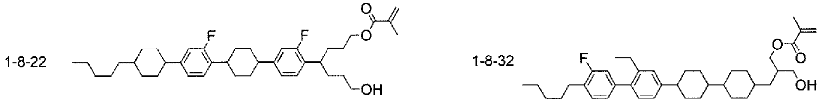

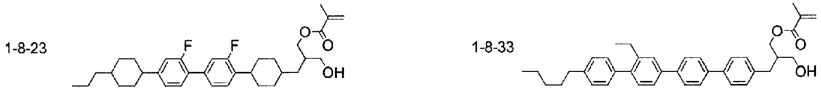

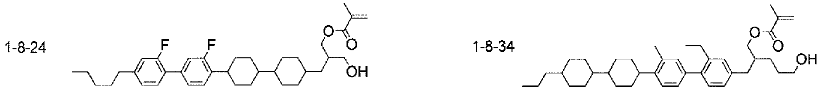

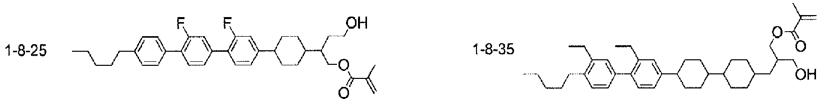

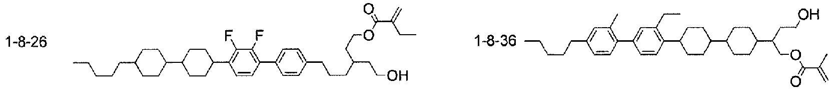

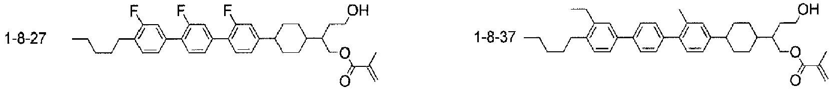

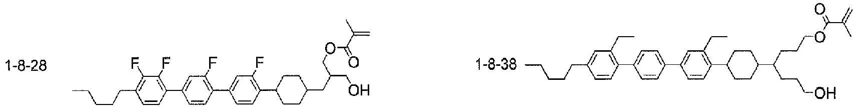

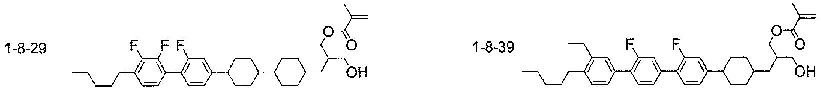

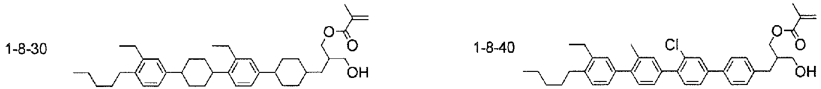

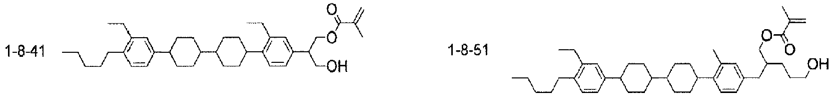

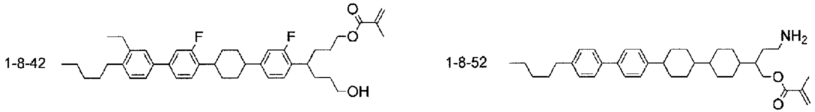

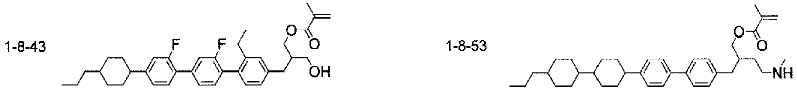

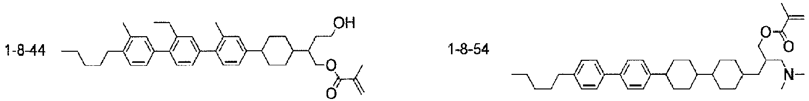

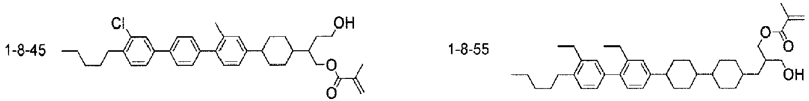

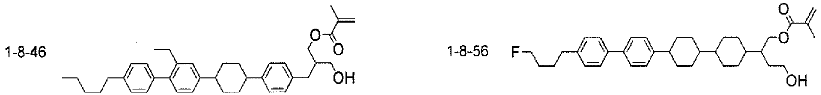

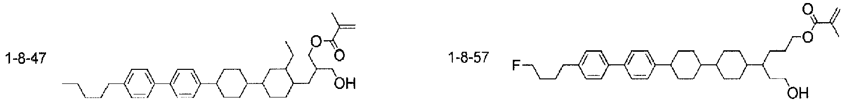

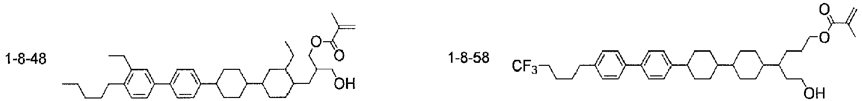

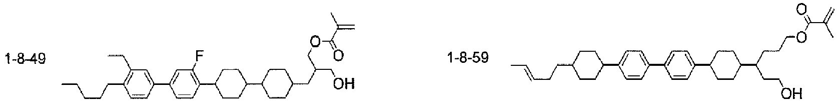

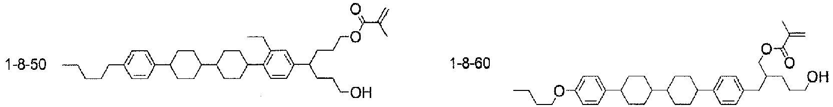

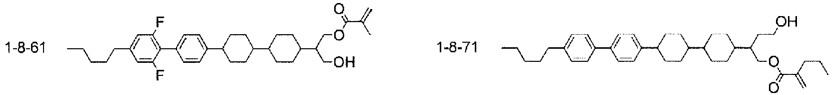

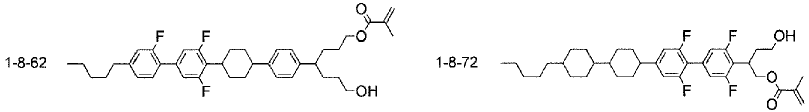

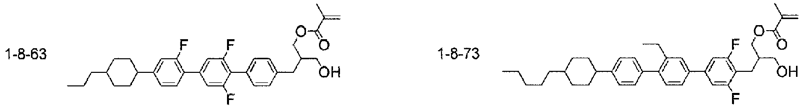

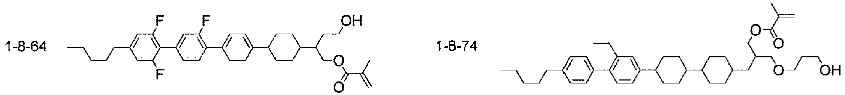

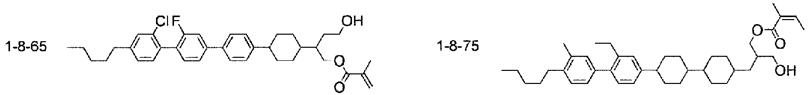

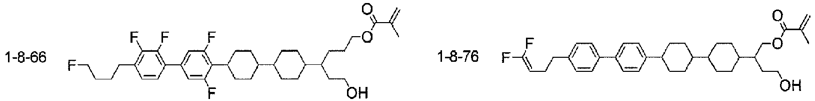

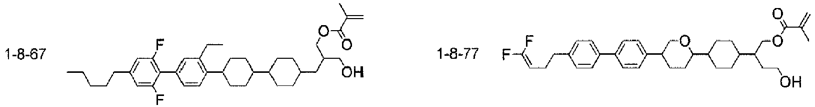

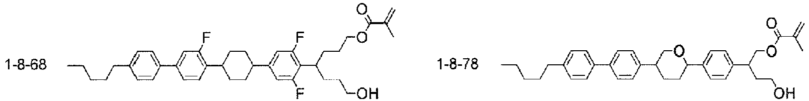

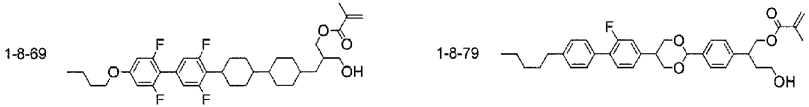

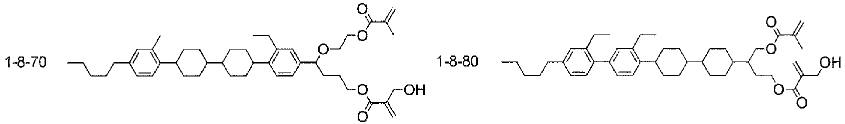

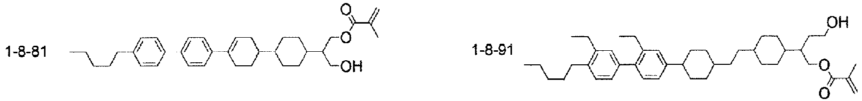

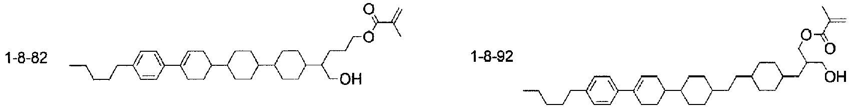

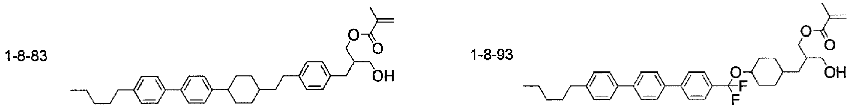

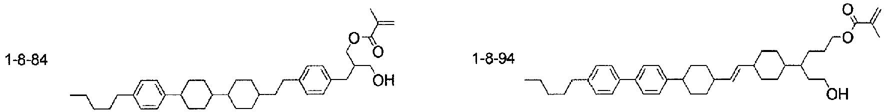

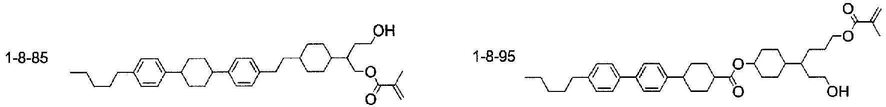

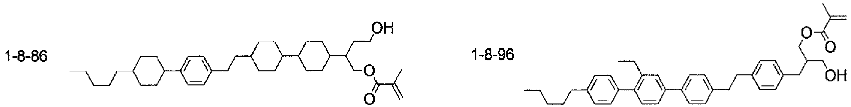

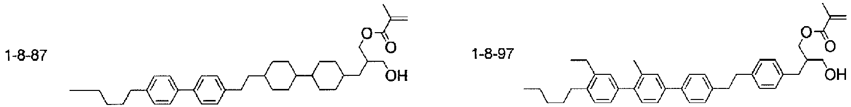

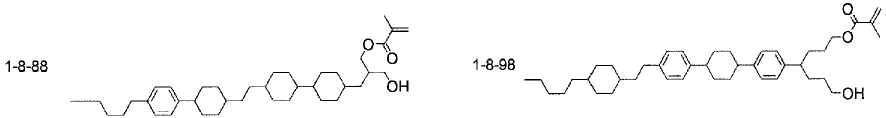

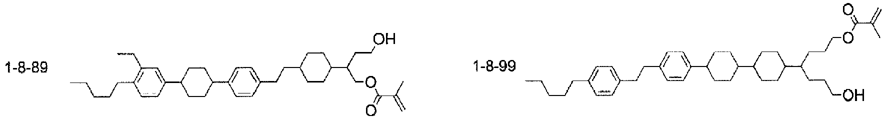

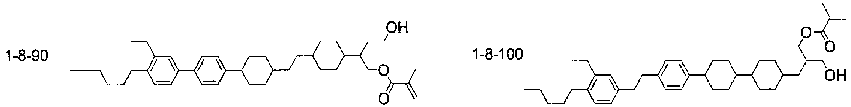

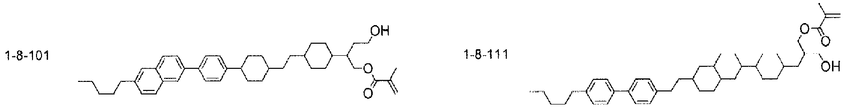

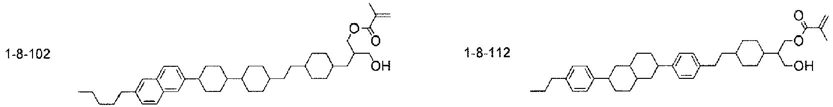

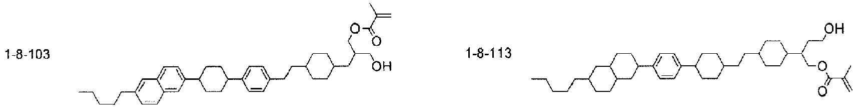

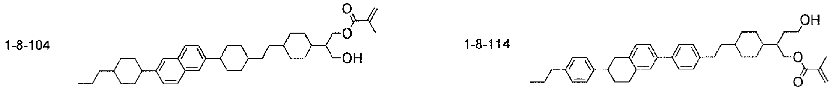

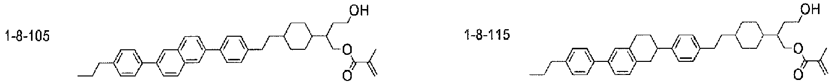

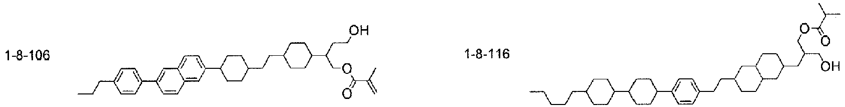

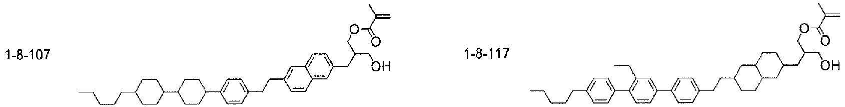

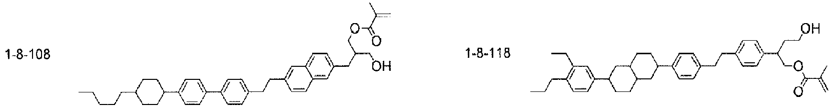

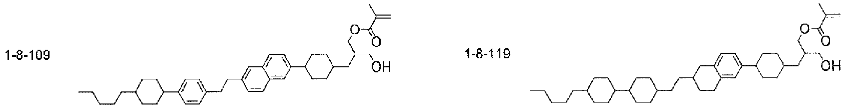

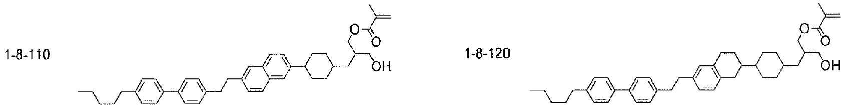

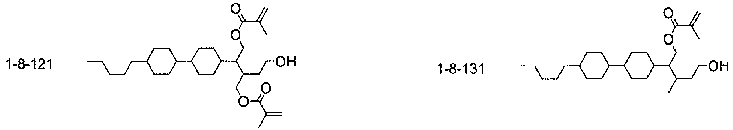

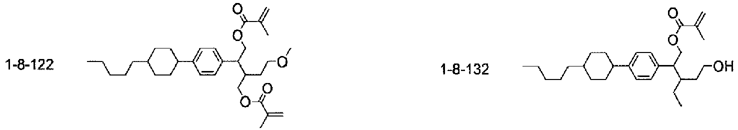

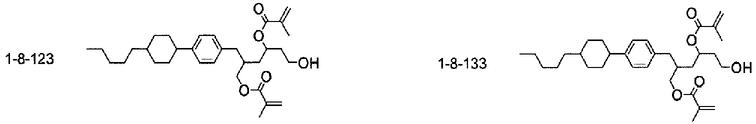

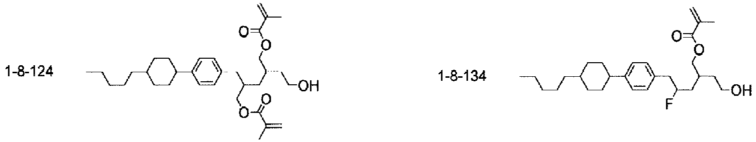

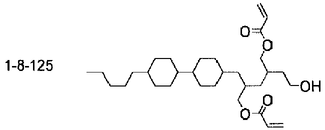

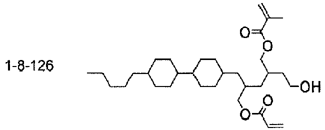

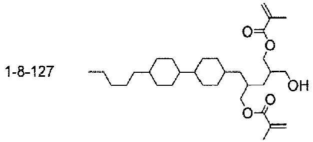

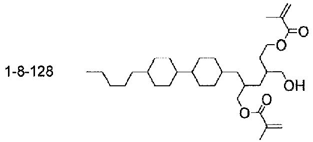

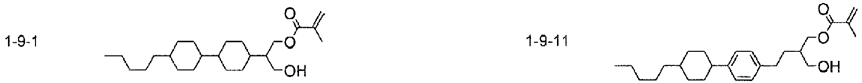

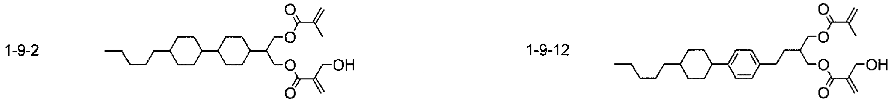

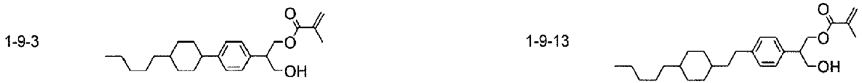

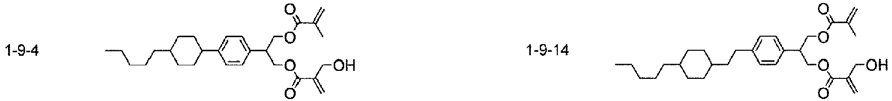

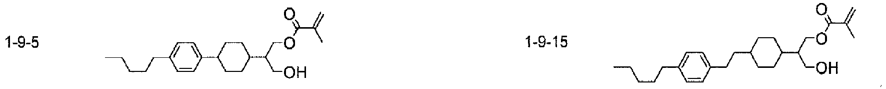

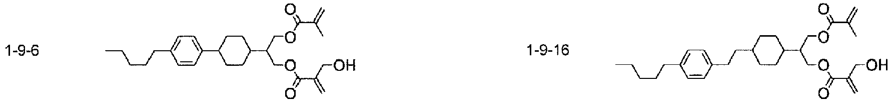

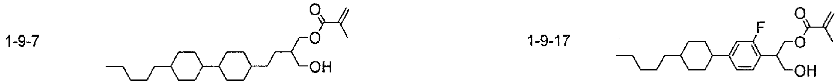

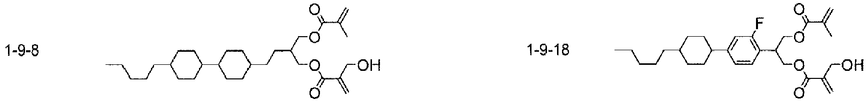

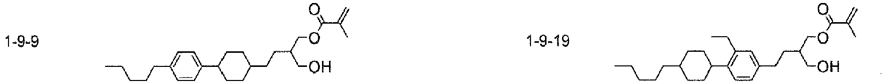

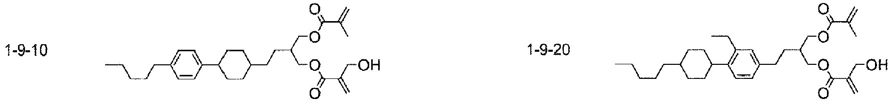

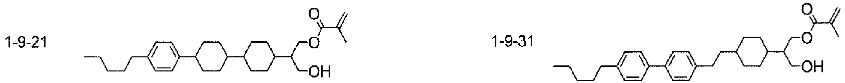

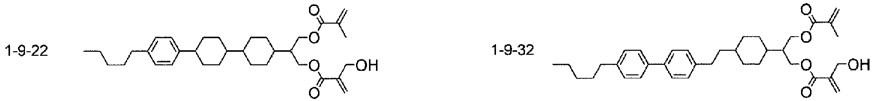

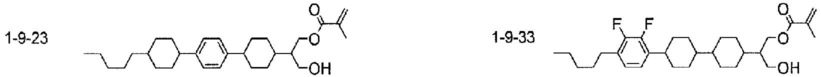

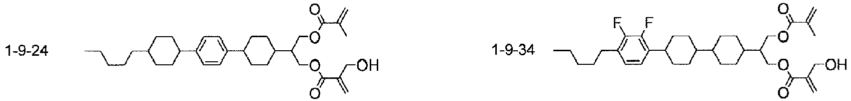

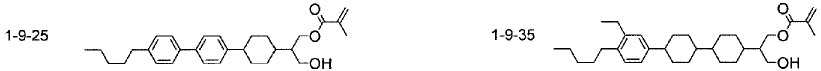

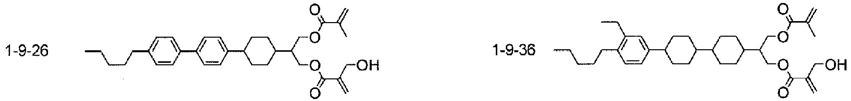

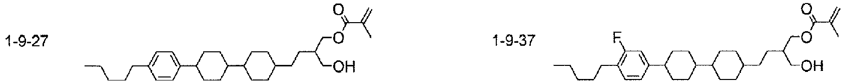

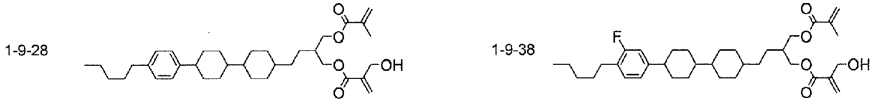

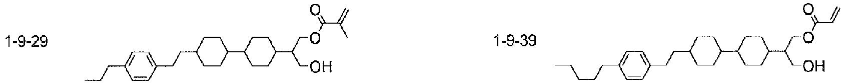

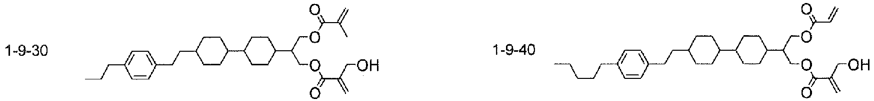

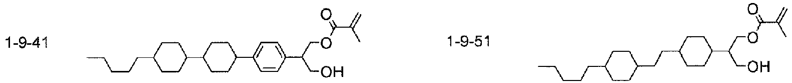

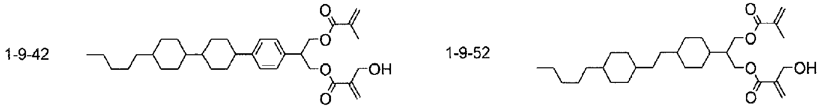

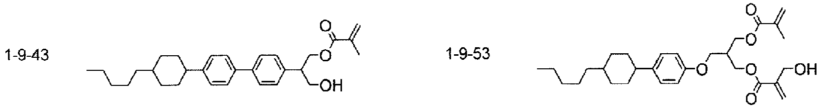

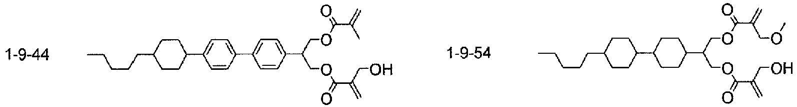

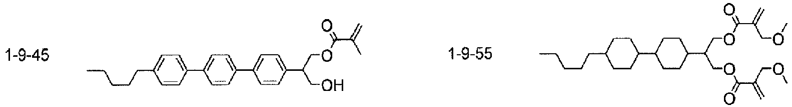

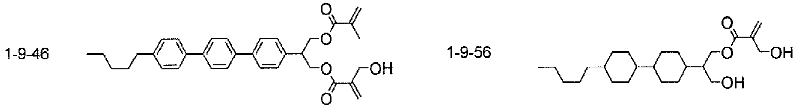

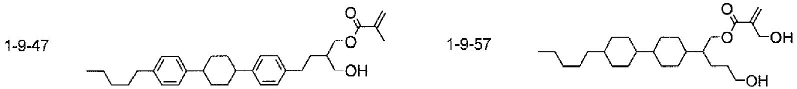

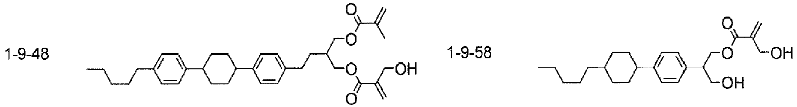

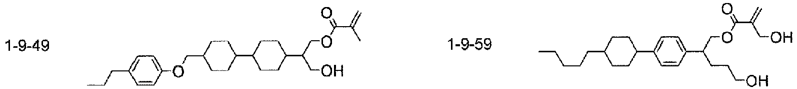

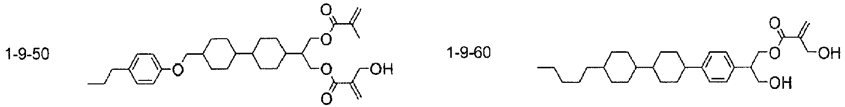

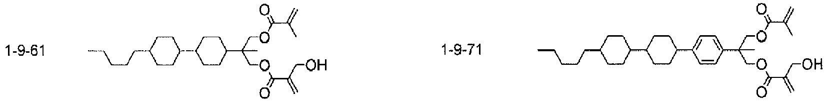

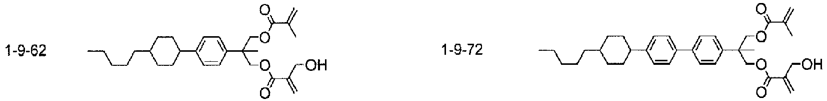

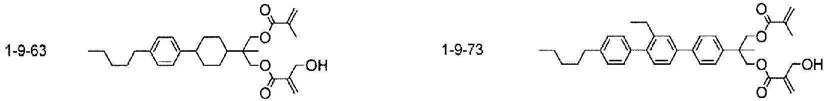

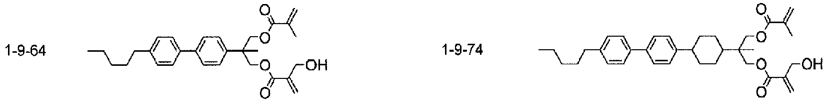

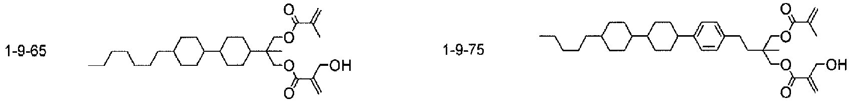

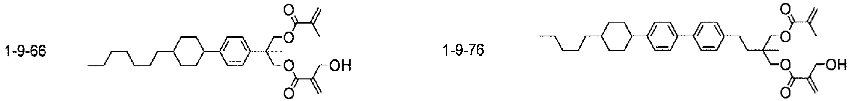

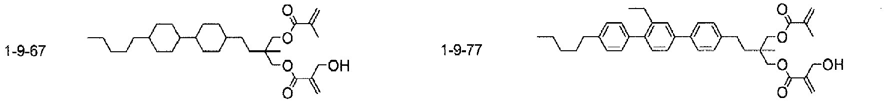

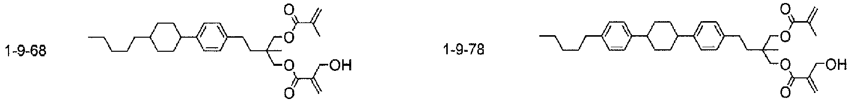

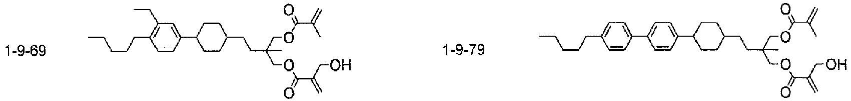

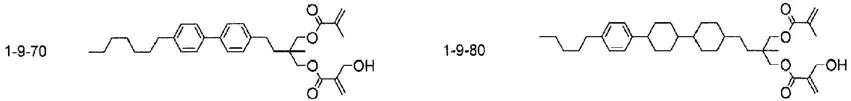

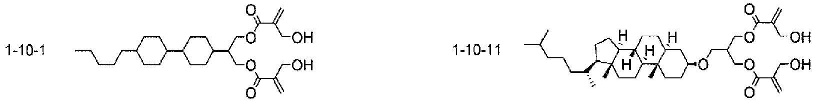

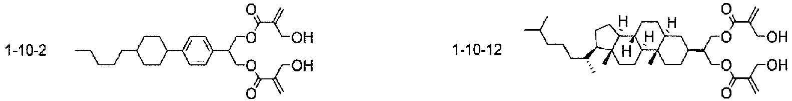

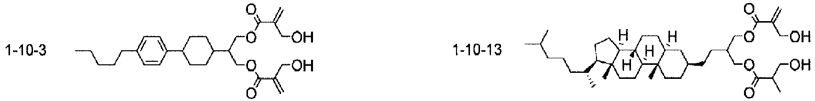

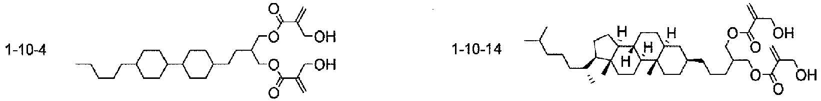

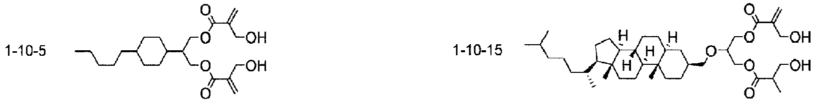

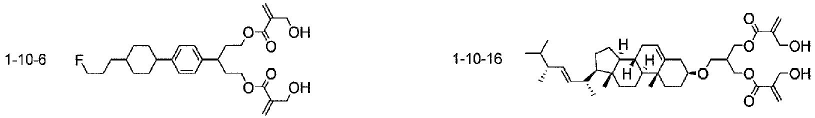

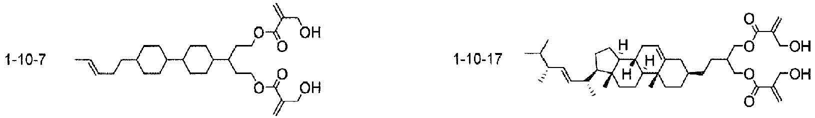

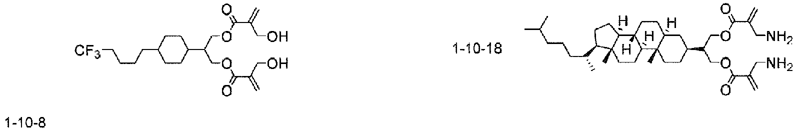

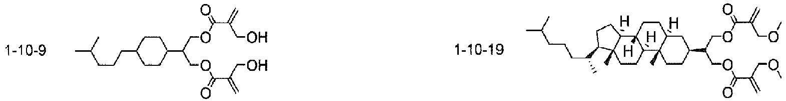

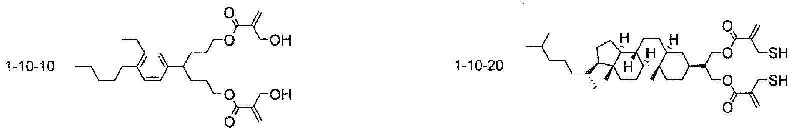

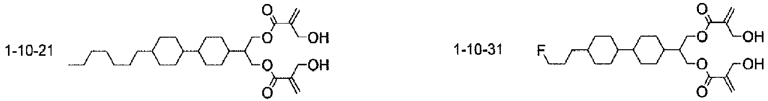

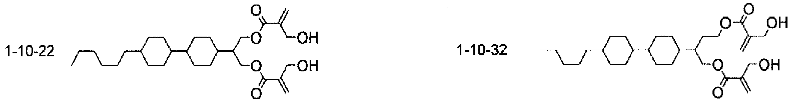

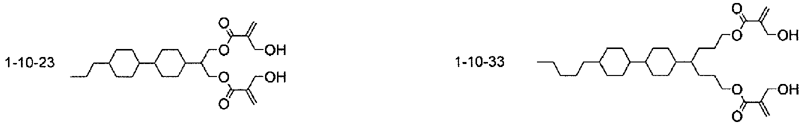

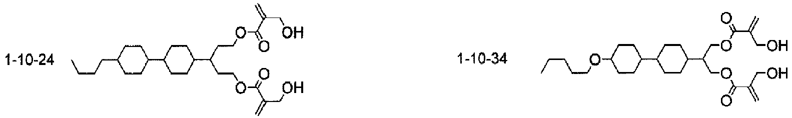

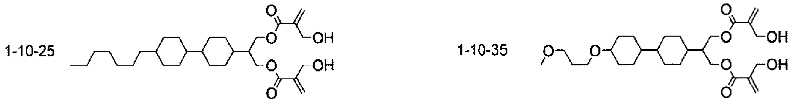

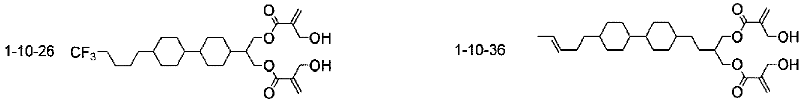

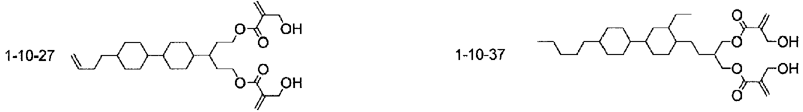

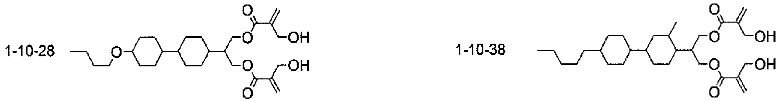

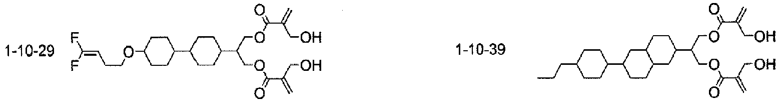

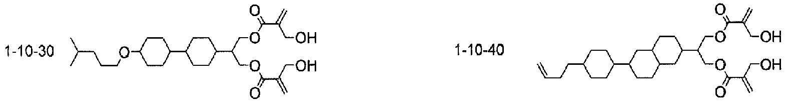

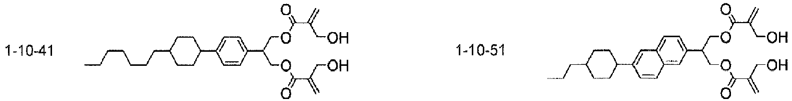

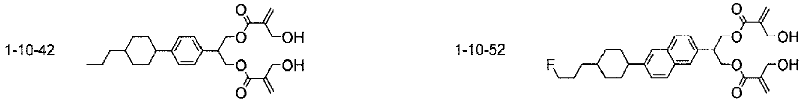

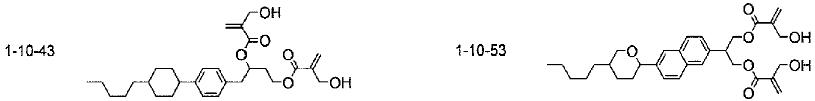

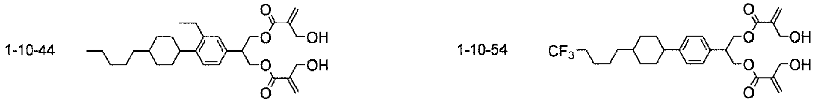

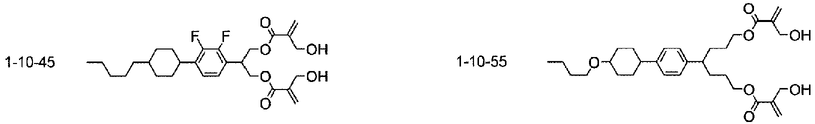

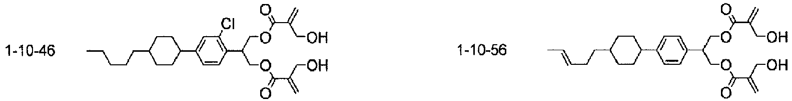

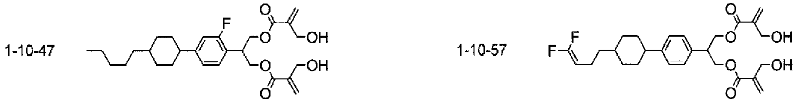

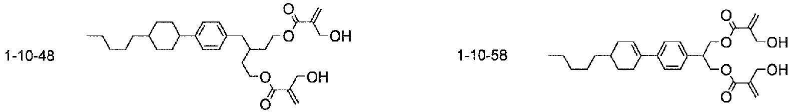

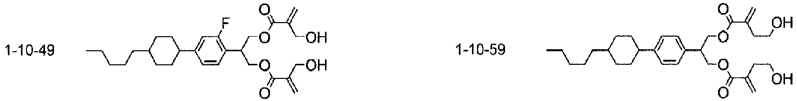

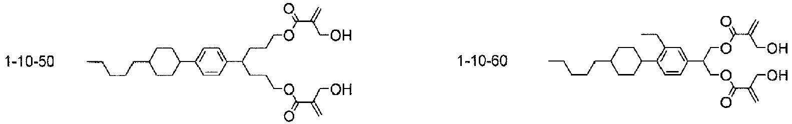

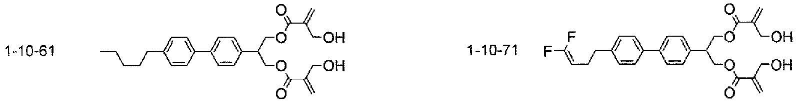

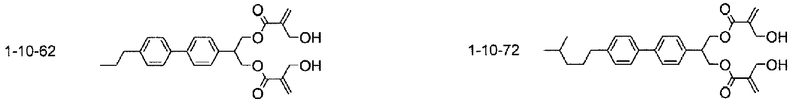

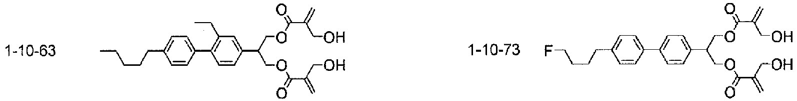

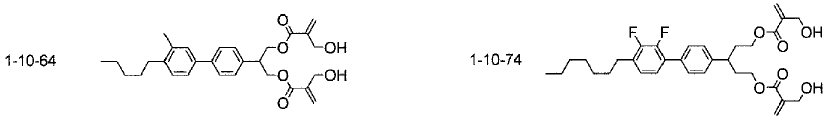

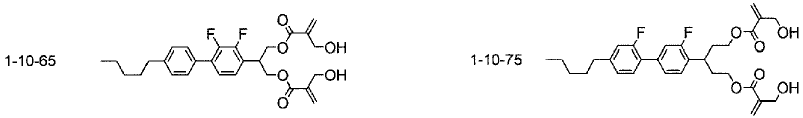

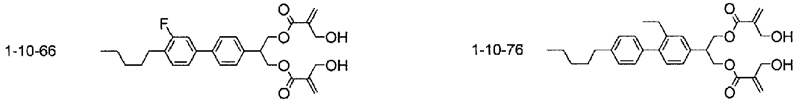

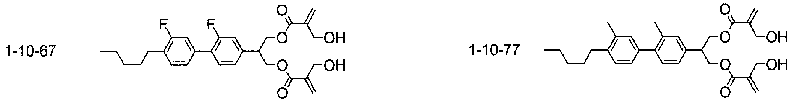

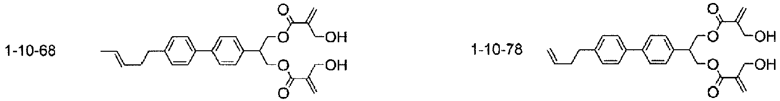

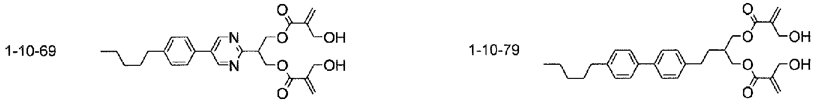

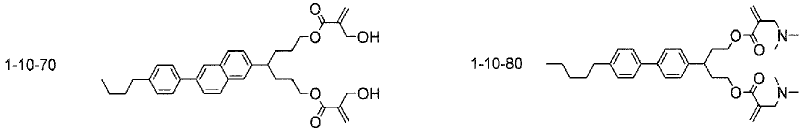

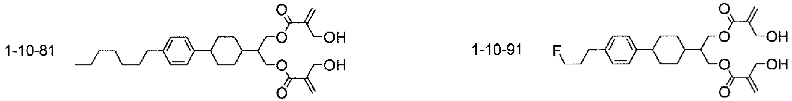

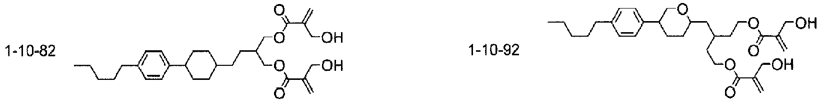

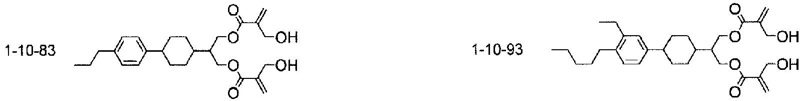

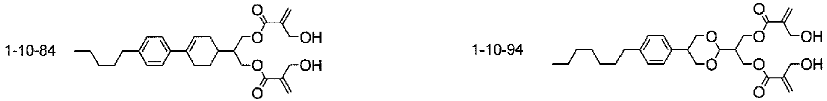

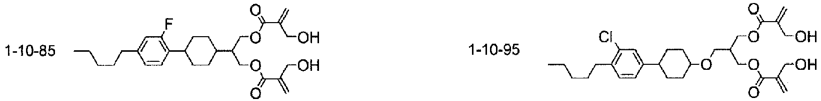

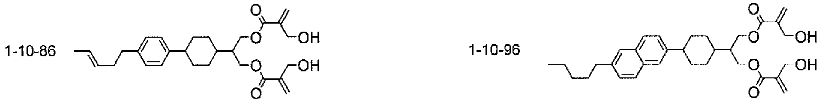

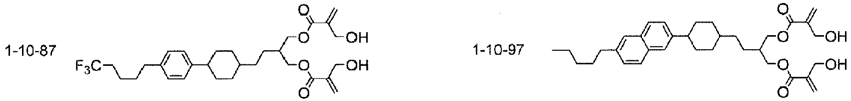

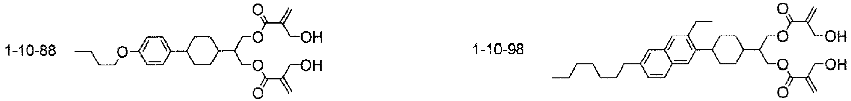

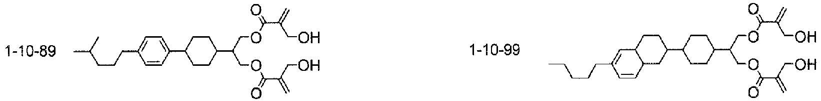

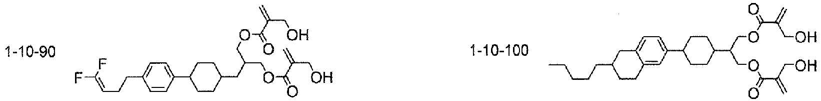

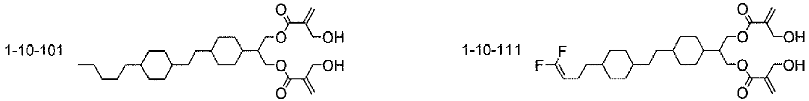

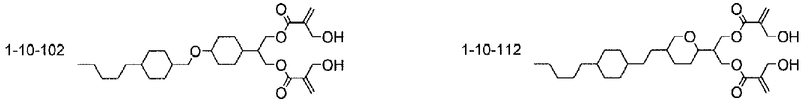

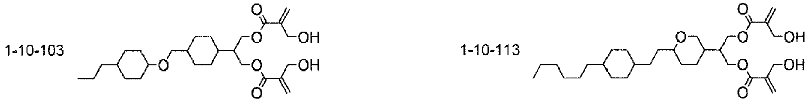

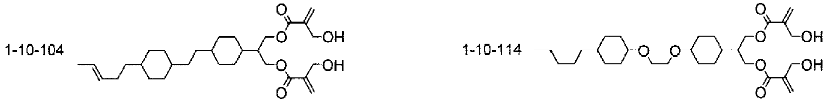

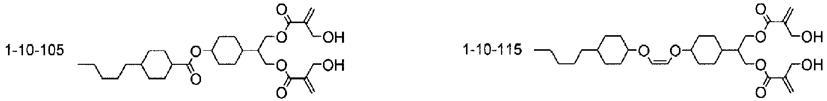

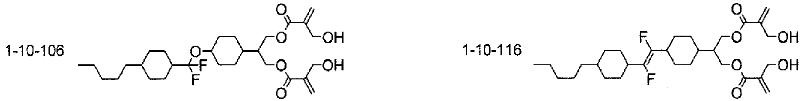

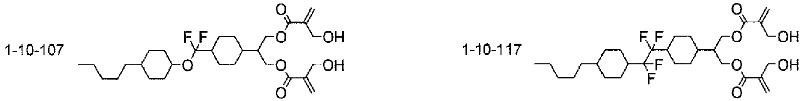

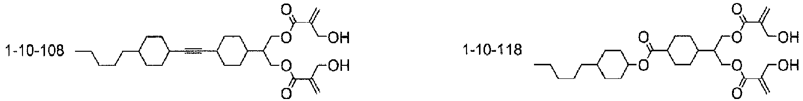

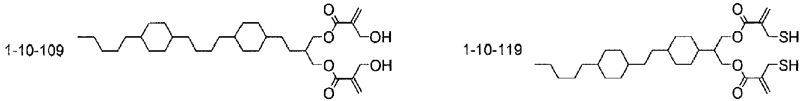

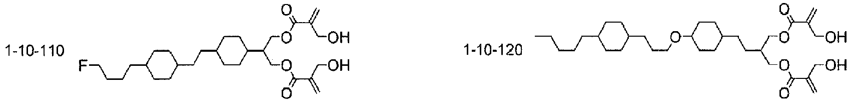

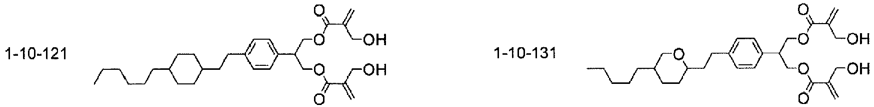

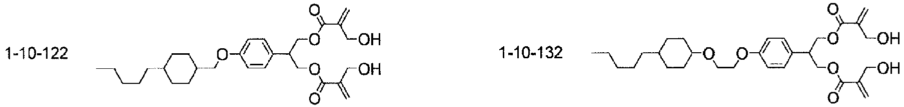

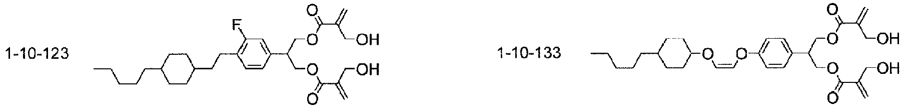

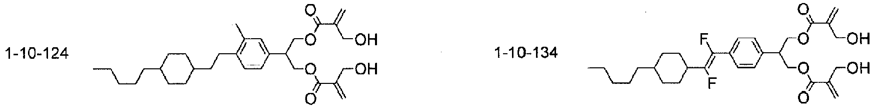

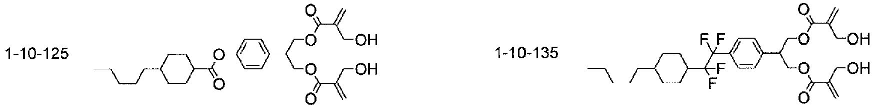

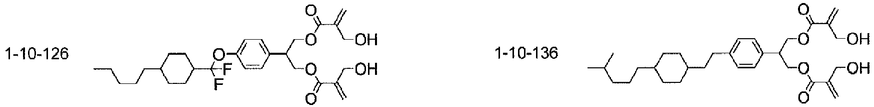

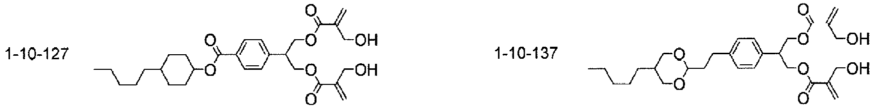

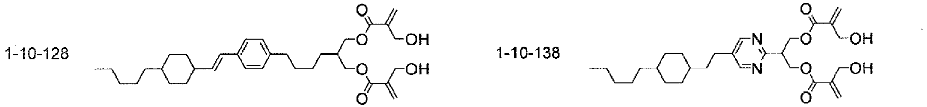

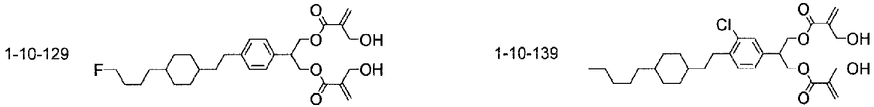

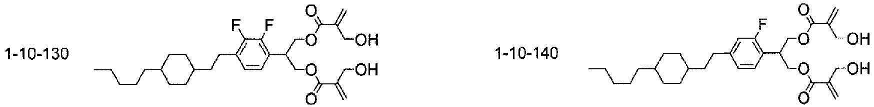

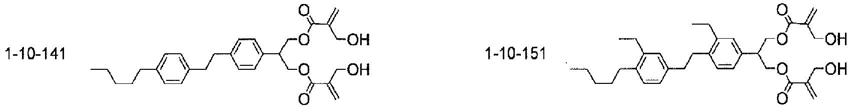

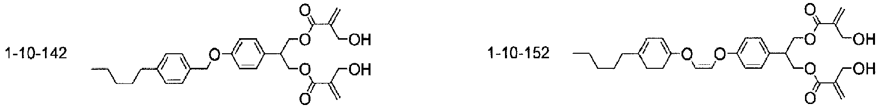

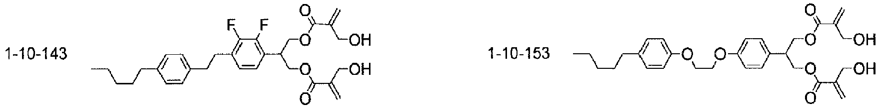

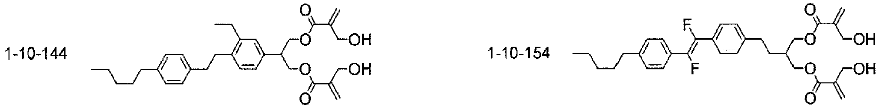

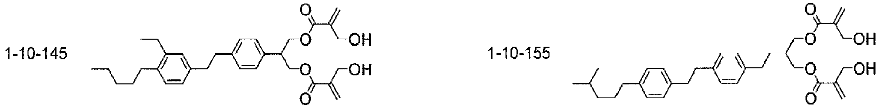

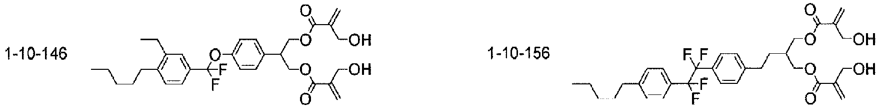

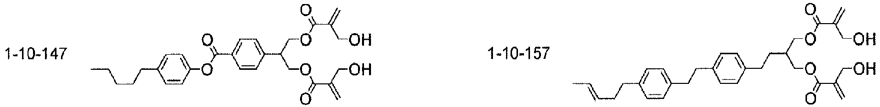

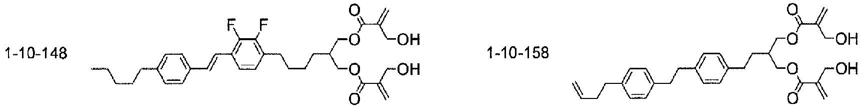

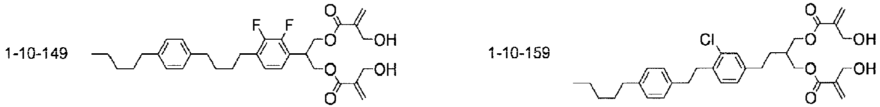

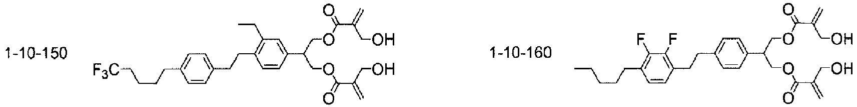

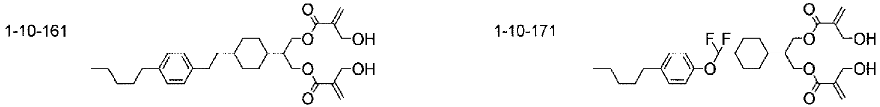

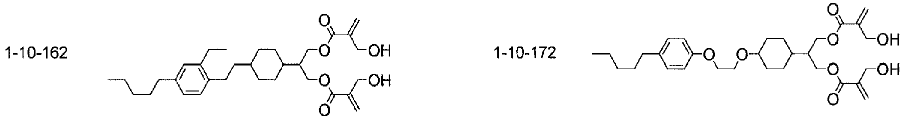

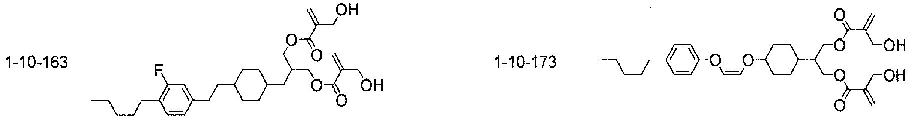

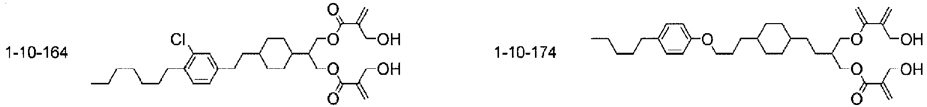

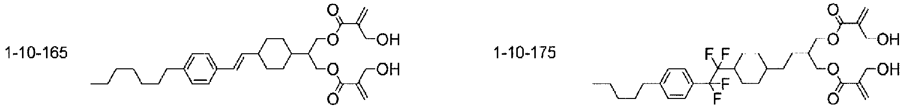

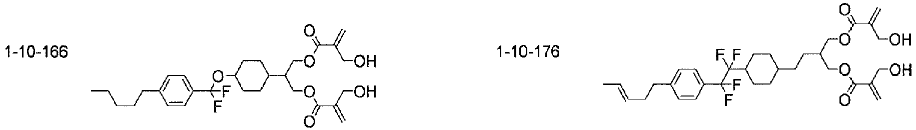

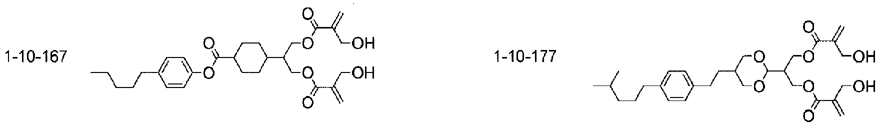

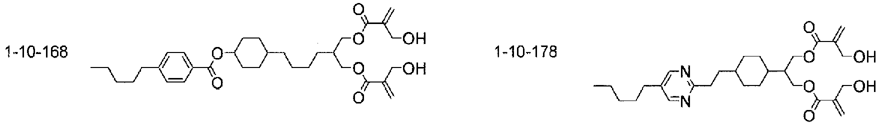

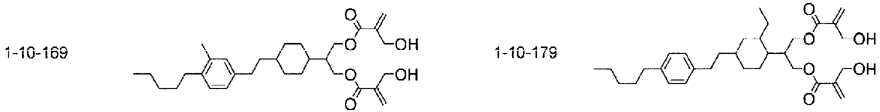

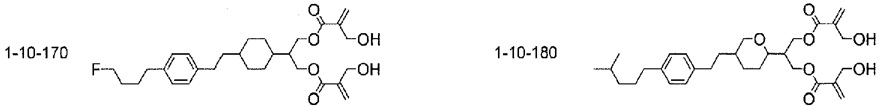

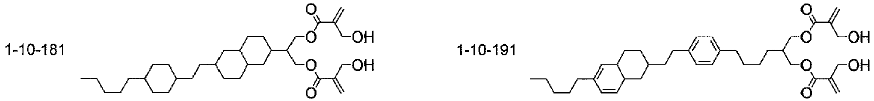

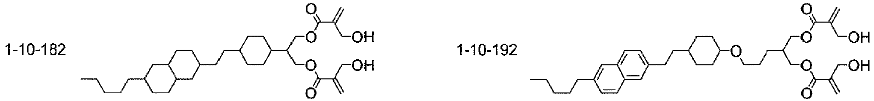

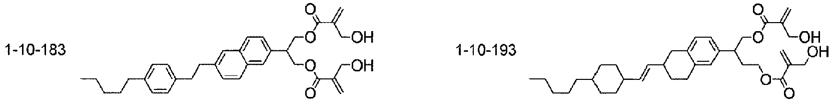

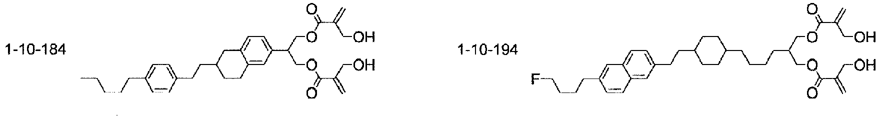

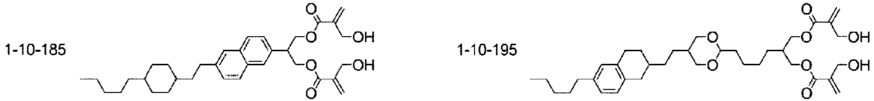

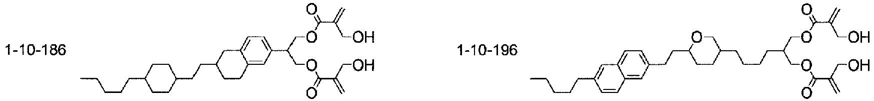

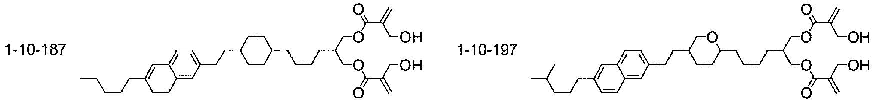

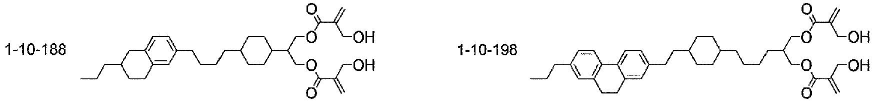

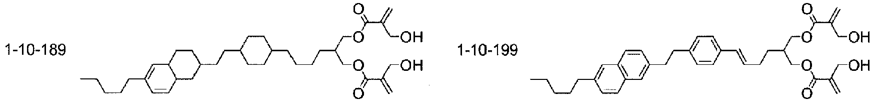

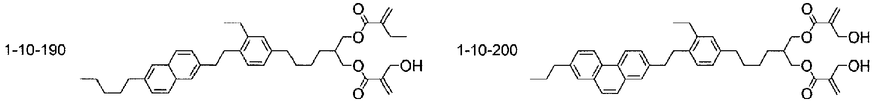

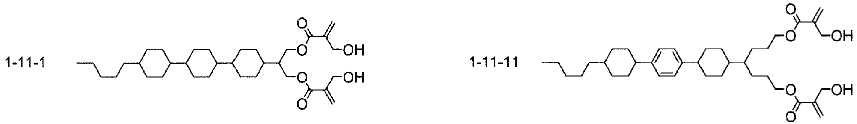

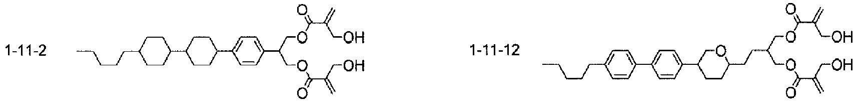

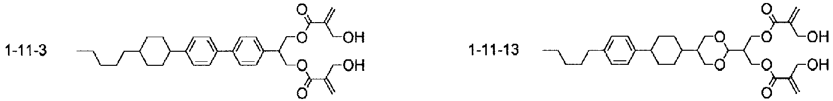

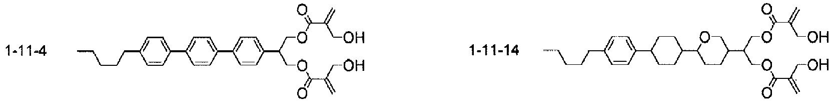

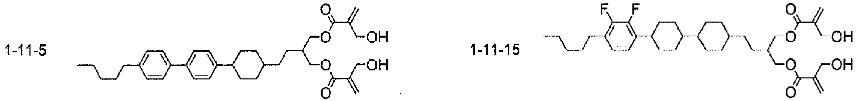

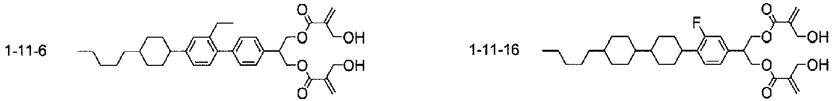

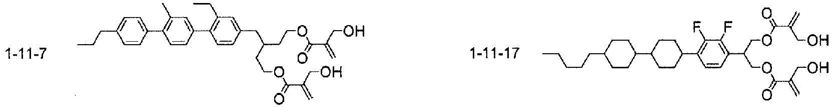

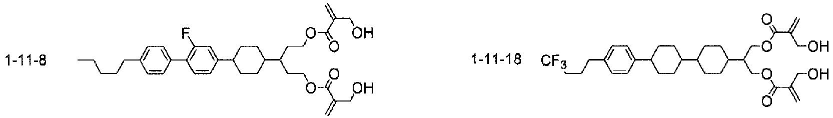

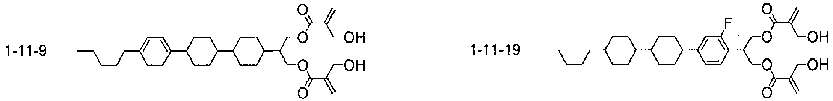

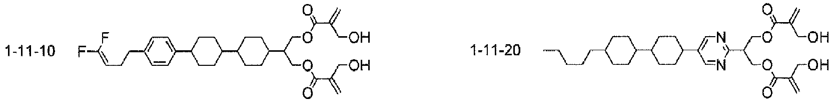

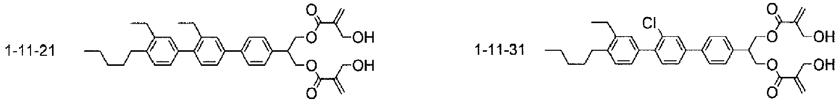

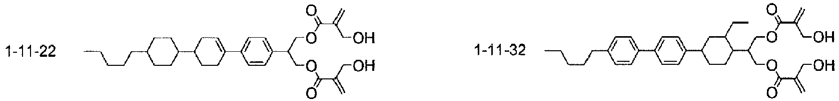

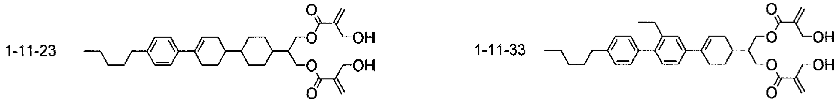

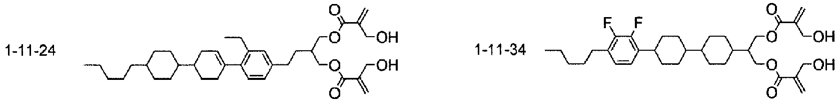

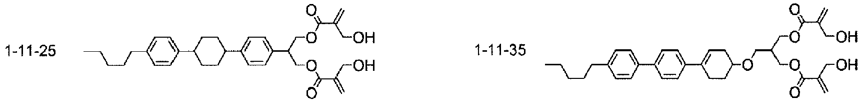

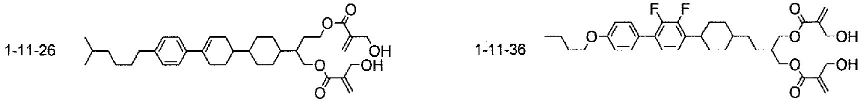

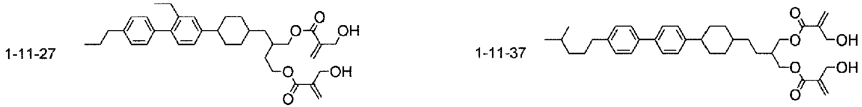

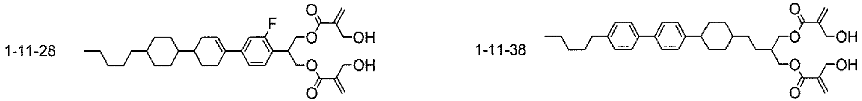

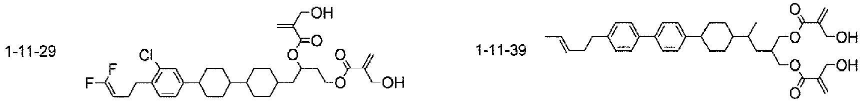

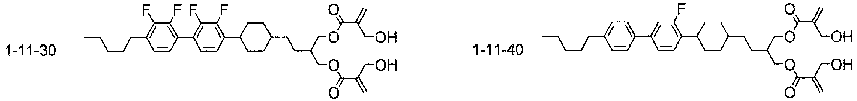

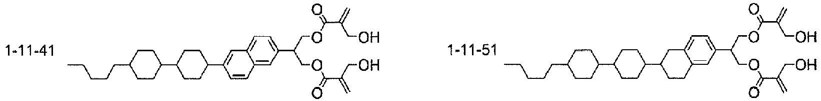

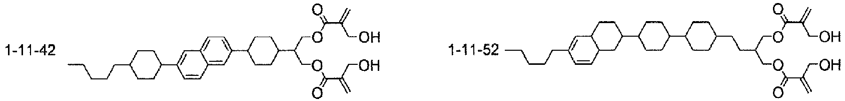

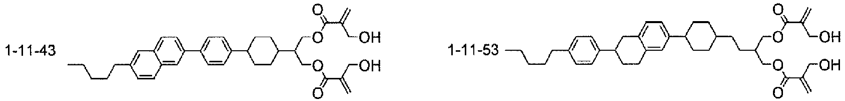

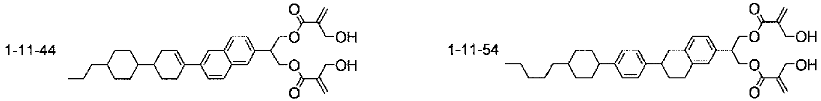

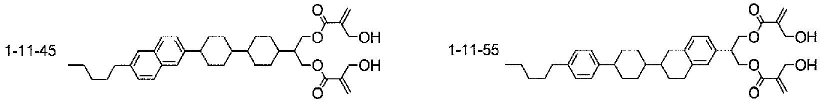

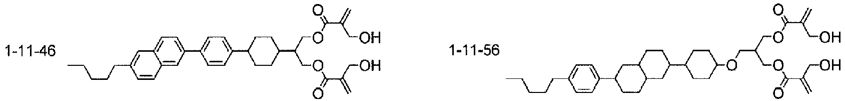

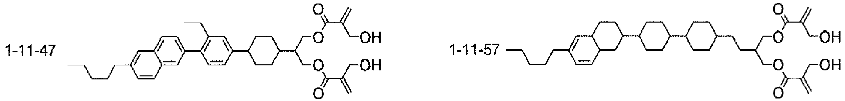

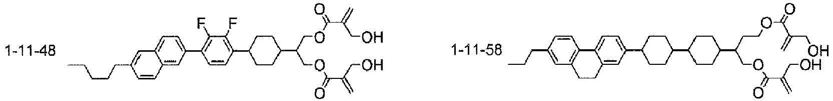

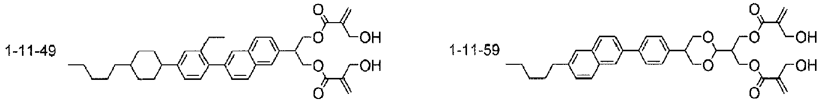

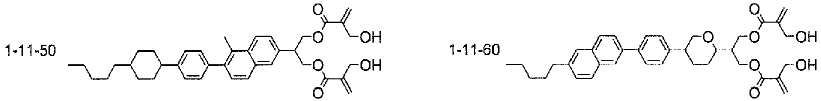

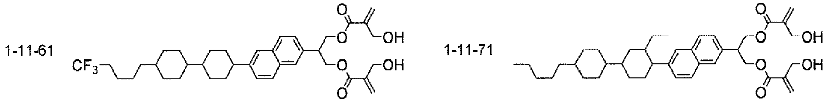

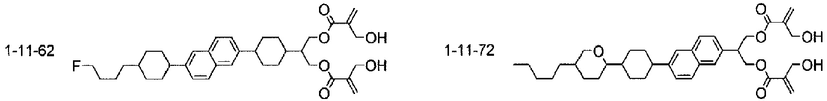

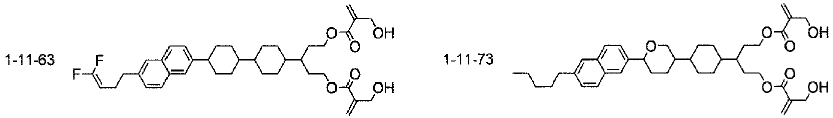

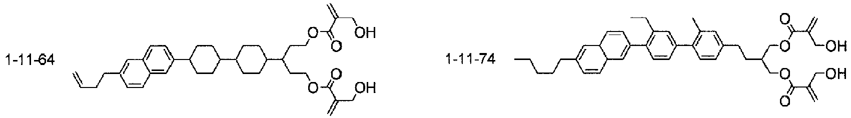

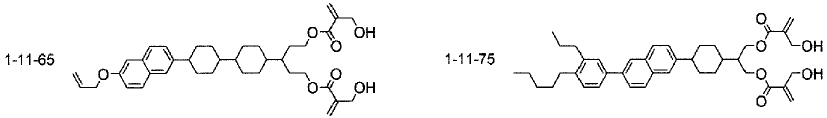

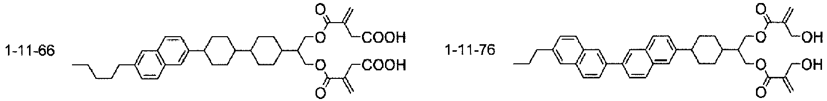

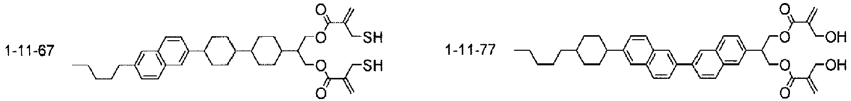

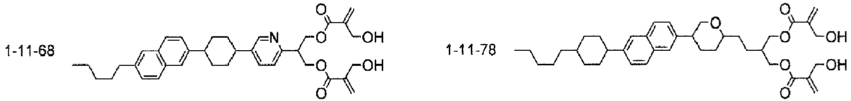

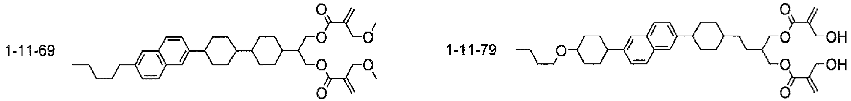

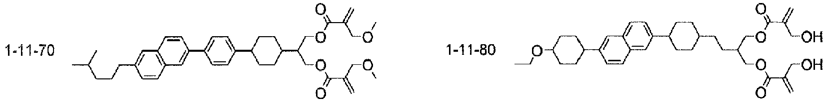

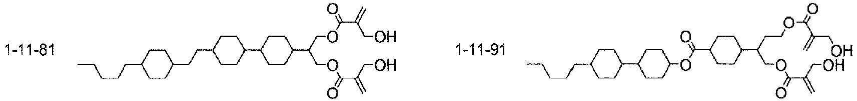

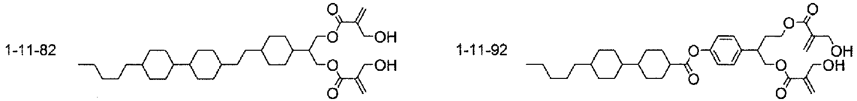

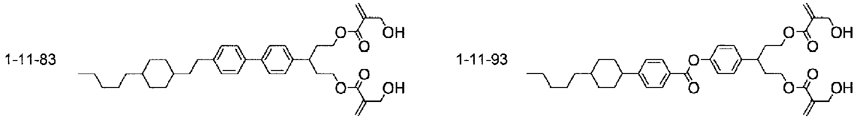

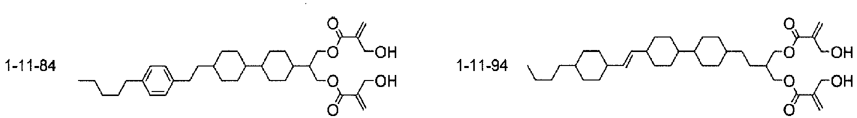

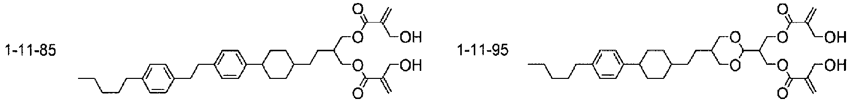

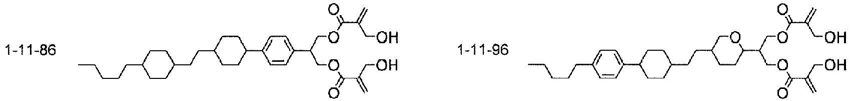

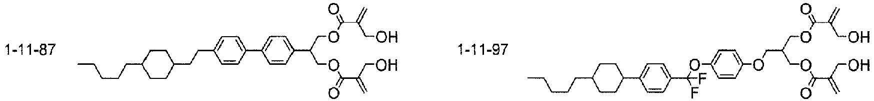

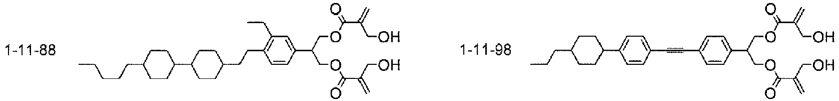

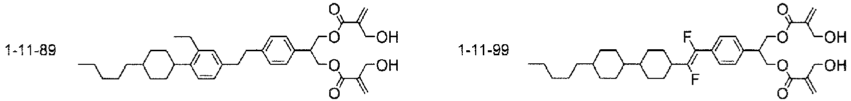

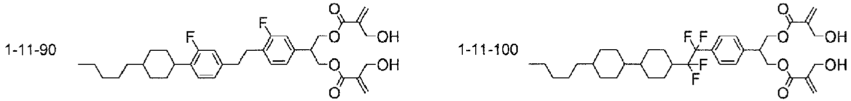

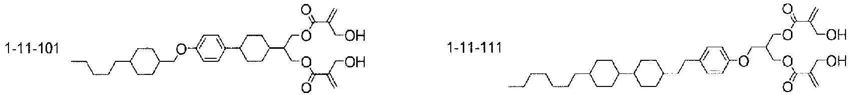

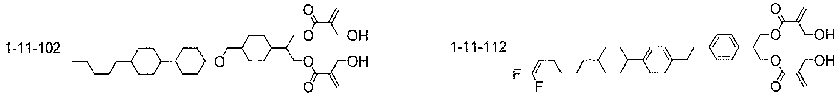

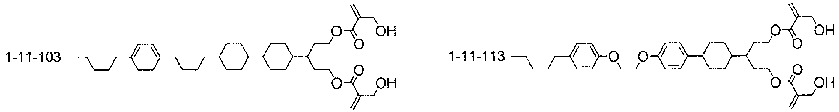

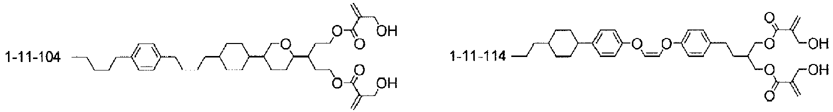

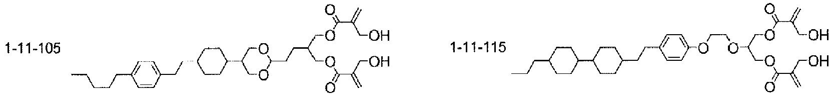

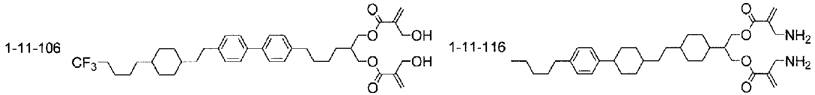

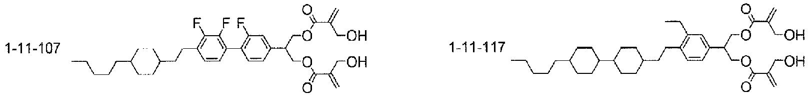

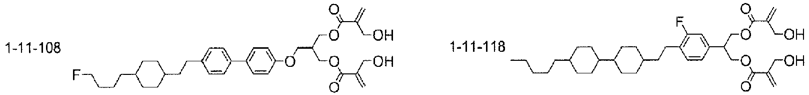

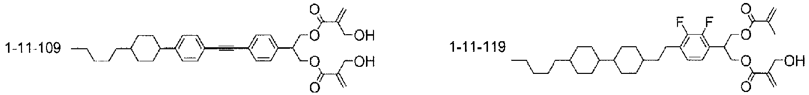

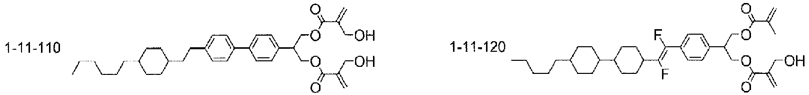

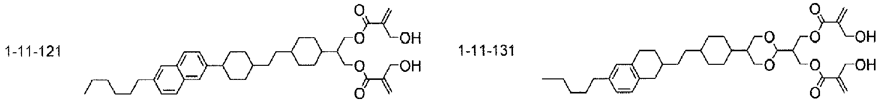

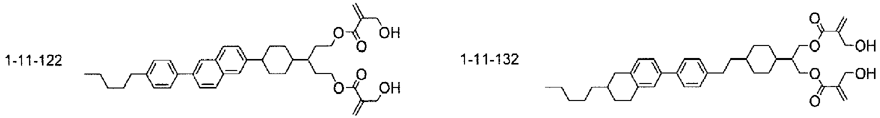

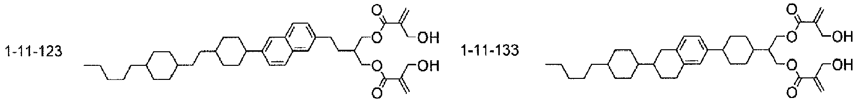

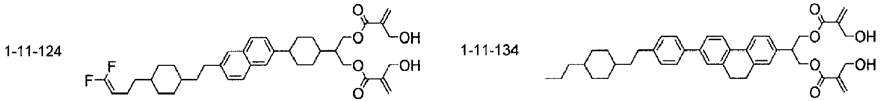

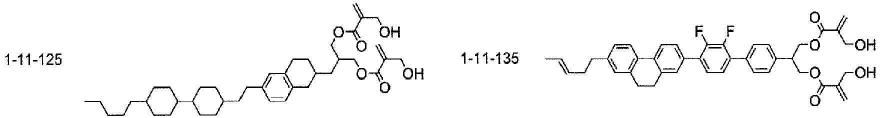

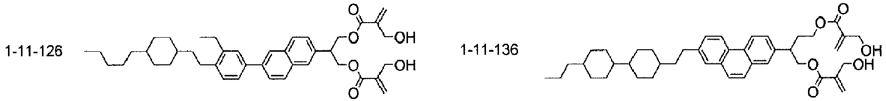

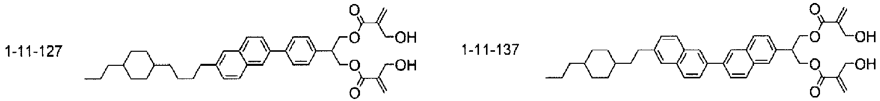

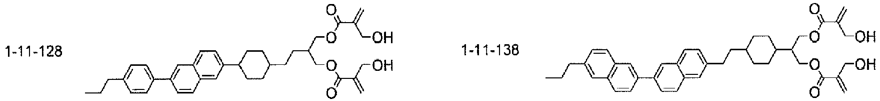

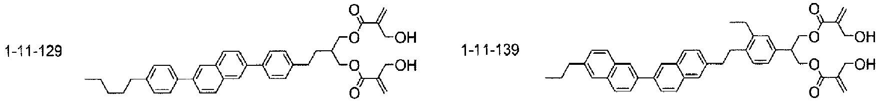

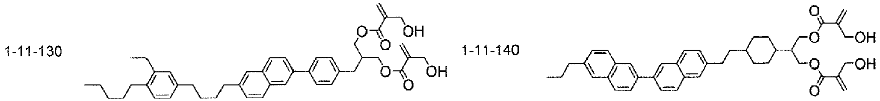

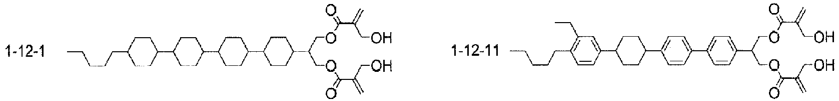

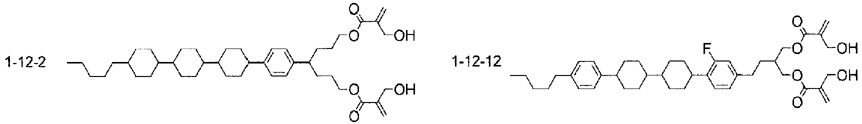

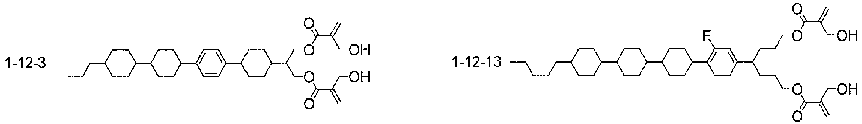

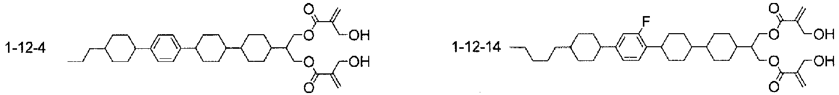

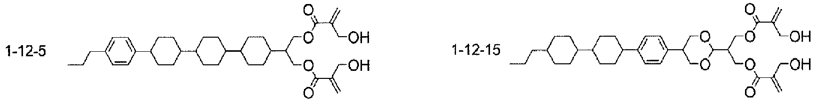

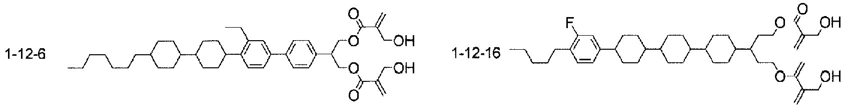

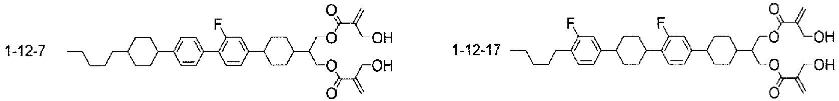

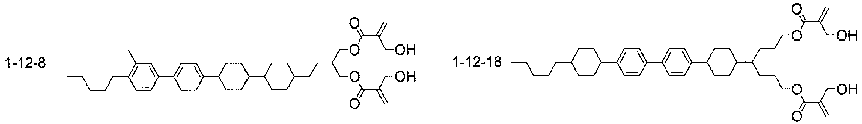

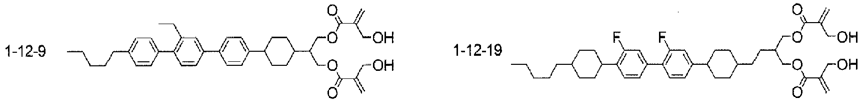

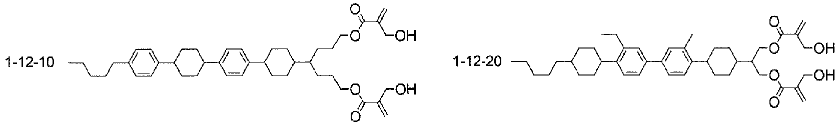

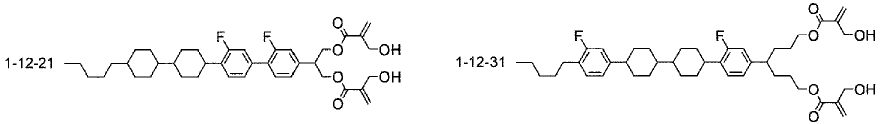

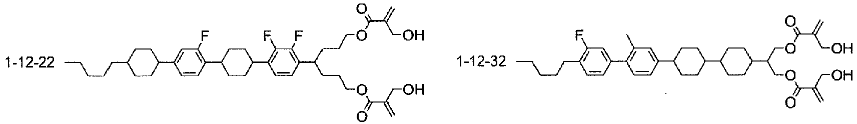

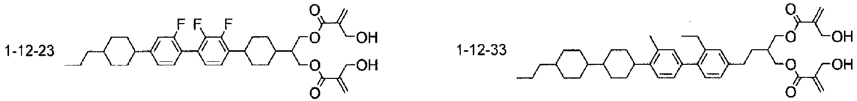

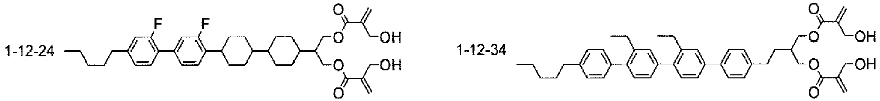

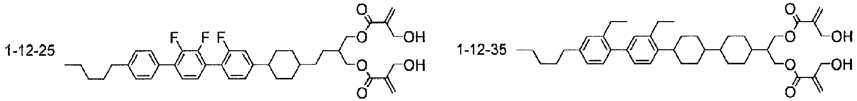

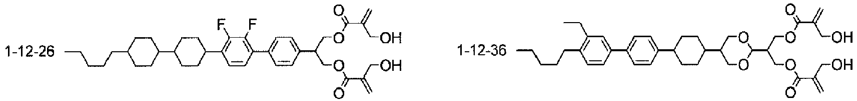

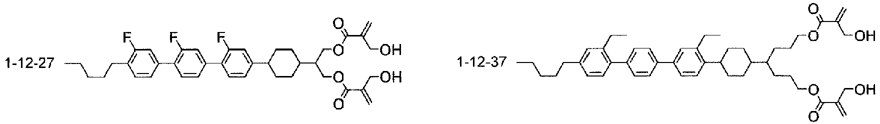

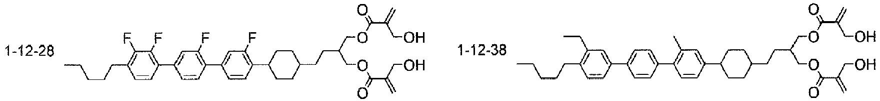

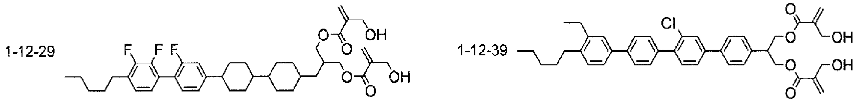

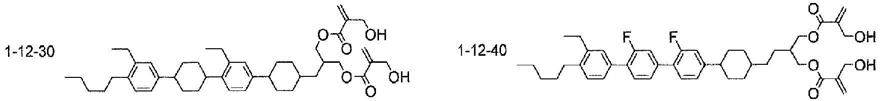

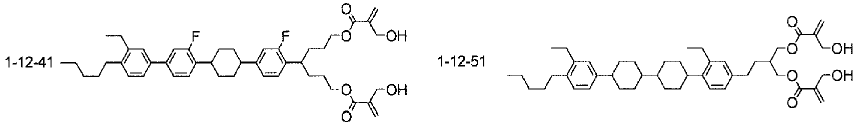

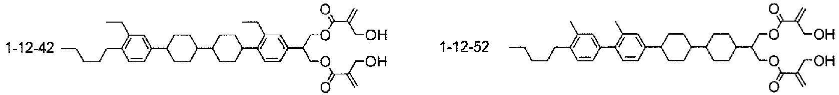

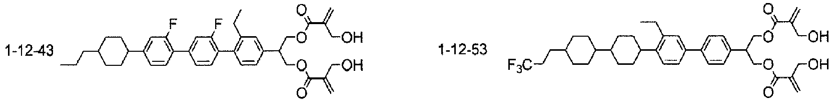

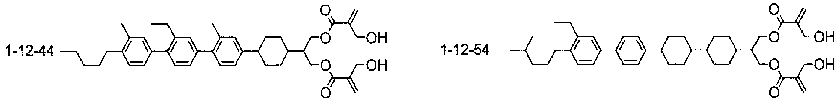

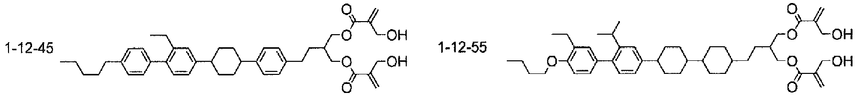

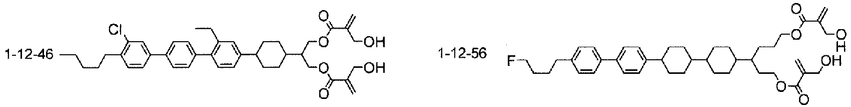

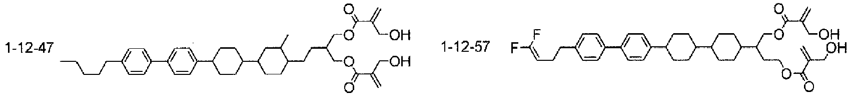

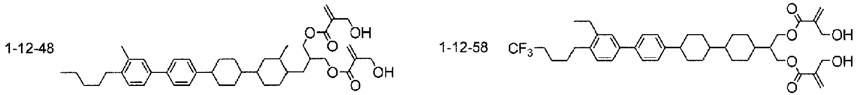

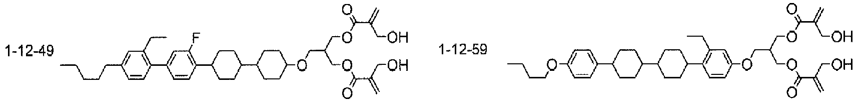

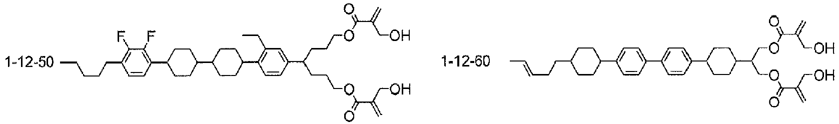

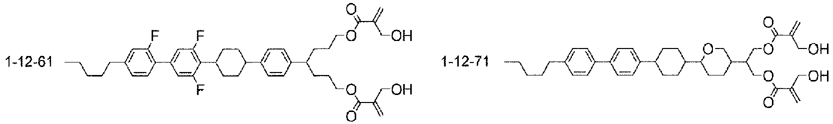

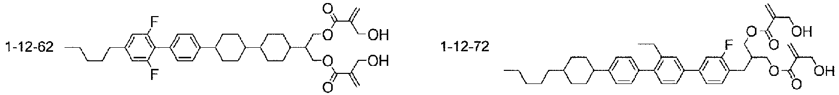

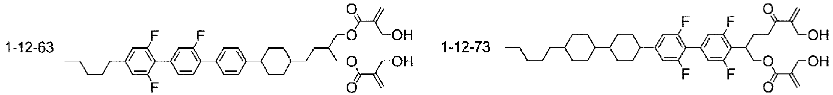

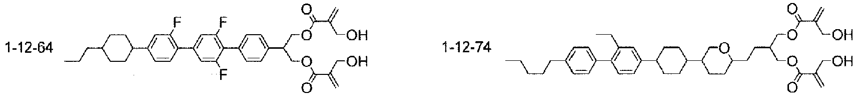

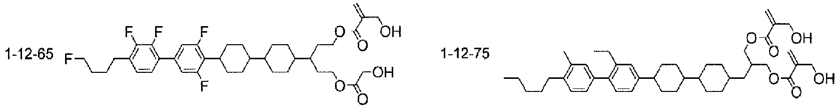

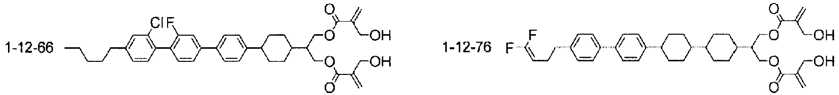

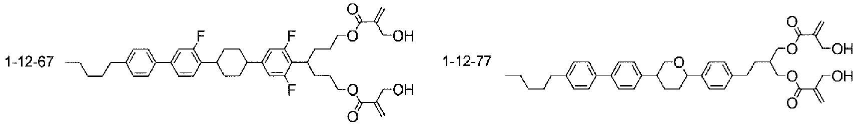

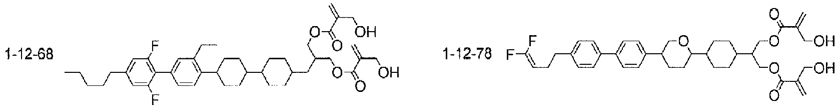

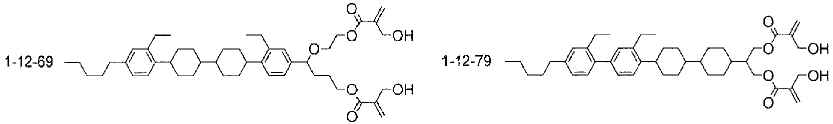

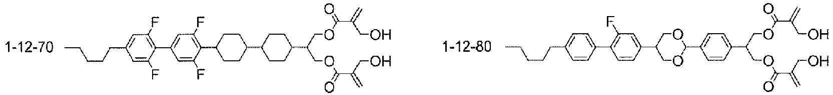

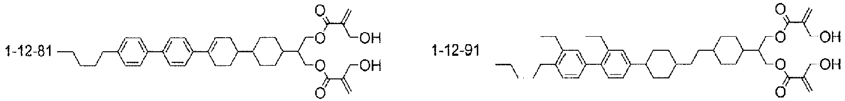

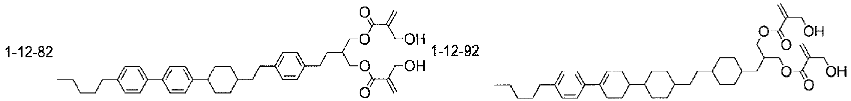

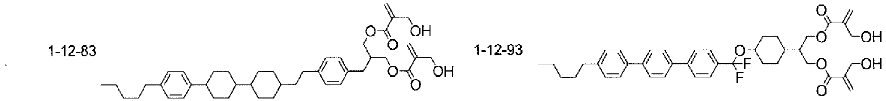

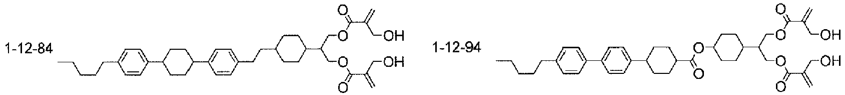

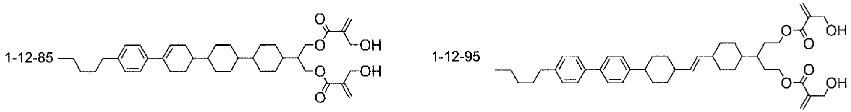

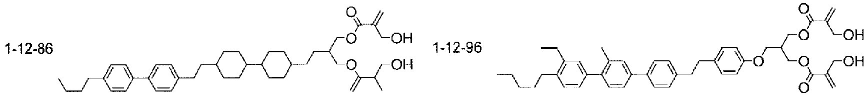

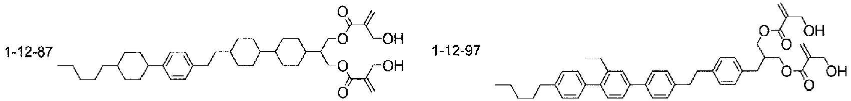

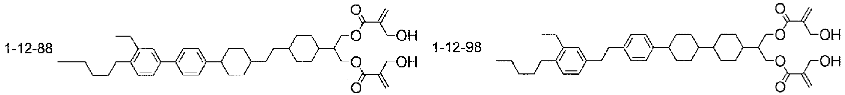

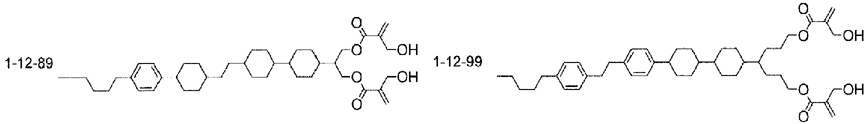

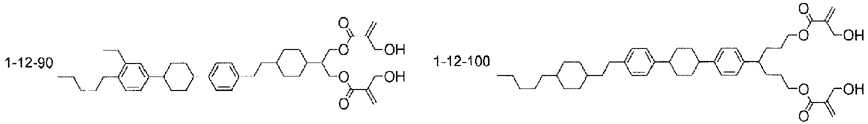

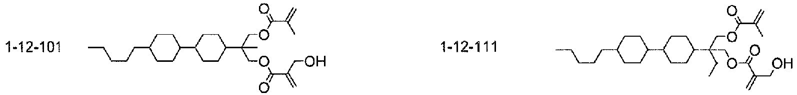

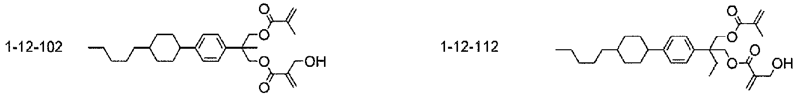

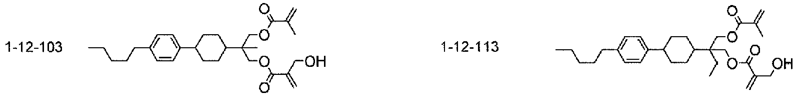

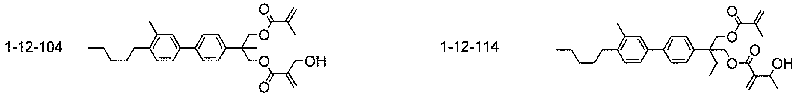

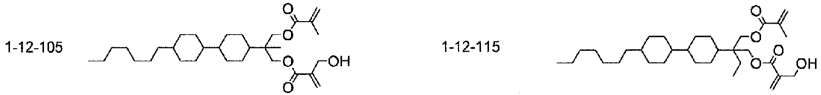

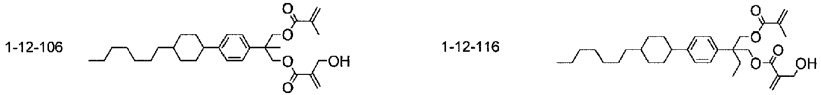

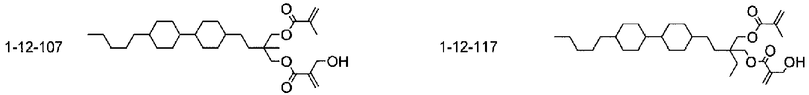

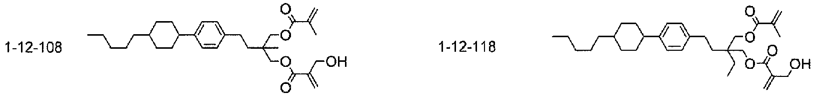

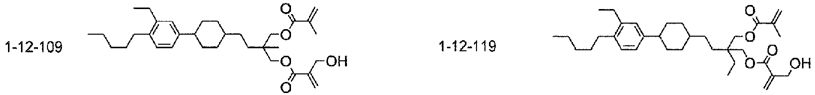

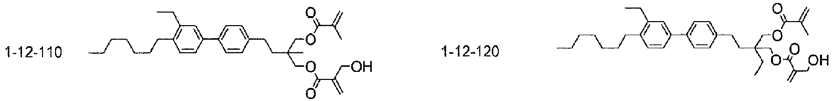

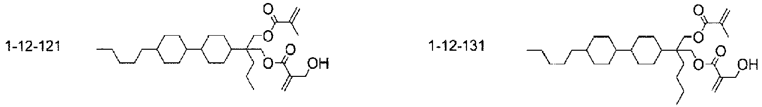

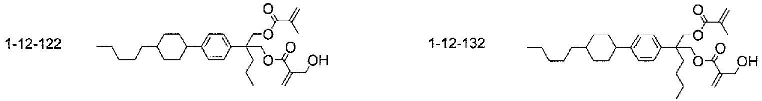

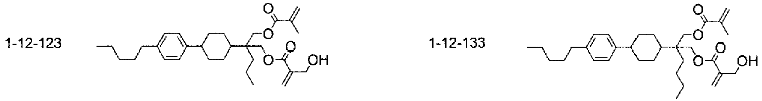

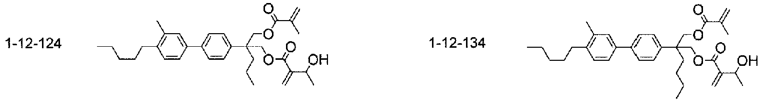

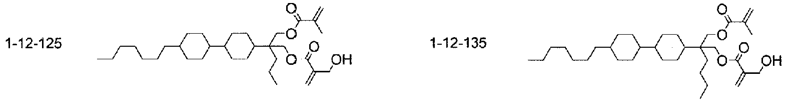

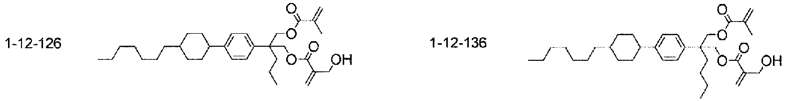

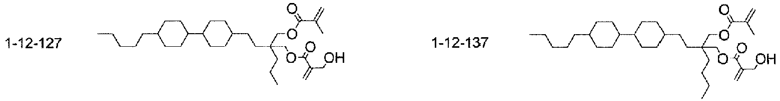

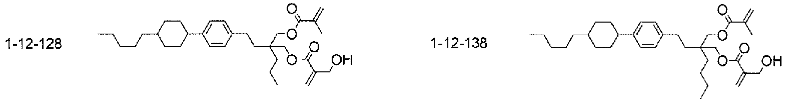

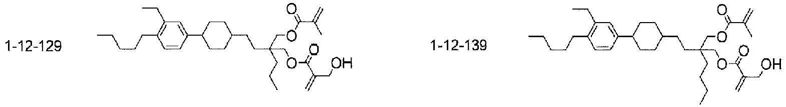

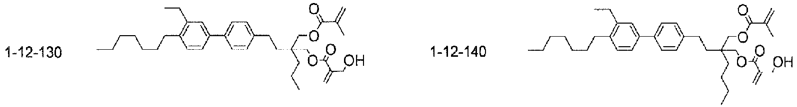

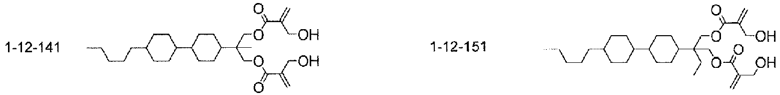

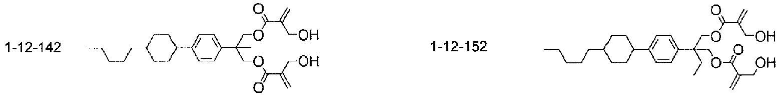

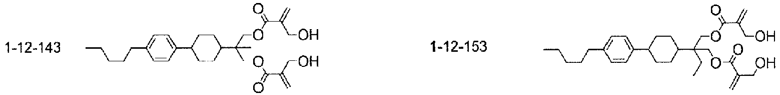

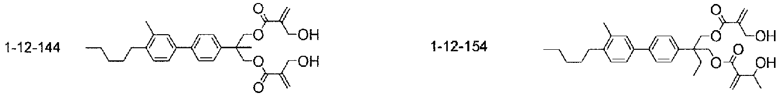

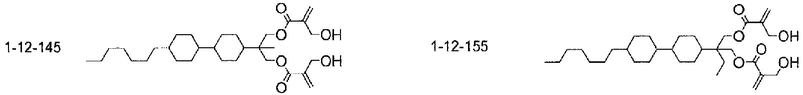

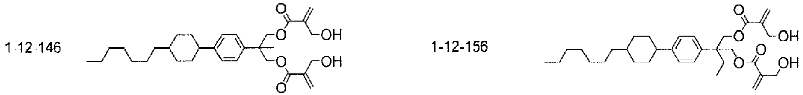

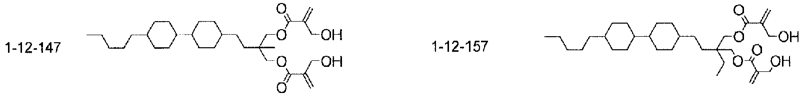

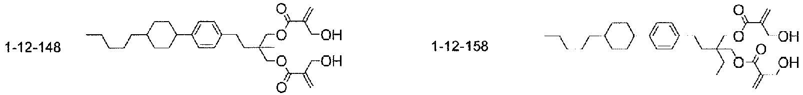

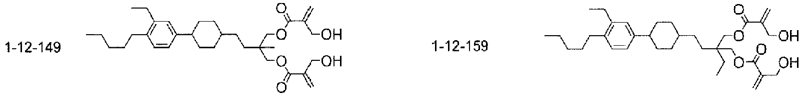

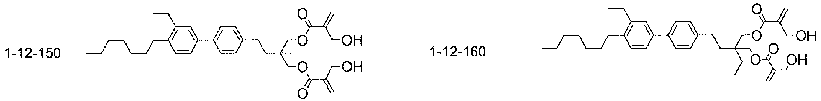

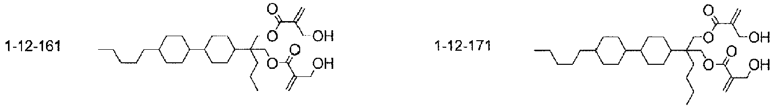

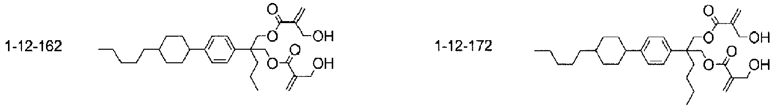

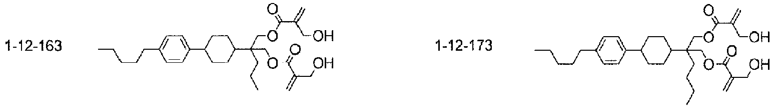

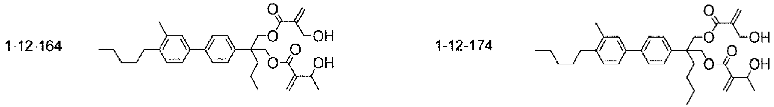

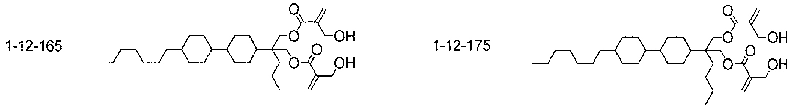

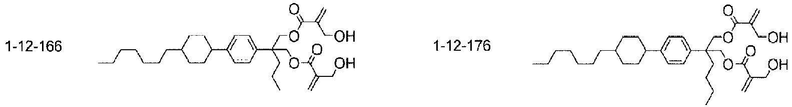

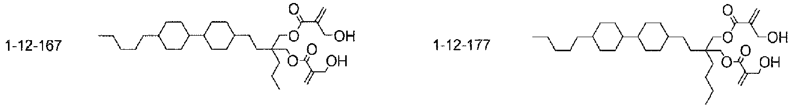

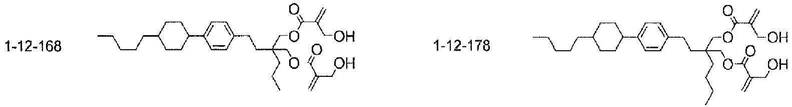

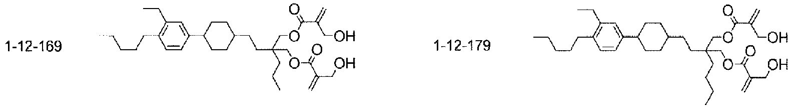

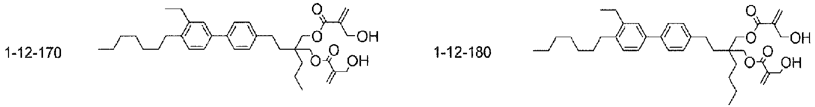

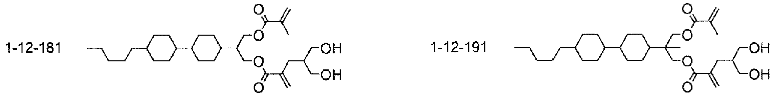

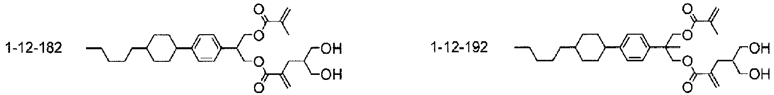

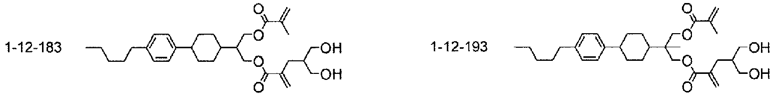

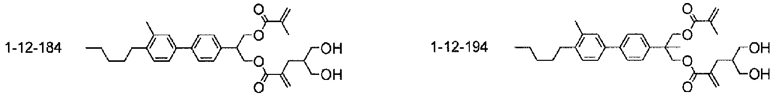

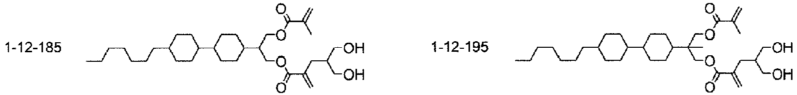

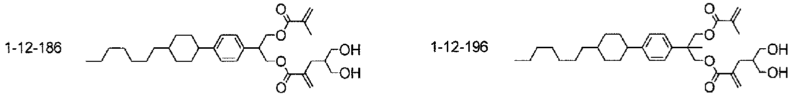

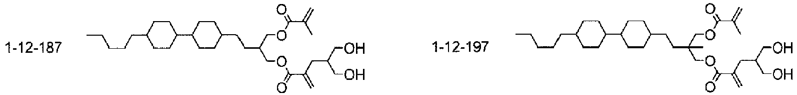

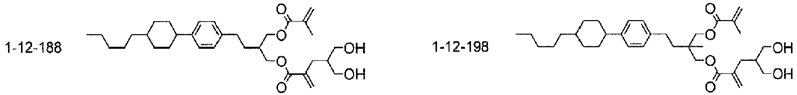

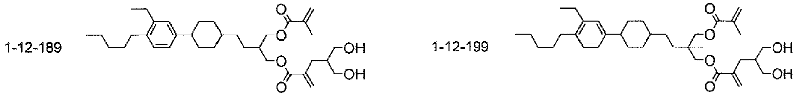

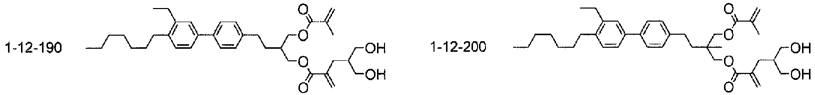

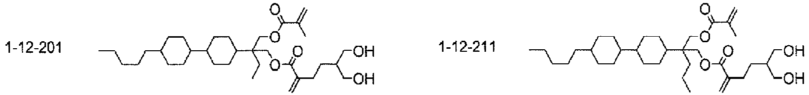

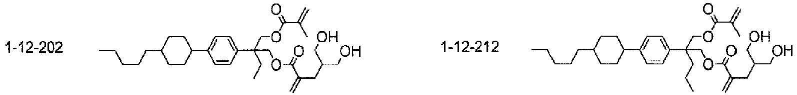

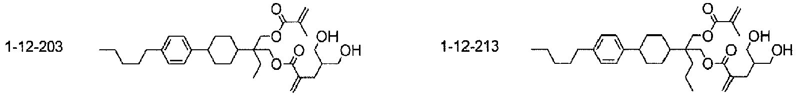

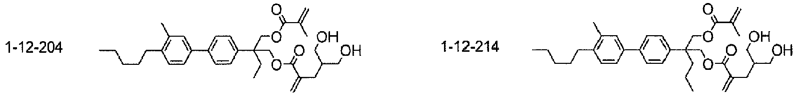

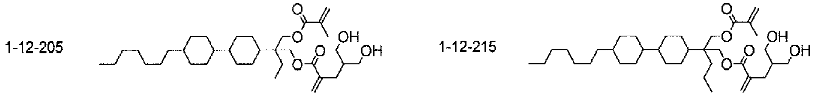

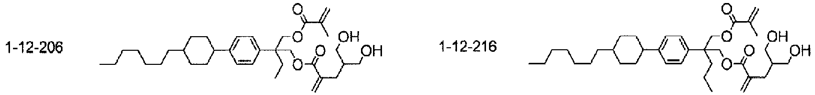

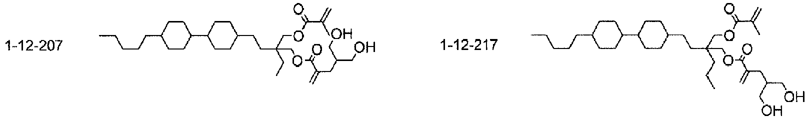

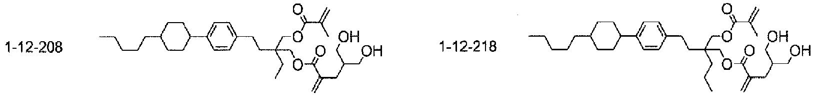

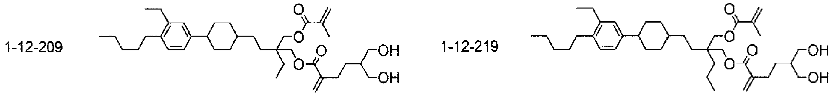

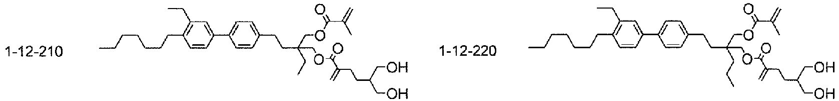

### 2. Examples of composition

The compounds in Examples were represented using symbols according to definitions in Table 4 described below. In Table 4, the configuration of 1,4-cyclohexylene is trans. A parenthesized number next to a symbolized compound corresponds to the number of the compound. A symbol (-) means any other liquid crystal compound. A proportion (percentage) of the liquid crystal compound is expressed in terms of weight percent (% by weight) based on the weight of the liquid crystal composition. Values of the characteristics of the composition were summarized in a last part. The characteristics were measured according to the methods described above, and measured values were directly described (without extrapolation).

### Table 4

**Table 4. Method of description of compounds using symbols**

| R-(A₁)-Z₁-·····-Zₙ-(Aₙ)-R' | | | |
|---|---|---|---|
| 1) Left-terminal group R- | Symbol | 4) Ring structure -Aₙ- | Symbol |
| CₙH₂ₙ₊₁- | n- | 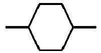 | H |
| CₙH₂ₙ₊₁O- | nO- | | |
| CₘH₂ₘ₊₁OCₙH₂ₙ- | mOn- | 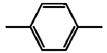 | B |
| CH₂=CH- | V- | | |
| CₙH₂ₙ₊₁-CH=CH- | nV- | | |
| CH₂=CH-CₙH₂ₙ- | Vn- | 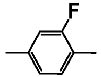 | B(F) |
| CₘH₂ₘ₊₁-C H=CH-CₙH₂ₙ- | mVn- | | |
| CF₂=CH- | \/FF- | 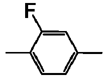 | B(2F) |
| CF₂=CH-CₙH₂ₙ- | VFFn- | | |
| 2) Right-terminal group -R' | Symbol | | |
| -CₙH₂ₙ₊₁ | -n | | |
| -OCₙH₂ₙ₊₁ | -On | 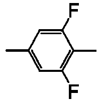 | B(F,F) |
| -COOCH₃ | -EMe | | |
| -CH=CH₂ | -V | | |
| -CH=CH-CₙH₂ₙ₊₁ | -Vn | 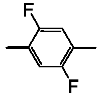 | B(2F,5F) |
| -CₙH₂ₙ-CH=CH₂ | -nV | | |
| -CₘH₂ₘ-CH=CH-CₙH₂ₙ₊₁ | -mVn | | |
| -CH=CF₂ | -VFF | 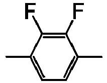 | B(2F,3F) |
| -F | -F | | |
| -Cl | -CL | | |
| -OCF₃ | -OCF3 | 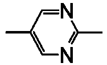 | Py |
| -OCF₂H | -OCF2H | | |
| -CF₃ | -CF3 | | |
| -OCH=CH-CF₃ | -OVCF3 | 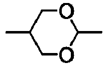 | G |
| -C≡N | -C | | |
| 3) Bonding group -Zₙ- | Symbol | | |
| -CₙH₂ₙ- | n | 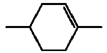 | ch |
| -COO- | E | | |
| -CH=CH- | V | | |
| -CH₂O- | 1O | | |
| -OCH₂- | O1 | | |
| -CF₂O- | X | | |
| -C≡C- | T | | |
| 5) Examples of description | | | |
| Example 1. 3-HB-CL | | Example 2. 5-HHBB(F,F)-F | |
| 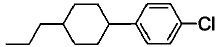 | | 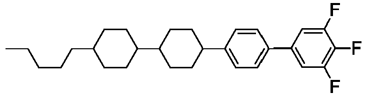 | |
| Example 3. 3-HB-O2 | | Example 4. 3-HBB(F,F)-F | |
| 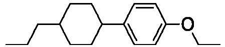 | | 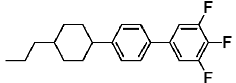 | |

### Use Example 1

| | | |
|---|---|---|
| 3-HHB(F,F)-F | (6-3) | 10% |
| 3-H2HB(F,F)-F | (6-15) | 7% |
| 4-H2HB(F,F)-F | (6-15) | 7% |
| 5-H2HB(F,F)-F | (6-15) | 9% |
| 3-HBB(F,F)-F | (6-24) | 19% |
| 5-HBB(F,F)-F | (6-24) | 20% |
| 3-H2BB(F,F)-F | (6-27) | 11% |
| 5-HHBB(F,F)-F | (7-6) | 3% |
| 5-HHEBB-F | (7-17) | 2% |
| 3-HH2BB(F,F)-F | (7-15) | 3% |
| 1O1-HBBH-4 | (4-1) | 5% |
| 1O1-HBBH-5 | (4-1) | 4% |

Compound (1-2-1) described below was added to the above composition in a proportion of 3% by weight.

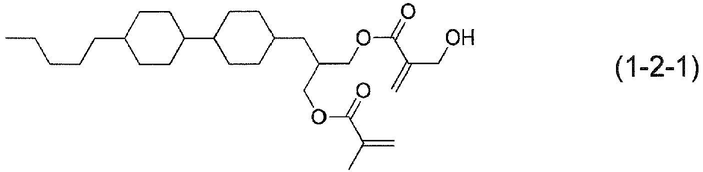

NI = 100.2°C; η = 35.1 mPa·s; Δn = 0.117; Δε = 8.9.

### Use Example 2

| | | |
|---|---|---|
| 2-HB-C | (8-1) | 6% |
| 3-HB-C | (8-1) | 13% |
| 3-HB-O2 | (2-5) | 16% |
| 2-BTB-1 | (2-10) | 4% |
| 3-HHB-F | (6-1) | 5% |
| 3-HHB-1 | (3-1) | 7% |
| 3-HHB-O1 | (3-1) | 4% |
| 3-HHB-3 | (3-1) | 13% |
| 3-HHEB-F | (6-10) | 3% |
| 5-HHEB-F | (6-10) | 3% |
| 2-HHB(F)-F | (6-2) | 6% |
| 3-HHB(F)-F | (6-2) | 6% |
| 5-HHB(F)-F | (6-2) | 8% |
| 3-HHB(F,F)-F | (6-3) | 6% |

Compound (1-2-2) described below was added to the above composition in a proportion of 2% by weight.

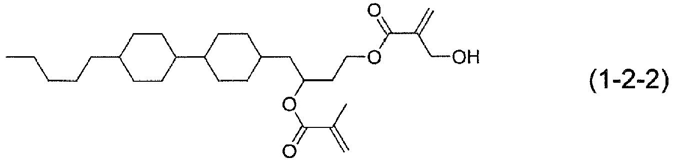

NI = 93.1°C; η = 15.9 mPa·s; Δn = 0.100; Δε = 4.8.

### Use Example 3

| | | |
|---|---|---|
| 7-HB(F,F)-F | (5-4) | 5% |
| 3-HB-O2 | (2-5) | 9% |
| 2-HHB(F)-F | (6-2) | 12% |
| 3-HHB(F)-F | (6-2) | 8% |
| 5-HHB(F)-F | (6-2) | 8% |
| 2-HBB(F)-F | (6-23) | 7% |
| 3-HBB(F)-F | (6-23) | 10% |
| 5-HBB(F)-F | (6-23) | 15% |
| 2-HBB-F | (6-22) | 5% |
| 3-HBB-F | (6-22) | 5% |
| 5-HBB-F | (6-22) | 4% |
| 3-HBB(F,F)-F | (6-24) | 4% |
| 5-HBB(F,F)-F | (6-24) | 8% |

Compound (1-2-18) described below was added to the above composition in a proportion of 4% by weight.

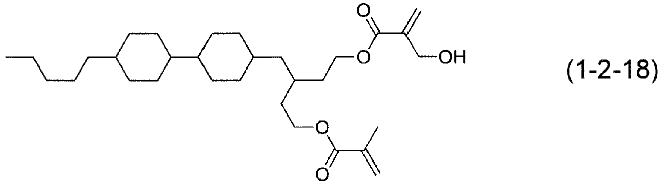

NI = 82.0°C; η = 23.3 mPa·s; Δn = 0.113; Δε = 5.3.

### Use Example 4

| | | |
|---|---|---|
| 5-HB-CL | (5-2) | 5% |
| 7-HB(F)-F | (5-3) | 5% |
| 3-HH-4 | (2-1) | 8% |
| 3-HH-5 | (2-1) | 10% |
| 3-HB-O2 | (2-5) | 10% |
| 3-HHEB-F | (6-10) | 10% |
| 5-HHEB-F | (6-10) | 9% |
| 3-HHEB(F,F)-F | (6-12) | 9% |
| 4-HHEB(F,F)-F | (6-12) | 5% |
| 3-GHB(F,F)-F | (6-109) | 5% |
| 4-GHB(F,F)-F | (6-109) | 6% |
| 5-GHB(F,F)-F | (6-109) | 7% |
| 2-HHB(F,F)-F | (6-3) | 5% |
| 3-HHB(F,F)-F | (6-3) | 6% |

Compound (1-2-28) described below was added to the above composition in a proportion of 7% by weight.

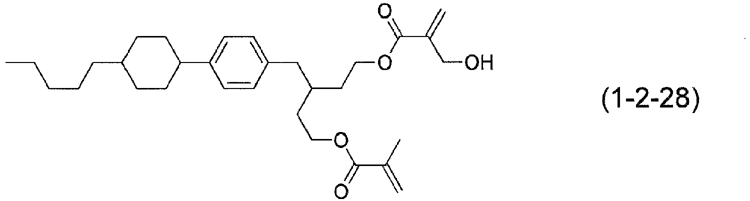

NI = 75.5°C; η = 20.4 mPa·s; Δn = 0.069; Δε = 6.1.

### Use Example 5

| | | |
|---|---|---|
| 5-HB-CL | (5-2) | 15% |
| 3-HH-4 | (2-1) | 12% |
| 3-HH-5 | (2-1) | 4% |
| 3-HHB-F | (6-1) | 4% |
| 3-HHB-CL | (6-1) | 3% |
| 4-HHB-CL | (6-1) | 4% |
| 3-HHB(F)-F | (6-2) | 11% |
| 4-HHB(F)-F | (6-2) | 10% |
| 5-HHB(F)-F | (6-2) | 8% |
| 7-HHB(F)-F | (6-2) | 8% |
| 5-HBB(F)-F | (6-23) | 3% |
| 1O1-HBBH-5 | (4-1) | 3% |
| 3-HHBB(F,F)-F | (7-6) | 3% |
| 4-HHBB(F,F)-F | (7-6) | 3% |
| 5-HHBB(F,F)-F | (7-6) | 3% |
| 3-HH2BB(F,F)-F | (7-15) | 3% |
| 4-HH2BB(F,F)-F | (7-15) | 3% |

Compound (1-3-23) described below was added to the above composition in a proportion of 6% by weight.

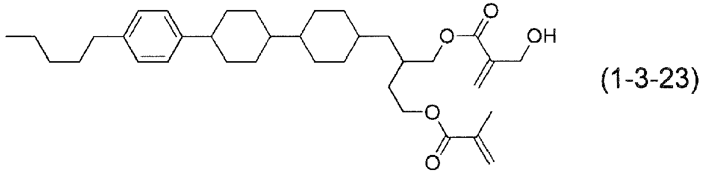

NI = 115.9°C; η = 19.6 mPa·s; Δn = 0.091; Δε = 3.8.

### Use Example 6

| | | |
|---|---|---|
| 3-HB-CL | (5-2) | 11% |
| 3-HH-4 | (2-1) | 14% |
| 3-HB-O2 | (2-5) | 6% |
| 3-HHB(F,F)-F | (6-3) | 5% |
| 3-HBB(F,F)-F | (6-24) | 32% |
| 5-HBB(F,F)-F | (6-24) | 22% |
| 5-HBB(F)B-2 | (4-5) | 5% |
| 5-HBB(F)B-3 | (4-5) | 5% |

Compound (1-2-77) described below was added to the above composition in a proportion of 3% by weight.

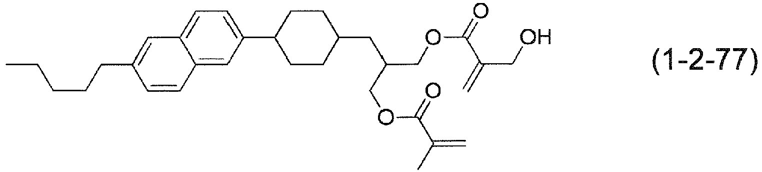

NI = 72.9°C; η = 19.7 mPa·s; Δn = 0.115; Δε = 5.5.

### Use Example 7

| | | |
|---|---|---|
| 5-HB-F | (5-2) | 12% |
| 6-HB-F | (5-2) | 9% |
| 7-HB-F | (5-2) | 7% |
| 2-HHB-OCF3 | (6-1) | 5% |
| 3-HHB-OCF3 | (6-1) | 8% |
| 4-HHB-OCF3 | (6-1) | 7% |
| 5-HHB-OCF3 | (6-1) | 5% |
| 3-HH2B-OCF3 | (6-4) | 5% |
| 5-HH2B-OCF3 | (6-4) | 4% |
| 3-HHB(F,F)-OCF2H | (6-3) | 3% |
| 3-HHB(F,F)-OCF3 | (6-3) | 4% |
| 3-HH2B(F)-F | (6-5) | 3% |
| 3-HBB(F)-F | (6-23) | 10% |
| 5-HBB(F)-F | (6-23) | 10% |
| 5-HBBH-3 | (4-1) | 5% |
| 3-HB(F)BH-3 | (4-2) | 3% |

Compound (1-2-24) described below was added to the above composition in a proportion of 5% by weight.

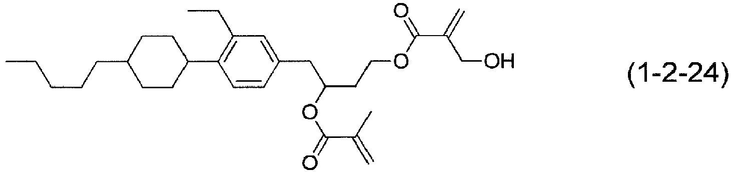

NI = 89.1°C; η = 15.1 mPa·s; Δn = 0.094; Δε = 4.3.

### Use Example 8

| | | |
|---|---|---|
| 5-HB-CL | (5-1) | 14% |
| 7-HB(F,F)-F | (5-4) | 5% |
| 3-HH-4 | (2-1) | 11% |
| 3-HH-5 | (2-1) | 6% |
| 3-HB-O2 | (2-5) | 12% |
| 3-HHB-1 | (3-1) | 10% |
| 3-HHB-O1 | (3-1) | 3% |
| 2-HHB(F)-F | (6-2) | 5% |
| 3-HHB(F)-F | (6-2) | 8% |
| 5-HHB(F)-F | (6-2) | 7% |
| 3-HHB(F,F)-F | (6-3) | 7% |
| 3-H2HB(F,F)-F | (6-15) | 7% |
| 4-H2HB(F,F)-F | (6-15) | 5% |

Compound (1-3-68) described below was added to the above composition in a proportion of 2% by weight.

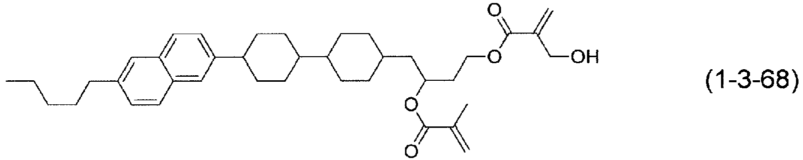

NI = 71.4°C; η = 14.4 mPa·s; Δn = 0.071; Δε = 2.9.

### Use Example 9

| | | |
|---|---|---|
| 5-HB-CL | (5-1) | 13% |
| 3-HH-4 | (2-5) | 5% |
| 3-HHB-1 | (3-1) | 5% |
| 3-HHB(F,F)-F | (6-3) | 7% |
| 3-HBB(F,F)-F | (6-24) | 21% |
| 5-HBB(F,F)-F | (6-24) | 13% |
| 3-HHEB(F,F)-F | (6-12) | 11% |
| 4-HHEB(F,F)-F | (6-12) | 3% |
| 5-HHEB(F,F)-F | (6-12) | 3% |
| 2-HBEB(F,F)-F | (6-39) | 4% |
| 3-HBEB(F,F)-F | (6-39) | 3% |
| 5-HBEB(F,F)-F | (6-39) | 3% |
| 3-HHBB(F,F)-F | (7-6) | 6% |
| 5-HB-O2 | (2-5) | 3% |

Compound (1-2-25) described below was added to the above composition in a proportion of 4% by weight.

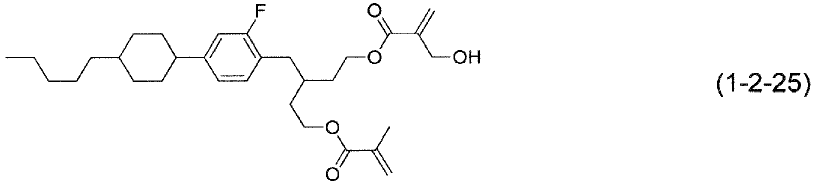

NI = 116.7°C; η = 20.6 mPa·s; Δn = 0.093; Δε = 4.0.

### Use Example 10

| | | |
|---|---|---|
| 1V2-BEB(F,F)-C | (8-15) | 5% |
| 3-HB-C | (8-1) | 20% |
| 2-BTB-1 | (2-10) | 10% |
| 5-HH-VFF | (2-1) | 27% |
| 3-HHB-1 | (3-1) | 5% |
| VFF-HHB-1 | (3-1) | 9% |
| VFF2-HHB-1 | (3-1) | 12% |
| 3-H2BTB-2 | (3-17) | 4% |
| 3-H2BTB-3 | (3-17) | 4% |
| 3-H2BTB-4 | (3-17) | 4% |

Compound (1-4-127) described below was added to the above composition in a proportion of 3% by weight.

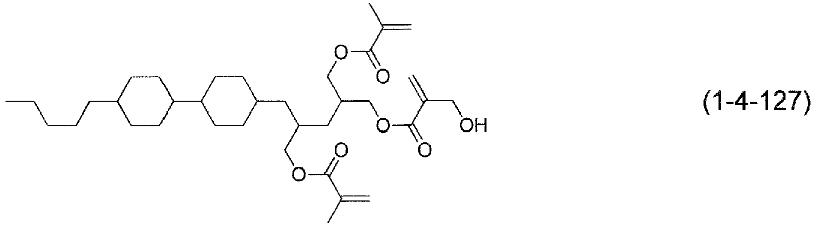

NI = 83.4°C; η = 12.2 mPa·s; Δn = 0.131; Δε = 6.1.

### Use Example 11

| | | |
|---|---|---|
| 3-HB-CL | (5-2) | 5% |
| 5-HB-CL | (5-2) | 3% |
| 3-HHB-OCF3 | (6-1) | 4% |
| 3-H2HB-OCF3 | (6-13) | 5% |
| 5-H4HB-OCF3 | (6-19) | 15% |
| V-HHB(F)-F | (6-2) | 4% |
| 3-HHB(F)-F | (6-2) | 6% |
| 5-HHB(F)-F | (6-2) | 6% |
| 3-H4HB(F,F)-CF3 | (6-21) | 8% |
| 5-H4HB(F,F)-CF3 | (6-21) | 10% |
| 5-H2HB(F,F)-F | (6-15) | 7% |
| 5-H4HB(F,F)-F | (6-21) | 7% |
| 2-H2BB(F)-F | (6-26) | 5% |
| 3-H2BB(F)-F | (6-26) | 10% |
| 3-HBEB(F,F)-F | (6-39) | 5% |

Compound (1-2-17) described below was added to the above composition in a proportion of 5% by weight.

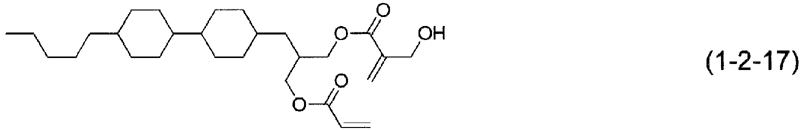

NI = 71.5°C; η = 26.3 mPa·s; Δn = 0.097; Δε = 8.3.

### Use Example 12

| | | |
|---|---|---|
| 3-HB-O2 | (2-5) | 13% |
| 2-BTB-1 | (2-10) | 4% |
| 3-HHB-1 | (3-1) | 9% |
| 3-HHB-O1 | (3-1) | 4% |
| 3-HHB-3 | (3-1) | 13% |
| 3-HHB-F | (6-1) | 4% |
| 2-HHB(F)-F | (6-2) | 7% |
| 3-HHB(F)-F | (6-2) | 7% |
| 5-HHB(F)-F | (6-2) | 7% |
| 3-HHB(F,F)-F | (6-3) | 6% |
| 3-HHEB-F | (6-10) | 4% |
| 5-HHEB-F | (6-10) | 5% |
| 2-HB-C | (8-1) | 5% |
| 3-HB-C | (8-1) | 12% |

Compound (1-9-1) described below was added to the above composition in a proportion of 2% by weight.

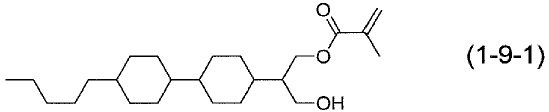

NI = 101.6°C; η = 18.9 mPa·s; Δn = 0.102; Δε = 4.7.

### Use Example 13

| | | |
|---|---|---|
| 3-HH-4 | (2-1) | 15% |
| 3-HB-O2 | (2-5) | 9% |
| 5-HBB(F)B-2 | (4-5) | 6% |
| 5-HBB(F)B-3 | (4-5) | 4% |
| 3-HB-CL | (5-2) | 14% |
| 3-HHB(F,F)-F | (6-3) | 5% |
| 3-HBB(F,F)-F | (6-24) | 26% |
| 5-HBB(F,F)-F | (6-24) | 21% |

Compound (1-9-2) described below was added to the above composition in a proportion of 3% by weight.

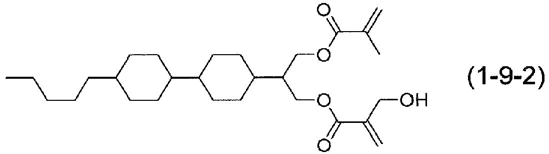

NI = 70.2°C; η = 16.3 mPa·s; Δn = 0.112; Δε = 4.9.

### Use Example 14

| | | |
|---|---|---|
| 3-HB-O2 | (2-5) | 8% |
| 7-HB(F,F)-F | (5-4) | 5% |
| 2-HHB(F)-F | (6-2) | 7% |
| 3-HHB(F)-F | (6-2) | 8% |
| 5-HHB(F)-F | (6-2) | 9% |
| 2-HBB-F | (6-22) | 5% |
| 3-HBB-F | (6-22) | 5% |
| 5-HBB-F | (6-22) | 5% |
| 2-HBB(F)-F | (6-23) | 10% |
| 3-HBB(F)-F | (6-23) | 6% |
| 5-HBB(F)-F | (6-23) | 14% |
| 3-HBB(F,F)-F | (6-24) | 6% |
| 5-HBB(F,F)-F | (6-24) | 12% |

Compound (1-9-3) described below was added to the above composition in a proportion of 1% by weight.

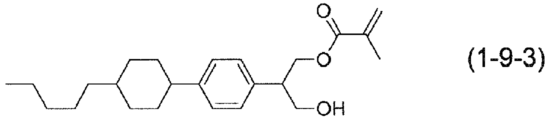

NI = 80.9°C; η = 24.3 mPa·s; Δn = 0.115; Δε = 5.4.

### Use Example 15

| | | |
|---|---|---|
| 3-HH-4 | (2-1) | 10% |
| 3-HH-5 | (2-1) | 4% |
| 1O1-HBBH-5 | (4-1) | 4% |
| 5-HB-CL | (5-2) | 16% |
| 3-HHB-F | (6-1) | 5% |
| 3-HHB-CL | (6-1) | 5% |
| 4-HHB-CL | (6-1) | 4% |
| 3-HHB(F)-F | (6-2) | 12% |
| 4-HHB(F)-F | (6-2) | 7% |
| 5-HHB(F)-F | (6-2) | 7% |
| 7-HHB(F)-F | (6-2) | 6% |
| 5-HBB(F)-F | (6-23) | 3% |
| 3-HHBB(F,F)-F | (7-6) | 3% |
| 4-HHBB(F,F)-F | (7-6) | 4% |
| 5-HHBB(F,F)-F | (7-6) | 3% |
| 3-HH2BB(F,F)-F | (7-15) | 3% |
| 4-HH2BB(F,F)-F | (7-15) | 4% |

Compound (1-9-4) described below was added to the above composition in a proportion of 4% by weight.

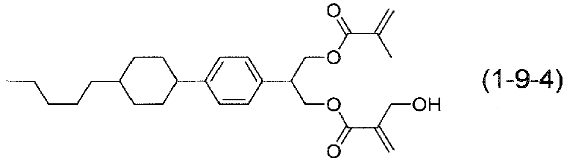

NI = 120.3°C; η = 21.4 mPa·s; Δn = 0.096; Δε = 4.0.

### Use Example 16

| | | |
|---|---|---|
| 1O1-HBBH-4 | (4-1) | 3% |
| 1O1-HBBH-5 | (4-1) | 3% |
| 3-HHB(F,F)-F | (6-3) | 8% |
| 3-H2HB(F,F)-F | (6-15) | 7% |
| 4-H2HB(F,F)-F | (6-15) | 6% |
| 5-H2HB(F,F)-F | (6-15) | 10% |
| 3-HBB(F,F)-F | (6-24) | 20% |
| 5-HBB(F,F)-F | (6-24) | 22% |
| 3-H2BB(F,F)-F | (6-27) | 12% |
| 5-HHBB(F,F)-F | (7-6) | 3% |
| 3-HH2BB(F.F)-F | (7-15) | 4% |
| 5-HHEBB-F | (7-17) | 2% |

Compound (1-9-5) described below was added to the above composition in a proportion of 7% by weight.

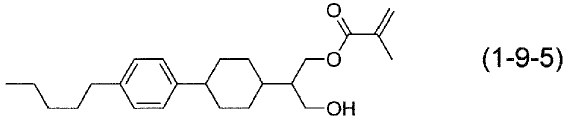

NI = 95.7°C; η = 34.8 mPa·s; Δn = 0.116; Δε = 9.1.

### Use Example 17

| | | |
|---|---|---|
| 5-HBBH-3 | (4-1) | 5% |
| 3-HB(F)BH-3 | (4-2) | 3% |
| 5-HB-F | (5-2) | 12% |
| 6-HB-F | (5-2) | 9% |
| 7-HB-F | (5-2) | 7% |
| 2-HHB-OCF3 | (6-1) | 5% |
| 3-HHB-OCF3 | (6-1) | 5% |
| 4-HHB-OCF3 | (6-1) | 7% |
| 5-HHB-OCF3 | (6-1) | 6% |
| 3-HHB(F,F)-OCF2H | (6-3) | 5% |
| 3-HHB(F,F)-OCF3 | (6-3) | 5% |
| 3-HH2B-OCF3 | (6-4) | 5% |
| 5-HH2B-OCF3 | (6-4) | 4% |
| 3-HH2B(F)-F | (6-5) | 3% |
| 3-HBB(F)-F | (6-23) | 8% |
| 5-HBB(F)-F | (6-23) | 11% |

Compound (1-9-6) described below was added to the above composition in a proportion of 5% by weight.

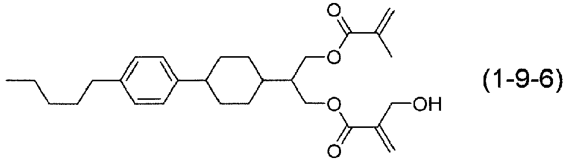

NI = 88.5°C; η = 15.7 mPa·s; Δn = 0.093; Δε = 4.4.

### Use Example 18

| | | |
|---|---|---|
| 3-HH-4 | (2-1) | 10% |
| 5-HB-O2 | (2-5) | 5% |
| 3-HHB-1 | (3-1) | 4% |
| 5-HB-CL | (5-2) | 10% |
| 3-HHB(F,F)-F | (6-3) | 8% |
| 3-HHEB(F,F)-F | (6-12) | 9% |
| 4-HHEB(F,F)-F | (6-12) | 3% |
| 5-HHEB(F,F)-F | (6-12) | 3% |
| 3-HBB(F,F)-F | (6-24) | 19% |
| 5-HBB(F,F)-F | (6-24) | 14% |
| 2-HBEB(F,F)-F | (6-39) | 3% |
| 3-HBEB(F,F)-F | (6-39) | 4% |
| 5-HBEB(F,F)-F | (6-39) | 3% |
| 3-HHBB(F,F)-F | (7-6) | 5% |

Compound (1-9-7) described below was added to the above composition in a proportion of 2% by weight.

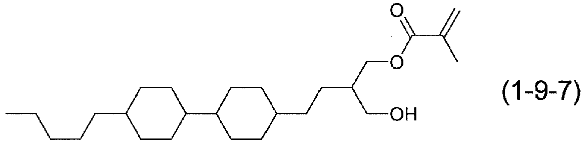

NI = 76.9°C; η = 19.3 mPa·s; Δn = 0.099; Δε = 8.2.

### Use Example 19

| | | |
|---|---|---|
| 3-HB-CL | (5-2) | 4% |
| 5-HB-CL | (5-2) | 6% |
| 3-HHB-OCF3 | (6-1) | 6% |
| V-HHB(F)-F | (6-2) | 4% |
| 3-HHB(F)-F | (6-2) | 4% |
| 5-HHB(F)-F | (6-2) | 6% |
| 3-H2HB-OCF3 | (6-13) | 5% |
| 5-H2HB(F,F)-F | (6-15) | 6% |
| 5-H4HB-OCF3 | (6-19) | 15% |
| 3-H4HB(F,F)-CF3 | (6-21) | 8% |
| 5-H4HB(F,F)-CF3 | (6-21) | 10% |
| 5-H4HB(F,F)-F | (6-21) | 7% |
| 2-H2BB(F)-F | (6-26) | 5% |
| 3-H2BB(F)-F | (6-26) | 8% |
| 3-HBEB(F,F)-F | (6-39) | 6% |

Compound (1-9-8) described below was added to the above composition in a proportion of 3% by weight.

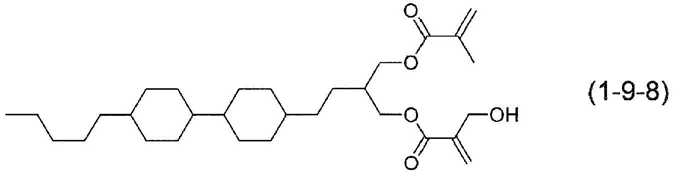

NI = 70.4°C; η = 25.7 mPa·s; Δn = 0.096; Δε = 8.5.

### Use Example 20

| | | |
|---|---|---|
| 3-HH-4 | (2-1) | 8% |
| 3-HH-5 | (2-1) | 6% |
| 3-HB-O2 | (2-5) | 14% |
| 3-HHB-1 | (3-1) | 8% |
| 3-HHB-O1 | (3-1) | 6% |
| 5-HB-CL | (5-2) | 15% |
| 7-HB(F,F)-F | (5-4) | 5% |
| 2-HHB(F)-F | (6-2) | 7% |
| 3-HHB(F)-F | (6-2) | 7% |
| 5-HHB(F)-F | (6-2) | 7% |
| 3-HHB(F,F)-F | (6-3) | 7% |
| 3-H2HB(F,F)-F | (6-15) | 5% |
| 4-H2HB(F,F)-F | (6-15) | 5% |

Compound (1-10-1) described below was added to the above composition in a proportion of 1% by weight.

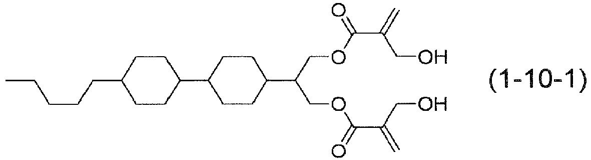

NI = 71.4°C; η = 14.7 mPa·s; Δn = 0.073; Δε = 3.0.

### Use Example 21

| | | |
|---|---|---|
| 3-HH-4 | (2-1) | 10% |
| 3-HH-5 | (2-1) | 11% |
| 3-HB-O2 | (2-5) | 10% |
| 5-HB-CL | (5-2) | 3% |
| 7-HB(F)-F | (5-3) | 8% |
| 2-HHB(F,F)-F | (6-3) | 5% |
| 3-HHB(F,F)-F | (6-3) | 4% |
| 3-HHEB-F | (6-10) | 9% |
| 5-HHEB-F | (6-10) | 8% |
| 3-HHEB(F,F)-F | (6-12) | 8% |
| 4-HHEB(F,F)-F | (6-12) | 5% |
| 3-GHB(F,F)-F | (6-109) | 4% |
| 4-GHB(F,F)-F | (6-109) | 7% |
| 5-GHB(F,F)-F | (6-109) | 8% |

Compound (1-10-2) described below was added to the above composition in a proportion of 2% by weight.

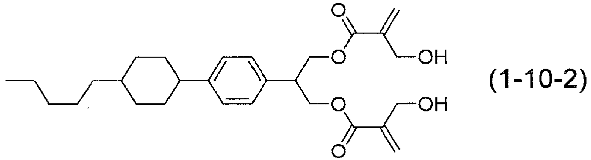

NI = 70.9°C; η = 19.0 mPa·s; Δn = 0.065; Δε = 5.9.

### Use Example 22

| | | |
|---|---|---|
| 3-HH-4 | (2-1) | 10% |
| 3-HH-5 | (2-1) | 8% |
| 1O1-HBBH-5 | (4-1) | 3% |
| 5-HB-CL | (5-2) | 10% |
| 3-HHB-F | (6-1) | 5% |
| 3-HHB-CL | (6-1) | 3% |
| 4-HHB-CL | (6-1) | 4% |
| 3-HHB(F)-F | (6-2) | 8% |
| 4-HHB(F)-F | (6-2) | 9% |
| 5-HBB(F)-F | (6-2) | 10% |
| 7-HHB(F)-F | (6-2) | 8% |
| 5-HBB(F)-F | (6-23) | 5% |
| 3-HHBB(F,F)-F | (7-6) | 4% |
| 4-HHBB(F,F)-F | (7-6) | 3% |
| 5-HHBB(F,F)-F | (7-6) | 4% |
| 3-HH2BB(F,F)-F | (7-15) | 3% |
| 4-HH2BB(F,F)-F | (7-15) | 3% |

Compound (1-10-3) described below was added to the above composition in a proportion of 4% by weight.

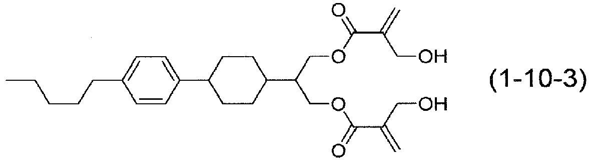

NI = 124.8°C; η = 22.6 mPa·s; Δn = 0.094; Δε = 3.9.

### Use Example 23

| | | |
|---|---|---|
| 3-HH-4 | (2-1) | 10% |
| 3-HB-O2 | (2-5) | 4% |
| 5-HB-O2 | (2-5) | 5% |
| 3-HHB-1 | (3-1) | 5% |
| 5-HB-CL | (5-2) | 6% |
| 3-HHB(F,F)-F | (6-3) | 6% |
| 3-HHEB(F,F)-F | (6-12) | 8% |
| 4-HHEB(F,F)-F | (6-12) | 3% |
| 5-HHEB(F,F)-F | (6-12) | 3% |
| 3-HBB(F,F)-F | (6-24) | 18% |
| 5-HBB(F,F)-F | (6-24) | 13% |
| 2-HBEB(F,F)-F | (6-39) | 4% |
| 3-HBEB(F,F)-F | (6-39) | 5% |
| 5-HBEB(F,F)-F | (6-39) | 4% |
| 3-HHBB(F,F)-F | (7-6) | 6% |

Compound (1-10-4) described below was added to the above composition in a proportion of 3% by weight.

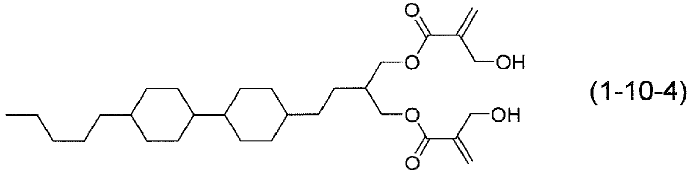

NI = 79.9°C; η = 20.0 mPa·s; Δn = 0.101; Δε = 8.3.

### Use Example 24

| | | |
|---|---|---|
| 3-HB-O2 | (2-5) | 9% |
| 7-HB(F,F)-F | (5-4) | 5% |
| 2-HHB(F)-F | (6-2) | 9% |
| 3-HHB(F)-F | (6-2) | 9% |
| 5-HHB(F)-F | (6-2) | 9% |
| 2-HBB-F | (6-22) | 5% |
| 3-HBB-F | (6-22) | 5% |
| 5-HBB-F | (6-22) | 5% |
| 2-HBB(F)-F | (6-23) | 7% |
| 3-HBB(F)-F | (6-23) | 5% |
| 5-HBB(F)-F | (6-23) | 13% |
| 3-HBB(F,F)-F | (6-24) | 8% |
| 5-HBB(F,F)-F | (6-24) | 11% |

Compound (1-9-11) described below was added to the above composition in a proportion of 1% by weight.

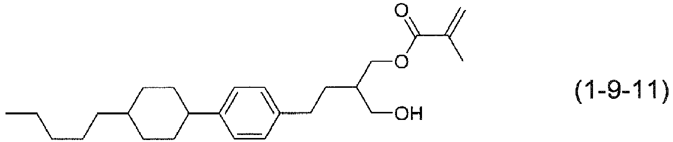

NI = 80.8°C; η = 203.7 mPa·s; Δn = 0.112; Δε = 5.4.

### Use Example 25

| | | |
|---|---|---|
| 5-HBBH-3 | (4-1) | 6% |
| 3-HB(F)BH-3 | (4-2) | 3% |
| 5-HB-F | (5-2) | 12% |
| 6-HB-F | (5-2) | 9% |
| 7-HB-F | (5-2) | 7% |
| 2-HHB-OCF3 | (6-1) | 7% |
| 3-HHB-OCF3 | (6-1) | 6% |
| 4-HHB-OCF3 | (6-1) | 7% |
| 5-HHB-OCF3 | (6-1) | 4% |
| 3-HHB(F,F)-OCF2H | (6-3) | 4% |
| 3-HBB(F,F)-OCF3 | (6-3) | 4% |
| 3-HH2B-OCF3 | (6-4) | 5% |
| 5-HH2B-OCF3 | (6-4) | 4% |
| 3-HH2B(F)-F | (6-5) | 3% |
| 3-HBB(F)-F | (6-23) | 6% |
| 5-HBB(F)-F | (6-23) | 13% |

Compound (1-9-12) described below was added to the above composition in a proportion of 3% by weight.

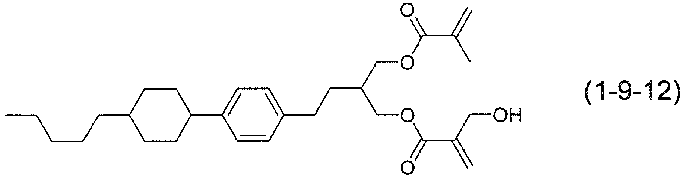

NI = 90.1°C; η = 15.4 mPa·s; Δn = 0.093; Δε = 4.3.

### Use Example 26

| | | |
|---|---|---|
| 3-HH-4 | (2-1) | 12% |
| 3-HH-5 | (2-1) | 12% |
| 3-HB-O2 | (2-5) | 10% |
| 5-HB-CL | (5-2) | 4% |
| 7-HB(F)-F | (5-3) | 5% |
| 2-HHB(F,F)-F | (6-3) | 5% |
| 3-HHB(F,F)-F | (6-3) | 5% |
| 3-HHEB-F | (6-10) | 7% |
| 5-HHEB-F | (6-10) | 7% |
| 3-HHEB(F,F)-F | (6-12) | 10% |
| 4-HHEB(F,F)-F | (6-12) | 3% |
| 3-GHB(F,F)-F | (6-109) | 6% |
| 4-GHB(F,F)-F | (6-109) | 8% |
| 5-GHB(F,F)-F | (6-109) | 6% |

Compound (1-9-9) described below was added to the above composition in a proportion of 3% by weight.

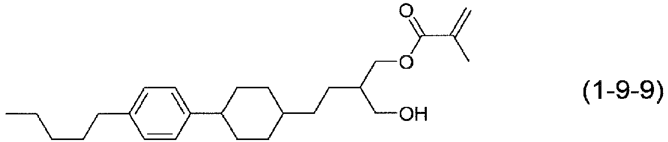

NI = 70.8°C; η = 18.8 mPa·s; Δn = 0.065; Δε = 6.1.

### Use Example 27

| | | |
|---|---|---|
| 3-HH-4 | (2-1) | 7% |
| 3-HB-O2 | (2-5) | 3% |
| 5-HB-O2 | (2-5) | 4% |
| 3-HHB-1 | (3-1) | 5% |
| 5-HB-CL | (5-2) | 8% |
| 3-HHB(F,F)-F | (6-3) | 9% |
| 3-HHEB(F,F)-F | (6-12) | 9% |
| 4-HHEB(F,F)-F | (6-12) | 4% |
| 5-HHEB(F,F)-F | (6-12) | 4% |
| 3-HBB(F,F)-F | (6-24) | 18% |
| 5-HBB(F,F)-F | (6-24) | 13% |
| 2-HBEB(F,F)-F | (6-39) | 4% |
| 3-HBEB(F,F)-F | (6-39) | 4% |
| 5-HBEB(F,F)-F | (6-39) | 4% |
| 3-HHBB(F,F)-F | (7-6) | 4% |

Compound (1-9-10) described below was added to the above composition in a proportion of 3% by weight.

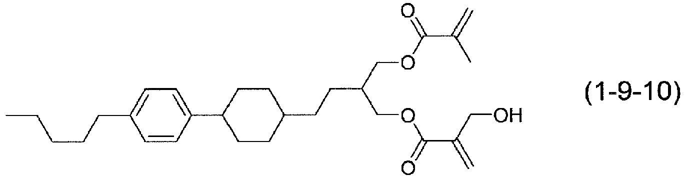

NI = 77.9°C; η = 20.6 mPa·s; Δn = 0.100; Δε = 8.5.

### Use Example 28

| | | |
|---|---|---|
| 3-HH-4 | (2-1) | 9% |
| 3-HB-O2 | (2-5) | 5% |
| 5-HB-O2 | (2-5) | 5% |
| 3-HHB-1 | (3-1) | 6% |
| 5-HB-CL | (5-2) | 5% |
| 3-HHB(F,F)-F | (6-3) | 5% |
| 3-HHEB(F,F)-F | (6-12) | 6% |
| 4-HHEB(F,F)-F | (6-12) | 5% |
| 5-HHEB(F,F)-F | (6-12) | 4% |
| 3-HBB(F,F)-F | (6-24) | 17% |
| 5-HBB(F,F)-F | (6-24) | 13% |
| 2-HBEB(F,F)-F | (6-39) | 5% |
| 3-HBEB(F,F)-F | (6-39) | 5% |
| 5-HBEB(F,F)-F | (6-39) | 5% |
| 3-HHBB(F,F)-F | (7-6) | 5% |

Compound (1-9-41) described below was added to the above composition in a proportion of 2% by weight.

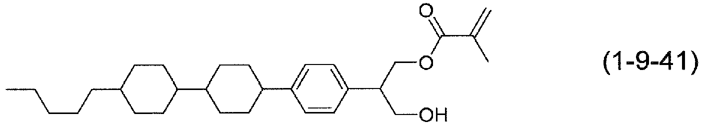

NI = 80.2°C; η = 20.5 mPa·s; Δn = 0.101; Δε = 8.5.

### Use Example 29

| | | |
|---|---|---|
| 3-HB-O2 | (2-5) | 16% |
| 2-BTB-1 | (2-10) | 4% |
| 3-HHB-1 | (3-1) | 6% |
| 3-HHB-O1 | (3-1) | 5% |
| 3-HHB-3 | (3-1) | 10% |
| 3-HHB-F | (6-1) | 5% |
| 2-HHB(F)-F | (6-2) | 5% |
| 3-HHB(F)-F | (6-2) | 5% |
| 5-HHB(F)-F | (6-2) | 5% |
| 3-HHB(F,F)-F | (6-3) | 6% |
| 3-HHEB-F | (6-10) | 6% |
| 5-HHEB-F | (6-10) | 6% |
| 2-HB-C | (8-1) | 6% |
| 3-HB-C | (8-1) | 15% |

Compound (1-9-42) described below was added to the above composition in a proportion of 2% by weight.

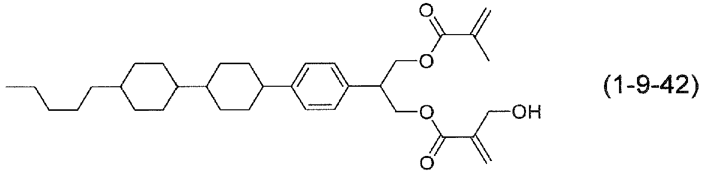

NI = 98.9°C; η = 19.5 mPa·s; Δn = 0.105; Δε = 4.9.

### Use Example 30

| | | |
|---|---|---|
| 3-HH-4 | (2-1) | 10% |
| 3-HH-5 | (2-1) | 5% |
| 1O1-HBBH-5 | (4-1) | 5% |
| 5-HB-CL | (5-2) | 15% |
| 3-HHB-F | (6-1) | 4% |
| 3-HHB-CL | (6-1) | 4% |
| 4-HHB-CL | (6-1) | 4% |
| 3-HHB(F)-F | (6-2) | 10% |
| 4-HHB(F)-F | (6-2) | 5% |
| 5-HHB(F)-F | (6-2) | 5% |
| 7-HHB(F)-F | (6-2) | 7% |
| 5-HBB(F)-F | (6-23) | 5% |
| 3-HHBB(F,F)-F | (7-6) | 5% |
| 4-HHBB (F,F)-F | (7-6) | 5% |
| 5-HHBB(F,F)-F | (7-6) | 5% |
| 3-HH2BB(F,F)-F | (7-15) | 3% |
| 4-HH2BB(F,F)-F | (7-15) | 3% |

Compound (1-9-52) described below was added to the above composition in a proportion of 3% by weight.

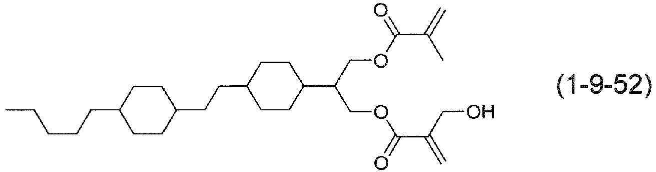

NI = 126.8°C; η = 25.3 mPa·s; Δn = 0.100; Δε = 4.2.

### Use Example 31

| | | |
|---|---|---|
| 3-HB-CL | (5-2) | 4% |
| 5-HB-CL | (5-2) | 5% |
| 3-HHB-OCF3 | (6-1) | 6% |
| V-HHB(F)-F | (6-2) | 6% |
| 3-HHB(F)-F | (6-2) | 6% |
| 5-HHB(F)-F | (6-2) | 6% |
| 3-H2HB-OCF3 | (6-13) | 5% |
| 5-H2HB(F,F)-F | (6-15) | 5% |
| 5-H4HB-OCF3 | (6-19) | 15% |
| 3-H4HB(F,F)-CF3 | (6-21) | 8% |
| 5-H4HB(F,F)-CF3 | (6-21) | 10% |
| 5-H4HB(F,F)-F | (6-21) | 7% |
| 2-H2BB(F)-F | (6-26) | 5% |
| 3-H2BB(F)-F | (6-26) | 6% |
| 3-HBEB(F,F)-F | (6-39) | 6% |

Compound (1-9-51) described below was added to the above composition in a proportion of 1.5% by weight.

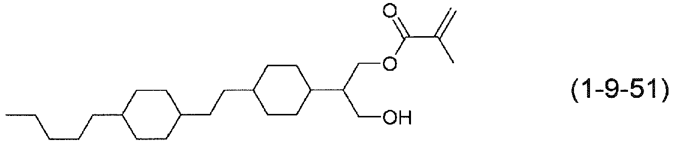

NI = 72.0°C; η = 25.9 mPa·s; Δn = 0.096; Δε = 8.5.

### Use Example 32

| | | |
|---|---|---|
| 1O1-HBBH-4 | (4-1) | 5% |
| 1O1-HBBH-5 | (4-1) | 5% |
| 3-HHB(F,F)-F | (6-3) | 5% |
| 3-H2HB(F,F)-F | (6-15) | 6% |
| 4-H2HB(F,F)-F | (6-15) | 7% |
| 5-H2HB(F,F)-F | (6-15) | 7% |
| 3-HBB(F,F)-F | (6-24) | 20% |
| 5-HBB(F,F)-F | (6-24) | 22% |
| 3-H2BB(F,F)-F | (6-27) | 15% |
| 5-HHBB(F,F)-F | (7-6) | 3% |
| 3-HH2BB(F,F)-F | (7-15) | 3% |
| 5-HHEBB-F | (7-17) | 2% |

Compound (1-9-53) described below was added to the above composition in a proportion of 3% by weight.

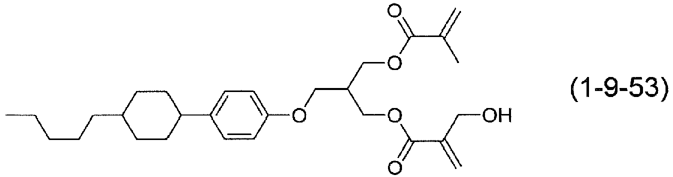

NI = 100.2°C; η = 35.9 mPa·s; Δn = 0.121; Δε = 9.0.

### Use Example 33

| | | |
|---|---|---|
| 3-HH-4 | (2-1) | 16% |
| 3-HB-O2 | (2-5) | 10% |
| 5-HBB(F)B-2 | (4-5) | 5% |
| 5-HBB(F)B-3 | (4-5) | 4% |
| 3-HB-CL | (5-2) | 10% |
| 3-HHB(F,F)-F | (6-3) | 6% |
| 3-HBB(F,F)-F | (6-24) | 27% |
| 5-HBB(F,F)-F | (6-24) | 22% |

Compound (1-9-54) described below was added to the above composition in a proportion of 2% by weight.

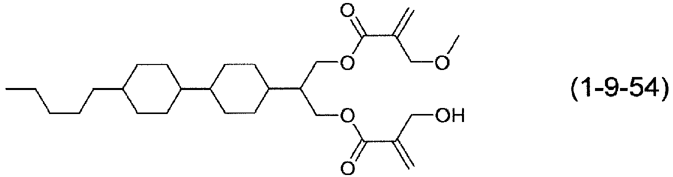

NI = 71.2°C; η = 17.1 mPa·s; Δn = 0.111; Δε = 5.0.

### Use Example 34

| | | |
|---|---|---|
| 3-HH-4 | (2-1) | 6% |
| 3-HH-5 | (2-1) | 6% |
| 3-HB-O2 | (2-5) | 15% |
| 3-HHB-1 | (3-1) | 6% |
| 3-HHB-O1 | (3-1) | 9% |
| 5-HB-CL | (5-2) | 15% |
| 7-HB(F,F)-F | (5-4) | 6% |
| 2-HHB(F)-F | (6-2) | 6% |
| 3-HHB(F)-F | (6-2) | 6% |
| 5-HHB(F)-F | (6-2) | 7% |
| 3-HHB(F,F)-F | (6-3) | 6% |
| 3-H2HB(F,F)-F | (6-15) | 6% |
| 4-H2HB(F,F)-F | (6-15) | 6% |

Compound (1-9-55) described below was added to the above composition in a proportion of 1% by weight.

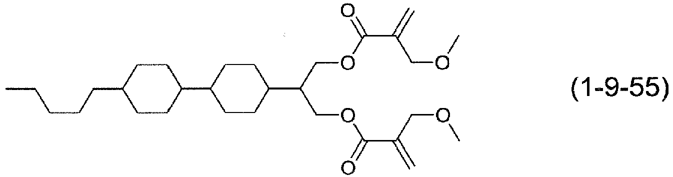

NI = 70.7°C; η = 15.3 mPa·s; Δn = 0.074; Δε = 3.0.

### Use Example 35

| | | |
|---|---|---|
| 3-HH-4 | (2-1) | 13% |
| 3-HH-5 | (2-1) | 6% |
| 1O1-HBBH-5 | (4-1) | 4% |
| 5-HB-CL | (5-2) | 13% |
| 3-HHB-F | (6-1) | 3% |
| 3-HHB-CL | (6-1) | 4% |
| 4-HHB-CL | (6-1) | 4% |
| 3-HHB(F)-F | (6-2) | 7% |
| 4-HHB(F)-F | (6-2) | 7% |
| 5-HHB(F)-F | (6-2) | 7% |
| 7-HHB(F)-F | (6-2) | 7% |
| 5-HBB(F)-F | (6-23) | 7% |
| 3-HHBB(F,F)-F | (7-6) | 4% |
| 4-HHBB(F,F)-F | (7-6) | 4% |
| 5-HHBB(F,F)-F | (7-6) | 4% |
| 3-HH2BB(F,F)-F | (7-15) | 3% |
| 4-HH2BB(F,F)-F | (7-15) | 3% |

Compound (1-9-56) described below was added to the above composition in a proportion of 3% by weight.

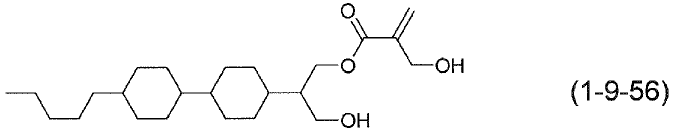

NI = 124.8°C; η = 23.5 mPa·s; Δn = 0.096; Δε = 3.9.

### Use Example 36

| | | |
|---|---|---|
| 3-HH-4 | (2-1) | 11% |
| 3-HH-5 | (2-1) | 5% |
| 1O1-HBBH-5 | (4-1) | 5% |
| 5-HB-CL | (5-2) | 15% |
| 3-HHB-F | (6-1) | 5% |
| 3-HHB-CL | (6-1) | 3% |
| 4-HHB-CL | (6-1) | 4% |
| 3-HHB(F)-F | (6-2) | 10% |
| 4-HHB(F)-F | (6-2) | 8% |
| 5-HHB(F)-F | (6-2) | 7% |
| 7-HHB(F)-F | (6-2) | 6% |
| 5-HBB(F)-F | (6-23) | 5% |
| 3-HHBB(F,F)-F | (7-6) | 4% |
| 4-HHBB(F,F)-F | (7-6) | 3% |
| 5-HHBB(F,F)-F | (7-6) | 3% |
| 3-HH2BB(F,F)-F | (7-15) | 3% |
| 4-HH2BB(F,F)-F | (7-15) | 3% |

Compound (1-9-57) described below was added to the above composition in a proportion of 2% by weight.

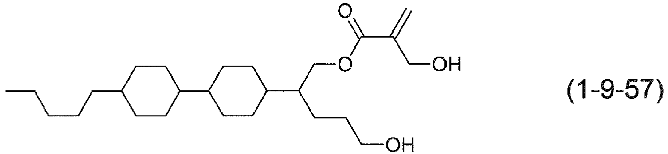

NI = 121.9°C; η = 21.8 mPa·s; Δn = 0.096; Δε = 3.9.

### Industrial Applicability

A liquid crystal composition containing compound (1) can be used in a display device such as a liquid crystal projector and a liquid crystal television.

## Claims

1. A compound, represented by formula (1):
R¹-MES-Sp¹-P¹ (1)
wherein, in formula (1),
R¹ is alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen;
MES is a mesogen group having at least one ring;
Sp¹ is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen, and in the groups, at least one hydrogen is replaced by a group selected from groups represented by formula (1a), formula (1b), formula (1c) and formula (1d); 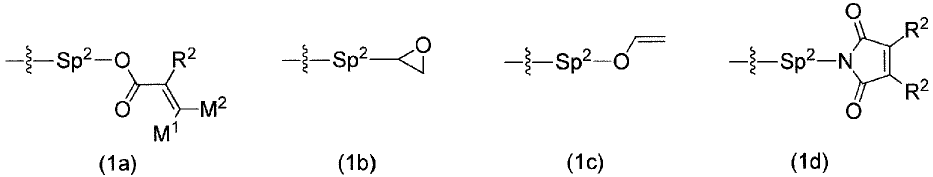 wherein, in formula (1a), formula (1b), formula (1c) and formula (1d),
Sp² is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by halogen;
M¹ and M² are independently hydrogen, halogen, alkyl having 1 to 5 carbons, or alkyl having 1 to 5 carbons in which at least one hydrogen is replaced by halogen;
R² is hydrogen or alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by halogen; and in formula (1),
P¹ is a group selected from groups represented by formula (1e) and formula (1f); 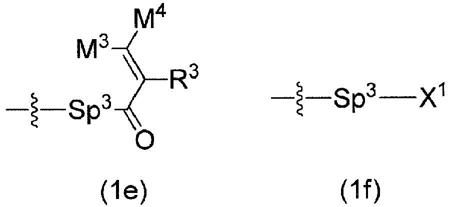 wherein, in formula (1e) and (1f),
Sp³ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
M³ and M⁴ are independently hydrogen, halogen, alkyl having 1 to 5 carbons, or alkyl having 1 to 5 carbons in which at least one hydrogen is replaced by halogen;
X¹ is -OH, -NH₂, -OR⁵, -N(R⁵)₂, -COOH, -SH, -B(OH)₂ or -Si(R⁵)₃; and
R³ is a group selected from groups represented by formula (1g), formula (1h) and formula (1i); 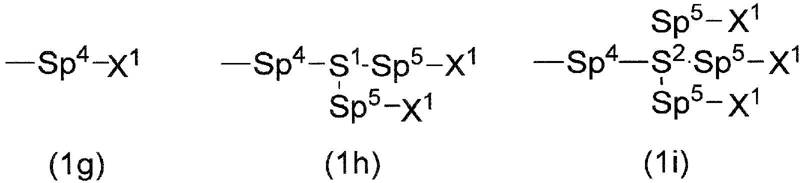 wherein, in formula (1g), formula (1h) and formula (1i),
Sp⁴ and Sp⁵ are independently a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
S¹ is >CH- or >N-;
S² is >C< or >Si<;
X¹ is -OH, -NH₂, -OR⁵, -N(R⁵)₂, -COOH, -SH, -B(OH)₂ or -Si(R⁵)₃; and in -OR⁵, -N(R⁵)₂ and -N(R⁵)₂,
R⁵ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by halogen.

2. The compound according to claim 1, represented by formula (1-1) : 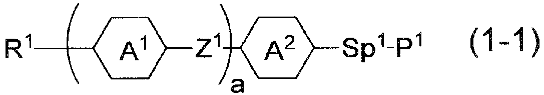 wherein, in formula (1-1),
R¹ is alkyl having 1 to 12 carbons, and in the alkyl, at least one piece of -CH₂- may be replaced by -O-, and at least one piece of -(CH₂)₂- may be replaced by -CH=CH- or -C≡C-, and in the groups, at least one hydrogen may be replaced by fluorine;
ring A¹ and ring A² are independently 1,4-cycloxylene, 1,4-cyclohexenylene, 1,4-phenylene, naphthalene-2,6-diyl, decahydronaphthalene-2,6-diyl, 1,2,3,4-tetrahydronaphthalene-2,6-diyl, tetrahydropyran-2,5-diyl, 1,3-dioxane-2,5-diyl, pyrimidine-2,5-diyl, pyridine-2,5-diyl, fluorene-2,7-diyl, phenanthrene-2,7-diyl, anthracene-2,6-diyl, perhydrocyclopenta[a]phenanthrene-3,17-diyl, or 2,3,4,7,8,9,10,11,12,13,14,15,16,17-tetradecahydrocyclopenta[a]phe nanthrene-3,17-diyl, and in the rings, at least one hydrogen may be replaced by fluorine, chlorine, alkyl having 1 to 12 carbons, alkenyl having 2 to 12 carbons, alkoxy having 1 to 11 carbons or alkenyloxy having 2 to 11 carbons, and in the groups, at least one hydrogen may be replaced by fluorine or chlorine;
a is 0, 1, 2, 3 or 4;
Z¹ is a single bond or alkylene having 1 to 6 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
Sp¹ is a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine, and in the groups, at least one hydrogen is replaced by a polymerizable group represented by formula (1a); 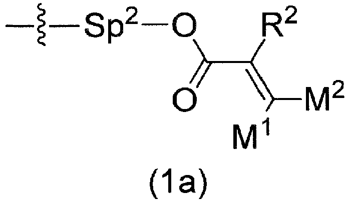
wherein, in formula (1a),
Sp² is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
M¹ and M² are independently hydrogen, fluorine, chlorine, alkyl having 1 to 5 carbons, or alkyl having 1 to 5 carbons in which at least one hydrogen is replaced by fluorine or chlorine;
R² is hydrogen or alkylene having 1 to 15 carbons, and in the alkylene, at least one piece of -CH₂- may be replaced by -O- or -S-, and at least one piece of -(CH₂)₂- may be replaced by -CH=CH- or -C≡C-, and in the groups, at least one hydrogen may be replaced by fluorine or chlorine; and
in formula (1-1),
P¹ is a group selected from groups represented by formula (1e) and formula (1f); 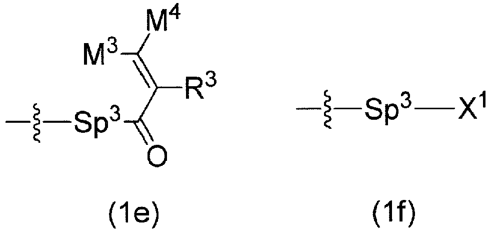
wherein, in formula (1e) and formula (1f),
Sp³ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
M³ and M⁴ are independently hydrogen, fluorine, chlorine, alkyl having 1 to 5 carbons, or alkyl having 1 to 5 carbons in which at least one hydrogen is replaced by fluorine or chlorine;
X¹ is -OH, -NH₂, -OR⁵, -N(R⁵)₂, -COOH, -SH or -Si(R⁵)₃; and
R³ is a group selected from groups represented by formula (1g) and formula (1h); 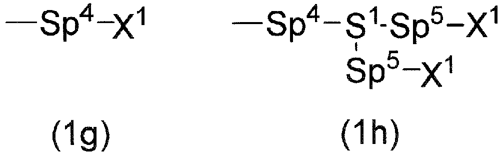
wherein, in formula (1g) and formula (1h),
Sp⁴ and Sp⁵ are independently a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
S¹ is >CH- or >N-;
X¹ is -OH, -NH₂, -OR⁵, -N(R⁵)₂, -COOH, -SH or -Si(R⁵)₃; and
in -OR⁵, -N(R⁵)₂ and -Si(R⁵)₃,
R⁵ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine or chlorine.

3. The compound according to claim 2,
wherein, in formula (1-1),
Z¹ is a single bond, - (CH₂)₂-, -(CH₂)₄-, -CH=CH-, -C≡C-, -COO-, -OCO-, -CF₂O-, -OCF₂-, -CH₂O-, -OCH₂- or -CF=CF-; and
in formula (1a),
M¹ and M⁴ are independently hydrogen, fluorine, alkyl having 1 to 5 carbons, or alkyl having 1 to 5 carbons in which at least one hydrogen is replaced by fluorine; and
in formula (1e),
M³ and M⁴ are independently hydrogen, fluorine, alkyl having 1 to 5 carbons, or alkyl having 1 to 5 carbons in which at least one hydrogen is replaced by fluorine; and
R³ is a group represented by formula (1g).

4. The compound according to claim 2 or 3,
wherein, in formula (1-1),
ring A¹ and ring A² are independently 1,4-cycloxylene, 1,4-cyclohexenylene, 1,4-phenylene, naphthalene-2,6-diyl, decahydronaphthalene-2,6-diyl, tetrahydropyran-2,5-diyl, 1,3-dioxane-2,5-diyl, fluorene-2,7-diyl, phenanthrene-2,7-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine;
Sp¹ is a single bond or alkylene having 1 to 8 carbons, and in the alkylene, at least one piece of -CH₂- may be replaced by -O-, -CO-, -COO-, -OCO- or -OCOO-, and at least one piece of - (CH₂)₂- may be replaced by -CH=CH- or -C≡C-, and in the groups, at least one hydrogen may be replaced by fluorine, and in the groups, at least one hydrogen is replaced by a group represented by formula (1a); 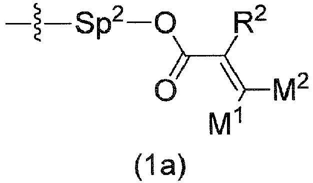 wherein, in formula (1a),
Sp² is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by halogen;
M¹ and M² are independently hydrogen, fluorine, methyl, ethyl or trifluoromethyl;
R² is hydrogen or alkylene having 1 to 8 carbons, and in the alkylene, at least one piece of -CH₂- may be replaced by -O-, and at least one piece of - (CH₂)₂- may be replaced by -CH=CH- or -C≡C-, and in the groups, at least one hydrogen may be replaced by fluorine; and in formula (1-1)
P¹ is a group selected from groups represented by formula (1e) and formula (1f); 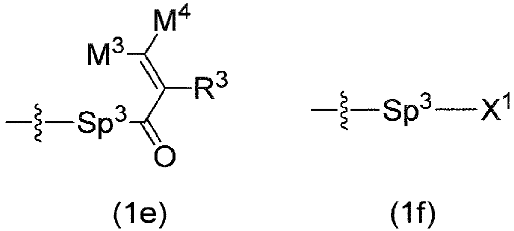
wherein, in formula (1e) and formula (1f),
Sp³ is a single bond or alkylene having 1 to 5 carbons, and in the alkylene, at least one piece of -CH₂- may be replaced by -O-, -CO-, -COO-, -OCO- or -OCOO-, and at least one piece of - (CH₂)₂- may be replaced by -CH=CH- or -C≡C-, and in the groups, at least one hydrogen may be replaced by fluorine;
M³ and M⁴ are independently hydrogen, fluorine, methyl, ethyl or trifluoromethyl;
X¹ is -OH, -NH₂ or -N(R⁵)₂; and
R³ is a group represented by formula (1g); -S
p⁴-X¹ (ig)
wherein, in formula (1g),
Sp⁴ is a single bond or alkylene having 1 to 5 carbons, and in the alkylene, at least one piece of -CH₂- may be replaced by -O-, -CO-, -COO-, -OCO- or -OCOO-, and at least one piece of - (CH₂)₂- may be replaced by -CH=CH- or -C≡C-, and in the groups, at least one hydrogen may be replaced by fluorine;
X¹ is -OH, -NH₂ or -N(R⁵)₂; and
in -N(R⁵)₂,
R⁵ is hydrogen or alkyl having 1 to 5 carbons, and in the alkyl, at least one piece of -CH₂- may be replaced by -0-, and at least one piece of -(CH₂)₂- may be replaced by -CH=CH-, and in the groups, at least one hydrogen may be replaced by fluorine.

5. The compound according to any one of claims 1 to 4, represented by formula (1-2) or formula (1-3): 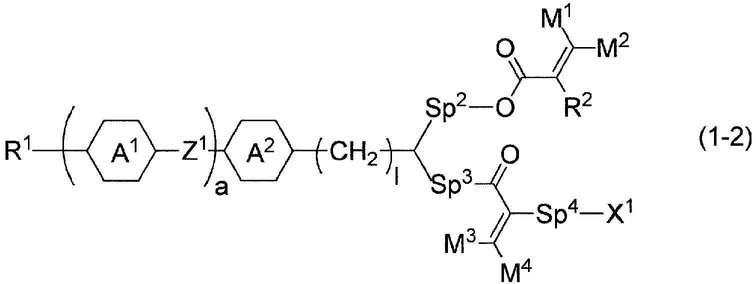 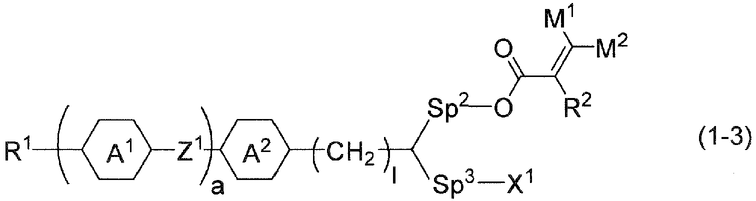 wherein, in formula (1-2) and formula (1-3),
R¹ is alkyl having 1 to 12 carbons, and in the alkyl, at least one piece of -CH₂- may be replaced by -O-, and at least one piece of -(CH₂)₂- may be replaced by -CH=CH- or -C≡C-, and in the groups, at least one hydrogen may be replaced by fluorine;
ring A¹ and ring A² are independently 1,4-cycloxylene, 1,4-cyclohexenylene, 1,4-phenylene, naphthalene-2,6-diyl, tetrahydropyran-2,5-diyl, 1,3-dioxane-2,5-diyl, fluorene-2,7-diyl, phenanthrene-2,7-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine, alkyl having 1 to 8 carbons, alkenyl having 2 to 8 carbons, alkoxy having 1 to 7 carbons or alkenyloxy having 2 to 7 carbons, and in the groups, at least one hydrogen may be replaced by fluorine;
Z¹ is a single bond, -(CH₂)₂-, -(CH₂)₄-, -CH=CH-, -C≡C-, -CF₂O-, -OCF₂-, -CH₂O-, -OCH₂- or -CF=CF-;
a is 0, 1, 2, 3 or 4;
1 is 0, 1, 2, 3, 4, 5 or 6, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
Sp² is a single bond or alkylene having 1 to 5 carbons, and in the alkylene, at least one piece of -CH₂- may be replaced by -O-, -CO-, -COO-, -OCO- or -OCOO-, and at least one piece of - (CH₂)₂- may be replaced by -CH=CH- or -C≡C-, and in the groups, at least one hydrogen may be replaced by fluorine;
M¹ and M² are independently hydrogen, fluorine, methyl, ethyl or trifluoromethyl;
R² is hydrogen or alkyl having 1 to 5 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by fluorine;
Sp³ is a single bond or alkylene having 1 to 5 carbons, and in the alkylene, at least one piece of -CH₂- may be replaced by -O-, -CO- or -COO-, and in the groups, at least one piece of - (CH₂)₂- may be replaced by -CH=CH- or -C≡C-, and in the groups, at least one hydrogen may be replaced by fluorine;
M³ and M⁴ are independently hydrogen, fluorine, methyl, ethyl or trifluoromethyl;
Sp⁴ is a single bond, alkylene having 1 to 5 carbons, and in the alkylene, at least one piece of -CH₂- may be replaced by -O-, -CO- or -COO-, and at least one piece of -(CH₂)₂- may be replaced by -CH=CH- or -C≡C-, and in the groups, at least one hydrogen may be replaced by fluorine;
X¹ is -OH or -N(R⁵)₂; and
in N(R⁵)₂,
R⁵ is hydrogen or alkyl having 1 to 5 carbons, and in the alkyl, at least one piece of -CH₂- may be replaced by -0-, and at least one piece of -(CH₂)₂- may be replaced by -CH=CH-, and in the groups, at least one hydrogen may be replaced by fluorine.

6. The compound according to claim 5,
wherein, in formula (1-2) and formula (1-3),
R¹ is alkyl having 1 to 10 carbons, alkenyl having 2 to 10 carbons or alkoxy having 1 to 9 carbons, and in the groups, at least one hydrogen may be replaced by fluorine;
ring A¹ and ring A² are independently 1,4-cycloxylene, 1,4-phenylene, naphthalene-2,6-diyl, perhydrocyclopenta[a]phenanthrene-3,17-diyl or 2,3,4,7,8,9,10,11,12,13,14,15,16,17-tetradecahydrocyclopenta[a]phe nanthrene-3,17-diyl, and in the rings, at least one hydrogen may be replaced by fluorine or alkyl having 1 to 5 carbons;
a is 0, 1, 2, 3 or 4;
1 is 0, 1, 2, 3, 4, 5 or 6, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine;
Z¹ is a single bond, -(CH₂)₂-, -(CH₂)₄-, -CH=CH-, -CF₂O- -OCF₂-, -CH₂O- or -OCH₂-;
Sp² is a single bond or alkylene having 1 to 5 carbons, and in the alkylene, at least one piece of -CH₂- may be replaced by -O-, and at least one piece of -(CH₂)₂- may be replaced by -CH=CH-;
M¹ and M² are independently hydrogen, methyl or ethyl;
R² is hydrogen or alkyl having 1 to 5 carbons, and in the alkyl, at least one piece of -CH₂- may be replaced by -0-, and at least one piece of -(CH₂)₂- may be replaced by -CH=CH-;
Sp³ is a single bond or alkylene having 1 to 5 carbons, and in the alkylene, at least one piece of -CH₂- may be replaced by -O-, and at least one piece of -(CH₂)₂- may be replaced by -CH=CH-;
M³ and M⁴ are independently hydrogen, fluorine, methyl or ethyl; Sp⁴ is a single bond or alkylene having 1 to 5 carbons, and in the alkylene, at least one piece of -CH₂- may be replaced by -O-, and at least one piece of -(CH₂)₂- may be replaced by -CH=CH-;
X¹ is -OH or -N(R⁵)₂; and
in -N(R⁵)₂,
R⁵ is hydrogen or alkyl having 1 to 3 carbons, and in the alkyl, at least one piece of -CH₂- may be replaced by -O-.

7. The compound according to claim 5,
wherein, in formula (1-2) and formula (1-3),
R¹ is alkyl having 1 to 10 carbons, alkenyl having 2 to 10 carbons or alkoxy having 1 to 9 carbons;
ring A¹ and ring A² are independently 1,4-cycloxylene, 1,4-phenylene or naphthalene-2,6-diyl, and in the rings, at least one hydrogen may be replaced by fluorine or alkyl having 1 to 5 carbons;
a is 0, 1, 2 or 3;
1 is 0, 1, 2, 3, 4, 5 or 6, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-;
Z¹ is a single bond, -(CH₂)₂- or -(CH₂)₄-;
Sp² is a single bond or alkylene having 1 to 3 carbons, and in the alkylene, at least one piece of -CH₂- may be replaced by -O-;
M¹ and M² are independently hydrogen or methyl;
R² is hydrogen or alkyl having 1 to 5 carbons, and in the alkyl, at least one piece of -CH₂- may be replaced by -O-;
Sp³ is a single bond or alkylene having 1 to 3 carbons, and in the alkylene, at least one piece of -CH₂- may be replaced by -O-;
M³ and M⁴ are independently hydrogen or methyl;
Sp⁴ is a single bond or alkylene having 1 to 3 carbons, and in the alkylene, at least one piece of -CH₂- may be replaced by -O-; and
X¹ is -OH.

8. The compound according to claim 1, represented by any one of formula (1-4) to formula (1-41): 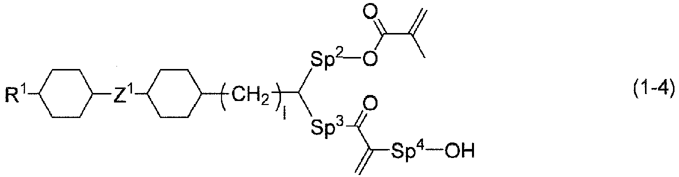 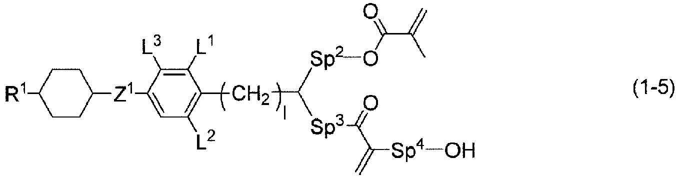 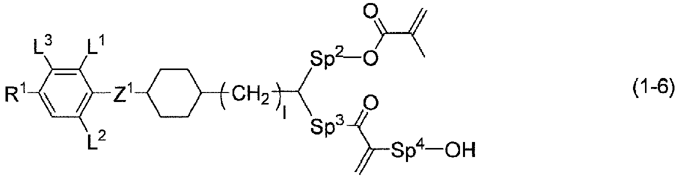 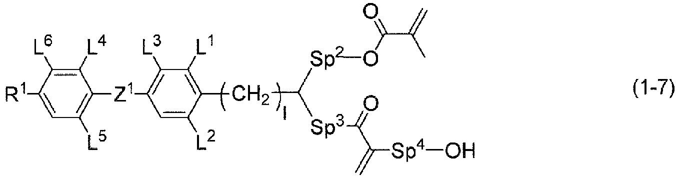 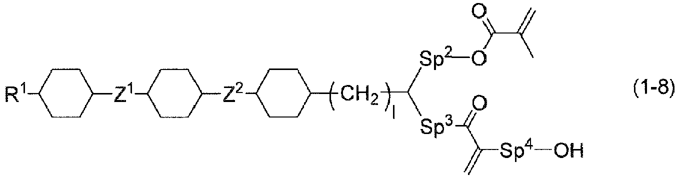 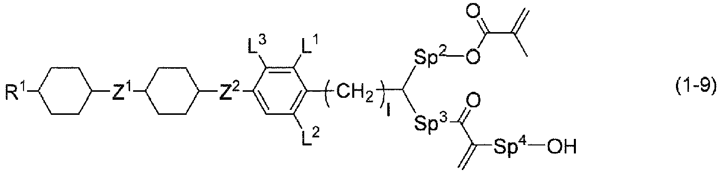 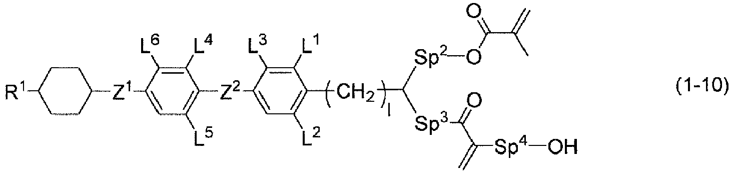 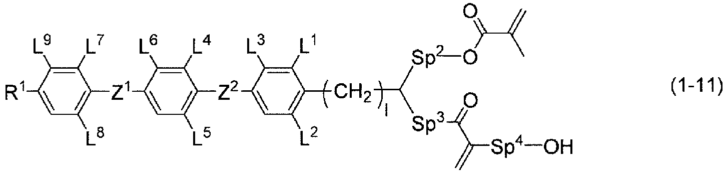 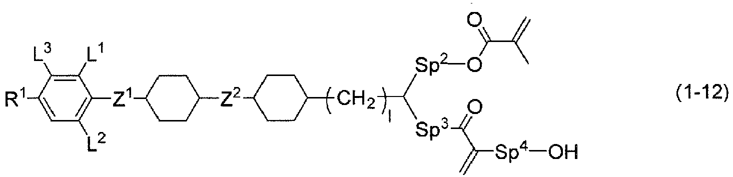 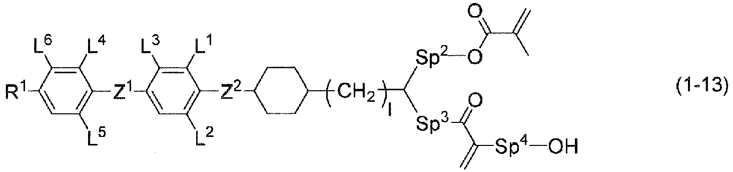 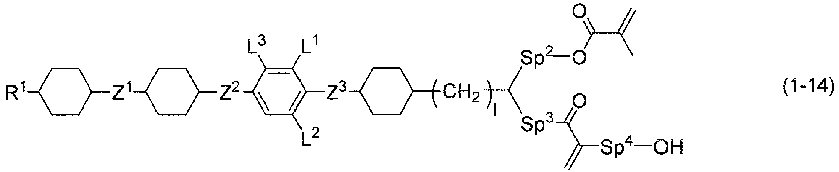 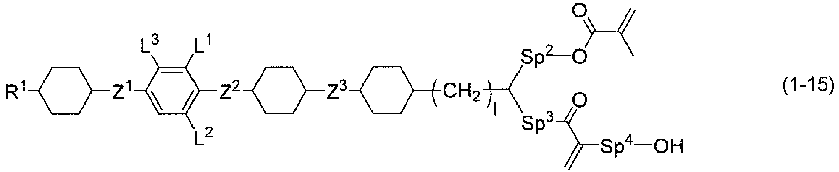 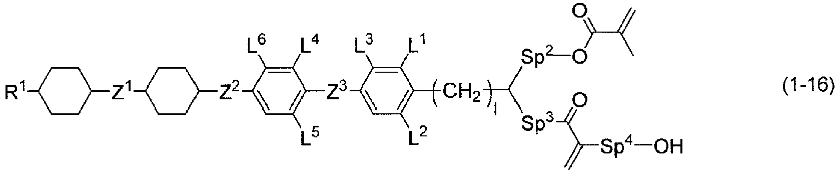 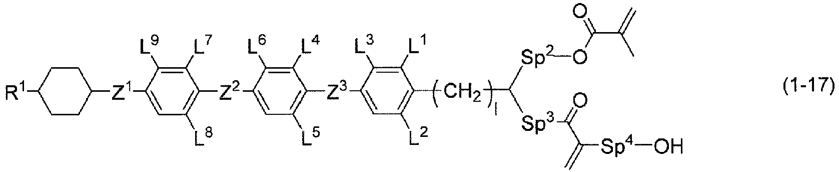 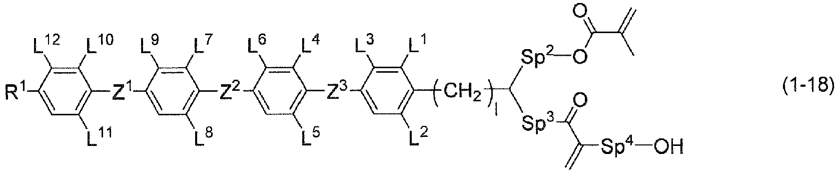 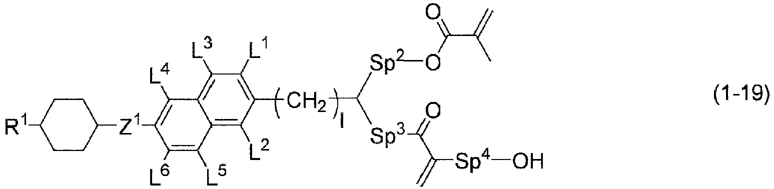 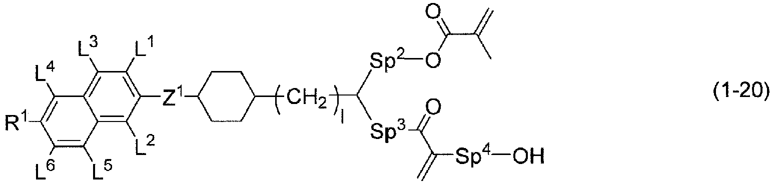 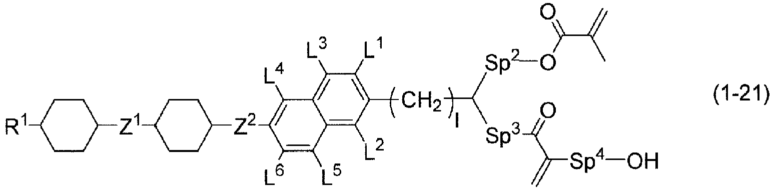 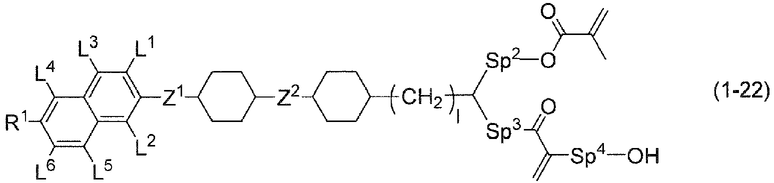 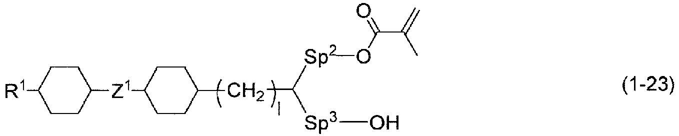 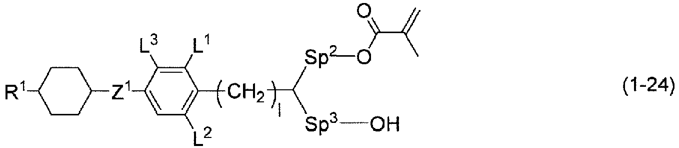 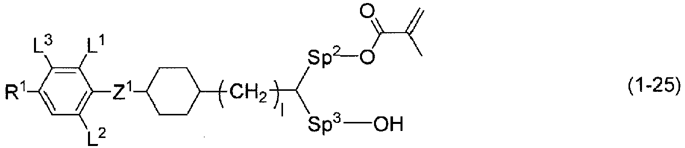 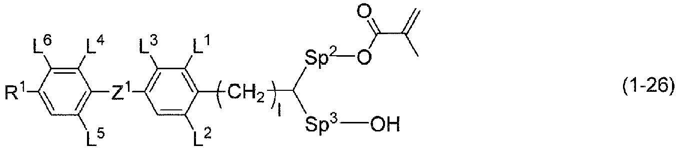 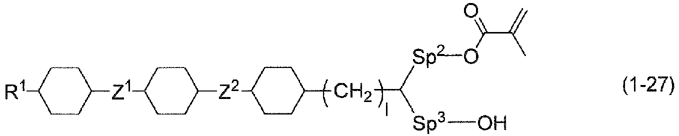 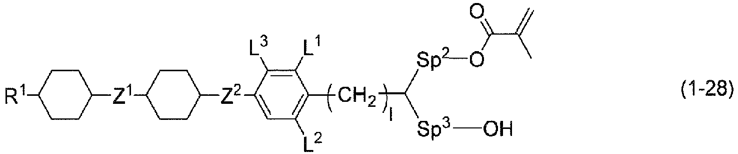 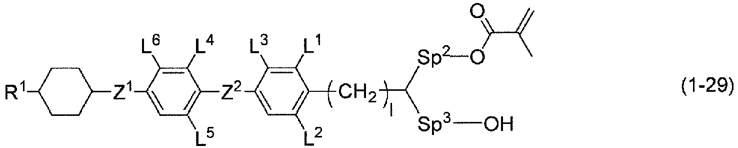 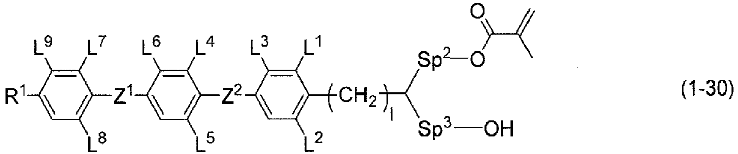 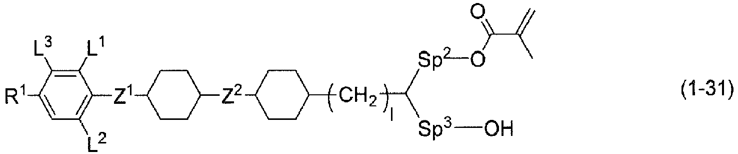 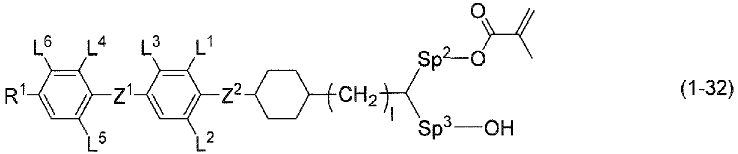 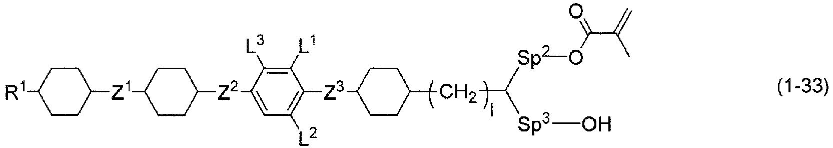 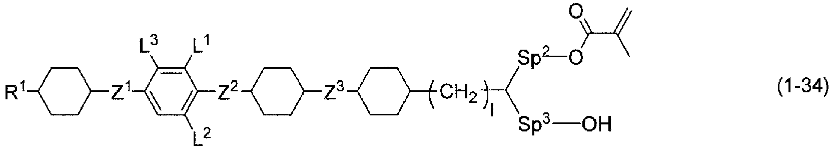 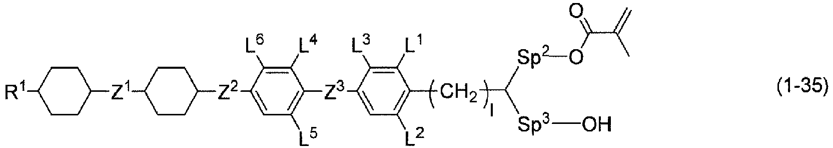 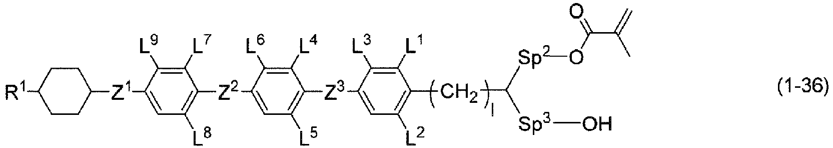 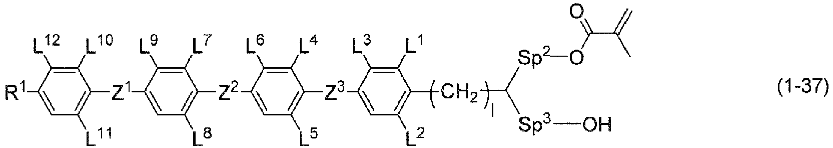 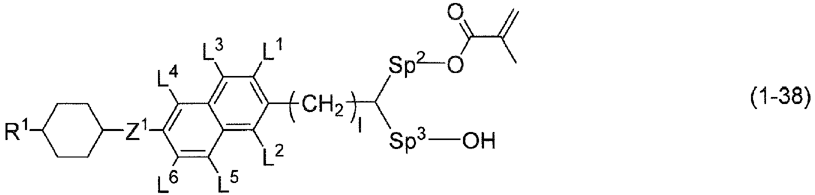 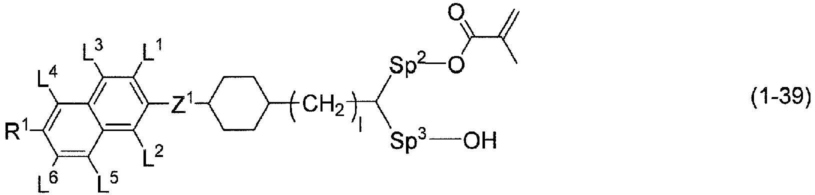 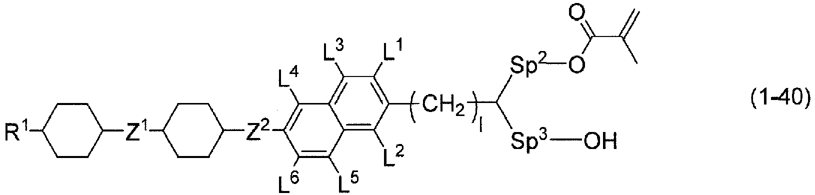 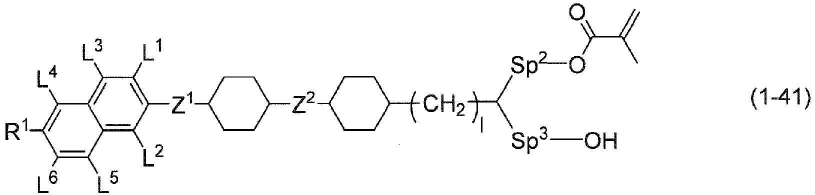 wherein, in formula (1-4) to formula (1-41),
R¹ is alkyl having 1 to 10 carbons;
Z¹, Z² and Z³ are independently a single bond, -(CH₂)₂- or -(CH₂)₄-;
Sp², Sp³ and Sp⁴ are independently alkylene having 1 to 5 carbons, and in the alkylene, at least one piece of -CH₂- may be replaced by -0-;
L¹, L², L³, L⁴, L⁵, L⁶, L⁷, L⁸, L⁹, L¹⁰, L¹¹ and L¹² are independently hydrogen, fluorine, methyl or ethyl; and
1 is 0, 1, 2, 3, 4, 5 or 6.

9. The compound according to claim 1, represented by any one of formula (1-42) to formula (1-60): 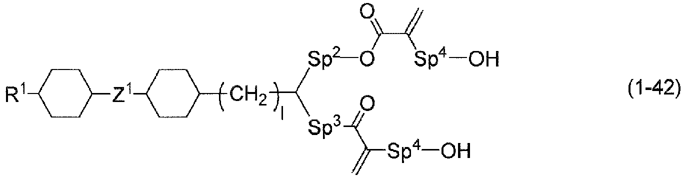 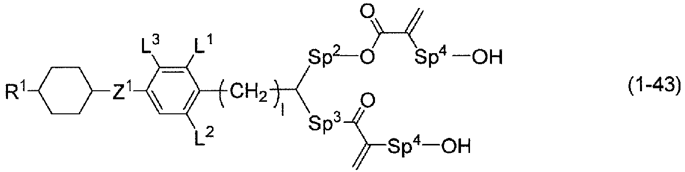 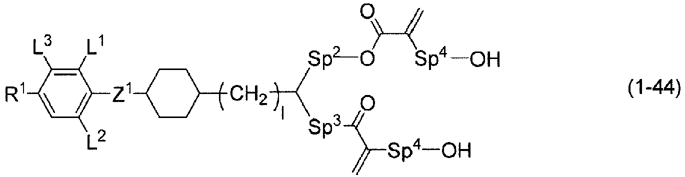 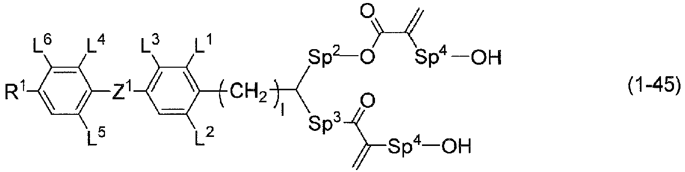 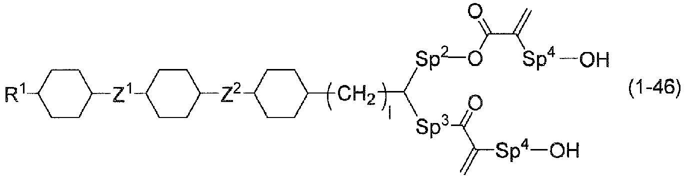 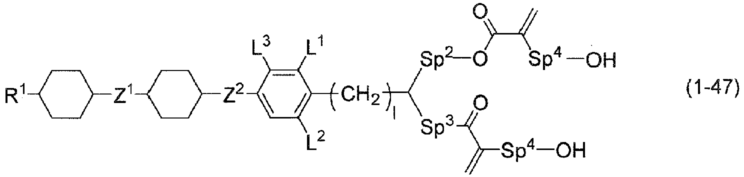 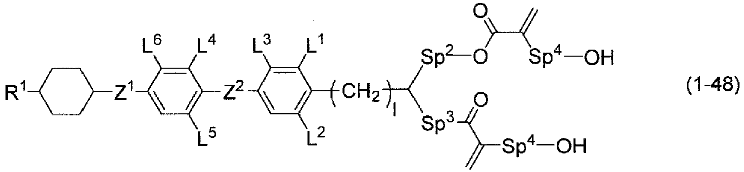 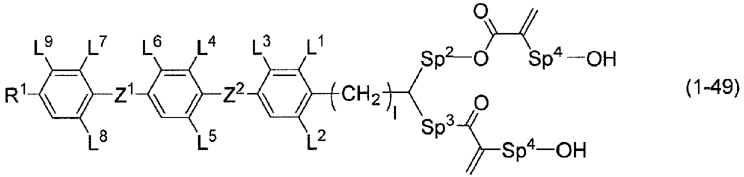 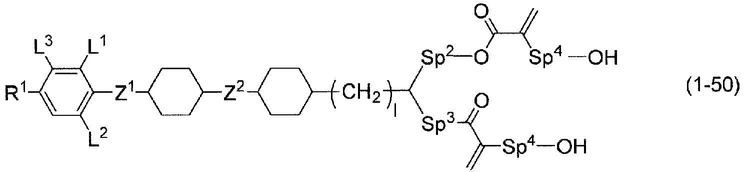 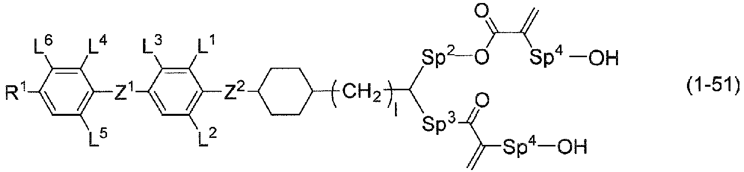 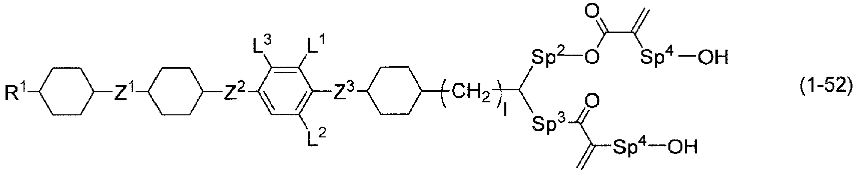 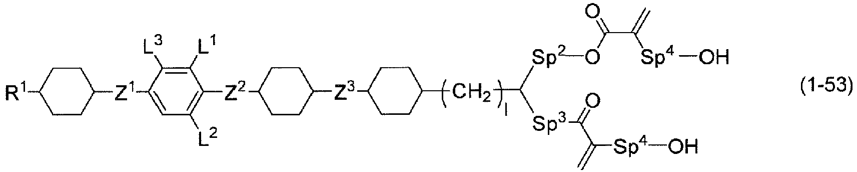 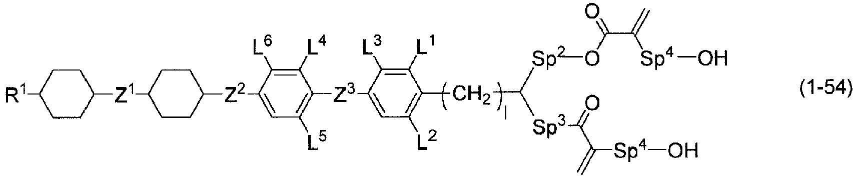 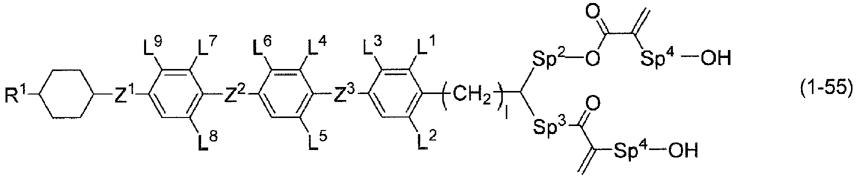 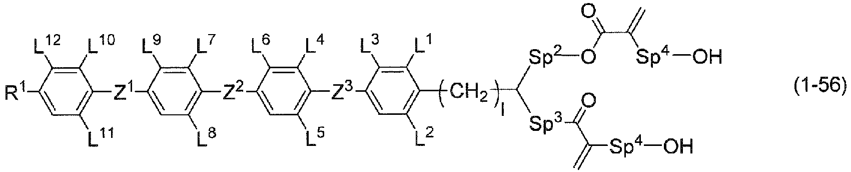 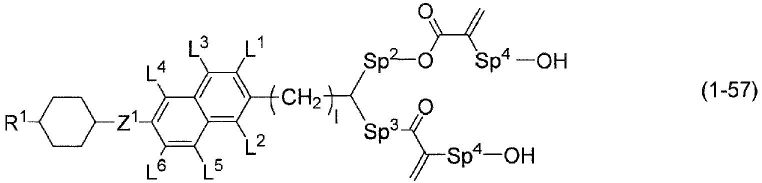 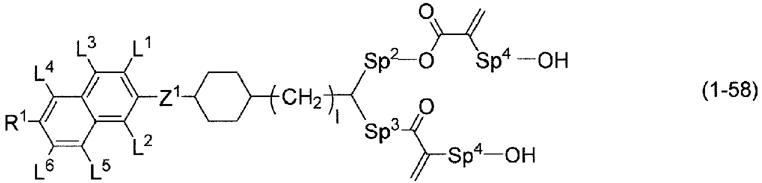 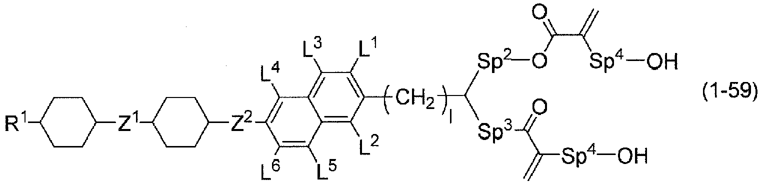 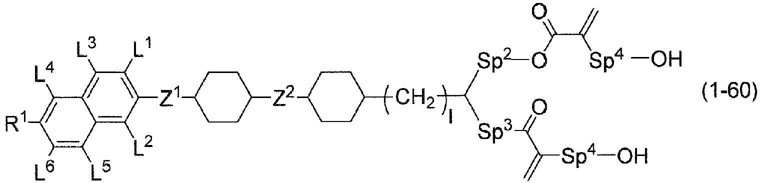 wherein, in formula (1-42) to formula (1-60),
R¹ is alkyl having 1 to 10 carbons;
Z¹, Z² and Z³ are independently a single bond, -(CH₂)₂- or -(CH₂)₄-;
Sp², Sp³ and Sp⁴ are independently alkylene having 1 to 5 carbons,
and in the alkylene, at least one piece of -CH₂- may be replaced by -0-;
L¹, L², L³, L⁴, L⁵, L⁶, L⁷, L⁸, L⁹, L¹⁰, L¹¹ and L¹² are independently hydrogen, fluorine, methyl or ethyl; and
1 is 0, 1, 2, 3, 4, 5 or 6.

10. The compound according to claim 1, represented by any one of formula (1-61) to formula (1-98): 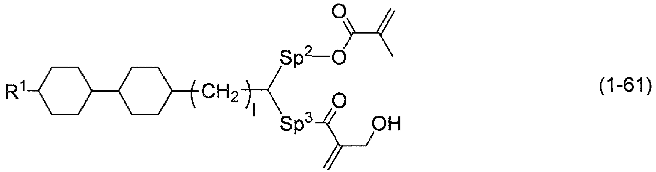 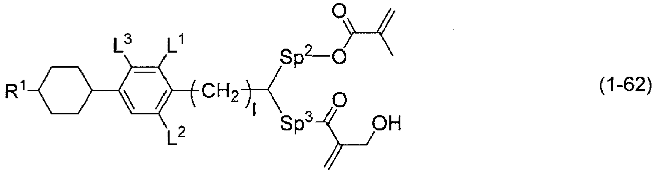 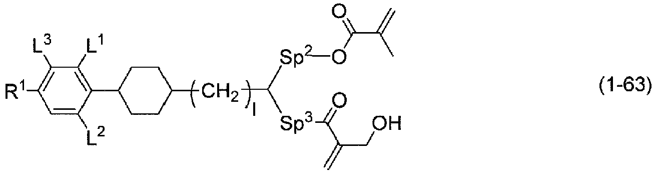 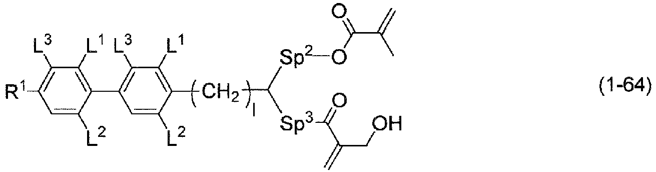 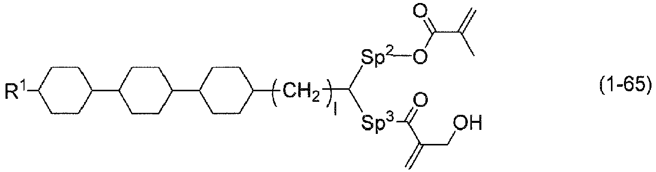 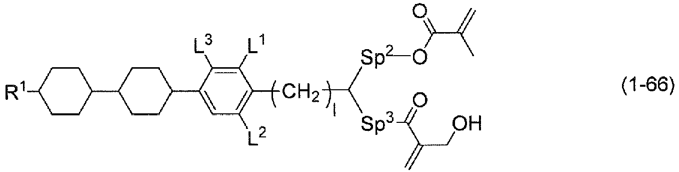 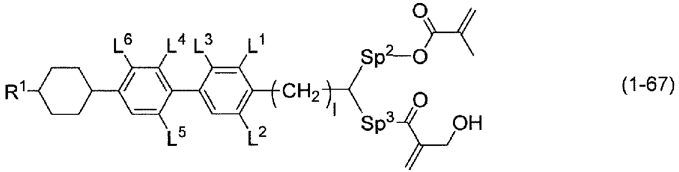 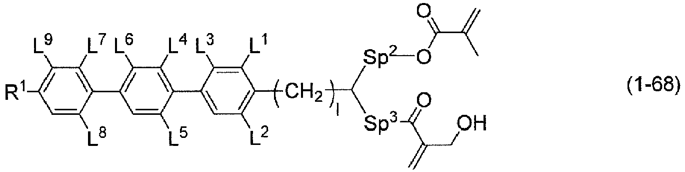 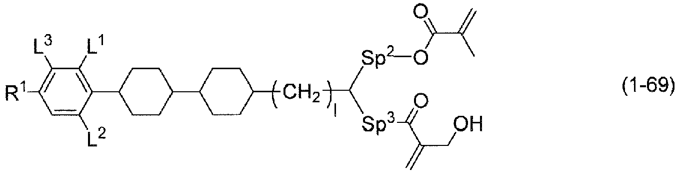 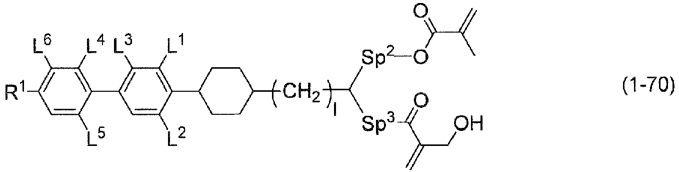 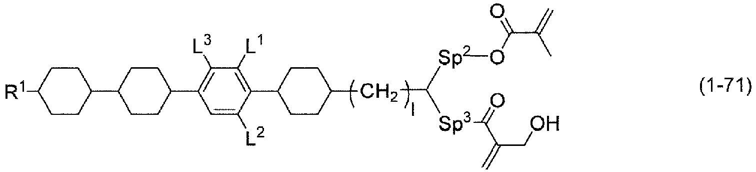 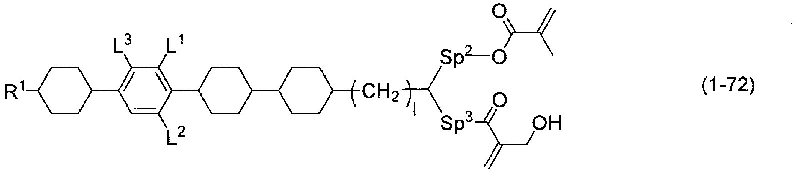 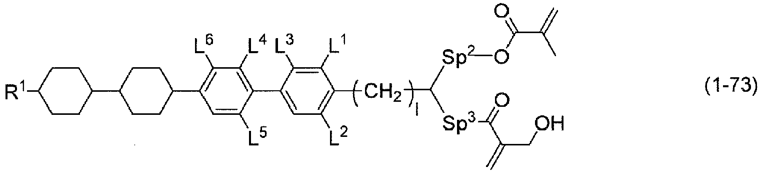 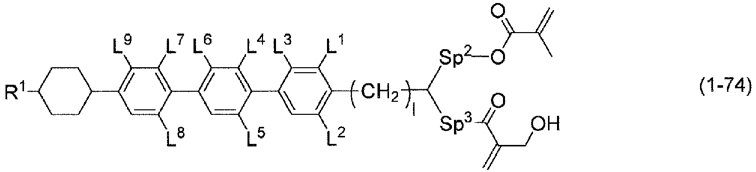 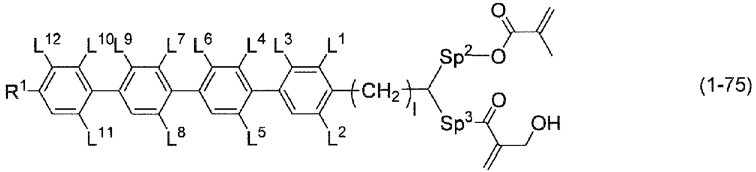 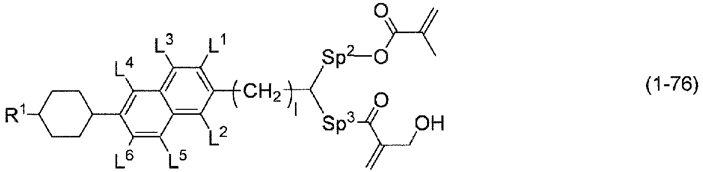 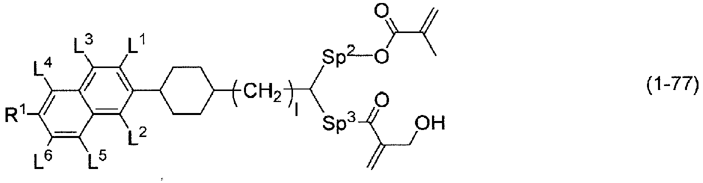 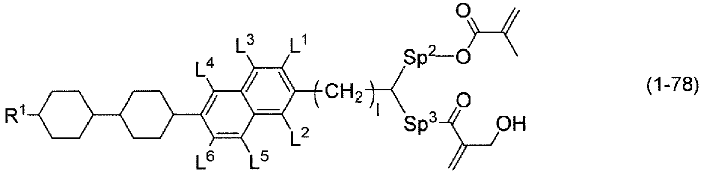 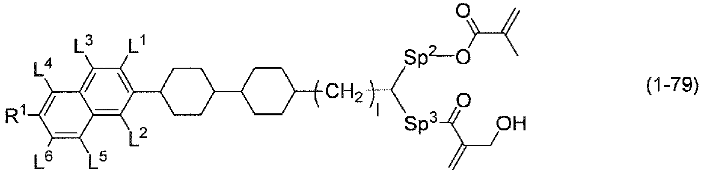 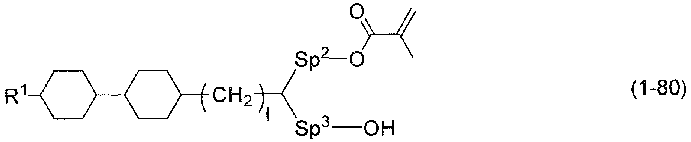 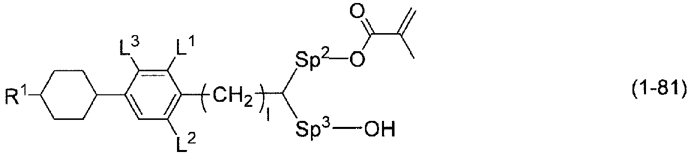 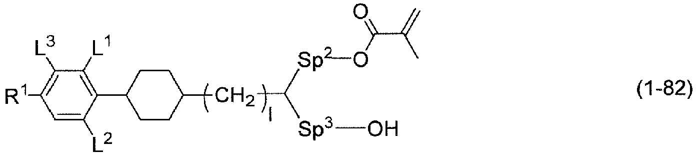 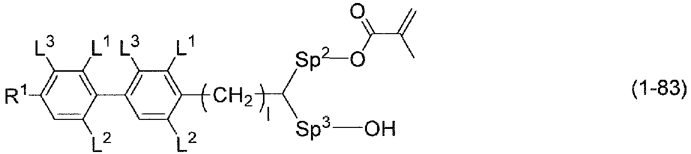 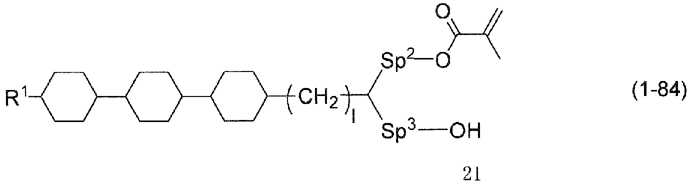 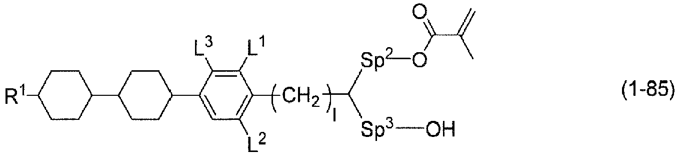 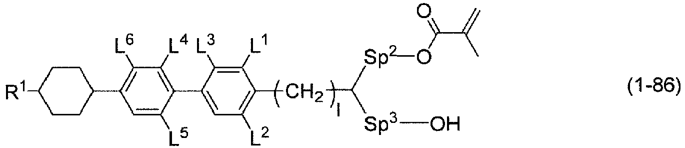 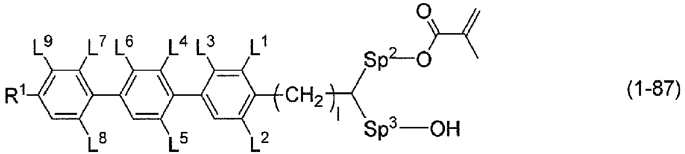 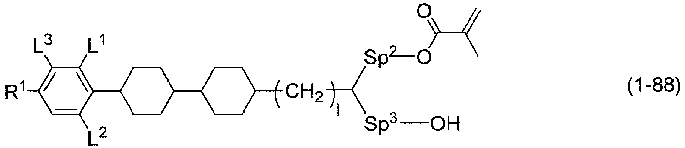 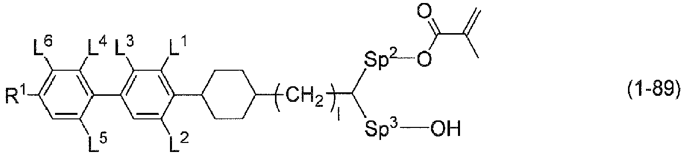 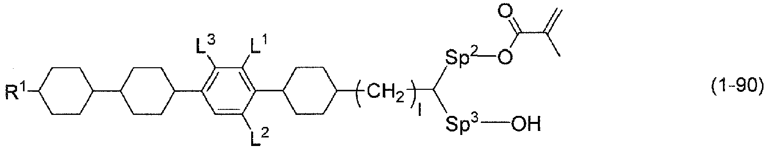 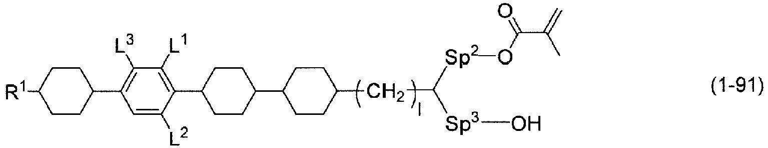 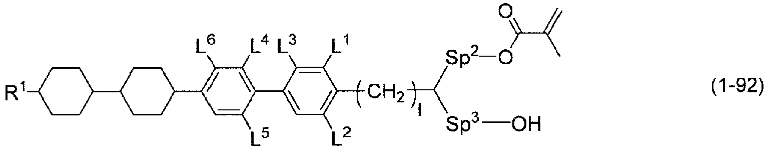 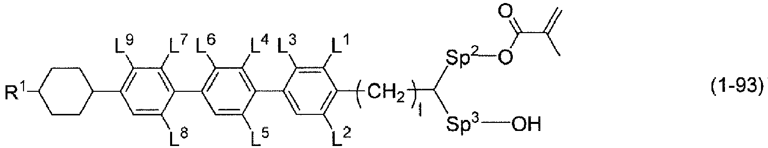 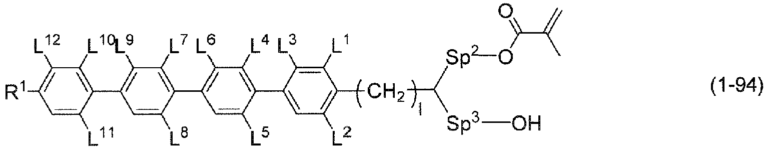 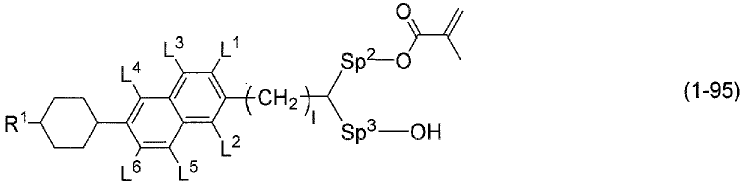 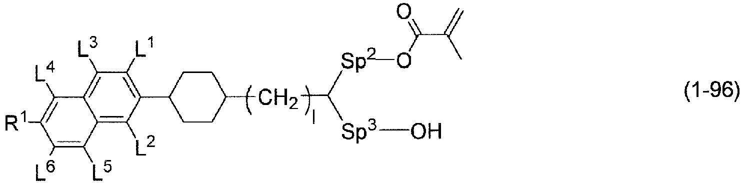 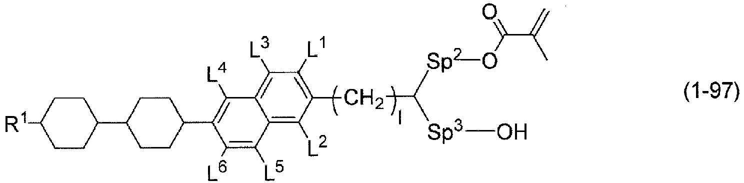 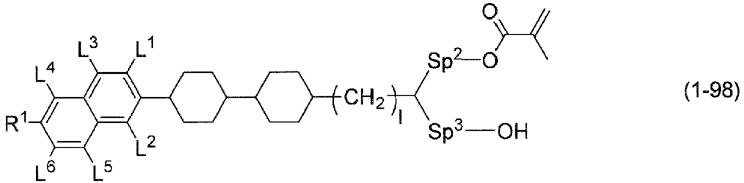 wherein, in formula (1-61) to formula (1-98),
R¹ is alkyl having 1 to 10 carbons;
Sp² and Sp³ are independently alkylene having 1 to 3 carbons, and in the alkylene, at least one piece of -CH₂- may be replaced by -O-;
L¹, L², L³, L⁴, L⁵, L⁶, L⁷, L⁸, L⁹, L¹⁰, L¹¹ and L¹² are independently hydrogen, fluorine or methyl; and
1 is 1, 2, 3 or 4, and in the alkylene, at least one piece of -CH₂- may be replaced by -O-.

11. The compound according to claim 1, represented by any one of formula (1-99) to formula (1-117): 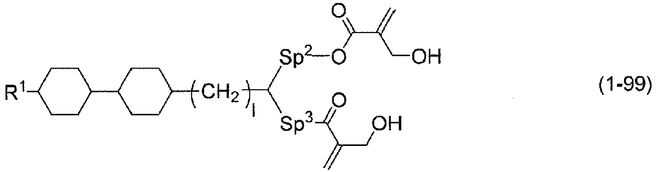 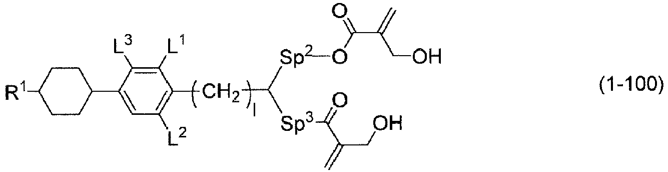 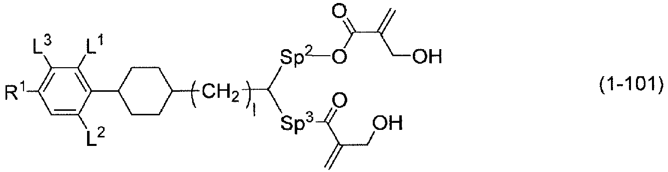 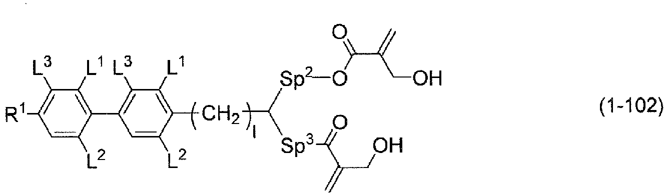 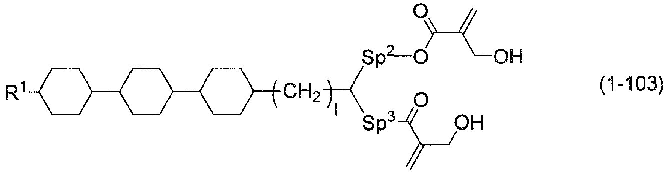 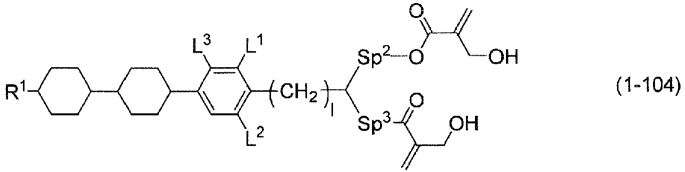 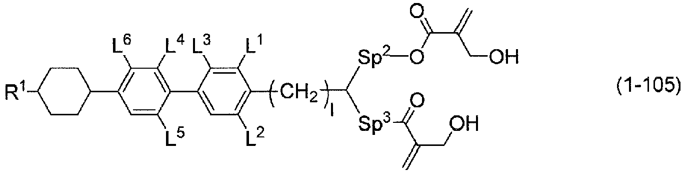 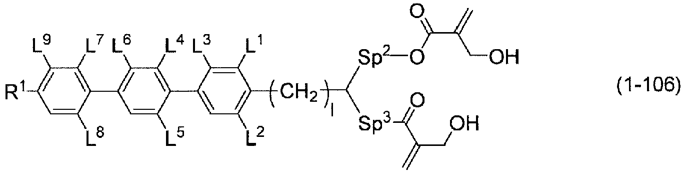 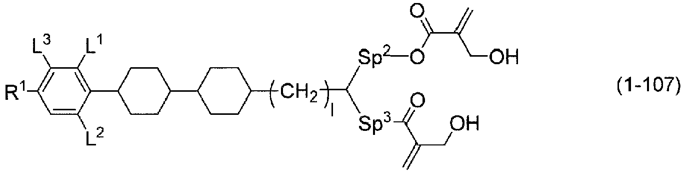 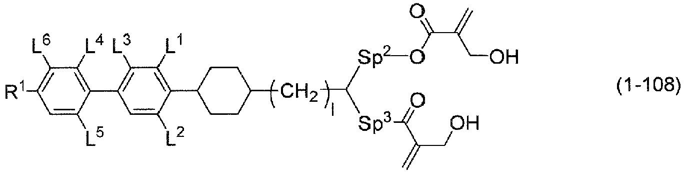 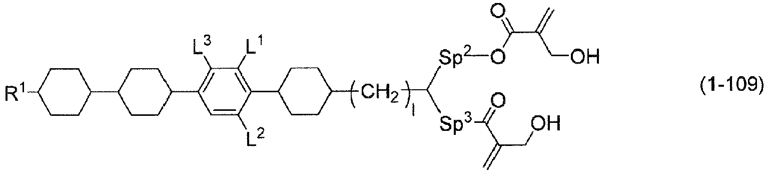 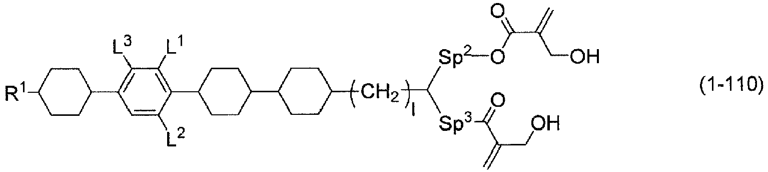 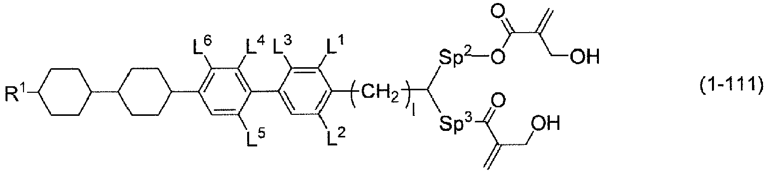 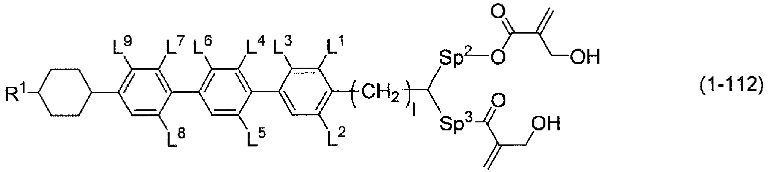 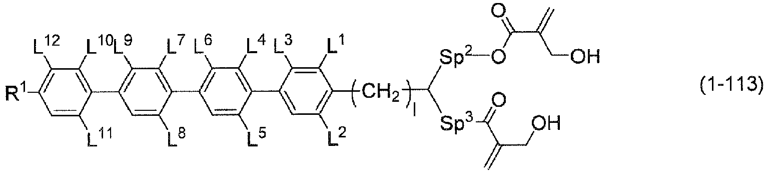 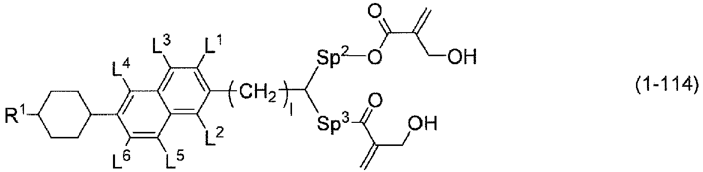 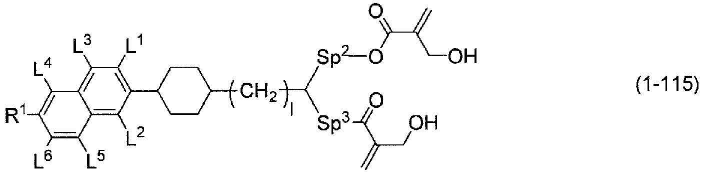 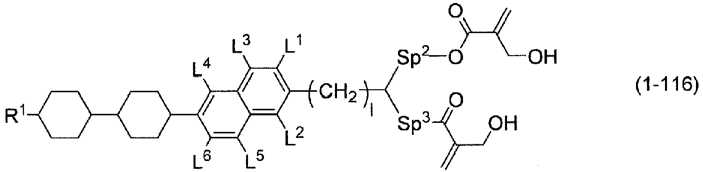 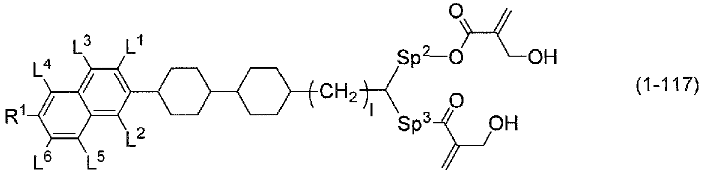 wherein, in formula (1-99) to formula (1-117),
R¹ is alkyl having 1 to 10 carbons;
Sp² and Sp³ are independently alkylene having 1 to 3 carbons, and in the alkylene, at least one piece of -CH₂- may be replaced by -O-;
L¹, L², L³, L⁴, L⁵, L⁶, L⁷, L⁸, L⁹, L¹⁰, L¹¹ and L¹² are independently hydrogen, fluorine or methyl; and
1 is 1, 2, 3 or 4, and in the alkylene, at least one piece of -CH₂- may be replaced by -O-.

12. A liquid crystal composition, containing at least one of the compounds according to any one of claims 1 to 11.

13. The liquid crystal composition according to claim 12, further containing at least one compound selected from the group of compounds represented by formula (2) to formula (4): 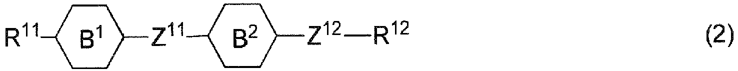 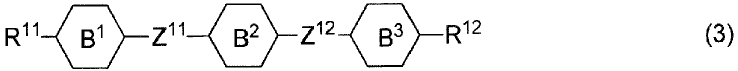 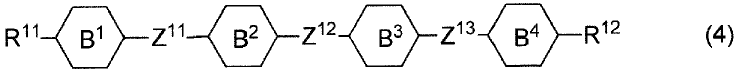 wherein, in formula (2) to formula (4),
R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -CH₂CH₂-, -CH=CH-, -C≡C- or -COO-.

14. The liquid crystal composition according to claim 12 or 13, further containing at least one compound selected from the group of compounds represented by formula (5) to formula (7): 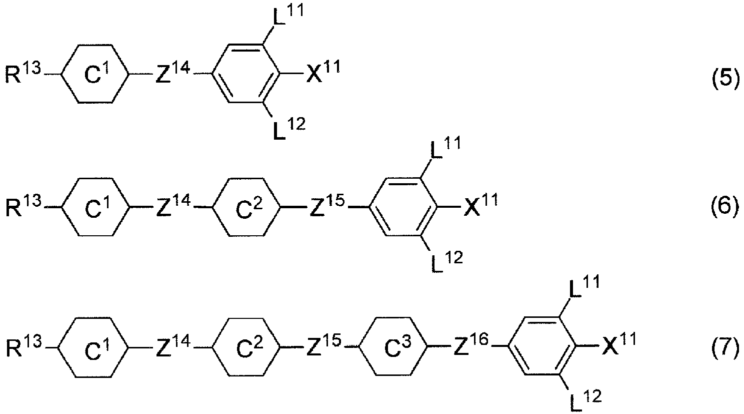 wherein, in formula (5) to formula (7),
R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹¹ is fluorine, chlorine, -OCF₃, -OCHF₂, -CF₃, -CHF₂, -CH₂F, -OCF₂CHF₂ or -OCF₂CHFCF₃;
ring C¹, ring C² and ring C³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁴, Z¹⁵ and Z¹⁶ are independently a single bond, -CH₂CH₂-, -CH=CH-, -C≡C-, -COO-, -CF2O-, -OCF2-, -CH2O- or -(CH₂)₄-; and
L¹¹ and L¹² are independently hydrogen or fluorine.

15. The liquid crystal composition according to any one of claims 12 to 14, further containing at least one compound of compounds represented by formula (8): 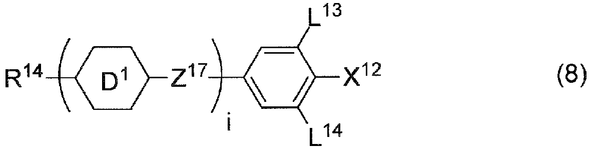 wherein, in formula (8),
R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹² is -C≡N or -C≡C-C≡N;
ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁷ is a single bond, -CH₂CH₂-, -C≡C-, -COO-, -CF₂O-, -OCF₂- or -CH₂O-;
L¹³ and L¹⁴ are independently hydrogen or fluorine; and
i is 1, 2, 3 or 4.

16. The liquid crystal composition according to any one of claims 12 to 15, further containing at least one compound selected from the group of compounds represented by formula (9) to formula (15): 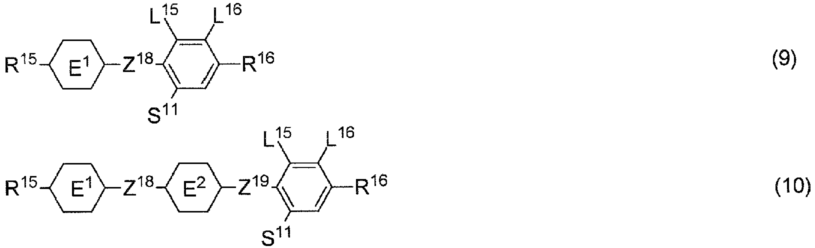 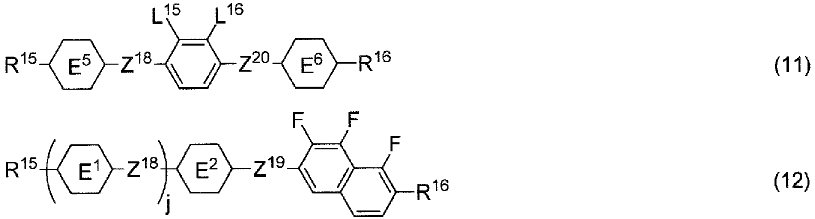 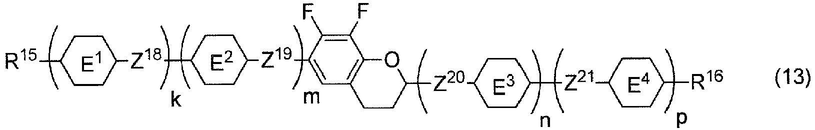 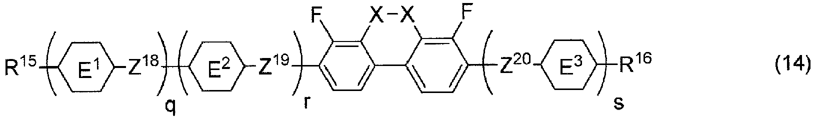 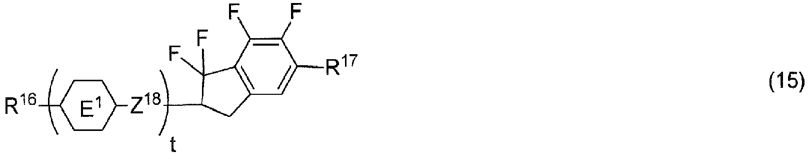 wherein, in formula (9) to formula (15),
R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine;
ring E¹, ring E², ring E³ and ring E⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
Z¹⁸, Z¹⁹, Z²⁰ and Z²¹ are independently a single bond, -CH₂CH₂-, -COO-, -CH₂O-, -OCF₂- or -OCF₂CH₂CH₂-;
L¹⁵ and L¹⁶ are independently fluorine or chlorine;
S¹¹ is hydrogen or methyl;
X is -CHF- or -CF₂-; and
j, k, m, n, p, q, r and s are independently 0 or 1, a sum of k, m, n and p is 1 or 2, a sum of q, r and s is 0, 1, 2 or 3, and t is 1, 2 or 3.

17. The liquid crystal composition according to any one of claims 12 to 16, further containing at least one compound of polymerizable compounds represented by formula (16): 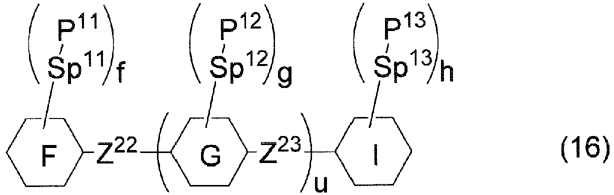 wherein, in formula (16),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cycloxylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃) - or -C(CH₃)=C(CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

18. The liquid crystal composition according to claim 17, wherein, in formula (16),
P¹¹, P¹² and P¹³ are independently a group selected from the group of polymerizable groups represented by formula (P-1) to formula (P-5) : 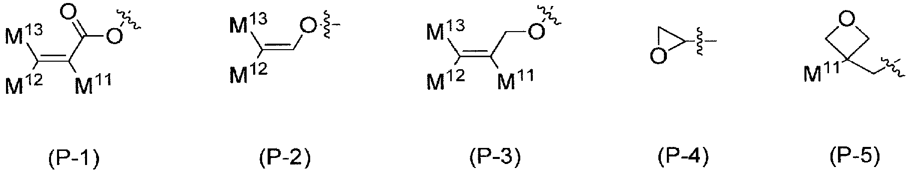 wherein, in formula (P-1) to formula (P-5),
M¹¹, M¹² and M¹³ are independently hydrogen, fluorine, alkyl having 1 to 5 carbons, or alkyl having 1 to 5 carbons in which at least one hydrogen is replaced by halogen.

19. The liquid crystal composition according to claim 17 or 18, wherein the polymerizable compound represented by formula (16) is at least one compound selected from the group of polymerizable compounds represented by formula (16-1) to formula (16-7): 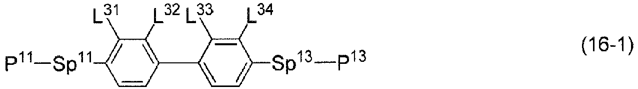 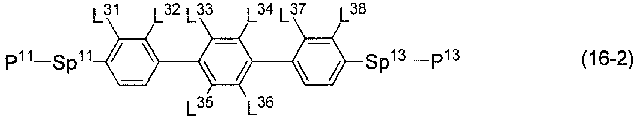 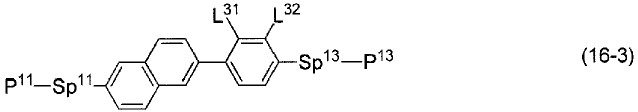 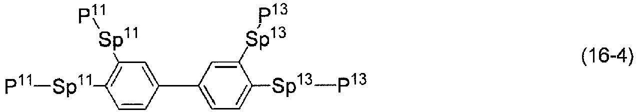 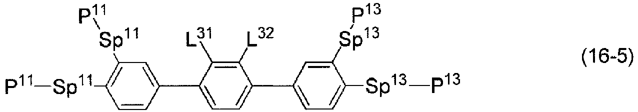 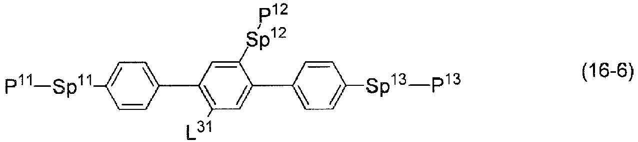 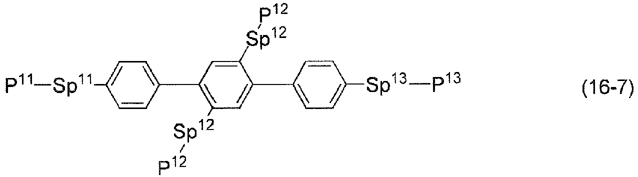 wherein, in formula (16-1) to formula (16-7),
L³¹, L³², L³³, L³⁴, L³⁵, L³⁶, L³⁷ and L³⁸ are independently hydrogen, fluorine or methyl;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine; and
P¹¹, P¹² and P¹³ are independently a group selected from the group of polymerizable groups represented by formula (P-1) to formula (P-3) : 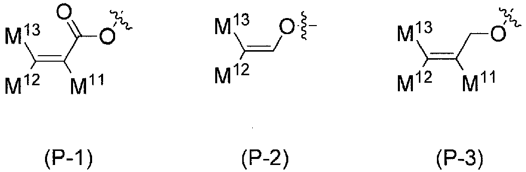
wherein, in formula (P-1) to formula (P-3),
M¹¹, M¹² and M¹³ are independently hydrogen, fluorine, alkyl having 1 to 5 carbons, or alkyl having 1 to 5 carbons in which at least one hydrogen is replaced by halogen.

20. The liquid crystal composition according to any one of claims 12 to 19, further containing at least one of a polymerizable compound different from the compound represented by formula (1), or formula (16) described below, a polymerization initiator, a polymerization inhibitor, an optically active compound, an antioxidant, an ultraviolet light absorber, a light stabilizer, a heat stabilizer and an antifoaming agent: 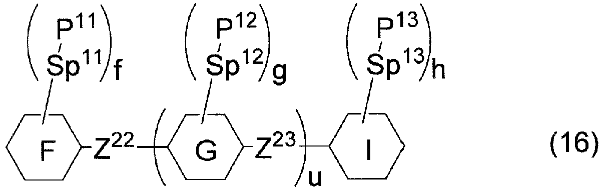 wherein, in formula (16),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cycloxylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

21. A liquid crystal display device, including at least one of the liquid crystal compositions according to any one of claims 12 to 20.
